(19) Europäisches Patentamt
European Patent Office
Office européen des brevets

(11) EP 1 957 115 B1

(12) **EUROPEAN PATENT SPECIFICATION**

(45) Date of publication and mention of the grant of the patent:
**15.01.2014 Bulletin 2014/03**

(21) Application number: **06827784.7**

(22) Date of filing: **13.11.2006**

(51) Int Cl.:
***A61K 39/395*** *(2006.01)* ***A61P 35/00*** *(2006.01)*

(86) International application number:
**PCT/US2006/044090**

(87) International publication number:
**WO 2007/059082 (24.05.2007 Gazette 2007/21)**

(54) **METHOD OF TREATING OVARIAN AND RENAL CANCER USING ANTIBODIES AGAINST T CELL IMMUNOGLOBULIN DOMAIN AND MUCIN DOMAIN 1 (TIM-1) ANTIGEN**

VERFAHREN ZUR BEHANDLUNG VON OVARIAL- UND NIERENKARZINOM MIT ANTIKÖRPERN GEGEN T-ZELL-IMMUNGLOBULIN-DOMÄNEN- UND MUCIN-DOMÄNEN-1-(TIM-1)-ANTIGEN

METHODE DE TRAITEMENT DU CANCER DE L'OVAIRE ET DU REIN UTILISANT DES ANTICORPS DIRIGES CONTRE L'ANTIGENE A DOMAINE 1 DE MUCINE ET A DOMAINE IMMUNOGLOBULINE DES LYMPHOCYTES T (TIM-1)

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.11.2005 US 735574 P**

(43) Date of publication of application:
**20.08.2008 Bulletin 2008/34**

(60) Divisional application:
**12174847.9 / 2 548 583**

(73) Proprietor: **Curagen Corporation**
**Branford, CT 06405 (US)**

(72) Inventors:
- **TSE, Kam, Fai**
  **Clinton, CT 06413 (US)**
- **POLLACK, Vincent, A.**
  **Gales Ferry, CT 06335 (US)**
- **MACDOUGALL, John**
  **Hamden, CT 06517 (US)**
- **SHENOY, Suresh, G.**
  **Branford, CT 06405 (US)**
- **MANSFIELD, Traci**
  **Guilford, CT 06437 (US)**
- **LICHENSTEIN, Henri**
  **Guilford, CT 06437 (US)**
- **JEFFERS, Michael, E.**
  **Branford, CT 06405 (US)**
- **LAROCHELLE, William, J.**
  **Madison, CT 06443 (US)**

(74) Representative: **Mintz Levin Cohn Ferris Glovsky and Popeo LLP**
**Alder Castle**
**10 Noble Street**
**London EC2V 7JX (GB)**

(56) References cited:
**WO-A2-2004/084823**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Background of the Invention

Field of the Invention

The invention disclosed herein is related to antibodies directed to the antigen T cell, immunoglobulin domain and mucin domain 1 (TIM-1) or binding fragments thereof, for us in treating ovarian cancer or renal cancer wherein said antibody or binding fragment thereof is conjugated to a monomethyl auristatin E (MMAE) toxin.

Description of the Related Art

A new family of genes encoding T cell, immunoglobulin domain and mucin domain (TIM) proteins (three in humans and eight in mice) have been described recently with emerging roles in immunity. Kuchroo et al., Nat Rev Immunol 3:454-462 (2003); McIntire et al., Nat Immunol 2:1109-1116 (2001). The TIM gene family members reside in chromosomal regions, 5q33.2 in human and 11B1.1 in mouse, and have been linked to allergy and autoimmune diseases. Shevach, Nat Rev Immunol 2:389-400 (2002); Wills-Karp et al., Nat Immunol 4:1050-1052 (2003).

One TIM family member, TIM-1, is also known as Hepatitis A virus cellular receptor (HAVcr-1) and was originally discovered as a receptor for Hepatitis A virus (HAV) (Kaplan et al, EMBO J 15(16):4282-96 (1996)). This gene was later cloned as kidney injury molecule 1 (KIM-1) (Ichimura et al., J Biol Chem 273:4135-4142 (1998); Han et al., Kidney Int 62:237-244 (2002)).

Kaplan *et al.* isolated the cellular receptor for hepatitis A virus from a cDNA library from a primary African Green Monkey Kidney (AGMK) cell line expressing the receptor. *See* U.S. Patent No. 5,622,861. The disclosed utility of the polypeptides and nucleic acids was to diagnose infection by hepatitis A virus, to separate hepatitis A virus from impurities in a sample, to treat infection as well as to prevent infection by hepatitis A virus. Furthermore, the polypeptides could be expressed in transformed cells and used to test efficacy of compounds in an anti-hepatitis A virus binding assay.

The human homolog, hHAVcr-1 (aka TIM-1), was described by Feigelstock et al., J Virology 72(8): 6621-6628 (1998). The same molecules were described in PCT Publication Nos: WO 97/44460 and WO 98/53071 and U.S. Patent No. 6,664,385 as Kidney Injury-related Molecules (KIM) that were found to be upregulated in renal tissue after injury to the kidney. The molecules were described as being useful in a variety of therapeutic interventions, specifically, renal disease, disorder or injury. For example, PCT Publication No. WO 02/098920 describes antibodies to KIM and describes antibodies that inhibit the shedding of KIM-1 polypeptide from KIM-1 expressing cells e.g., renal cells, or renal cancer cells.

TIM-1 is a type 1 membrane protein that contains a novel six-cysteine immunoglobulin-like domain and a mucin threonine/serine.proline-rich (T/S/P) domain. TIM-1 was originally identified in rat. TIM-1 has been found in mouse, African green monkey, and humans (Feigelstock et al., J Virol 72(8):6621-8 (1998). The African green monkey ortholog is most closely related to human TIM-1 showing 77.6% amino acid identity over 358 aligned amino acids. Rat and mouse orthologs exhibit 50% (155/310) and 45.6% (126/276) amino acid identity respectively, although over shorter segments of aligned sequence than for African green monkey. Monoclonal antibodies to the Ig-like domain of TIM-1 have been shown to be protective against Hepatitis A Virus infection *in vitro*. Silberstein et al., J Virol 75(2):717-25 (2001). In addition, Kim-1 was shown to be expressed at low levels in normal kidney but its expression is increased dramatically in postischemic kidney. Ichimura et al., J Biol Chem 273(7):4135-42 (1998). HAVCR-1 is also expressed at elevated levels in clear cell carcinomas and cancer cell lines derived from the same.

TIM-1 shows homology to the P-type "trefoil" domain suggesting that it may have similar biological activity to other P-type trefoil family members. Some trefoil domain containing proteins have been shown to induce cellular scattering and invasion when used to treat kidney, colon and breast tumor cell lines. Prest et al., FASEB J 16(6):592-4 (2002). In addition, some trefoil containing proteins confer cellular resistance to anoikis, an anchorage-related apoptosis phenomenon in epithelium. Chen et al., Biochem Biophys Res Commun 274(3):576-82 (2000).

TIM-1 maps to a region of human chromosome 5 known as Tapr in the murine sytenic region that has been implicated in asthma. Tapr, a major T cell regulatory locus, controls the development of airway hyperreactivity. Wills-Karp, Nature Immunology 2:1095-1096 (2001); McIntire et al., Nature Immunology 2:1109-1116 (2001).

WO 2004/084823 discloses antibodies against T cell immunoglobulin domain and mucin domain 1 (TIM-1) antigen and their use in the treatment of cancer. The antibodies disclosed in WO 2004/084823 may be conjugated to therapeutic agents such as toxins, radioactive isotopes and chemotherapeutic agents.

Summary of the Invention and Disclosure

TIM-1 is expressed at elevated levels in pathologies, such as neoplasms and inflammatory diseases. Inhibition of the biological activity of TIM-1 can thus prevent inflammation and other desired effects, including TIM-1 induced cell proliferation. The invention is based upon the generation and identification of isolated antibodies, or binding fragments thereof, that bind specifically to TIM-1.

Accordingly, the invention provides an antibody, or binding fragment thereof, that specifically binds to TIM-1 for use in treating ovarian cancer or renal cancer wherein said antibody or binding fragment thereof is conjugated to a monomethyl auristatin E (MMAE) toxin. As known in the art, the antibodies can advantageously be, for example, monoclonal, chimeric and/or fully human antibodies. Also described herein are cells for producing these antibodies.

Some embodiments of the invention described herein relate to monoclonal antibodies that bind TIM-1 and affect TIM-1 function. Other embodiments relate to fully human anti-TIM-1 antibodies and anti-TIM-1 antibody preparations with desirable properties from a therapeutic perspective, including strong binding affinity for TIM-1, the ability to neutralize TIM-1 *in vitro* and *in vivo*, and the ability to inhibit TIM-1 induced cell proliferation.

In a preferred embodiment, antibodies described herein bind to TIM-1 with very high affinities (Kd). For example a human, rabbit, mouse, chimeric or humanized antibody that is capable of binding TIM-1 with a Kd less than, but not limited to, $10^{-7}$, $10^{-8}$, $10^{-9}$, $10^{-10}$, $10^{-11}$, $10^{-12}$, $10^{-13}$ or $10^{-14}$ M, or any range or value therein. Affinity and/or avidity measurements can be measured by KinExA® and/or BIACORE®, as described herein.

There is herein described an isolated antibody that specifically binds to T cell, immunoglobulin domain and mucin domain 1 (TIM-1).

The isolated antibody may have a heavy chain polypeptide comprising an amino acid sequence selected from the group consisting of SEQ ID NOs: 2, 6, 10, 14, 18, 22, 26, 30, 34, 38, 42, 46, and 50.

Also described herein is an isolated antibody that specifically binds to T cell, immunoglobulin domain and mucin domain 1 (TIM-1) and has a light chain polypeptide comprising an amino acid sequence selected from the group consisting of SEQ ID NOs: 4, 8, 12, 16, 20, 24, 28, 32, 36, 40, 44, 48, and 52.

Also described herein is an isolated antibody that specifically binds to TIM-1 and has a heavy chain polypeptide comprising an amino acid sequence selected from the group consisting of SEQ ID NOs: 2, 6, 10, 14, 18, 22, 26, 30, 34, 38, 42, 46, and 50 and has a light chain polypeptide comprising an amino acid sequence selected from the group consisting of SEQ ID NOs: 4, 8, 12, 16, 20, 24, 28, 32, 36, 40, 44, 48, and 52.

Also described herein is a fully human antibody that specifically binds to TIM-1 and has a heavy chain polypeptide comprising an amino acid sequence comprising the complementarity determining region (CDR) with one of the sequences shown in Table 4. It is noted that CDR determinations can be readily accomplished by those of ordinary skill in the art. See for example, Kabat et al., Sequences of Proteins of Immunological Interest, Fifth Edition, NIH Publication 91-3242, Bethesda MD [1991], vols. 1-3.

Also described herein is an antibody that specifically binds to TIM-1 and has a light chain polypeptide comprising an amino acid sequence comprising a CDR comprising one of the sequences shown in Table 5. The antibody may be a fully human monoclonal antibody.

Also described herein is an antibody that binds to TIM-1 and comprises a heavy chain polypeptide comprising an amino acid sequence comprising one of the CDR sequences shown in Table 4 and a light chain polypeptide comprising an amino acid sequence comprising one of the CDR sequences shown in Table 5. The antibody may be a fully human monoclonal antibody.

Also described herein is a fully human antibody that binds to orthologs of TIM-1. Also described herein is an antibody that cross-competes for binding to TIM-1 with the fully human antibodies described herein.

The disclosure includes methods of producing high affinity antibodies to TIM-1 by immunizing a mammal with human TIM-1, or a fragment thereof, and one or more orthologous sequences or fragments thereof.

It will be appreciated that embodiments of the invention and the disclosure are not limited to any particular form of an antibody. For example, the anti-TIM-1 antibody can be a full length antibody (e.g., having an intact human Fc region) or an antibody fragment (e.g., a Fab, Fab', $F(ab')_2$, Fv, or single chain antibodies). In addition, the antibody can be manufactured from a hybridoma that secretes the antibody, or from a recombinantly produced cell that has been transformed or transfected with a gene or genes encoding the antibody.

Also described herein are isolated nucleic acid molecules encoding any of the anti-TIM-1 antibodies described herein, vectors having an isolated nucleic acid molecule encoding the anti-TIM-1 antibody, and a host cell transformed with such a nucleic acid molecule. In addition, a method of producing an anti-TIM-1 antibody by culturing host cells under conditions wherein a nucleic acid molecule is expressed to produce the antibody followed by recovering the antibody from the host cell is herein described.

Also herein described are compositions, including an antibody, or functional fragment thereof, and a pharmaceutically acceptable carrier.

The disclosure includes pharmaceutical compositions having an effective amount of an anti-TIM-1 antibody in admixture with a pharmaceutically acceptable carrier or diluent. The anti-TIM-1 antibody, or a fragment thereof, may be conjugated to a therapeutic agent. The therapeutic agent can be, for example, a toxin, a radioisotope, or a chemotherapeutic agent. Preferably, such antibodies can be used for the treatment of pathologies, including for example, tumors and cancers, such as ovarian, stomach, endometrial, salivary gland, lung, kidney, colon, colorectal, thyroid, pancreatic, prostate and bladder cancer, as well as other inflammatory conditions. More preferably, the antibodies can be used to treat renal and ovarian carcinomas.

The antibodies described herein can be used for the preparation of a medicament for the effective treatment of TIM-1 induced cell proliferation in an animal, wherein said monoclonal antibody specifically binds to TIM-1.

The use of an anti-TIM-1 antibody in the preparation of a medicament for the treatment of diseases such as neoplasms and inflammatory conditions is described herein. The neoplasm may include, without limitation, tumors and cancers, such as ovarian, stomach, endometrial, salivary gland, lung, kidney, colon, colorectal, thyroid, pancreatic, prostate and bladder cancer.

Also herein described is a method for effectively treating pathologies associated with the expression of TIM-1. These methods include selecting an animal in need of treatment for a condition associated with the expression of TIM-1, and administering to said animal a therapeutically effective dose of a fully human monoclonal antibody, wherein said antibody specifically binds to TIM-1.

Preferably a mammal and, more preferably, a human, receives the anti-TIM-1 antibody. Preferably, neoplasms are treated, including, without limitation, renal and pancreatic tumors, head and neck cancer, ovarian cancer, gastric (stomach) cancer, melanoma, lymphoma, prostate cancer, liver cancer, lung cancer, renal cancer, bladder cancer, colon cancer, esophageal cancer, and brain cancer.

Also herein described is the use of an antibody in the preparation of medicament for the effective treatment of neoplastic disease in an animal, wherein said monoclonal antibody specifically binds to TIM-1. Treatable neoplastic diseases include, for example, ovarian cancer, bladder cancer, lung cancer, glioblastoma, stomach cancer, endometrial cancer, kidney cancer, colon cancer, pancreatic cancer, and prostrate cancer.

Also described herein is a method for inhibiting cell proliferation associated with the expression of TIM-1. These methods include selecting an animal in need of treatment for TIM-1 induced cell proliferation and administering to said animal a therapeutically effective dose of a fully human monoclonal antibody, wherein the antibody specifically binds TIM-1. Cells expressing TIM-1 may be treated with an effective amount of an anti-TIM-1 antibody or a fragment thereof. The method can be performed *in vivo*.

The methods described herein can be performed *in vivo* and the patient is preferably a human patient. Preferably, the methods concern the treatment of neoplastic diseases, for example, tumors and cancers, such as renal (kidney) cancer, pancreatic cancer, head and neck cancer, ovarian cancer, gastric (stomach) cancer, melanoma, lymphoma, prostate cancer, liver cancer, breast cancer, lung cancer, bladder cancer, colon cancer, esophageal cancer, and brain cancer.

The anti-TIM-1 antibody may be administered to a patient, followed by administration of a clearing agent to remove excess circulating antibody from the blood.

Anti-TIM-1 antibodies can be modified to enhance their capability of fixing complement and participating in complement-dependent cytotoxicity (CDC). Anti-TIM-1 antibodies can be modified, such as by an amino acid substitution, to alter their clearance from the body. Alternatively, some other amino acid substitutions can slow clearance of the antibody from the body.

Also described herein is an article of manufacture including a container. The container includes a composition containing an anti-TIM-1 antibody, and a package insert or label indicating that the composition can be used to treat neoplastic or inflammatory diseases characterized by the overexpression of TIM-1.

Also described herein are methods for assaying the level of TIM-1 in a patient sample, comprising contacting an anti-TIM-1 antibody with a biological sample from a patient, and detecting the level of binding between said antibody and TIM-1 in said sample. The biological sample may be blood.

There is also described herein an assay kit for detecting TIM-1 and TIM-1 orthologs in mammalian tissues or cells to screen for neoplastic diseases or inflammatory conditions. The kit includes an antibody that binds to TIM-1 and a means for indicating the reaction of the antibody with TIM-1, if present. Preferably the antibody is a monoclonal antibody. The antibody that binds TIM-1 may be labeled. Alternatively the antibody is an unlabeled first antibody and the kit further includes a means for detecting the first antibody. The means includes a labeled second antibody that is an anti-immunoglobulin. Preferably the antibody is labeled with a marker selected from the group consisting of a fluorochrome, an enzyme, a radionuclide and a radiopaque material.

There is also herein described a method of diagnosing diseases or conditions in which an antibody prepared as described herein is utilized to detect the level of TIM-1 in a patient sample. The patient sample may be blood or blood serum. Methods for the identification of risk factors, diagnosis of disease, and staging of disease which involve the identification of the overexpression of TIM-1 using anti-TIM-1 antibodies are herein described.

Also described herein are variants of a TIM-1 protein that function as either TIM-1 agonists (mimetics) or as TIM-1 antagonists.

Another embodiment of the invention is the use of monoclonal antibodies directed against the TIM-1 antigen coupled to cytotoxic chemotherapic agents or radiotherapic agents such as anti-tumor therapeutics.

There is herein described an isolated antibody that blocks simultaneous binding to TIM-1 antigen by an antibody having a heavy chain sequence comprising an amino acid sequence selected from the group consisting of SEQ ID NOS: 2, 6, 10, 14, 18, 22, 26, 30, 34, 38, 42, 46, and 50. Also described herein is an isolated antibody that binds to TIM-1 antigen and that cross reacts with an antibody having a heavy chain sequence comprising the amino acid sequence from the group consisting of SEQ ID NOS: 2, 6, 10, 14, 18, 22, 26, 30, 34, 38, 42, 46, and 50.

There is herein described an isolated antibody that binds to an epitope of SEQ ID NO: 87 on the TIM-1 antigen of SEQ ID NO. 54, and that cross reacts with an antibody having a heavy chain sequence comprising the amino acid sequence selected from the group consisting of SEQ ID NOS: 2, 6, 10, 14, 18, 22, 26, 30, 34, 38, 42, 46, and 50. Also herein described is an isolated antibody that binds to an epitope of SEQ ID NO: 87 on the TIM-1 antigen of SEQ ID NO. 54, wherein said antibody blocks simultaneous binding to TIM-1 antigen by an antibody having a heavy chain sequence comprising the amino acid sequence selected from the group comprising SEQ ID NOS: 2, 6, 10, 14, 18, 22, 26, 30, 34, 38, 42, 46, and 50.

Brief Description of the Drawings

Figure 1 is a bar graph of the results of an ELISA assay of anti-TIM-1 monoclonal antibodies 1.29, 2.56.2, 2.59.2, and 2.45.1 against the TIM-1 antigen.

Figure 2 is a bar graph of the results of an ELISA assay of anti-TIM-1 monoclonal antibodies 1.29, 2.56.2, 2.59.2, and 2.45.1 against irrelevant protein.

Figure 3 shows staining of Renal Cell Cancer (3A) and Pancreatic Cancer (3B) with the anti-TIM-1 mAb 2.59.2.

Figure 4 is a bar graph of clonogenic assay results of anti-TIM-1 monoclonal antibody mediated toxin killing in the ACHN kidney cancer cell line.

Figure 5 is a bar graph of clonogenic assay results of anti-TIM-1 monoclonal antibody mediated toxin killing in the BT549 breast cancer cell line.

Figure 6 is a bar graph of the results of a clonogenic assay of CAKI-1 cells treated with Auristatin E (AE) conjugated antibodies.

Figure 7 is a bar graph of the results of a clonogenic assay of BT549 cells treated with Auristatin E (AE) conjugated antibodies.

Figure 8 is a bar graph showing that anti-TIM-1 monoclonal antibodies 2.59.2, 2.56.2 and 2.45.1 significantly inhibit IL-4 release from Th1 cells compared to the control PK16.3 mAb.

Figure 9 is a bar graph showing that anti-TIM-1 monoclonal antibodies 2.59.2 and 2.45.1 significantly inhibit IL-4 release from Th2 cells compared to control PK16.3 mAb.

Figure 10 is a bar graph showing that anti-TIM-1 monoclonal antibody 2.59.2 significantly inhibited IL-5 release from Th1 cells compared to control PK16.3 mAb.

Figure 11 is a bar graph showing that anti-TIM-1 monoclonal antibodies 2.59.2 and 1.29 significantly inhibited IL-5 release from Th2 cells compared to control PK16.3 mAb.

Figure 12 is a bar graph showing that anti-TIM-1 monoclonal antibodies 2.59.2, 1.29 and 2.56.2 significantly inhibited IL-10 release from Th1 cells compared to control PK16.3 mAb.

Figure 13 is a bar graph showing that anti-TIM-1 monoclonal antibodies 2.59.2, 1.29 and 2.45.1 significantly inhibited IL-10 release from Th2 cells compared to control PK16.3 mAb.

Figure 14 is a bar graph showing that anti-TIM-1 monoclonal antibodies 2.59.2, 1.29 and 2.56.2 significantly inhibited IL-13 release from Th1 cells compared to control PK16.3 mAb.

Figure 15 is a bar graph showing that anti-TIM-1 monoclonal antibodies 2.59.2 and 1.29 significantly inhibited IL-13 release from Th2 cells compared to control PK16.3 mAb.

Figure 16 is a bar graph showing that anti-TIM-1 monoclonal antibodies did not inhibit IFN$\gamma$ release from Th1 cells compared to control PK16.3 mAb.

Figure 17 is a bar graph showing that anti-TIM-1 monoclonal antibodies 2.59.2 and 2.45.1 significantly inhibited IFN$\gamma$ release from Th2 cells compared to control PK16.3 mAb.

Figures 18A-18T are bar graphs showing BrdU incorporation assay results from experiments in which the neutralization of various human anti-TIM-1 monoclonal antibodies was assessed.

Figures 19A through 19D are line graphs showing the results of antibody conjugate studies performed using the plant toxin Saporin conjugated to TIM-1-specific antibodies and irrelevant antibodies (Figures 19A-19C). Additional negative controls included irrelevant antibodies alone without toxin (Figure 19D).

Figure 20 is a graph showing tumor growth inhibition and complete regression of IGROV1 ovarian carcinoma xenografts in athymic mice after treatment with 6.25 to 50 mg/kg i.v. every 4 days for 4 treatments. The responses of tumor-bearing animals to reference drugs such as vinblastine (1.7 mg/kg i.v. q4d X4) and paclitaxel (15.0 mg/kg i.v. q2d X4) are also shown. Control groups were treated with either phosphate-buffered saline (PBS) or physiological saline. CR014-vcMMAE was toxic to the test animals at 50 mg/kg/treatment (n=1/6) and at 100 mg/kg/treatment (n= 6/6).

Detailed Description

The invention and disclosures described herein are based upon the generation and identification of isolated antibodies that bind specifically to T cell, immunoglobulin domain and mucin domain 1 (TIM-1). As discussed below, TIM-1 is expressed at elevated levels in clear cell carcinomas and cancer cell lines derived from the same. Accordingly, antibodies that bind to TIM-1 are useful for the treatment and inhibition of carcinomas. In addition, antibodies that bind TIM-1 are also useful for reducing cell migration and enhancing apoptosis of kidney cancer cells.

Accordingly, there is herein described isolated antibodies, or fragments of those antibodies, that bind to TIM-1. As known in the art, the antibodies can advantageously be, *e.g.*, monoclonal, chimeric and/or human antibodies. Also herein described are cells for producing these antibodies.

These antibodies may be used for diagnostic or therapeutic purposes. For example, there is herein described methods and antibodies for inhibiting the expression of TIM-1 associated with cell proliferation. Preferably, the antibodies are used to treat neoplasms such as renal and pancreatic tumors, head and neck cancer, ovarian cancer, gastric (stomach) cancer, melanoma, lymphoma, prostate cancer, liver cancer, breast cancer, lung cancer, renal cancer, bladder cancer, colon cancer, esophageal cancer, and brain cancer. In association with such treatment, articles of manufacture comprising these antibodies are described herein. Additionally, an assay kit comprising these antibodies described herein to screen for cancers or tumors.

Additionally, the nucleic acids described herein, and fragments and variants thereof, may be used, by way of nonlimiting example, (a) to direct the biosynthesis of the corresponding encoded proteins, polypeptides, fragments and variants as recombinant or heterologous gene products, (b) as probes for detection and quantification of the nucleic acids disclosed herein, (c) as sequence templates for preparing antisense molecules, and the like. Such uses are described more fully in the following disclosure.

Furthermore, the TIM-1 proteins and polypeptides described herein, and fragments and variants thereof, may be used, in ways that include (a) serving as an immunogen to stimulate the production of an anti-TIM-1 antibody, (b) a capture antigen in an immunogenic assay for such an antibody, (c) as a target for screening for substances that bind to a TIM-1 polypeptide described herein, and (d) a target for a TIM-1 specific antibody such that treatment with the antibody affects the molecular and/or cellular function mediated by the target. TIM-1 polypeptide expression or activity can promote cell survival and/or metastatic potential. Conversely, a decrease in TIM-1 polypeptide expression or inhibition of its function reduces tumor cell survival and invasiveness in a therapeutically beneficial manner.

Single chain antibodies (scFv's) and bispecific antibodies specific for TIM-1 are useful particularly because it may more readily penetrate a tumor mass due to its smaller size relative to a whole IgG molecule. Studies comparing the tumor penetration between whole IgG molecules and scFv's have been have been described in the literature. The scFv can be derivatized with a toxin or radionuclide in order to destroy tumor cells expressing the TIM-1 antigen, in a manner similar to the IgG2 or IgG4 anti-TIM-1 toxin labeled or radionuclide derivatized whole antibodies already discussed, but with the advantage of being able to penetrate the tumor more fully, which may translate into increased efficacy in eradicating the tumor. A specific example of a biologically active anti-TIM-1 scFv is provided herein.

Sequence Listing

The heavy chain and light chain variable region nucleotide and amino acid sequences of representative human anti-TIM-1 antibodies are provided in the sequence listing, the contents of which are summarized in Table 1 below.

Table 1

| mAb ID No.: | Sequence | SEQ ID NO: |
|---|---|---|
| **1.29** | Nucleotide sequence encoding the variable region and a portion of the constant region of the heavy chain | 1 |
| | Amino acid sequence of the variable region of the heavy chain | 2 |
| | Nucleotide sequence encoding the variable region and a portion of the constant region of the light chain | 3 |
| | Amino acid sequence of the variable region of the light chain | 4 |

(continued)

| mAb ID No.: | Sequence | SEQ ID NO: |
|---|---|---|
| 1.37 | Nucleotide sequence encoding the variable region and a portion of the constant region of the heavy chain | 5 |
| | Amino acid sequence of the variable region of the heavy chain | 6 |
| | Nucleotide sequence encoding the variable region and a portion of the constant region of the light chain | 7 |
| | Amino acid sequence of the variable region of the light chain | 8 |
| 2.16 | Nucleotide sequence encoding the variable region and a portion of the constant region of the heavy chain | 9 |
| | Amino acid sequence of the variable region of the heavy chain | 10 |
| | Nucleotide sequence encoding the variable region and a portion of the constant region of the light chain | 11 |
| | Amino acid sequence of the variable region of the light chain | 12 |
| 2.17 | Nucleotide sequence encoding the variable region and a portion of the constant region of the heavy chain | 13 |
| | Amino acid sequence of the variable region of the heavy chain | 14 |
| | Nucleotide sequence encoding the variable region and a portion of the constant region of the light chain | 15 |
| | Amino acid sequence of the variable region of the light chain | 16 |
| 2.24 | Nucleotide sequence encoding the variable region and a portion of the constant region of the heavy chain | 17 |
| | Amino acid sequence of the variable region of the heavy chain | 18 |
| | Nucleotide sequence encoding the variable region and a portion of the constant region of the light chain | 19 |
| | Amino acid sequence of the variable region of the light chain | 20 |
| 2.45 | Nucleotide sequence encoding the variable region and a portion of the constant region of the heavy chain | 21 |
| | Amino acid sequence of the variable region of the heavy chain | 22 |
| | Nucleotide sequence encoding the variable region and a portion of the constant region of the light chain | 23 |
| | Amino acid sequence of the variable region of the light chain | 24 |
| 2.54 | Nucleotide sequence encoding the variable region and a portion of the constant region of the heavy chain | 25 |
| | Amino acid sequence of the variable region of the heavy chain | 26 |
| | Nucleotide sequence encoding the variable region and a portion of the constant region of the light chain | 27 |
| | Amino acid sequence of the variable region of the light chain | 28 |
| 2.56 | Nucleotide sequence encoding the variable region and a portion of the constant region of the heavy chain | 29 |
| | Amino acid sequence of the variable region of the heavy chain | 30 |
| | Nucleotide sequence encoding the variable region and a portion of the constant region of the light chain | 31 |
| | Amino acid sequence of the variable region of the light chain | 32 |

(continued)

| mAb ID No.: | Sequence | SEQ ID NO: |
|---|---|---|
| 2.59 | Nucleotide sequence encoding the variable region and a portion of the constant region of the heavy chain | 33 |
| | Amino acid sequence of the variable region of the heavy chain | 34 |
| | Nucleotide sequence encoding the variable region and a portion of the constant region of the light chain | 35 |
| | Amino acid sequence of the variable region of the light chain | 36 |
| 2.61 | Nucleotide sequence encoding the variable region and a portion of the constant region of the heavy chain | 37 |
| | Amino acid sequence of the variable region of the heavy chain | 38 |
| | Nucleotide sequence encoding the variable region and a portion of the constant region of the light chain | 39 |
| | Amino acid sequence of the variable region of the light chain | 40 |
| 2.70 | Nucleotide sequence encoding the variable region and a portion of the constant region of the heavy chain | 41 |
| | Amino acid sequence of the variable region of the heavy chain | 42 |
| | Nucleotide sequence encoding the variable region and a portion of the constant region of the light chain | 43 |
| | Amino acid sequence of the variable region of the light chain | 44 |
| 2.76 | Nucleotide sequence encoding the variable region and a portion of the constant region of the heavy chain | 45 |
| | Amino acid sequence of the variable region of the heavy chain | 46 |
| | Nucleotide sequence encoding the variable region and a portion of the constant region of the light chain | 47 |
| | Amino acid sequence of the variable region of the light chain | 48 |
| 2.70.2 | Nucleotide sequence encoding the variable region and a portion of the constant region of the heavy chain | 49 |
| | Amino acid sequence of the variable region and a portion of the constant region of the heavy chain | 50 |
| | Nucleotide sequence encoding the variable region and a portion of the constant region of the light chain | 51 |
| | Amino acid sequence of the variable region and a portion of the constant region of the light chain | 52 |

Definitions

Unless otherwise defined, scientific and technical terms used in connection with the invention and disclosure described herein shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (*e.g.*, electroporation, lipofection). Enzymatic reactions and purification techniques are performed according to manufacturer's specifications or as commonly accomplished in the art or as described herein. The foregoing techniques and procedures are generally performed according to conventional methods well known in the art and as described in various general and more specific references that are cited and discussed throughout the present specification. *See e.g.*, Sambrook et al. Molecular Cloning: A Laboratory Manual (2d ed., Cold Spring Harbor Laboratory Press, Cold Spring Harbor, N.Y. (1989)). The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

As utilized in accordance with the present disclosure, the following terms, unless otherwise indicated, shall be understood to have the following meanings:

The term "TIM-1" refers to T cell, immunoglobulin domain and mucin domain 1. TIM-1 may refer to a polypeptide comprising the amino acid sequence of SEQ ID NO: 54.

The term "polypeptide" is used herein as a generic term to refer to native protein, fragments, or analogs of a polypeptide sequence. Hence, native protein, fragments, and analogs are species of the polypeptide genus. Preferred polypeptides in accordance with the invention comprise human heavy chain immunoglobulin molecules and human kappa light chain immunoglobulin molecules, as well as antibody molecules formed by combinations comprising the heavy chain immunoglobulin molecules with light chain immunoglobulin molecules, such as the kappa light chain immunoglobulin molecules, and vice versa, as well as fragments and analogs thereof.

The term "polynucleotide" as referred to herein means a polymeric form of nucleotides of at least 10 bases in length, either ribonucleotides or deoxynucleotides or a modified form of either type of nucleotide. The term includes single and double stranded forms of DNA.

The term "isolated polynucleotide" as used herein shall mean a polynucleotide of genomic, cDNA, or synthetic origin or some combination thereof, which by virtue of its origin the isolated polynucleotide (1) is not associated with all or a portion of a polynucleotide in which the isolated polynucleotide is found in nature, (2) is operably linked to a polynucleotide which it is not linked to in nature, or (3) does not occur in nature as part of a larger sequence.

The term "isolated protein" referred to herein means a protein of cDNA, recombinant RNA, or synthetic origin or some combination thereof, which by virtue of its origin, or source of derivation, the "isolated protein" (1) is not associated with proteins found in nature, (2) is free of other proteins from the same source, e.g., free of murine proteins, (3) is expressed by a cell from a different species, or (4) does not occur in nature.

The term "oligonucleotide" referred to herein includes naturally occurring, and modified nucleotides linked together by naturally occurring, and non-naturally occurring oligonucleotide linkages. Oligonucleotides are a polynucleotide subset generally comprising a length of 200 bases or fewer. Preferably oligonucleotides are 10 to 60 bases in length and most preferably 12, 13, 14, 15, 16, 17, 18, 19, or 20 to 40 bases in length. Oligonucleotides are usually single stranded, *e.g.* for probes; although oligonucleotides may be double stranded, *e.g.* for use in the construction of a gene mutant. Oligonucleotides described herein can be either sense or antisense oligonucleotides.

Similarly, unless specified otherwise, the lefthand end of single-stranded polynucleotide sequences is the 5' end; the lefthand direction of double-stranded polynucleotide sequences is referred to as the 5' direction. The direction of 5' to 3' addition of nascent RNA transcripts is referred to as the transcription direction; sequence regions on the DNA strand having the same sequence as the RNA and which are 5' to the 5' end of the RNA transcript are referred to as upstream sequences; sequence regions on the DNA strand having the same sequence as the RNA and which are 3' to the 3' end of the RNA transcript are referred to as downstream sequences.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory or otherwise is naturally-occurring.

The term "naturally occurring nucleotides" referred to herein includes deoxyribonucleotides and ribonucleotides. The term "modified nucleotides" referred to herein includes nucleotides with modified or substituted sugar groups and the like. The term "oligonucleotide linkages" referred to herein includes oligonucleotides linkages such as phosphorothioate, phosphorodithioate, phosphoroselenoate, phosphorodiselenoate, phosphoroanilothioate, phoshoraniladate, phosphoroamidate, and the like. *See, e.g.*, La Planche et al., Nucl. Acids Res. 14:9081 (1986); Stec et al., J. Am. Chem. Soc. 106:6077 (1984); Stein et al., Nucl. Acids Res. 16:3209 (1988); Zon et al., Anti-Cancer Drug Design 6:539 (1991); Zon et al., Oligonucleotides and Analogues: A Practical Approach, pp. 87-108 (F. Eckstein, ed., Oxford University Press, Oxford England (1991)); Stec et al., U.S. Patent No. 5,151,510; Uhlmann and Peyman, Chemical Reviews 90:543 (1990). An oligonucleotide can include a label for detection, if desired.

The term "operably linked" as used herein refers to positions of components so described are in a relationship permitting them to function in their intended manner. A control sequence operably linked to a coding sequence is ligated in such a way that expression of the coding sequence is achieved under conditions compatible with the control sequences.

The term "control sequence" as used herein refers to polynucleotide sequences which are necessary to effect the expression and processing of coding sequences to which they are ligated. The nature of such control sequences differs depending upon the host organism; in prokaryotes, such control sequences generally include promoter, ribosomal binding site, and transcription termination sequence; in eukaryotes, generally, such control sequences include promoters and transcription termination sequence. The term control sequences is intended to include, at a minimum, all components whose presence is essential for expression and processing, and can also include additional components whose presence is advantageous, for example, leader sequences and fusion partner sequences.

The term "selectively hybridize" referred to herein means to detectably and specifically bind. Polynucleotides, oligonucleotides and fragments thereof described herein selectively hybridize to nucleic acid strands under hybridization and wash conditions that minimize appreciable amounts of detectable binding to nonspecific nucleic acids. High stringency conditions can be used to achieve selective hybridization conditions as known in the art and discussed herein. Generally, the nucleic acid sequence homology between the polynucleotides, oligonucleotides, and fragments described herein and a nucleic acid sequence of interest will be at least 80%, and more typically with preferably increasing homologies of at least 85%, 90%, 95%, 99%, and 100%.

Two amino acid sequences are homologous if there is a partial or complete identity between their sequences. For example, 85% homology means that 85% of the amino acids are identical when the two sequences are aligned for maximum matching. Gaps (in either of the two sequences being matched) are allowed in maximizing matching; gap lengths of 5 or less are preferred with 2 or less being more preferred. Alternatively and preferably, two protein sequences (or polypeptide sequences derived from them of at least 30 amino acids in length) are homologous, as this term is used herein, if they have an alignment score of at more than 5 (in standard deviation units) using the program ALIGN with the mutation data matrix and a gap penalty of 6 or greater. *See* Dayhoff, M.O., in Atlas of Protein Sequence and Structure, pp. 101-110 (Volume 5, National Biomedical Research Foundation (1972)) and Supplement 2 to this volume, pp. 1-10. The two sequences or parts thereof are more preferably homologous if their amino acids are greater than or equal to 50% identical when optimally aligned using the ALIGN program.

The term "corresponds to" is used herein to mean that a polynucleotide sequence is homologous (*i.e.*, is identical, not strictly evolutionarily related) to all or a portion of a reference polynucleotide sequence, or that a polypeptide sequence is identical to a reference polypeptide sequence.

In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA."

The following terms are used to describe the sequence relationships between two or more polynucleotide or amino acid sequences: "reference sequence," "comparison window," "sequence identity," "percentage of sequence identity," and "substantial identity." A "reference sequence" is a defined sequence used as a basis for a sequence comparison; a reference sequence may be a subset of a larger sequence, for example, as a segment of a full-length cDNA or gene sequence given in a sequence listing or may comprise a complete cDNA or gene sequence. Generally, a reference sequence is at least 18 nucleotides or 6 amino acids in length, frequently at least 24 nucleotides or 8 amino acids in length, and often at least 48 nucleotides or 16 amino acids in length. Since two polynucleotides or amino acid sequences may each (1) comprise a sequence (i.e., a portion of the complete polynucleotide or amino acid sequence) that is similar between the two molecules, and (2) may further comprise a sequence that is divergent between the two polynucleotides or amino acid sequences, sequence comparisons between two (or more) molecules are typically performed by comparing sequences of the two molecules over a comparison window to identify and compare local regions of sequence similarity. A "comparison window," as used herein, refers to a conceptual segment of at least 18 contiguous nucleotide positions or 6 amino acids wherein a polynucleotide sequence or amino acid sequence may be compared to a reference sequence of at least 18 contiguous nucleotides or 6 amino acid sequences and wherein the portion of the polynucleotide sequence in the comparison window may comprise additions, deletions, substitutions, and the like (i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman, Adv. Appl. Math., 2:482 (1981), by the homology alignment algorithm of Needleman and Wunsch, J. Mol. Biol., 48:443 (1970), by the search for similarity method of Pearson and Lipman, Proc. Natl. Acad. Sci. (U.SA.), 85:2444 (1988), by computerized implementations of these algorithms (GAP, BESTFIT, FASTA, and TFASTA in the Wisconsin Genetics Software Package Release 7.0, (Genetics Computer Group, 575 Science Dr., Madison, Wis.), Geneworks, or MacVector software packages), or by inspection, and the best alignment (i.e., resulting in the highest percentage of homology over the comparison window) generated by the various methods is selected.

The term "sequence identity" means that two polynucleotide or amino acid sequences are identical (i.e., on a nucleotide-by-nucleotide or residue-by-residue basis) over the comparison window. The term percentage of sequence identity is calculated by comparing two optimally aligned sequences over the window of comparison, determining the number of positions at which the identical nucleic acid base (*e.g.*, A, T, C, G, U, or I) or residue occurs in both sequences to yield the number of matched positions, dividing the number of matched positions by the total number of positions in the comparison window (*i.e.*, the window size), and multiplying the result by 100 to yield the percentage of sequence identity. The terms "substantial identity" as used herein denotes a characteristic of a polynucleotide or amino acid sequence, wherein the polynucleotide or amino acid comprises a sequence that has at least 85 percent sequence identity, preferably at least 90 to 95 percent sequence identity, more usually at least 99 percent sequence identity as compared to a reference sequence over a comparison window of at least 18 nucleotide (6 amino acid) positions, frequently over a window of at least 24-48 nucleotide (8-16 amino acid) positions, wherein the percentage of sequence identity is calculated by comparing the reference sequence to the sequence which may include deletions or additions which total 20 percent or less of the reference sequence over the comparison window. The reference sequence may be a subset of a larger sequence.

As used herein, the twenty conventional amino acids and their abbreviations follow conventional usage. *See* Immunology - A Synthesis (2nd Edition, E.S. Golub and D.R. Gren, Eds., Sinauer Associates, Sunderland, Mass. (1991)). Stereoisomers (*e.g.*, D-amino acids) of the twenty conventional amino acids, unnatural amino acids such as α-, α-disubstituted amino acids, N-alkyl amino acids, lactic acid, and other unconventional amino acids may also be suitable components for polypeptides described herein. Examples of unconventional amino acids include: 4-hydroxyproline, γ -carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine, and other similar amino acids and imino acids (*e.g.*, 4-hydroxyproline). In the polypeptide notation used herein, the lefthand direction is the amino terminal direction and the righthand direction is the carboxy-terminal direction, in accordance with standard usage and convention.

As applied to polypeptides, the term "substantial identity" means that two peptide sequences, when optimally aligned, such as by the programs GAP or BESTFIT using default gap weights, share at least 80 percent sequence identity, preferably at least 90 percent sequence identity, more preferably at least 95 percent sequence identity, and most preferably at least 99 percent sequence identity. Preferably, residue positions which are not identical differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, glutamic-aspartic, and asparagine-glutamine.

As discussed herein, minor variations in the amino acid sequences of antibodies or immunoglobulin molecules are contemplated herein, providing that the variations in the amino acid sequence maintain at least 75%, more preferably at least 80%, 90%, 95%, and most preferably 99% sequence identity to the antibodies or immunoglobulin molecules described herein. In particular, conservative amino acid replacements are contemplated. Conservative replacements are those that take place within a family of amino acids that are related in their side chains. Genetically encoded amino acids are generally divided into families: (1) acidic=aspartate, glutamate; (2) basic=lysine, arginine, histidine; (3) non-polar=alanine, valine, leucine, isoleucine, proline, phenylalanine, methionine, tryptophan; and (4) uncharged polar=glycine, asparagine, glutamine, cysteine, serine, threonine, tyrosine. More preferred families are: serine and threonine are aliphatic-hydroxy family; asparagine and glutamine are an amide-containing family; alanine, valine, leucine and isoleucine are an aliphatic family; and phenylalanine, tryptophan, and tyrosine are an aromatic family. For example, it is reasonable to expect that an isolated replacement of a leucine with an isoleucine or valine, an aspartate with a glutamate, a threonine with a serine, or a similar replacement of an amino acid with a structurally related amino acid will not have a major effect on the binding or properties of the resulting molecule, especially if the replacement does not involve an amino acid within a framework site. Whether an amino acid change results in a functional peptide can readily be determined by assaying the specific activity of the polypeptide derivative. Assays are described in detail herein. Fragments or analogs of antibodies or immunoglobulin molecules can be readily prepared by those of ordinary skill in the art. Preferred amino- and carboxy-termini of fragments or analogs occur near boundaries of functional domains. Structural and functional domains can be identified by comparison of the nucleotide and/or amino acid sequence data to public or proprietary sequence databases. Preferably, computerized comparison methods are used to identify sequence motifs or predicted protein conformation domains that occur in other proteins of known structure and/or function. Methods to identify protein sequences that fold into a known three-dimensional structure are known. Bowie et al., Science, 253:164 (1991). Thus, the foregoing examples demonstrate that those of skill in the art can recognize sequence motifs and structural conformations that may be used to define structural and functional domains described herein.

Preferred amino acid substitutions are those which: (1) reduce susceptibility to proteolysis, (2) reduce susceptibility to oxidation, (3) alter binding affinity for forming protein complexes, (4) alter binding affinities, and (4) confer or modify other physicochemical or functional properties of such analogs. Analogs can include various muteins of a sequence other than the naturally-occurring peptide sequence. For example, single or multiple amino acid substitutions (preferably conservative amino acid substitutions) may be made in the naturally-occurring sequence (preferably in the portion of the polypeptide outside the domain(s) forming intermolecular contacts). A conservative amino acid substitution should not substantially change the structural characteristics of the parent sequence (*e.g.*, a replacement amino acid should not tend to break a helix that occurs in the parent sequence, or disrupt other types of secondary structure that characterizes the parent sequence). Examples of art-recognized polypeptide secondary and tertiary structures are described in Proteins, Structures and Molecular Principles (Creighton, Ed., W. H. Freeman and Company, New York (1984)); Introduction to Protein Structure (C. Branden and J. Tooze, eds., Garland Publishing, New York, N.Y. (1991)); and Thornton et al., Nature, 354:105 (1991).

The term "polypeptide fragment" as used herein refers to a polypeptide that has an amino-terminal and/or carboxy-terminal deletion, but where the remaining amino acid sequence is identical to the corresponding positions in the naturally-occurring sequence deduced, for example, from a full-length cDNA sequence. Fragments typically are at least 5, 6, 8 or 10 amino acids long, preferably at least 14 amino acids long, more preferably at least 20 amino acids long, usually at least 50 amino acids long, and even more preferably at least 70 amino acids long. The term "analog" as used herein refers to polypeptides which are comprised of a segment of at least 25 amino acids that has substantial identity to a portion of a deduced amino acid sequence and which has at least one of the following properties: (1) specific binding to a TIM-1, under suitable binding conditions, (2) ability to block appropriate TIM-1 binding, or (3) ability to inhibit the growth and/or survival of TIM-1 expressing cells *in vitro* or *in vivo*. Typically, polypeptide analogs comprise a conservative amino acid substitution (or addition or deletion) with respect to the naturally occurring sequence. Analogs typically are at least 20 amino acids long, preferably at least 50 amino acids long or longer, and can often be as long as a full-length naturally-occurring polypeptide.

Peptide analogs are commonly used in the pharmaceutical industry as non-peptide drugs with properties analogous to those of the template peptide. These types of non-peptide compounds are termed peptide mimetics or peptidomimetics. Fauchere, J. Adv. Drug Res., 15:29 (1986); Veber and Freidinger, TINS, p.392 (1985); and Evans et al., J. Med. Chem., 30:1229 (1987). Such compounds are often developed with the aid of computerized molecular modeling. Peptide mimetics that are structurally similar to therapeutically useful peptides may be used to produce an equivalent therapeutic or prophylactic effect. Generally, peptidomimetics are structurally similar to a paradigm polypeptide (i.e., a polypeptide that has a biochemical property or pharmacological activity), such as human antibody, but have one or more peptide linkages optionally replaced by a linkage selected from the group consisting of: $--CH_2NH--$, $--CH_2S--$, $--CH_2-CH_2--$, --CH=CH--(cis and trans), $--COCH_2--$, $--CH(OH)CH_2--$, and $-CH_2SO--$, by methods well known in the art. Systematic substitution of one or more amino acids of a consensus sequence with a D-amino acid of the same type (*e.g.*, D-lysine in place of L-lysine) may be used to generate more stable peptides. In addition, constrained peptides comprising a consensus sequence or a substantially identical consensus sequence variation may be generated by methods known in the art (Rizo and Gierasch, Ann. Rev. Biochem., 61:387 (1992); for example, by adding internal cysteine residues capable of forming intramolecular disulfide bridges which cyclize the peptide.

"Antibody" or "antibody peptide(s)" refer to an intact antibody, or a binding fragment thereof that competes with the intact antibody for specific binding. Binding fragments are produced by recombinant DNA techniques, or by enzymatic or chemical cleavage of intact antibodies. Binding fragments include Fab, Fab', $F(ab')_2$, Fv, and single-chain antibodies. An antibody other than a bispecific or bifunctional antibody is understood to have each of its binding sites identical. An antibody substantially inhibits adhesion of a receptor to a counterreceptor when an excess of antibody reduces the quantity of receptor bound to counterreceptor by at least about 20%, 40%, 60% or 80%, and more usually greater than about 85% (as measured in *an in vitro* competitive binding assay).

Digestion of antibodies with the enzyme, papain, results in two identical antigen-binding fragments, known also as "Fab" fragments, and a "Fc" fragment, having no antigen-binding activity but having the ability to crystallize. Digestion of antibodies with the enzyme, pepsin, results in the a "$F(ab')_2$" fragment in which the two arms of the antibody molecule remain linked and comprise two-antigen binding sites. The $F(ab')_2$ fragment has the ability to crosslink antigen.

"Fv" when used herein refers to the minimum fragment of an antibody that retains both antigen-recognition and antigen-binding sites.

"Fab" when used herein refers to a fragment of an antibody which comprises the constant domain of the light chain and the CH1 domain of the heavy chain.

The term "epitope" includes any protein determinant capable of specific binding to an immunoglobulin or T-cell receptor. Epitopic determinants usually consist of chemically active surface groupings of molecules such as amino acids or sugar side chains and usually have specific three dimensional structural characteristics, as well as specific charge characteristics. An antibody is said to specifically bind an antigen when the dissociation constant is $\leq 1$ $\mu$M, preferably $\leq$ 100 nM and most preferably $\leq$ 10 nM.

The term "agent" is used herein to denote a chemical compound, a mixture of chemical compounds, a biological macromolecule, or an extract made from biological materials.

The term "pharmaceutical agent" or "drug" as used herein refers to a chemical compound or composition capable of inducing a desired therapeutic effect when properly administered to a patient. Other chemistry terms herein are used according to conventional usage in the art, as exemplified by The McGraw-Hill Dictionary of Chemical Terms (Parker, S., Ed., McGraw-Hill, San Francisco (1985)).

The term "antineoplastic agent" is used herein to refer to agents that have the functional property of inhibiting a development or progression of a neoplasm in a human, particularly a malignant (cancerous) lesion, such as a carcinoma, sarcoma, lymphoma, or leukemia. Inhibition of metastasis is frequently a property of antineoplastic agents.

As used herein, "substantially pure" means an object species is the predominant species present (i.e., on a molar basis it is more abundant than any other individual species in the composition), and preferably a substantially purified fraction is a composition wherein the object species comprises at least about 50 percent (on a molar basis) of all macromolecular species present Generally, a substantially pure composition will comprise more than about 80 percent of all macromolecular species present in the composition, more preferably more than about 85%, 90%, 95%, and 99%. Most preferably, the object species is purified to essential homogeneity (contaminant species cannot be detected in the composition by conventional detection methods) wherein the composition consists essentially of a single macromolecular species.

"Active" or "activity" in regard to a TIM-1 polypeptide refers to a portion of a TIM-1 polypeptide which has a biological or an immunological activity of a native TIM-1 polypeptide. "Biological" when used herein refers to a biological function that results from the activity of the native TIM-1 polypeptide. A preferred biological activity includes, for example, regulation of cellular growth.

"Label" or "labeled" as used herein refers to the addition of a detectable moiety to a polypeptide, for example, a radiolabel, fluorescent label, enzymatic label chemiluminescent labeled or a biotinyl group. Radioisotopes or radionuclides may include $^{3}H$, $^{14}C$, $^{15}N$, $^{35}S$, $^{90}Y$, $^{99}Tc$, $^{111}In$, $^{125}I$, $^{131}I$, fluorescent labels may include rhodamine, lanthanide phosphors or FITC and enzymatic labels may include horseradish peroxidase, β-galactosidase, luciferase, alkaline phosphatase.

"Mammal" when used herein refers to any animal that is considered a mammal. Preferably, the mammal is human.

"Liposome" when used herein refers to a small vesicle that may be useful for delivery of drugs that may include the TIM-1 polypeptide described herein or antibodies to such a TIM-1 polypeptide to a mammal.

The term "patient" includes human and veterinary subjects.

Antibody Structure

The basic whole antibody structural unit is known to comprise a tetramer. Each tetramer is composed of two identical pairs of polypeptide chains, each pair having one "light" (about 25 kDa) and one "heavy" chain (about 50-70 kDa). The amino-terminal portion of each chain includes a variable domain of about 100 to 110 or more amino acids primarily responsible for antigen recognition. The carboxy-terminal portion of each chain defines a constant region primarily responsible for effector function. Human light chains are classified as kappa and lambda light chains. Human heavy chains are classified as mu, delta, gamma, alpha, or epsilon, and define the antibody's isotype as IgM, IgG, IgA, and IgE, respectively. Within light and heavy chains, the variable and constant regions are joined by a "J" region of about 12 or more amino acids, with the heavy chain also including a "D" region of about 10 more amino acids. *See generally,* Fundamental Immunology Ch. 7 (Paul, W., ed., 2d ed. Raven Press, N.Y. (1989)). The variable regions of each light/heavy chain pair form the antibody binding site.

The variable domains all exhibit the same general structure of relatively conserved framework regions (FR) joined by three hyper variable regions, also called complementarity determining regions or CDRs. The CDRs from the heavy and light chains of each pair are aligned by the framework regions, enabling binding to a specific epitope. From N-terminal to C-terminal, both light and heavy chains comprise the domains FR1, CDR1, FR2, CDR2, FR3, CDR3 and FR4. The assignment of amino acids to each region is in accordance with the definitions of Kabat, Sequences of Proteins of Immunological Interest (National Institutes of Health, Bethesda, Md. (1987 and 1991)), or Chothia & Lesk, J. Mol. Biol. 196:901-917 (1987); Chothia et al., Nature 342:878-883 (1989).

A bispecific or bifunctional antibody is an artificial hybrid antibody having two different heavy/light chain pairs and two different binding sites. Bispecific antibodies can be produced by a variety of methods including fusion of hybridomas or linking of Fab' fragments. *See, e.g.*, Songsivilai & Lachmann, Clin. Exp. Immunol. 79: 315-321 (1990), Kostelny et al., J. Immunol. 148:1547-1553 (1992). Bispecific antibodies do not exist in the form of fragments having a single binding site (e.g., Fab, Fab', and Fv).

It will be appreciated that such bifunctional or bispecific antibodies are contemplated herein. A bispecific single chain antibody with specificity to TIM-1 and to the CD3 antigen on cytotoxic T lymphocytes can be used to direct these T cells to tumor cells expressing TIM-1 and cause apoptosis and eradication of the tumor. Two bispecific scFv constructs for this purpose are described herein. The scFv components specific for TIM-1 can be derived from anti-TIM-1 antibodies described herein. The anti-TIM-1 antibody components disclosed in Tables 4 and 5 can be used to generate a biologically active scFv directed against TIM-1. Preferably, the scFv components are derived from mAb 2.70. The anti-CD3 scFv component of the therapeutic bispecific scFv was derived from a sequence deposited in Genbank (accession number CAE85148). Alternative antibodies known to target CD3 or other T cell antigens may similarly be effective in treating malignancies when coupled with anti-TIM-1, whether on a single-chain backbone or a full IgG.

Human Antibodies and Humanization of Antibodies

Human antibodies avoid certain of the problems associated with antibodies that possess murine or rat variable and/or constant regions. The presence of such murine or rat derived proteins can lead to the rapid clearance of the antibodies or can lead to the generation of an immune response against the antibody by a mammal other than a rodent.

Human Antibodies

The ability to clone and reconstruct megabase-sized human loci in YACs and to introduce them into the mouse germline provides a powerful approach to elucidating the functional components of very large or crudely mapped loci as well as generating useful models of human disease. An important practical application of such a strategy is the "humanization" of the mouse humoral immune system. Introduction of human immunoglobulin (Ig) loci into mice in which the endogenous Ig genes have been inactivated offers the opportunity to develop human antibodies in the mouse. Fully human antibodies are expected to minimize the immunogenic and allergic responses intrinsic to mouse or mouse-derivatized Mabs and thus to increase the efficacy and safety of the antibodies administered to humans. The use of fully human antibodies can be expected to provide a substantial advantage in the treatment of chronic and recurring human diseases, such as inflammation, autoimmunity, and cancer, which require repeated antibody administrations.

One approach toward this goal was to engineer mouse strains deficient in mouse antibody production with large fragments of the human Ig loci in anticipation that such mice would produce a large repertoire of human antibodies in the absence of mouse antibodies. This general strategy was demonstrated in connection with our generation of the first XenoMouse® strains as published in 1994. *See* Green et al., Nature Genetics 7:13-21 (1994). The XenoMouse® strains were engineered with yeast artificial chromosomes (YACs) containing 245 kb and 190 kb-sized germline configuration fragments of the human heavy chain locus and kappa light chain locus, respectively, which contained core variable and constant region sequences. Id. The XENOMOUSE® strains are available from Abgenix, Inc. (Fremont, CA). Greater than approximately 80% of the human antibody repertoire has been introduced through introduction of megabase sized, germline configuration YAC fragments of the human heavy chain loci and kappa light chain loci, respectively, to produce XenoMouse® mice.

The production of the XENOMOUSE® is further discussed and delineated in U.S. Patent Application Serial Nos. 07/466,008, filed January 12, 1990, 07/610,515, filed November 8, 1990, 07/919,297, filed July 24, 1992, 07/922,649, filed July 30, 1992, filed 08/031,801, filed March 15,1993, 08/112,848, filed August 27, 1993, 08/234,145, filed April 28, 1994, 08/376,279, filed January 20, 1995, 08/430, 938, April 27, 1995, 08/464,584, filed June 5, 1995, 08/464,582, filed June 5, 1995, 08/463,191, filed June 5, 1995, 08/462,837, filed June 5, 1995, 08/486,853, filed June 5, 1995, 08/486,857, filed June 5, 1995, 08/486,859, filed June 5, 1995, 08/462,513, filed June 5, 1995, 08/724,752, filed October 2, 1996, and 08/759,620, filed December 3, 1996 and U.S. Patent Nos. 6,162,963, 6,150,584, 6,114,598, 6,075,181, and 5,939,598 and Japanese Patent Nos. 3 068 180 B2, 3 068 506 B2, and 3 068 507 B2. *See also* Mendez et al., Nature Genetics 15:146-156 (1997) and Green and Jakobovits, J. Exp. Med. 188:483-495 (1998). *See also* European Patent No. EP 0 463 151 B1, grant published June 12, 1996, International Patent Application No., WO 94/02602, published February 3, 1994, International Patent Application No., WO 96/34096, published October 31, 1996, WO 98/24893, published June 11, 1998, WO 00/76310, published December 21, 2000.

Alternative approaches have utilized a "minilocus" approach, in which an exogenous Ig locus is mimicked through the inclusion of pieces (individual genes) from the Ig locus. Thus, one or more $V_H$ genes, one or more $D_H$ genes, one or more $J_H$ genes, a mu constant region, and a second constant region (preferably a gamma constant region) are formed into a construct for insertion into an animal. This approach is described in U.S. Patent No. 5,545,807 to Surani et al. and U.S. Patent Nos. 5,545,806, 5,625,825, 5,625,126, 5,633,425, 5,661,016, 5,770,429, 5,789,650, 5,814,318, 5,877,397, 5,874,299, and 6,255,458 each to Lonberg and Kay, U.S. Patent No. 5,591,669 and 6,023,010 to Krimpenfort and Berns, U.S. Patent Nos. 5,612,205, 5,721,367, and 5,789,215 to Berns et al., and U.S. Patent No. 5,643,763 to Choi and Dunn, and GenPharm International U.S. Patent Application Serial Nos. 07/574,748, filed August 29, 1990, 07/575,962, filed August 31, 1990, 07/810,279, filed December 17, 1991, 07/853,408, filed March 18, 1992, 07/904,068, filed June 23, 1992, 07/990,860, filed December 16, 1992, 08/053,131, filed April 26, 1993, 08/096,762, filed July 22, 1993, 08/155,301, filed November 18, 1993, 08/161,739, filed December 3, 1993, 08/165,699, filed December 10, 1993, 08/209,741, filed March 9, 1994. *See also* European Patent No. 0 546 073 B1, International Patent Application Nos. WO 92/03918, WO 92/22645, WO 92/22647, WO 92/22670, WO 93/12227, WO 94/00569, WO 94/25585, WO 96/14436, WO 97/13852, and WO 98/24884 and U.S. Patent No. 5,981,175. *See further* Taylor *et al.*, 1992, Chen *et al.*, 1993, Tuaillon *et al.*, 1993, *Choi et al.*, 1993, Lonberg *et al.*, (1994), Taylor *et al.*, (1994), and Tuaillon *et al.*, (1995), Fishwild *et al.*, (1996).

While chimeric antibodies have a human constant region and a murine variable region, it is expected that certain human anti-chimeric antibody (HACA) responses will be observed, particularly in chronic or multi-dose utilizations of the antibody. Thus, it would be desirable to provide fully human antibodies against TIM-1 in order to vitiate concerns and/or effects of human anti-mouse antibody (HAMA) or HACA response.

Humanization and Display Technologies

Antibodies with reduced immunogenicity can be generated using humanization and library display techniques. It will be appreciated that antibodies can be humanized or primatized using techniques well known in the art. *See e.g.*, Winter and Harris, Immunol Today 14:43-46 (1993) and Wright et al., Crit, Reviews in Immunol. 12:125-168 (1992). The antibody of interest can be engineered by recombinant DNA techniques to substitute the CH1, CH2, CH3, hinge domains, and/or the framework domain with the corresponding human sequence (*see* WO 92/02190 and U.S. Patent Nos. 5,530,101, 5,585,089, 5,693,761, 5,693,792, 5,714,350, and 5,777,085). Also, the use of Ig cDNA for construction of chimeric immunoglobulin genes is known in the art (Liu et al., P.N.A.S 84:3439 (1987) and J. Immunol. 139:3521 (1987)). mRNA is isolated from a hybridoma or other cell producing the antibody and used to produce cDNA. The cDNA of interest can be amplified by the polymerase chain reaction using specific primers (U.S. Pat. Nos. 4,683,195 and 4,683,202). Alternatively, an expression library is made and screened to isolate the sequence of interest encoding the variable region of the antibody is then fused to human constant region sequences. The sequences of human constant regions genes can be found in Kabat et al., "Sequences of Proteins of Immunological Interest," N.I.H. publication no. 91-3242 (1991). Human C region genes are readily available from known clones. The choice of isotype will be guided by the desired effector functions, such as complement fixation, or activity in antibody-dependent cellular cytotoxicity. Preferred isotypes are IgG1, IgG2 and IgG4. Either of the human light chain constant regions, kappa or lambda, can be used. The chimeric, humanized antibody is then expressed by conventional methods. Expression vectors include plasmids, retroviruses, YACs, EBV derived episomes, and the like.

Antibody fragments, such as Fv, $F(ab')_2$ and Fab can be prepared by cleavage of the intact protein, e.g., by protease or chemical cleavage. Alternatively, a truncated gene is designed. For example, a chimeric gene encoding a portion of the $F(ab')_2$ fragment would include DNA sequences encoding the CH1 domain and hinge region of the H chain, followed by a translational stop codon to yield the truncated molecule.

Consensus sequences of H and L J regions can be used to design oligonucleotides for use as primers to introduce useful restriction sites into the J region for subsequent linkage of V region segments to human C region segments. C region cDNA can be modified by site directed mutagenesis to place a restriction site at the analogous position in the human sequence.

Expression vectors include plasmids, retroviruses, YACs, EBV derived episomes, and the like. A convenient vector is one that encodes a functionally complete human CH or CL immunoglobulin sequence, with appropriate restriction sites engineered so that any VH or VL sequence can be easily inserted and expressed. In such vectors, splicing usually occurs between the splice donor site in the inserted J region and the splice acceptor site preceding the human C region, and also at the splice regions that occur within the human CH exons. Polyadenylation and transcription termination occur at native chromosomal sites downstream of the coding regions. The resulting chimeric antibody can be joined to any strong promoter, including retroviral LTRs, e.g., SV-40 early promoter, (Okayama et al., Mol. Cell. Bio. 3:280 (1983)), Rous sarcoma virus LTR (Gorman et al., P.N.A.S. 79:6777 (1982)), and moloney murine leukemia virus LTR (Grosschedl et al., Cell 41:885 (1985)). Also, as will be appreciated, native Ig promoters and the like can be used.

Further, human antibodies or antibodies from other species can be generated through display-type technologies, including, without limitation, phage display, retroviral display, ribosomal display, and other techniques, using techniques well known in the art and the resulting molecules can be subjected to additional maturation, such as affinity maturation, as such techniques are well known in the art. Wright and Harris, *supra.*, Hanes and Plucthau, PNAS USA 94:4937-4942 (1997) (ribosomal display), Parmley and Smith, Gene 73:305-318 (1988) (phage display), Scott, TIBS 17:241-245 (1992), Cwirla et al., PNAS USA 87:6378-6382 (1990), Russel et al., Nucl. Acids Res. 21:1081-1085 (1993), Hoganboom et al., Immunol. Reviews 130:43-68 (1992), Chiswell and McCafferty, TIBTECH 10:80-84 (1992), and U.S. Patent No. 5,733,743. If display technologies are utilized to produce antibodies that are not human, such antibodies can be humanized as described above.

Using these techniques, antibodies can be generated to TIM-1 expressing cells, TIM-1 itself, forms of TIM-1, epitopes or peptides thereof, and expression libraries thereto (*see e.g.* U.S. Patent No. 5,703,057) which can thereafter be screened as described above for the activities described above.

Antibody Therapeutics

In certain respects, it can be desirable in connection with the generation of antibodies as therapeutic candidates against TIM-1 that the antibodies be capable of fixing complement and participating in complement-dependent cytotoxicity (CDC). Such antibodies include, without limitation, the following: murine IgM, murine IgG2a, murine IgG2b, murine IgG3, human IgM, human IgG1, and human IgG3. It will be appreciated that antibodies that are generated need not initially possess such an isotype but, rather, the antibody as generated can possess any isotype and the antibody can be isotype switched thereafter using conventional techniques that are well known in the art. Such techniques include the use of direct recombinant techniques (*see, e.g.*, U.S. Patent No. 4,816,397), cell-cell fusion techniques (*see*, *e.g.*, U.S. Patent Nos. 5,916,771 and 6,207,418), among others.

In the cell-cell fusion technique, a myeloma or other cell line is prepared that possesses a heavy chain with any desired isotype and another myeloma or other cell line is prepared that possesses the light chain. Such cells can, thereafter, be fused and a cell line expressing an intact antibody can be isolated.

By way of example, the TIM-1 antibody discussed herein is a human anti-TIM-1 IgG2 antibody. If such antibody possessed desired binding to the TIM-1 molecule, it could be readily isotype switched to generate a human IgM, human IgG1, or human IgG3 isotype, while still possessing the same variable region (which defines the antibody's specificity and some of its affinity). Such molecule would then be capable of fixing complement and participating in CDC.

Design and Generation of Other Therapeutics

Due to their association with renal and pancreatic tumors, head and neck cancer, ovarian cancer, gastric (stomach) cancer, melanoma, lymphoma, prostate cancer, liver cancer, breast cancer, lung cancer, renal cancer, bladder cancer, colon cancer, esophageal cancer, and brain cancer, antineoplastic agents comprising anti-TIM-1 antibodies are described herein.

Moreover, based on the activity of the antibodies that are produced and characterized herein with respect to TIM-1, the design of other therapeutic modalities beyond antibody moieties is facilitated. Such modalities include, without limitation, advanced antibody therapeutics, such as bispecific antibodies, immunotoxins, and radiolabeled therapeutics, generation of peptide therapeutics, gene therapies, particularly intrabodies, antisense therapeutics, and small molecules.

In connection with the generation of advanced antibody therapeutics, where complement fixation is a desirable attribute, it can be possible to sidestep the dependence on complement for cell killing through the use of bispecifics, immunotoxins, or radiolabels, for example.

For example, in connection with bispecific antibodies, bispecific antibodies can be generated that comprise (i) two antibodies one with a specificity to TIM-1 and another to a second molecule that are conjugated together, (ii) a single antibody that has one chain specific to TIM-1 and a second chain specific to a second molecule, or (iii) a single chain antibody that has specificity to TIM-1 and the other molecule. Such bispecific antibodies can be generated using techniques that are well known for example, in connection with (i) and (ii) *see, e.g.*, Fanger et al., Immunol Methods 4:72-81 (1994) and Wright and Harris, *supra* and in connection with (iii) *see, e.g.,* Traunecker et al., Int. J. Cancer (Suppl.) 7: 51-52 (1992). In each case, the second specificity can be made to the heavy chain activation receptors, including, without limitation, CD16 or CD64 (*see, e.g.*, Deo et al., 18:127 (1997)) or CD89 (*see, e.g.*, Valerius et al., Blood 90: 4485-4492 (1997)). Bispecific antibodies prepared in accordance with the foregoing would be likely to kill cells expressing TIM-1, and particularly those cells in which the TIM-1 antibodies described herein are effective.

With respect to immunotoxins, antibodies can be modified to act as immunotoxins utilizing techniques that are well known in the art. *See, e.g.*, Vitetta, Immunol Today 14:252 (1993). *See also* U.S. Patent No. 5,194,594. In connection with the preparation of radiolabeled antibodies, such modified antibodies can also be readily prepared utilizing techniques that are well known in the art. *See, e.g.,* Junghans et al., in Cancer Chemotherapy and Biotherapy 655-686 (2d ed., Chafner and Longo, eds., Lippincott Raven (1996)). *See also* U.S. Patent Nos. 4,681,581, 4,735,210, 5,101,827, 5,102,990 (RE 35,500), 5,648,471, and 5,697,902. Each of immunotoxins and radiolabeled molecules would be likely to kill cells expressing TIM-1, and particularly those cells in which the antibodies described herein are effective.

In connection with the generation of therapeutic peptides, through the utilization of structural information related to TIM-1 and antibodies thereto, such as the antibodies described herein (as discussed below in connection with small molecules) or screening of peptide libraries, therapeutic peptides can be generated that are directed against TIM-1. Design and screening of peptide therapeutics is discussed in connection with Houghten et al., Biotechniques 13:412-421 (1992), Houghten, PNAS USA 82:5131-5135 (1985), Pinalla et al., Biotechniques 13:901-905 (1992), Blake and Litzi-Davis, BioConjugate Chem. 3:510-513 (1992). Immunotoxins and radiolabeled molecules can also be prepared, and in a similar manner, in connection with peptidic moieties as discussed above in connection with antibodies.

Assuming that the TIM-1 molecule (or a form, such as a splice variant or alternate form) is functionally active in a disease process, it will also be possible to design gene and antisense therapeutics thereto through conventional techniques. Such modalities can be utilized for modulating the function of TIM-1. In connection therewith the antibodies, as described herein, facilitate design and use of functional assays related thereto. A design and strategy for antisense therapeutics is discussed in detail in International Patent Application No. WO 94/29444. Design and strategies for gene therapy are well known. However, in particular, the use of gene therapeutic techniques involving intrabodies could prove to be particularly advantageous. *See, e.g.*, Chen et al., Human Gene Therapy 5:595-601 (1994) and Marasco, Gene Therapy 4:11-15 (1997). General design of and considerations related to gene therapeutics is also discussed in International Patent Application No. WO 97/38137.

Small molecule therapeutics can also be envisioned. Drugs can be designed to modulate the activity of TIM-1, as described herein. Knowledge gleaned from the structure of the TIM-1 molecule and its interactions with other molecules, as described herein, such as the antibodies described herein, and others can be utilized to rationally design additional therapeutic modalities. In this regard, rational drug design techniques such as X-ray crystallography, computer-aided (or assisted) molecular modeling (CAMM), quantitative or qualitative structure-activity relationship (QSAR), and similar technologies can be utilized to focus drug discovery efforts. Rational design allows prediction of protein or synthetic structures which can interact with the molecule or specific forms thereof which can be used to modify or modulate the activity of TIM-1. Such structures can be synthesized chemically or expressed in biological systems. This approach has been reviewed in Capsey et al., Genetically Engineered Human Therapeutic Drugs (Stockton Press, NY (1988)). Further, combinatorial libraries can be designed and synthesized and used in screening programs, such as high throughput screening efforts.

TIM-1 Agonists And Antagonists

Also herein described are variants of a TIM-1 protein that function as either TIM-1 agonists (mimetics) or as TIM-1 antagonists. Variants of a TIM-1 protein can be generated by mutagenesis, *e.g.*, discrete point mutation or truncation of the TIM-1 protein. An agonist of the TIM-1 protein can retain substantially the same, or a subset of, the biological activities of the naturally occurring form of the TIM-1 protein. An antagonist of the TIM-1 protein can inhibit one or more of the activities of the naturally occurring form of the TIM-1 protein by, for example, competitively binding to a downstream or upstream member of a cellular signaling cascade which includes the TIM-1 protein. Thus, specific biological effects can be elicited by treatment with a variant of limited function. Treatment of a subject with a variant having a subset of the biological activities of the naturally occurring form of the protein has fewer side effects in a subject relative to treatment with the naturally occurring form of the TIM-1 protein.

Variants of the TIM-1 protein that function as either TIM-1 agonists (mimetics) or as TIM-1 antagonists can be identified by screening combinatorial libraries of mutants, *e.g.*, truncation mutants, of the TIM-1 protein for protein agonist or antagonist activity. A variegated library of TIM-1 variants is generated by combinatorial mutagenesis at the nucleic acid level and is encoded by a variegated gene library. A variegated library of TIM-1 variants can be produced by, for example, enzymatically ligating a mixture of synthetic oligonucleotides into gene sequences such that a degenerate set of potential TIM-1 sequences is expressible as individual polypeptides, or alternatively, as a set of larger fusion proteins (*e.g.*, for phage display) containing the set of TIM-1 sequences therein. There are a variety of methods which can be used to produce libraries of potential TIM-1 variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential TIM-1 variant sequences. Methods for synthesizing degenerate oligonucleotides are known in the art (*see, e.g.*, Narang, Tetrahedron 39:3 (1983); Itakura et al., Annu. Rev. Biochem. 53:323 (1984); Itakura et al., Science 198:1056 (1984); Ike et al., Nucl. Acid Res. 11:477 (1983).

Radioimmuno & Immunochemotherapeutic Antibodies

Cytotoxic chemotherapy or radiotherapy of cancer is limited by serious, sometimes life-threatening, side effects that arise from toxicities to sensitive normal cells because the therapies are not selective for malignant cells. Therefore, there is a need to improve the selectivity. One strategy is to couple therapeutics to antibodies that recognize tumor-associated antigens. This increases the exposure of the malignant cells to the ligand-targeted therapeutics but reduces the exposure of normal cells to the same agent. *See* Allen, Nat. Rev. Cancer 2(10):750-63 (2002).

The TIM-1 antigen is one of these tumor-associated antigens, as shown by its specific expression on cellular membranes of tumor cells by FACS and IHC. Therefore monoclonal antibodies directed against the TIM-1 antigen coupled to cytotoxic chemotherapic agents or radiotherapic agents may be used as anti-tumor therapeutics.

Radiolabels are known in the art and have been used for diagnostic or therapeutic radioimmuno conjugates. Examples of radiolabels includes, but are not limited to, the following: radioisotopes or radionuclides (e.g., 3H, 14C, 15N, 35S, 90Y, 99Tc, 111In, 125I, 131I, 177Lu, Rhenium-186, Rhenium-188, Samarium-153, Copper-64, Scandium-47). For example, radionuclides which have been used in radioimmunoconjugate guided clinical diagnosis include, but are not limited to: 131 I, 125 I, 123 I, 99 Tc, 67 Ga, as well as 111 In. Antibodies have also been labeled with a variety of radionuclides for potential use in targeted immunotherapy (*see* Peirersz *et al.*, 1987). Monoclonal antibody conjugates have also been used for the diagnosis and treatment of cancer (e.g., Immunol. Cell Biol. 65:111-125). These radionuclides include, for example, 188 Re and 186 Re as well as 90 Y, and to a lesser extent 199 Au and 67 Cu. I-(131) have also been used for therapeutic purposes. U.S. Patent No. 5,460,785 provides a listing of such radioisotopes. Radiotherapeutic chelators and chelator conjugates are known in the art. *See* U.S. Patent Nos. 4,831,175, 5,099,069, 5,246,692, 5,286,850, and 5,124,471.

Immunoradiopharmaceuticals utilizing anti-TIM-1 antibodies can be prepared utilizing techniques that are well known in the art. *See, e.g.,* Junghans et al., in Cancer Chemotherapy and Biotherapy 655-686 (2d ed., Chafner and Longo, eds., Lippincott Raven (1996)), U.S. Patent Nos. 4,681,581, 4,735,210, 5,101,827, RE 35,500, 5,648,471, and 5,697,902.

Cytotoxic immunoconjugates are known in the art and have been used as therapeutic agents. Such immunoconjugates may for example, use maytansinoids (U.S. Patent No. 6,441,163), tubulin polymerization inhibitor, auristatin (Mohammad et al., Int. J. Oncol. 15(2):367-72 (1999); Doronina et al., Nature Biotechnology 21(7):778-784 (2003)), dolastatin derivatives (Ogawa et al., Toxicol Lett. 121(2):97-106 (2001); 21(3)778-784), Mylotarg® (Wyeth Laboratories, Philadelphia, PA); maytansinoids (DM1), taxane or mertansine (ImmunoGen Inc.). Immunotoxins utilizing anti-TIM-1 antibodies may be prepared by techniques that are well known in the art. *See, e.g.*, Vitetta, Immunol Today 14:252 (1993); U.S. Patent No. 5,194,594.

Bispecific antibodies may be generated using techniques that are well known in the art for example, *see, e.g.*, Fanger et al., Immunol Methods 4:72-81 (1994); Wright and Harris, *supra*; Traunecker et al., Int. J. Cancer (Suppl.) 7: 51-52 (1992). In each case, the first specificity is to TIM-1, the second specificity may be made to the heavy chain activation receptors, including, without limitation, CD16 or CD64 (*see, e.g.*, Deo *et al.*, **18**:127 (1997)) or CD89 (*see, e.g.*, Valerius et al., Blood 90:4485-4492 (1997)). Bispecific antibodies prepared in accordance with the foregoing would kill cells expressing TIM-1.

Depending on the intended use of the antibody, i.e., as a diagnostic or therapeutic reagent, radiolabels are known in the art and have been used for similar purposes. For example, radionuclides which have been used in clinical diagnosis include, but are not limited to: $^{131}$I, $^{125}$I, $^{123}$I, $^{99}$Tc, $^{67}$Ga, as well as $^{111}$In. Antibodies have also been labeled with a variety of radionuclides for potential use in targeted immunotherapy. *See* Peirersz *et al.*, (1987). Monoclonal antibody conjugates have also been used for the diagnosis and treatment of cancer. *See, e.g.*, Immunol. Cell Biol. 65: 111-125. These radionuclides include, for example, .sup.188 Re and .sup.186 Re as well as .sup.90 Y, and to a lesser extent .sup.199 Au and .sup.67 Cu. I-(131) have also been used for therapeutic purposes. U.S. Pat. No. 5,460,785 provides a listing of such radioisotopes.

Patents relating to radiotherapeutic chelators and chelator conjugates are known in the art. For example, U.S. Pat. No. 4,831,175 of Gansow is directed to polysubstituted diethylenetriaminepentaacetic acid chelates and protein conjugates containing the same, and methods for their preparation. U.S. Pat. Nos. 5,099,069, 5,246,692, 5,286,850, and 5,124,471 of Gansow also relate to polysubstituted DTPA chelates.

Cytotoxic chemotherapies are known in the art and have been used for similar purposes. For example, U.S. Pat. No. 6,441,163 describes the process for the production of cytotoxic conjugates of maytansinoids and antibodies. The anti-tumor activity of a tubulin polymerization inhibitor, auristatin PE, is also known in the art. Mohammad et al., Int. J. Oncol. 15(2):367-72 (Aug 1999).

<u>Preparation of Antibodies</u>

Briefly, XenoMouse® lines of mice were immunized with TIM-1 protein, lymphatic cells (such as B-cells) were recovered from the mice that express antibodies and were fused with a myeloid-type cell line to prepare immortal hybridoma cell lines, and such hybridoma cell lines were screened and selected to identify hybridoma cell lines that produce antibodies specific to TIM-1. Alternatively, instead of being fused to myeloma cells to generate hybridomas, the recovered B cells, isolated from immunized XenoMouse® lines of mice, with reactivity against TIM-1 (determined by e.g. ELISA with TIM-1-His protein), were then isolated using a TIM-1-specific hemolytic plaque assay. Babcook et al., Proc. Natl. Acad. Sci. USA, 93:7843-7848 (1996). In this assay, target cells such as sheep red blood cells (SRBCs) were coated with the TIM-1 antigen. In the presence of a B cell culture secreting the anti-TIM-1 antibody and complement, the formation of a plaque indicates specific TIM-1-mediated lysis of the target cells. Single antigen-specific plasma cells in the center of the plaques were isolated and the genetic information that encodes the specificity of the antibody isolated from single plasma cells.

Using reverse-transcriptase PCR, the DNA encoding the variable region of the antibody secreted was cloned and inserted into a suitable expression vector, preferably a vector cassette such as a pcDNA, more preferably the pcDNA vector containing the constant domains of immunoglobulin heavy and light chain. The generated vector was then be transfected into host cells, preferably CHO cells, and cultured in conventional nutrient media modified as appropriate for inducing promoters, selecting transformants, or amplifying the genes encoding the desired sequences.

In general, antibodies produced by the above-mentioned cell lines possessed fully human IgG2 heavy chains with human kappa light chains. The antibodies possessed high affinities, typically possessing Kd's of from about 10-6 through about 10-11 M, when measured by either solid phase and solution phase. These mAbs can be stratified into groups or "bins" based on antigen binding competition studies, as discussed below.

As will be appreciated, antibodies, as described herein, can be expressed in cell lines other than hybridoma cell lines. Sequences encoding particular antibodies can be used for transformation of a suitable mammalian host cell. Transformation can be by any known method for introducing polynucleotides into a host cell, including, for example packaging the polynucleotide in a virus (or into a viral vector) and transducing a host cell with the virus (or vector) or by transfection procedures known in the art, as exemplified by U.S. Patent Nos. 4,399,216, 4,912,040, 4,740,461, and 4,959,455. The transformation procedure used depends upon the host to be transformed. Methods for introduction of heterologous polynucleotides into mammalian cells are well known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei.

Mammalian cell lines available as hosts for expression are well known in the art and include many immortalized cell lines available from the American Type Culture Collection (ATCC), including but not limited to Chinese hamster ovary (CHO) cells, HeLa cells, baby hamster kidney (BHK) cells, monkey kidney cells (COS), human hepatocellular carcinoma cells (e.g., Hep G2), and a number of other cell lines. Cell lines of particular preference are selected through determining which cell lines have high expression levels and produce antibodies with constitutive TIM-1 binding properties.

Therapeutic Administration and Formulations

The compounds of the disclosure are formulated according to standard practice, such as prepared in a carrier vehicle. The term "pharmacologically acceptable carrier" means one or more organic or inorganic ingredients, natural or synthetic, with which the mutant proto-oncogene or mutant oncoprotein is combined to facilitate its application. A suitable carrier includes sterile saline although other aqueous and nonaqueous isotonic sterile solutions and sterile suspensions known to be pharmaceutically acceptable are known to those of ordinary skill in the art. In this regard, the term "carrier" encompasses liposomes and the antibody (See Chen et al., Anal. Biochem. 227: 168-175 (1995) as well as any plasmid and viral expression vectors.

Any of the novel polypeptides of this disclosure may be used in the form of a pharmaceutically acceptable salt. Suitable acids and bases which are capable of forming salts with the polypeptides of the present disclosure are well known to those of skill in the art, and include inorganic and organic acids and bases.

A compound of the disclosure is administered to a subject in a therapeutically-effective amount, which means an amount of the compound which produces a medically desirable result or exerts an influence on the particular condition being treated. An effective amount of a compound of the disclosure is capable of ameliorating or delaying progression of the diseased, degenerative or damaged condition. The effective amount can be determined on an individual basis and will be based, in part, on consideration of the physical attributes of the subject, symptoms to be treated and results sought. An effective amount can be determined by one of ordinary skill in the art employing such factors and using no more than routine experimentation.

The compounds of the disclosure may be administered in any manner which is medically acceptable. This may include injections, by parenteral routes such as intravenous, intravascular, intraarterial, subcutaneous, intramuscular, intratumor, intraperitoneal, intraventricular, intraepidural, or others as well as oral, nasal, ophthalmic, rectal, or topical. Sustained release administration is also specifically included in the disclosure, by such means as depot injections or erodible implants. Localized delivery is particularly contemplated, by such means as delivery via a catheter to one or more arteries, such as the renal artery or a vessel supplying a localized tumor.

Biologically active anti-TIM-1 antibodies as described herein can be used in a sterile pharmaceutical preparation or formulation to reduce the level of serum TIM-1 thereby effectively treating pathological conditions where, for example, serum TIM-1 is abnormally elevated. Anti-TIM-1 antibodies preferably possess adequate affinity to potently suppress TIM-1 to within the target therapeutic range, and preferably have an adequate duration of action to allow for infrequent dosing. A prolonged duration of action will allow for less frequent and more convenient dosing schedules by alternate parenteral routes such as subcutaneous or intramuscular injection.

When used for *in vivo* administration, the antibody formulation must be sterile. This is readily accomplished, for example, by filtration through sterile filtration membranes, prior to or following lyophilization and reconstitution. The antibody ordinarily will be stored in lyophilized form or in solution. Therapeutic antibody compositions generally are placed into a container having a sterile access port, for example, an intravenous solution bag or vial having an adapter that allows retrieval of the formulation, such as a stopper pierceable by a hypodermic injection needle.

The route of antibody administration is in accord with known methods, e.g., injection or infusion by intravenous, intraperitoneal, intracerebral, intramuscular, intraocular, intraarterial, intrathecal, inhalation or intralesional routes, or by sustained release systems as noted below. The antibody is preferably administered continuously by infusion or by bolus injection.

An effective amount of antibody to be employed therapeutically will depend, for example, upon the therapeutic objectives, the route of administration, and the condition of the patient. Accordingly, it is preferred that the therapist titer the dosage and modify the route of administration as required to obtain the optimal therapeutic effect. Typically, the clinician will administer antibody until a dosage is reached that achieves the desired effect. The progress of this therapy is easily monitored by conventional assays or by the assays described herein.

Antibodies, as described herein, can be prepared in a mixture with a pharmaceutically acceptable carrier. This therapeutic composition can be administered intravenously or through the nose or lung, preferably as a liquid or powder aerosol (lyophilized). The composition can also be administered parenterally or subcutaneously as desired. When administered systemically, the therapeutic composition should be sterile, pyrogen-free and in a parenterally acceptable solution having due regard for pH, isotonicity, and stability. These conditions are known to those skilled in the art. Briefly, dosage formulations of the compounds described herein are prepared for storage or administration by mixing the compound having the desired degree of purity with physiologically acceptable carriers, excipients, or stabilizers. Such materials are non-toxic to the recipients at the dosages and concentrations employed, and include buffers such as TRIS HCl, phosphate, citrate, acetate and other organic acid salts; antioxidants such as ascorbic acid; low molecular weight (less than about ten residues) peptides such as polyarginine, proteins, such as serum albumin, gelatin, or immunoglobulins; hydrophilic polymers such as polyvinylpyrrolidinone; amino acids such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium and/or nonionic surfactants such as TWEEN, PLURONICS or polyethyleneglycol.

Sterile compositions for injection can be formulated according to conventional pharmaceutical practice as described in Remington: The Science and Practice of Pharmacy (20th ed, Lippincott Williams & Wilkens Publishers (2003)). For example, dissolution or suspension of the active compound in a vehicle such as water or naturally occurring vegetable oil like sesame, peanut, or cottonseed oil or a synthetic fatty vehicle like ethyl oleate or the like can be desired. Buffers, preservatives, antioxidants and the like can be incorporated according to accepted pharmaceutical practice.

Suitable examples of sustained-release preparations include semipermeable matrices of solid hydrophobic polymers containing the polypeptide, which matrices are in the form of shaped articles, films or microcapsules. Examples of sustained-release matrices include polyesters, hydrogels (e.g., poly(2-hydroxyethyl-methacrylate) as described by Langer et al., J. Biomed Mater. Res., (1981) 15:167-277 and Langer, Chem. Tech., (1982) 12:98-105, or poly(vinylalcohol)), polylactides (U.S. Pat. No. 3,773,919, EP 58,481), copolymers of L-glutamic acid and gamma ethyl-L-glutamate (Sidman et al., Biopolymers, (1983) 22:547-556), non-degradable ethylene-vinyl acetate (Langer *et al.*, *supra*), degradable lactic acid-glycolic acid copolymers such as the LUPRON Depot™ (injectable microspheres composed of lactic acid-glycolic acid copolymer and leuprolide acetate), and poly-D-(-)-3-hydroxybutyric acid (EP 133,988).

While polymers such as ethylene-vinyl acetate and lactic acid-glycolic acid enable release of molecules for over 100 days, certain hydrogels release proteins for shorter time periods. When encapsulated proteins remain in the body for a long time, they can denature or aggregate as a result of exposure to moisture at 37°C, resulting in a loss of biological activity and possible changes in immunogenicity. Rational strategies can be devised for protein stabilization depending on the mechanism involved. For example, if the aggregation mechanism is discovered to be intermolecular S-S bond formation through disulfide interchange, stabilization can be achieved by modifying sulfhydryl residues, lyophilizing from acidic solutions, controlling moisture content, using appropriate additives, and developing specific polymer matrix compositions.

Sustained-released compositions also include preparations of crystals of the antibody suspended in suitable formulations capable of maintaining crystals in suspension. These preparations when injected subcutaneously or intraperitonealy can produce a sustained release effect. Other compositions also include liposomally entrapped antibodies. Liposomes containing such antibodies are prepared by methods known per se: U.S. Pat. No. DE 3,218,121; Epstein et al., Proc. Natl. Acad. Sci. USA, (1985) 82:3688-3692; Hwang et al., Proc. Natl. Acad. Sci. USA, (1980) 77:4030-4034; EP 52,322; EP 36,676; EP 88,046; EP 143,949; 142,641; Japanese patent application 83-118008; U.S. Pat. Nos. 4,485,045 and 4,544,545; and EP 102,324.

The dosage of the antibody formulation for a given patient will be determined by the attending physician taking into consideration various factors known to modify the action of drugs including severity and type of disease, body weight, sex, diet, time and route of administration, other medications and other relevant clinical factors. Therapeutically effective dosages can be determined by either *in vitro* or *in vivo* methods.

An effective amount of the antibodies, described herein, to be employed therapeutically will depend, for example, upon the therapeutic objectives, the route of administration, and the condition of the patient. Accordingly, it is preferred for the therapist to titer the dosage and modify the route of administration as required to obtain the optimal therapeutic effect. A typical daily dosage might range from about 0.001mg/kg to up to 100mg/kg or more, depending on the factors mentioned above. Typically, the clinician will administer the therapeutic antibody until a dosage is reached that achieves the desired effect. The progress of this therapy is easily monitored by conventional assays or as described herein.

It will be appreciated that administration of therapeutic entities in accordance with the compositions and methods herein will be administered with suitable carriers, excipients, and other agents that are incorporated into formulations to provide improved transfer, delivery, tolerance, and the like. These formulations include, for example, powders, pastes, ointments, jellies, waxes, oils, lipids, lipid (cationic or anionic) containing vesicles (such as Lipofectin™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. Any of the foregoing mixtures can be appropriate in treatments and therapies in accordance with the present disclosure, provided that the active ingredient in the formulation is not inactivated by the formulation and the formulation is physiologically compatible and tolerable with the route of administration. *See also* Baldrick P. "Pharmaceutical excipient development: the need for preclinical guidance." Regul. Toxicol. Pharmacol. 32(2):210-8 (2000), Wang W. "Lyophilization and development of solid protein pharmaceuticals." Int. J. Pharm. 203(1-2):1-60 (2000), Charman WN "Lipids, lipophilic drugs, and oral drug delivery-some emerging concepts." J Pharm Sci .89(8):967-78 (2000), Powell et al. "Compendium of excipients for parenteral formulations" PDA J Pharm Sci Technol. 52:238-311 (1998) and the citations therein for additional information related to formulations, excipients and carriers well known to pharmaceutical chemists.

It is expected that the antibodies described herein will have therapeutic effect in treatment of symptoms and conditions resulting from TIM-1 expression. The antibodies and methods herein relate to the treatment of symptoms resulting from TIM-1 expression including symptoms of cancer. The antibodies and methods described herein may be used to treat cancers, such as cancer of the lung, colon, stomach, kidney, prostrate, or ovary.

## Diagnostic Use

TIM-1 has been found to be expressed at low levels in normal kidney but its expression is increased dramatically in postischemic kidney. Ichimura et al., J. Biol. Chem. 273(7):4135-42 (1998). As immunohistochemical staining with anti-TIM-1 antibody shows positive staining of renal, kidney, prostate and ovarian carcinomas (see below), TIM-1 overexpression relative to normal tissues can serve as a diagnostic marker of such diseases.

Antibodies, including antibody fragments, can be used to qualitatively or quantitatively detect the expression of TIM-1 proteins. As noted above, the antibody preferably is equipped with a detectable, *e.g.*, fluorescent label, and binding can be monitored by light microscopy, flow cytometry, fluorimetry, or other techniques known in the art. These techniques are particularly suitable if the amplified gene encodes a cell surface protein, e.g., a growth factor. Such binding assays are performed as known in the art.

*In situ* detection of antibody binding to the TIM-1 protein can be performed, for example, by immunofluorescence or immunoelectron microscopy. For this purpose, a tissue specimen is removed from the patient, and a labeled antibody is applied to it, preferably by overlaying the antibody on a biological sample. This procedure also allows for determining the distribution of the marker gene product in the tissue examined. It will be apparent for those skilled in the art that a wide variety of histological methods are readily available for *in situ* detection.

## Epitope Mapping

The specific part of the protein immunogen recognized by an antibody may be determined by assaying the antibody reactivity to parts of the protein, for example an N terminal and C terminal half. The resulting reactive fragment can then be further dissected, assaying consecutively smaller parts of the immunogen with the antibody until the minimal reactive peptide is defined. Anti-TIM-1 mAb 2.70.2 was assayed for reactivity against overlapping peptides designed from the antigen sequence and was found to specifically recognize the amino acid sequence PLPRQNHE (SEQ ID NO: 96) corresponding to amino acids 189-202 of the TIM-1 immunogen (SEQ ID NO:54). Furthermore using an alanine scanning technique, it has been determined that the second proline and the asparagine residues appear to be important for mAb 2.70.2 binding.

Alternatively, the epitope that is bound by the anti-TIM-1 antibodies of the disclosure may be determined by subjecting the TIM-1 immunogen to SDS-PAGE either in the absence or presence of a reduction agent and analyzed by immunoblotting. Epitope mapping may also be performed using SELDI. SELDI ProteinChip® (LumiCyte) arrays used to define sites of protein-protein interaction. TIM-1 protein antigen or fragments thereof may be specifically captured by antibodies covalently immobilized onto the PROTEINCHIP array surface. The bound antigens may be detected by a laser-induced desorption process and analyzed directly to determine their mass.

The epitope recognized by anti-TTM-1 antibodies described herein may be determined by exposing the PROTEINCHIP Array to a combinatorial library of random peptide 12-mer displayed on Filamentous phage (New England Biolabs). Antibody-bound phage are eluted and then amplified and taken through additional binding and amplification cycles to enrich the pool in favor of binding sequences. After three or four rounds, individual binding clones are further tested for binding by phage ELISA assays performed on antibody-coated wells and characterized by specific DNA sequencing of positive clones.

## Examples

The following examples, including the experiments conducted and results achieved are provided for illustrative purposes only and are not to be construed as limiting upon the invention described herein.

Example 1

Preparation of monoclonal antibodies that bind TIM-1

The soluble extracellular domain of TIM-1 was used as the immunogen to stimulate an immune response in XenoMouse® animals. A DNA (CG57008-02), which encodes the amino acid sequence for the TIM-1 extracellular domain (minus the predicted N-terminal signal peptide) was subcloned to the baculovirus expression vector, pMelV5His (CuraGen Corp., New Haven, CT), expressed using the pBlueBac baculovirus expression system (Invitrogen Corp., Carlsbad, CA), and confirmed by Western blot analyses. The nucleotide sequence below encodes the polypeptide used to generate antibodies.

TCTGTAAAGGTTGGTGGAGAGGCAGGTCCATCTGTCACACTACCCTGCCACTAC
AGTGGAGCTGTCACATCAATGTGCTGGAATAGAGGCTCATGTTCTCTATTCACA
TGCCAAAATGGCATTGTCTGGACCAATGGAACCCACGTCACCTATCGGAAGGA
CACACGCTATAAGCTATTGGGGGACCTTTCAAGAAGGGATGTCTCTTTGACCAT
AGAAAATACAGCTGTGTCTGACAGTGGCGTATATTGTTGCCGTGTTGAGCACCG
TGGGTGGTTCAATGACATGAAAATCACCGTATCATTGGAGATTGTGCCACCCAA
GGTCACGACTACTCCAATTGTCACAACTGTTCCAACCGTCACGACTGTTCGAAC
GAGCACCACTGTTCCAACGACAACGACTGTTCCAACGACAACTGTTCCAACAAC
AATGAGCATTCCAACGACAACGACTGTTCCGACGACAATGACTGTTTCAACGAC

AACGAGCGTTCCAACGACAACGAGCATTCCAACAACAACAAGTGTTCCAGTGA
CAACAACGGTCTCTACCTTTGTTCCTCCAATGCCTTTGCCCAGGCAGAACCATG
AACCAGTAGCCACTTCACCATCTTCACCTCAGCCAGCAGAAACCCACCCTACGA
CACTGCAGGGAGCAATAAGGAGAGAACCCACCAGCTCACCATTGTACTCTTAC
ACAACAGATGGGAATGACACCGTGACAGAGTCTTCAGATGGCCTTTGGAATAA
CAATCAAACTCAACTGTTCCTAGAACATAGTCTACTG (SEQ ID NO:53)

The amino acid sequence encoded thereby is as follows:

SVKVGGEAGPSVTLPCHYSGAVTSMCWNRGSCSLFTCQNGIVWTNGTHVTYRKDT
RYKLLGDLSRRDVSLTIENTAVSDSGVYCCRVEHRGWFNDMKITVSLEIVPPKVTT
TPIVTTVPTVTTVRTSTTVPTTTTVPTTTVPTTMSIPTTTTVPTTMTVSTTTSVPTTTSI
PTTTSVPVTTTVSTFVPPMPLPRQNHEPVATSPSSPQPAETHPTTLQGAIRREPTSSPL
YSYTTDGNDTVTESSDGLWNNNQTQLFLEHSLL (SEQ ID NO:54)

To facilitate purification of recombinant TIM-1, the expression construct can incorporate coding sequences for the V5 binding domain V5 and a HIS tag. Fully human IgG2 and IgG4 monoclonal antibodies (mAb), directed against TIM-1 were generated from human antibody-producing XenoMouse® strains engineered to be deficient in mouse antibody production and to contain the majority of the human antibody gene repertoire on megabase-sized fragments from the human heavy and kappa light chain loci as previously described in Yang et al., Cancer Res. (1999). Two XenoMouse® strains, an hIgG2 (xmg-2) strain and an IgG4 (3C-1) strain, were immunized with the TIM-1 antigen (SEQ ID NO: 54). Both strains responded well to immunization (Tables 2 and 3).

Table 2

| Serum titer of XENOMOUSE® hIgG$_2$ strain immunized with TIM-1 antigen. | | |
|---|---|---|
| Group 1: 5 mice (hIgG$_2$ strain); mode of immunization = footpad | | |
| | Reactivity to TIM-1 Titers via HIgG | |
| **Mouse ID** | **Bleed After 4 inj.** | **Bleed After 6 inj.** |
| M716-1 | 600,000 | 600,000 |

(continued)

| Serum titer of XENOMOUSE® hIgG$_2$ strain immunized with TIM-1 antigen. | | |
|---|---|---|
| Group 1: 5 mice (hIgG$_2$ strain); mode of immunization = footpad | | |
| | Reactivity to TIM-1<br>Titers via HIgG | |
| **Mouse ID** | **Bleed After 4 inj.** | **Bleed After 6 inj.** |
| M716-2 | 600,000 | 500,000 |
| M716-3 | 200,000 | 400,000 |
| M716-4 | 300,000 | 200,000 |
| M716-5 | 400,000 | 400,000 |
| Negative Control | 75 | 110 |
| Positive Control | - | 600,000 |

Table 3

| Serum titer of XENOMOUSE® IgG$_4$ strain immunized with TIM-1 antigen | | |
|---|---|---|
| Group 2: 5 mice (IgG$_4$ strain); mode of immunization = footpad | | |
| | Reactivity to TIM-1<br>Titers via hIgG | |
| **Mouse ID** | **Bleed After 4 inj.** | **Bleed After 6 inj.** |
| M326-2 | 15,000 | 73,000 |
| M326-3 | 7,500 | 60,000 |
| M329-1 | 27,000 | 30,000 |
| M329-3 | 6,500 | 50,000 |
| M337-1 | 2,500 | 16,000 |
| Negative Control | <100 | 90 |
| Positive Control | - | 600,000 |

Hybridoma cell lines were generated from the immunized mice. Selected hybridomas designated 1.29, 1.37, 2.16, 2.17, 2.24, 2.45, 2.54 2.56, 2.59, 2.61, 2.70, and 2.76 (and subclones thereof) were further characterized. The antibodies produced by cell lines 1.29 and 1.37 possess fully human IgG2 heavy chains with human kappa light chains while those antibodies produced by cell lines 2.16, 2.17, 2.24, 2.45, 2.54 2.56, 2.59, 2.61, 2.70, and 2.76 possess fully human IgG4 heavy chains with human kappa light chains.

The amino acid sequences of the heavy chain variable domain regions of twelve anti-TIM-1 antibodies with their respective germline sequences are shown in Table 4 below. The corresponding light chain variable domain regions amino acid sequence is shown in Table 5 below. "X" indicates any amino acid, preferably the germline sequence in the corresponding amino acid position. The CDRs (CDR1, CDR2, and CDR3) and FRs (FR1, FR2, and FR3) in the immunoglobulins are shown under the respective column headings.

Table 4. Heavy Chain Analysis

| mAb | SEQ ID NO: | D | FR1 | CDR1 | FR2 | CDR2 | FR3 | CDR3 | J |
|---|---|---|---|---|---|---|---|---|---|
| | 55 | Germline | QVQLVESGGGVVQPGRSLRLSCAAS | GFTFSSYGMH | WVRQAPGKGLEWVA | VIWYDGSNKYYADSVKG | RFTISRDNSKNTLYLQMNSLRAEDTAVYYCAR | XXDY | WGQGTLVTVSSA |
| 2.54 | 26 | VH3-33/--/JH4b | QVQLEQSGGGVVQPGRSLRLSCAAS | GFTFTNYGLH | WVRQAPGKGLDWVA | VIWYDGSHKFYADSVKG | RFTISRDNSKNTLFLQMNSLRAEDTAVYYCTR | DLDY | WGQGTLVTVSSA |
| | 56 | Germline | QVQLVESGGGVVQPGRSLRLSCAAS | GFTFSSYGMH | WVRQAPGKGLEWVA | VIWYDGSNKYYADSVKG | RFTISRDNSKNTLYLQMNSLRAEDTAVYYCAX | XXYDSSXXXYGMDV | WGQGTTVTVSSA |
| 2.76 | 46 | VH3-33/D3-22/JH6b | XXXXEQSGGGVVQPGRSLRLSCAAS | GFTFSSYGMY | WVRQAPGKGLEWVA | VIWYDGSNKYYADSVKG | RFTISRDNSKNTLYLQMNSLRAEDTAVYYCAR | DFYDSSRYHYGMDV | WGQGTTVTVSSA |
| | 57 | Germline | QVQLQESGPGLVKPSQTLSLTCTVS | GGSISSGGYYWS | WIRQHPGKGLEWIG | YIYYSGSTYYNPSLKS | RVTISVDTSKNQFSLKLSSVTAADTAVYYCAR | XXXXSSSWYXXFDY | WGQGTLVTVSSA |
| 2.59 | 34 | VH4-31/D6-13/JH4b | XXXXXQSGPRLVKPSQTLSLTCTVS | GGSISSDGYYWS | WIRQHPGKGLEWIG | YIYYSGSTFYNPSLKS | RVAISVDTSKNQFSLKLSSVTAADTAVYYCAR | ESPHSSNWYSGFDC | WGQGTLVTVSSA |
| | 58 | Germline | QVQLVESGGGVVQPGRSLRLSCAAS | GFTFSSYGMH | WVRQAPGKGLEWVA | VIWYDGSNKYYADSVKG | RFTISRDNSKNTLYLQMNSLRAEDTAVYYCAR | DYYDSSXXXXXFDY | WGQGTLVTVSSA |
| 2.70 | 42 | VH3-33/D3-22/JH4b | QVQLVESGGGVVQPGRSLRLSCAAS | GFIFSRYGMH | WVRQAPGKGLKWVA | VIWYDGSNKLYADSVKG | RFTISRDNSKNTLYLQMNSLRAEDTAVYYCAR | DYYDNSRHHWGFDY | WGQGTLVTVSSA |
| 2.24 | 18 | | QVQLEQSGGGVVQPGRSLRLSCAAS | GFTFSRYGMH | WVRQAPGKGLKWVA | VIWYDGSNKLYADSVKG | RFTISRDNSKNTLYLQMNSLRAEDTAVYYCAR | DYYDNSRHHWGFDY | WGQGTLVTVSSA |
| 2.61 | 38 | | QVQLVEAGGGVVQPGRSLRLSCAAS | GFTFRSYGMH | WVRQAPGKGLKWVA | VIWYDGSNKYYTDSVKG | RFTISRDNSKNTLYLQMNSLRAEDTAVYYCVR | DYYDNSRHHWGFDY | WGQGTLVTVSSA |
| 2.56 | 30 | | QVQLVESGGGVVQPGRSLRLSCAAS | GFTFSSYGMH | WVRQAPGKGLEWVA | VIWYDGSHKYYADSVKG | RFTISRDNSKNTLYLQMNSLRAEDTAVYYSAR | DYYDTSRHHWGFDC | WGQGTLVTVSSA |
| | 59 | Germline | EVQLVESGGGLVKPGGSLRLSCAAS | GFTFSNAWMS | WVRQAPGKGLEWVG | RIKSKTDGGTTDYAAPVKG | RFTISRDDSKNTLYLQMNSLKTEDTAVYYCTX | XDXXXDY | WGQGTLVTVSSA |
| 2.16 | 10 | VH3-15/D3-16/JH4b | XXXXEQSGGGVVKPGGSLRLSCAAS | GFTFSNAWMT | WVRQAPGKGLEWVG | RIKRRTDGGTTDYAAPVKG | RFTISRDDSKNTLYLQMNNLKNEDTAVYYCTS | VDNDVDY | WGQGTLVTVSSA |
| | 60 | Germline | QVQLQESGPGLVKPSETLSLTCTVS | GGSVSSGGYYWS | WIRQPPGKGLEWIG | YIYYSGSTNYNPSLKS | RVTISVDTSKNQFSLKLSSVTAADTAVYYCAR | XXXWXXXFDY | WGQGTLVTVSSA |
| 1.29 | 2 | VH4-61/D1-7/JH4b | QVQLQESGPGLVKPSETLSLTCTVS | GGSVSSGGYYWS | WIRQPPGKGLEWIG | FIYYTGSTNYNPSLKS | RVSISVDTSKNQFSLKLSSVTAADAAVYYCAR | DYDWSFHFDY | WGQGTLVTVSSA |
| | 61 | Germline | EVQLVESGGGLVKPGGSLRLSCAAS | GFTFSNAWMS | WVRQAPGKGLEWVG | RIKSKTDGGTTDYAAPVKG | RFTISRDDSKNTLYLQMNSLKTEDTAVYYCTT | XXXSGDY | WGQGTLVTVSSA |
| 2.45 | 22 | VH3-15/D6-19/JH4b | XXXXXQSGGGLVKPGGSLRLSCAAS | GFTFSNAWMT | WVRQAPGKGLEWVG | RIKRKTDGGTTDYAAPVKG | RFTISRDDSENTLYLQMNSLETEDTAVYYCTT | VDNSGDY | WGQGTLVTVSSA |
| | 62 | Germline | EVQLVESGGGLVQPGGSLRLSCAAS | GFTFSSYWMS | WVRQAPGKGLEWVA | NIKQDGSEKYYVDSVKG | RFTISRDNAKNSLYLQMNSLRAEDTAVYYCAR | XDY | WGQGTLVTVSSA |
| 1.37 | 6 | VH3-7/--/JH4b | EVQLVESGGGLVQPGGSLRLSCAAS | GFTFTNYWMS | WVRQAPGKGLEWVA | NIQQDGSEKYYVDSVRG | RFTISRDNAKNSLYLQMNSLRAEDSAVYYCAR | WDY | WGQGTLVTVSSA |
| | 63 | Germline | EVQLVESGGGLVQPGGSLRLSCAAS | GFTFSSYSMN | WVRQAPGKGLEWVS | YISSSSSTIYYADSVKG | RFTISRDNAKNSLYLQMNSLRDEDTAVYYCAX | XFDY | WGQGTLVTVSSA |
| 2.17 | 14 | VH3-48/--/JH4b | QVQLEQSGGGLVQPGGSLRLSCAAS | GFTFSTYSMN | WVRQAPGKGLEWVS | YIRSSTSTIYYAESLKG | RFTISSDNAKNSLYLQMNSLRDEDTAVYYCAR | DFDY | WGQGTLVTVSSA |

Table 5. Light Chain Analysis

| mAb | SEQ ID NO: | J | FR1 | CDR1 | FR2 | CDR2 | PR3 | CDR3 | J |
|---|---|---|---|---|---|---|---|---|---|
| | 64 | Germline | EIVLTQSPGTLSLS PGERATLSC | RASQSVSSSYLA | WYQQKPGQAPR LLIY | GASSRAT | GIPDRFSGSGSGTDFTLTISRL EPEDFAVYYC | QQYGSSXXLT | FGGGTKVEIKR |
| 2.54 | 28 | A27/JK4 | ETQLTQSPGTLSLS PGERVTLSC | RASQSVSNNYLA | WYQQKPGQAPR LLIY | GASSRAT | GIPDRFSGSGSGTDFTLTISRL EPEDCAECYC | QQYGSSLPLT | FGGGTKVEIKR |
| | 65 | Germline | DIVMTQSPLSLPVT PGEPASISC | RSSQSLLHSNGYN YLD | WYLQKPGQSPQ LLIY | LGSNRAS | GVPDRFSGSGSGTDFTLKISRV EAEDVGVYYC | MQALQTXXT | FGGGTKVEIKR |
| 2.16 | 12 | A3/JK4 | XXXLTQSPLSLPVT PGEPASISC | RSSQSLLHSNGYN YLD | WYLQKPGQSPQ LLIY | LGSNRAS | GVPDRFSGSGSGTDFTLKISRV EAEDIGLYYC | MQALQTPLT | FGGGTKVDIKR |
| 2.45 | 24 | | XXXXTQSPLSLPVT PGEPASISC | RSSQSLLHSNGYN YLD | WYLQKPGQSPQ LLIY | LGSNRAS | GVPDRFSGSGSGTDFTLKISRV EAEDVGVYYC | MQALQTPLT | FGGGTKVEIKR |
| | 66 | Germline | DIQMTQSPSSLSAS VGDRVTITC | RASQGIRNDLG | WYQQKPGKAPK RLIY | AASSLQS | GVPSRFSGSGSGTEFTLTISSL QPEDFATYYC | LQHNSYPLT | FGGGTKVEIKR |
| 1.29 | 4 | A30/JK4 | DIQMTQSPSSLSAS IGDRVTITC | RASQGIRNDLG | WYQQKPGKAPK RLIY | AASSLQS | GVPSRFSGSGSGTEFTLTISSL QPEDFATYYC | LQHNSYPLT | FGGGTKVEIKR |
| | 67 | Germline | DIVMTQTPLSSPVT LGOPASISC | RSSQSLVHSDGNT YLS | WLQQRPGQPPR LLIY | KISNRFS | GVPDRFSGSGAGTDFTLKISRV EAEDVGVYYC | MQATQFPXIT | FGQGTRLEIKR |
| 2.17 | 16 | A23/JK5 | EIQLTQSPLSSPVT LGQPASISC | RSSQSLVHSDGDT YLN | WLQQRPGQPPR LLIY | KISTRFS | GVPDRFSGSGAGTDFTLKISRV ETDDVGIYYC | MQTTQIPQIT | FGQGTRLEIKR |
| | 68 | Germline | DIQMTQSPSSLSAS VGDRVTITC | RASQSISSYLN | WYQQKPGKAPK LLIY | AASSLQS | GVPSRFSGSGSGTDFTLTISSL QPEDFATYYC | QQSYSTPPT | FGQGTKVEIKR |
| 2 24 | 20 | O12/JK1 | DIQLTQSPSSLSAS VGDRVTITC | RASQSIYSYLN | WYQQKPGKAPK LLIY | AASSLQS | GVPSRFSGSGSGTDFTLTISSL QPEDFATYYC | QQSYSTPPT | FGQGTKVEIKR |
| | 69 | Germline | DIVMTQTPLSSPVT LGQPASISC | RSSQSLVHSDGNT YLS | WLQQRPGQPPR LLIY | KISNRFS | GVPDRFSGSGAGTDFTLKISRV EAEDVGVYYC | MQATQFPQT | FGQGTKVEIKR |
| 1.37 | 8 | A23/JK1 | DIVMTQTPLSSTVI LGQPASISC | RSSQSLVHSDGNT YLN | WLQQRPGQPPR LLIY | MISNRFS | GVPDRFSGSGAGTDFTLKISRV EAEDVGVYYC | MQATESPQT | FGQGTKVEIKR |
| | 70 | Germline | DIVMTQTPLSLPVT PGEPASISC | RSSQSLLDSDDGN TYLD | WYLQKPGQSPQ LLIY | TLSYRAS | GVPDRFSGSGSGTDFTLKISRV EAEDVGVYYC | MQRIEFPIT | FGQGTRLEIKR |

(continued)

| mAb | SEQ ID NO: | J | FR1 | CDR1 | FR2 | CDR2 | PR3 | CDR3 | J |
|---|---|---|---|---|---|---|---|---|---|
| 2.70 | 44 | | DIVMTQTPLSLPVT PGEPASISC | RSSRSLLDSDDGN TYLD | WYLQKPGQSPQ LLIY | TLSYRAS | GVPDRFSGSGSGTDFTLKISRV EAEDVGVYYC | MQRVEFPIT | FGQGTRLEIKR |
| $2.5_5$ | 32 | O1/JK5 | EIVMTQTPLSLPVT PGEPASISC | RSSQSLLDSEDGN TYLD | WYLQKPGQSPQ LLIY | TLSHRAS | GVPDRFSGSGSGTDFTLKISRV EAEDVGVYCC | MQRVEFPIT | FGQGTRLEIKR |
| 2.76 | 48 | | XXXXTQCPLSLPVT PGEPASISC | RSSQSLLDSDDGN TYLD | WYLQKPGQSPQ LLIY | TVSYRAS | GVPDRFSGSGSGTDFTLKISRV EAEDVGVYYC | MQRIEFPIT | FGQGTRLEIKR |
| | 71 | Germline | EIVLTQSPDFQSVT PKEKVTITC | RASQSIGSSLH | WYQQKPDQSPK LLIK | YASQSFS | GVPSRFSGSGSGTDFTLTINSL EAEDAATYYC | HQSSSLPFT | FGPGTKVDIKR |
| 2.59 | 36 | A26/JK3 | XXXXTQSPDFQSVT PKEKVTITC | RASQSIGSRLH | WYQQKPDQSPK LLIK | YASQSFS | GVPSRFSGSGSGTDFTLTINSL EAEDAATYYC | HOSSNLPFT | FGPGTKVDIKR |
| | 72 | Germline | DIQMTQSPSSLSAS VGDRVTITC | RASQGIRNDLG | WYQQKPGKAPK RLIY | AASSLQS | GVPSRFSGSGSGTEFTLTISSL QPEDFATYYC | LQHNSYPXX | FGQGTKLEIKR |
| 2.61 | 40 | A30/JK2 | DIQMTQSPSSRCAS VGDRVTITC | RASQGIRNDLA | WYQQKPGKAPK RLIY | AASSLQS | GVPSRFSGSRSGTEFTLTISSL QPEDFAAYYC | LQHNSYPPS | FGQGTKLEIKR |

Human antibody heavy chain VH-3-33 was frequently selected in productive rearrangement for producing antibody successfully binding to TIM-1. Any variants of a human antibody VH3-33 germline in a productive rearrangement making antibody to TIM-1 is described herein. Other heavy chain V regions selected in TIM-1 binding antibodies included: VH4-31, VH3-15, VH4-61, VH3-7 and VH3-48. The light chain V regions selected included: A27, A3, A30, A23, 012, O1, and A26. It is understood that the λκ XenoMouse® may be used to generate anti-TIM-1 antibodies utilizing lambda V regions.

The heavy chain variable domain germ line usage of the twelve anti-TIM-1 antibodies is shown in Table 6. The light chain variable domain germ line usage is shown in Table 7 (below).

Table 6. Germ Line Usage of the Heavy Chain Variable Domain Regions

| mAb | V Heavy | V Sequence | #N's | N | D1 | D1 Sequence | #N's | N | D2 | D2 Sequence | #N's | N | JH | J Sequence | Constant Region | CDR1 | CDR2 | CDR3 |
|---|---|---|---|---|---|---|---|---|---|---|---|---|---|---|---|---|---|---|
| | | | | | | | | | | | | | | | | | | |
| 2.16 | VH3-15 (1-285) | TGTACC | 5 | TCA GT | D3-16 (291-296) | CGATAA | -N.A- | -N.A- | -N.A- | -N.A- | 7 | TGACGTG | JH4b (304-343) | GACTAC | G4 (344-529) | 64-93 | 136-192 | 289-309 |
| 2.70 | VH3-33 (1-290) | GAGAGA | 0 | | D3-22 (291-306) | TTACTATGAT AATAGT (SEQ ID NO: 73) | -N.A- | -N.A- | -N.A- | -N.A- | 15 | AGACATCA CTGGGGG (SEQ ID NO: 74) | JH4b (322-364) | TTTGAC | G4 (365-502) | 70-99 | 142-192 | 289-330 |
| 2.59 | VH4-31 (2-284) | GAGAGA | 8 | ATC CCC TC | D6-13 (293-309) | ATAGCAGCAA CTGGTAC (SEQ ID NO: 75) | -N.A- | -N.A- | -N.A- | -N.A- | 5 | TCGGG | JH4b (315-358) | CTTTGA | G4 (359-545) | 61-96 | 139-186 | 283-324 |
| 2.24 | VH3-33 (1-296) | GAGAGA | 0 | | D3-22 (297-312) | TTACTATGAT AATAGT (SEQ ID NO: 76) | -N.A- | -N.A- | -N.A- | -N.A- | 15 | AGACATCA CTGGGGG (SEQ ID NO: 77) | JH4b (328-370) | TTTGAC | G4 (371-568) | 76-105 | 148-198 | 295-336 |
| 1.29 | VH4-61 (1-293) | GAGAGA | 5 | TTA TG | D1-7 (299-304) | ACTGGA | -N.A- | -N.A- | -N.A- | -N.A- | 6 | GCTTCC | JH4b (311-355) | ACTTTG | G2 (356-491) | 70-105 | 148-195 | 292-321 |
| 2.61 | VH3-33 (1-296) | GAGAGA | 0 | | D3-22 (297-312) | TTACTATGAT AATAGT (SEQ ID NO: 78) | -N.A- | -N.A- | -N.A- | -N.A- | 15 | AGACATCA CTGGGGG (SEQ ID NO: 79) | JH4b (328-370) | TTTGAC | G4 (371-534) | 76-105 | 148-198 | 295-336 |
| 2.76 | VH3-33 (1-281) | TGCGAG | 6 | GGA TTT | D3-22 (288-300) | CTATGATAGT AGT (SEQ ID NO: 80) | -N.A- | -N.A- | -N.A- | -N.A- | 7 | CGTTACC | JH6b (308-358) | ACTACG | G4 (359-544) | 64-93 | 136-186 | 283-324 |
| 2.54 | VH3-33 (1-296) | GCGAGA | -N.A- | -N.A- | -N.A- | -N.A- | -N.A- | -N.A- | -N.A- | -N.A- | 2 | TC | JH4b (299-340) | TTGACT | G4 (341-537) | 76-105 | 148-198 | 295-306 |
| 1.37 | VH3-7 (7-300) | GCGAGA | -N.A- | -N.A- | -N.A- | -N.A- | -N.A- | -N.A- | -N.A- | -N.A- | 3 | TGG | JH4b (304-343) | GACTAC | G2 (344-469) | 82-111 | 154-204 | 301-309 |
| 2.17 | VH3-48 (2-291) | TGTGCG | -N.A- | -N.A- | -N.A- | -N.A- | -N.A- | -N.A- | -N.A- | -N.A- | 5 | CGGGA | JH4b (297-340) | CTTTGA | G4 (341-538) | 76-105 | 148-198 | 295-306 |
| 2.45 | VH3-15 (2-286) | CCACAG | 7 | TCG ATA A | D6-19 (294-299) | CAGTGG | -N.A- | -N.A- | -N.A- | -N.A- | 0 | | JH4b (300-340) | TGACTA | G4 (341-526) | 61-90 | 133-189 | 286-306 |
| 2.56 | VH3-33 (1-290) | GAGAGA | 0 | | D3-22 (291-301) | TTACTATGAT A (SEQ ID NO: 81) | -N.A- | -N.A- | -N.A- | -N.A- | 20 | CGAGTCGG CATCACTG GGGG (SEQ ID NO: 82) | JH4b (322-364) | TTTGAC | G4 (365-527) | 70-99 | 142-192 | 289-330 |

Table 7. Germ Line Usage of the Light Chain Variable Domain Regions

| mAb | VL | V Sequence | #N's | N | JL | J Sequence | Constant Region | CDR1 | CDR2 | CDR3 |
|---|---|---|---|---|---|---|---|---|---|---|
| 2.70 | 01 (46-348) | TTCCT | 0 | | JKS (349-385) | ATCACC | IGKC (386-522) | 115-165 | 211-231 | 328-354 |
| 2.59 | A26 (1-272) | TTTACC | 0 | | JK3 (273-310) | ATTCAC | IGKC (311-450) | 58-90 | 136-156 | 253-279 |
| 2.24 | 012 (1-287) | CCCTCC | 0 | | JK1 (288-322) | GACGTT | IGKC (323-472) | 70-102 | 148-168 | 265-291 |
| 1.29 | A30 (46-331) | ACCCTC | 0 | | JK4 (332-367) | TCACTT | IGKC (368-504) | 115-147 | 193-213 | 310-336 |
| 2.56 | 01 (46-348) | TTTCCT | 0 | | JK5 (349-385) | ATCACC | IGKC (386-521) | 115-165 | 211-231 | J28-354 |
| 2.61 | A30 (1-287) | CCCTCC | 3 | CAG | JK2 (291-322) | TTTTGG | IGKC (323-470) | 70-102 | 148-168 | 265-291 |
| 2.76 | 01 (1-290) | GTTTCC | 0 | | JK5 (291-328) | GATCAC | IGKC (329-419) | 58-108 | 154-174 | 271-297 |
| 1.37 | A23 (43-344) | TCCTCA | 0 | | JK1 (345-379) | GACGTT | IGKC (380-454) | 112-159 | 205-225 | 322-348 |
| 2.17 | A23 (1-302) | TCCTCA | 1 | A | JK5 (304-340) | ATCACC | IGKC (341-490) | 70-117 | 163-183 | 280-309 |
| 2.54 | A27 (1-286) | GCTCAC | 4 | TCCC | JK4 (291-328) | GCTCAC | IGKC (329-480) | 70-105 | 151-171 | 268-297 |
| 2.16 | A3 (2-290) | AACTCC | 2 | GC | JK4 (293-328) | TCACTT | IGKC (329-447) | 61-108 | 154-174 | 271-297 |
| 2.45 | A3 (1-287) | AACTCC | 2 | GC | JK4 (290-325) | TCACTT | IGKC (326-465) | 58-105 | 151-171 | 268-294 |

The sequences encoding monoclonal antibodies 1.29, 1.37, 2.16, 2.17, 2.24, 2.45, 2.54 2.56, 2.59, 2.61, 2.70, and 2.76, respectively, including the heavy chain nucleotide sequence (A), heavy chain amino acid sequence (B) and the light chain nucleotide sequence (C) with the encoded amino acid sequence (D) are provided in the sequence listing as summarized in Table 1 above. A particular monoclonal antibody, 2.70, was further subcloned and is designated 2.70.2, see Table 1.

Example 2

Antibody reactivity with membrane bound TIM-1 protein by FACS.

Fluorescent Activated Cell Sorter (FACS) analysis was performed to demonstrate the specificity of the anti-TIM-1 antibodies for cell membrane-bound TIM-1 antigen and to identify preferred antibodies for use as a therapeutic or diagnostic agent. The analysis was performed on two renal cancer cell lines, ACHN (ATCC#:CRL-1611) and CAKI-2 (ATCC#:HTB-47). A breast cancer cell line that does not express the TIM-1 antigen, BT549, was used as a control. Table 8 shows that both antibodies 2.59.2 and 2.70.2 specifically bound to TIM-1 antigen expressed on ACHN and CAKI-2 cells, but not antigen negative BT549 cells. Based on the Geo Mean Ratios normalized to the irrelevant antibody isotype control (pK16), ACHN cells had a higher cell surface expression of TIM-1 protein than CAKI-2 cells.

Table 8

| | | Geo Mean Ratio (relative to negative control) | | |
|---|---|---|---|---|
| **Antibody** | **BIN** | **ACHN** | **CAKI-2** | **BT549** |
| 2.59.2 | 1 | 15.2 | 7.7 | 1.4 |
| 2.70.2 | 6 | 19.4 | 8.8 | 1.8 |
| 1.29 | 1 | 17.9 | | 1.2 |
| 2.16.1 | 2 | 7.9 | | 1.5 |
| 2.56.2 | 5 | 12.2 | | 1.5 |
| 2.45.1 | 8 | 4.3 | | 1.1 |

Example 3

Specificity of the anti-TIM-1 monoclonal antibodies

The anti-TIM-1 antibodies bound specifically to TIM-1 protein but not an irrelevant protein in an ELISA assay. TIM-1 antigen (with a V5-HIS tag) specific binding results for four of the anti-TIM-1 monoclonal antibodies (1.29, 2.56.2, 2.59.2, and 2.45.1) as well as an isotype matched control mAb PK16.3 are shown in Figure 1. The X axis depicts the antibodies used in the order listed above and the Y axis is the optical density. The respective binding of these antibodies to the irrelevant protein (also with a V5-HIS tag) is shown in Figure 2.

ELISA Protocol.

A 96-well high protein binding ELISA plate (Corning Costar cat. no. 3590) was coated with 50 μL of the TIM-1 antigen at a concentration of 5 μg/mL diluted in coating buffer (0.1M Carbonate, pH9.5), and incubated overnight at 4 oC. The wells were then washed five times with 200-300 μL of 0.5% Tween-20 in PBS. Next, plates were blocked with 200μL of assay diluent (Pharmingen, San Diego, CA, cat. no. 26411E) for at least 1 hour at room temperature. Anti-TIM-1 monoclonal antibodies were then diluted in assay diluent with the final concentrations of 7, 15, 31.3, 62.5, 125, 250, 500 and 1000 ng/mL. An anti-V5-HRP antibody was used at 1:1000 to detect the V5 containing peptide as the positive control for the ELISA. Plates were then washed again as described above. Next 50 μL of each antibody dilution was added to the proper wells, then incubated for at least 2 hours at room temp. Plates were washed again as described above, then 50 μL of secondary antibody (goat anti-human-HRP) was added at 1:1000 and allowed to incubate for 1 hour at room temp. Plates were washed again as described above then developed with 100 μL of TMB substrate solution/well (1:1 ratio of solution A+B) (Pharmingen, San Diego, CA, cat. no. 2642KK). Finally, the reaction was stopped with 50 μL sulfuric acid and the plates read at 450nm with a correction of 550nm.

Example 4

Antibody Sequences

In order to analyze structures of antibodies, as described herein, genes encoding the heavy and light chain fragments out of the particular hybridoma were cloned. Gene cloning and sequencing was accomplished as follows. Poly(A)+ mRNA was isolated from approximately 2 X 105 hybridoma cells derived from immunized XenoMouse® mice using a Fast-Track kit (Invitrogen). The generation of random primed cDNA was followed by PCR. Human VH or human Vκ family specific variable domain primers (Marks et. al., 1991) or a universal human VH primer, MG-30 (CAGGTGCAGCTGGAGCAGTCIGG) (SEQ ID NO:83) were used in conjunction with primers specific for the human:

Cγ2 constant region (MG-40d; 5'-GCT GAG GGA GTA GAG TCC TGA GGA-3' (SEQ ID NO:84));
Cγ1 constant region (HG1; 5' CAC ACC GCG GTC ACA TGG C (SEQ ID NO:85)); or
Cγ3 constant region (HG3; 5' CTA CTC TAG GGC ACC TGT CC (SEQ ID NO:86))

or the human Cκ constant domain (hκP2; as previously described in Green *et* al., 1994). Sequences of human MAbs-derived heavy and kappa chain transcripts from hybridomas were obtained by direct sequencing of PCR products generated from poly($A^+$) RNA using the primers described above. PCR products were also cloned into pCRII using a TA cloning kit (Invitrogen) and both strands were sequenced using Prism dye-terminator sequencing kits and an ABI 377 sequencing machine. All sequences were analyzed by alignments to the "V BASE sequence directory" (Tomlinson *et al.*, MRC Centre for Protein Engineering, Cambridge, UK) using MacVector and Geneworks software programs.

In each of Tables 4-7 above, CDR domains were determined in accordance with the Kabat numbering system. See Kabat, Sequences of Proteins of Immunological Interest (National Institutes of Health, Bethesda, Md. (1987 and 1991)).

Example 5

Epitope binning and BiaCore® affinity determination

Epitope binning

Certain antibodies, described herein were "binned" in accordance with the protocol described in U.S. Patent Application Publication No. 20030157730, published on August 21, 2003, entitled "Antibody Categorization Based on Binding Characteristics."

MxhIgG conjugated beads were prepared for coupling to primary antibody. The volume of supernatant needed was calculated using the following formula: (n+10) x 50μL (where n = total number of samples on plate). Where the concentration was known, 0.5μg/mL was used. Bead stock was gently vortexed, then diluted in supernatant to a concentration of 2500 of each bead per well or 0.5X105 /mL and incubated on a shaker in the dark at room temperature overnight, or 2 hours if at a known concentration of 0.5μg/mL. Following aspiration, 50μL of each bead was added to each well of a filter plate, then washed once by adding 100μL/well wash buffer and aspirating. Antigen and controls were added to the filter plate 50μL/well then covered and allowed to incubate in the dark for 1 hour on shaker. Following a wash step, a secondary unknown antibody was added at 50μL/well using the same dilution (or concentration if known) as used for the primary antibody. The plates were then incubated in the dark for 2 hours at room temperature on shaker followed by a wash step. Next, 50μL/well biotinylated mxhIgG diluted 1:500 was added and allowed to incubate in the dark for 1 hour on shaker at room temperature. Following a wash step, 50μL/well Streptavidin-PE was added at 1:1000 and allowed to incubate in the dark for 15 minutes on shaker at room temperature. Following a wash step, each well was resuspended in 80μL blocking buffer and read using a Luminex system.

Table 9 shows that the monoclonal antibodies generated belong to eight distinct bins. Antibodies bound to at least three distinct epitopes on the TIM-1 antigen.

Determination of anti-TIM-1 mAb affinity using BiaCore® analysis

BiaCore® analysis was used to determine binding affinity of anti-TIM-1 antibody to TIM-1 antigen. The analysis was performed at 25°C using a BiaCore® 2000 biosensor equipped with a research-grade CM5 sensor chip. A high-density goat a human antibody surface over a CM5 BiaCore® chip was prepared using routine amine coupling. Antibody supernatants were diluted to ~ 5 μg/mL in HBS-P running buffer containing 100 μg/mL BSA and 10 mg/mL carboxymethyldextran. The antibodies were then captured individually on a separate surface using a 2 minute contact time, and a 5 minute wash for stabilization of antibody baseline.

TIM-1 antigen was injected at 292 nM over each surface for 75 seconds, followed by a 3-minute dissociation. Double-referenced binding data were obtained by subtracting the signal from a control flow cell and subtracting the baseline drift of a buffer inject just prior to the TIM-1 injection. TIM-1 binding data for each mAb were normalized for the amount of mAb captured on each surface. The normalized, drift-corrected responses were also measured. The kinetic analysis results of anti-TIM-1 mAB binding at 25°C are listed in Table 9 below.

Table 9

| Competition Bins and KDs for TIM-1-specific mAbs | | |
|---|---|---|
| **Bin** | **Antibody** | **Affinity nM by BIAcore** |
| 1 | 2.59 | 0.38 |
| | 1.29 | 3.64 |
| 2 | 2.16 | 0.79 |
| 3 | 2.17 | 2.42 |
| 4 | 1.37 | 2.78 |
| | 2.76 | 0.57 |
| | 2.61 | 1.0 |
| 5 | 2.24 | 2.42 |
| | 2.56 | 1.1 |
| 6 | 2.70 | 2.71 |
| 7 | 2.54 | 3.35 |
| 8 | 2.45 | 1.15 |

Example 6

Epitope Mapping

Anti-TIM-1 mAb 2.70.2 was assayed for reactivity against overlapping peptides designed from the TIM-1 antigen sequence. Assay plates were coated with the TIM-1 fragment peptides, using irrelevant peptide or no peptide as controls. Anti-TIM-1 mAb 2.70.2 was added to the plates, incubated, washed and then bound antibody was detected using anti-human Ig HRP conjugate. Human antibody not specific to TIM-1, an isotype control antibody or no antibody served as controls. Results showed that mAb 2.70.2 specifically reacted with a peptide having the amino acid sequence PMPLPRQNHEPVAT (SEQ ID NO:87), corresponding to amino acids 189-202 of the TIM-1 immunogen (SEQ ID NO:54).

Specificity of mAb 2.70.2 was further defined by assaying against the following peptides:

A) PMPLPRQNHEPVAT (SEQ ID NO:87)
B) PMPLPRQNHEPV (SEQ ID NO:88)
C) PMPLPRQNHE (SEQ ID NO:89)
D) PMPLPRQN (SEQ ID NO:90)
E) PMPLPR (SEQ ID NO:91)
F) PLPRQNHEPVAT (SEQ ID NO:92)
G) PRQNHEPVAT (SEQ ID NO:93)
H) QNHEPVAT (SEQ ID NO:94)
I) HEPVAT (SEQ ID NO:95)

Results showed mAb 2.70.2 specifically bound to peptides A, B, C, and F, narrowing the antibody epitope to PLPRNHE (SEQ ID NO:96)

As shown in Table 10, synthetic peptides were made in which each amino acid residue of the epitope was replace with an alanine and were assayed for reactivity with mAb 2.70.2. In this experiment, the third proline and the asparagines residues were determined to be critical for mAb 2.70.2 binding. Furthermore, assays of peptides with additional N or C terminal residues removed showed mAb 2.70.2 binding was retained by the minimal epitope LPRQNH (SEQ ID NO:97)

Table 10

| | | | | | | | | | | SEQ ID NO: | mAb 2.70.2 Reactivity |
|---|---|---|---|---|---|---|---|---|---|---|---|
| P | M | P | L | P | R | Q | N | H | E | 89 | + |
| P | M | P | A | P | R | Q | N | H | E | 98 | + |
| P | M | P | L | A | R | Q | N | H | E | 99 | - |
| P | M | P | L | P | A | Q | N | H | E | 100 | + |
| P | M | P | L | P | R | A | N | H | E | 101 | + |
| P | M | P | L | P | R | Q | A | H | E | 102 | - |
| P | M | P | L | P | R | Q | N | A | E | 103 | + |
| | | P | L | P | R | Q | N | H | E | 104 | + |
| | | | L | P | R | Q | N | H | E | 105 | + |
| | | P | L | P | R | Q | N | H | E | 106 | + |
| | | | L | P | R | Q | N | H | E | 107 | + |

Example 7

Immunohistochemical (IHC) analysis of TIM-1 expression in normal and tumor tissues

Immunohistochemical (IHC) analysis of TIM-1 expression in normal and tumor tissue specimens was performed with techniques known in the art. Biotinylated fully human anti-TIM-1 antibodies 2.59.2, 2.16.1 and 2.45.1 were analyzed. Streptavidin-HRP was used for detection.

Briefly, tissues were deparaffinized using conventional techniques, and then processed using a heat-induced epitope retrieval process to reveal antigenic epitopes within the tissue sample. Sections were incubated with 10% normal goat serum for 10 minutes. Normal goat serum solution was drained and wiped to remove excess solution. Sections were incubated with the biotinylated anti-TIM-1 mAb at 5 μg/mL for 30 minutes at 25°C, and washed thoroughly with PBS. After incubation with streptavidin-HRP conjugate for 10 minutes, a solution of diaminobenzidine (DAB) was applied onto the sections to visualize the immunoreactivity. For the isotype control, sections were incubated with a biotinylated isotype matched negative control mAb at 5 μg/mL for 30 minutes at 25°C instead of biotinylated anti-TIM-1 mAb. The results of the IHC studies are summarized in Tables 11 and 12.

The specimens were graded on a scale of 0-3, with a score of 1+ indicating that the staining is above that observed in control tissues stained with an isotype control irrelevant antibody. The corresponding histological specimens from one renal tumor and the pancreatic tumor are shown in Figure 3 (A and B). In addition to these the renal and pancreatic tumors, specimens from head and neck cancer, ovarian cancer, gastric cancer, melanoma, lymphoma, prostate cancer, liver cancer, breast cancer, lung cancer, bladder cancer, colon cancer, esophageal cancer, and brain cancer, as well the corresponding normal tissues were stained with anti-TIM-1 mAb 2.59.2. Overall, renal cancer tissue samples and pancreatic cancer tissue samples highly positive when stained with anti-TIM-1 mAb 2.59.2. No staining in normal tissues was seen. These results indicate that TIM-1 is a marker of cancer in these tissues and that anti-TIM-1 mAb can be used to differentiate cancers from normal tissues and to target TIM-1 expressing cells *in vivo.*

Table 11

| Immunohistology Renal tumors expression of TIM-1 protein detected by anti-TIM-1 mAb 2.59.2 | | | |
|---|---|---|---|
| **Specimen** | **Cell Type** | **Histology** | **Score** |
| 1 | Malignant cells | Not known | 0 |
| 1 | Other | Not cell associated | 2 |
| 2 | Malignant cells | Clear Cell | 2 |
| 3 | Malignant cells | Clear Cell | 0 |
| 4 | Malignant cells | Clear Cell | 3 |

(continued)

| Immunohistology Renal tumors expression of TIM-1 protein detected by anti-TIM-1 mAb 2.59.2 | | | |
|---|---|---|---|
| **Specimen** | **Cell Type** | **Histology** | **Score** |
| 5 | Malignant cells | Clear Cell | 2 (occasional) |
| 6 | Malignant cells | Not known | 2 |
| 7 | Malignant cells | Clear Cell | 2 |
| 8 | Malignant cells | Clear Cell | 0 |
| 9 | Malignant cells | Clear Cell | 2 (occasional) |
| 10 | Malignant cells | Clear Cell | 1-2 |
| 11 | Malignant cells | Not known | 3 (many) |
| 12 | Malignant cells | Clear Cell | 1-2 |
| 12 | Other | Not cell associated | 2 |
| 13 | Malignant cells | Clear Cell | 2 (occasional) |
| 14 | Malignant cells | Clear Cell | 1-2 |
| 15 | Malignant cells | Clear Cell | 3-4 |
| 16 | Malignant cells | Not known | 1-2 |
| 17 | Malignant cells | Not known | 4 (occasional) |
| 18 | Malignant cells | Not known | 1-2 |
| 19 | Malignant cells | Clear Cell | 0 |
| 20 | Malignant cells | Clear Cell | 3-4 |
| 21 | Malignant cells | Clear Cell | 2 (occasional) |
| 22 | Malignant cells | Clear Cell | 3 |
| 23 | Malignant cells | Clear Cell | 2 |
| 24 | Malignant cells | Not known | 3-4 occasional |
| 25 | Malignant cells | Not known | 2-3 |
| 26 | Malignant cells | Not known | 3 |
| 27 | Malignant cells | Clear Cell | 2 |
| 27 | Other | Not cell associated | 2 |
| 28 | Malignant cells | Not known | 2 |
| 29 | Malignant cells | Clear Cell | 2-3 |
| 30 | Malignant cells | Clear Cell | 2 |
| 31 | Malignant cells | Clear Cell | 2-3 |
| 32 | Malignant cells | Clear Cell | 0 |
| 33 | Malignant cells | Clear Cell | 0 |
| 34 | Malignant cells | Clear Cell | 2 |
| 34 | Other | Not cell associated | 2 |
| 35 | Malignant cells | Clear Cell | 2-3 |
| 36 | Malignant cells | Clear Cell | 3 |
| 37 | Malignant cells | Not known | 3 |
| 38 | Malignant cells | Clear Cell | 3 |

(continued)

| Immunohistology Renal tumors expression of TIM-1 protein detected by anti-TIM-1 mAb 2.59.2 | | | |
|---|---|---|---|
| **Specimen** | **Cell Type** | **Histology** | **Score** |
| 39 | Malignant cells | Not known | 2 |
| 40 | Malignant cells | Clear Cell | 2-3 |

Table 12

| Normal Human Tissue Immunohistology with anti-TIM-1 mAb 2.59.2 | | |
|---|---|---|
| **Tissue** | **Score** | |
| | **Specimen 1** | **Specimen 2** |
| Adrenal Cortex | 0 | 0 |
| Adrenal Medulla | 0 | 1 |
| Bladder:Smooth muscle | 0 | 0 |
| Bladder: Transitional Epithelium | 3 | 0 |
| Brain cortex: Blia | 0 | 0 |
| Brain cortex: Neurons | 0 | 0 |
| Breast: Epithelium | 0 | 0 |
| Breast: Stroma | 0 | 0 |
| Colon: Epithelium | 0 | 0 |
| Colon: Ganglia | 0 | NA |
| Colon: Inflammatory compartment | 3-4 (occasional) | 3 (occasional) |
| Colon: Smooth muscle | 1 (occasional) | 0 |
| Heart: Cardiac myocytes | 0 | 0 |
| Kidney cortex: Glomeruli | 2-3 | 2 |
| Kidney cortex: Tubular epithelium | 2 | 2-3 |
| Kidney medulla:Tubular epithelium | 2 | 0 |
| Kidney medulla: other | NA | 2-3 |
| Liver: Bile duct epithelium | 0 | 0 |
| Liver: Hepatocytes | 1-2 | 1 |
| Liver: Kupffer cells | 0 | 0 |
| Lung :Airway epithelium | 0 | 0 |
| Lung: Alveolar macrophages | 2 (occasional)-3 | 2-3 (occasional) |
| Lung: other | 3 | NA |
| Lung: Pneumocytes | 2-3 (occasional) | 2-3 (occasional) |
| Ovary: Follicle | 2 (occasional) | 1-2 |
| Ovary: Stroma | 1 | 1 (occasional) |
| Pancreas: Acinar epithelium | 0 | 1 (occasional) |
| Pancreas:Ductal epithelium | 0 | 0 |
| Pancreas:Islets of Langerhans | 0 | 0 |
| Placenta: Stroma | 0 | 0 |

(continued)

| Normal Human Tissue Immunohistology with anti-TIM-1 mAb 2.59.2 | | |
|---|---|---|
| **Tissue** | **Score** | |
| | **Specimen 1** | **Specimen 2** |
| Placenta:Trophoblasts | 0 | 0 |
| Prostate: Fibromuscular stroma | 0 | 0 |
| Prostate: Glandular epithelium | 0 | 0 |
| Skeletal muscle: Myocytes | 0 | 0 |
| Skin: Dermis | 0 | 0 |
| Skin: Epidermis | 0 | 0 |
| Small intestine: Epithelium | 0 | 0 |
| Small intestine: Ganglion | 0 | 0 |
| Small intestine: Inflammatory compartment | 0 | 0 |
| Small intestine: Smooth muscle cells | 0 | 0 |
| Spleen: Red pulp | 0 | 2 (rare) |
| Spleen: white pulp | 0 | 0 |
| Stomach: Epithelium | 0 | 0 |
| Stomach: Smooth Muscle Cells | 0 | 0 |
| Tstis: Leydig cells | 2 | 1-2 |
| Testis: Seminiferous epithelium | 1 | 2 |
| Thymus: Epithelium | 0 | 0 |
| Thymus: Lymphocytes | 2 (rare) | 2 (occasional) |
| Thyroid: Follicular epithelium | 0 | 0 |
| Tonsil: Epithelium | 0 | 0 |
| Tonsil: Lymphocytes | 3 (occasional) | 2 (occasional) |
| Uterus: Endometrium | 0 | 0 |
| Uterus: Myometrium | 0 | 0 |

Example 8

Antibody mediated toxin killing

A clonogenic assay as described in the art was used to determine whether primary antibodies can induce cancer cell death when used in combination with a saporin toxin conjugated secondary antibody reagent. Kohls and Lappi, Biotechniques, **28**(1):162-5 (2000).

Assay Protocol

ACHN and BT549 cells were plated onto flat bottom tissue culture plates at a density of 3000 cells per well. On day 2 or when cells reached ~25% confluency, 100 ng/well secondary mAb-toxin (goat anti-human IgG-saporin; Advanced Targeting Systems; HUM-ZAP; cat. no. IT-22) was added. A positive control anti-EGFR antibody, mAb 2.7.2, mAb 2.59.2, or an isotype control mAb was then added to each well at the desired concentration (typically 1 to 500 ng/mL). On day 5, the cells were trypsinized, transferred to a 150 mm tissue culture dish, and incubated at 37 °C. Plates were examined daily. On days 10-12, all plates were Giemsa stained and colonies on the plates were counted. Plating efficiency was determined by comparing the number of cells prior to transfer to 150 mm plates to the number of colonies that eventually formed.

The percent viability in antigen positive ACHN and antigen negative BT549 cell lines are presented in Figure 4 and Figure 5 respectively. In this study, the cytotoxic chemotherapy reagent 5 Fluorouracil (5-FU) was used as the positive control and induced almost complete killing, whereas the saporin conjugated-goat anti-human secondary antibody alone had no effect. A monoclonal antibody (NeoMarkers MS-269-PABX) generated against the EGF receptor expressed by both cell lines was used to demonstrate primary antibody and secondary antibody- saporin conjugate specific killing. The results indicate that both cell lines were susceptible to EGFR mAb mediated toxin killing at 100 ng/mL. At the same dose, both the anti-TIM-1 mAb 2.59.2 and the anti-TIM-1 mAb 2.702 induced over 90% ACHN cell death as compared to 0% BT549 cell death.

Antibody toxin conjugate mediated killing: Clonogenic Assay

CAKI-1 and BT549 cells were plated onto flat bottom tissue culture plates at a density of 3000 cells per well. On day 2 or when cells reach ~25% confluency, various concentrations (typically 1 to 1000 ng/ml) of unconjugated and Auristatin E (AE)-conjugated mAb, which included anti-EGFR, anti-TIM-1 mAb 2.7.2, anti-TIM-1 mAb 2.59.2 or isotype control mAb, were added to cells. Each of these antibodies was conjugated to AE. The monoclonal antibody (NeoMarkers MS-269-PABX) generated against the EGF receptor, which is expressed by both cell lines, was used as a positive control to demonstrate specific killing mediated by AE-conjugated antibody. On day 5, the cells were trypsinized, transferred to a 150 mm tissue culture dish, and incubated at 37 °C. Plates were examined daily. On days 10-12, all plates were Giemsa stained and colonies on the plates were counted. Plating efficiency was determined by counting the cells prior to transfer to 150 mm plates and compared to the number of colonies that eventually formed.

The percent viability in antigen positive CAKI-1 and antigen negative BT549 cell lines are presented in Figures 6 and 7, respectively.

The results indicate that unconjugated and AE-conjugated isotype control mAb had no effect on growth of both CAKI-1 and BT549 cells. However, both cell lines were susceptible to AE-EGFR mAb mediated toxin killing in a dose-dependent fashion. At the maximum dose, both anti-TIM-1 mAbs (2.59.2 and 2.70.2) induced over 90 % CAKI-1 cell death when compared to their unconjugated counterparts. The response was dose dependent. At the same dose range, both anti-TIM-1 mAbs 2.59.2 and 2.70.2 did not affect the survival of BT549 cells.

Example 9

Human Tumor Xenograft Growth Delay Assay

A tumor growth inhibition model was used according to standard testing methods. Geran *et al*., *Cancer Chemother. Rep.* **3**:1-104 (1972). Athymic nude mice (nu/nu) were implanted with either tumor cells or tumor fragments from an existing host, in particular, renal (CaKi-1) or ovarian (OVCAR) carcinoma tumor fragments were used. These animals were then treated with an anti-TIM-1 antibody immunotoxin conjugate, for example, mAb 2.70.2 AE conjugate at doses ranging from 1 to 20 mg/kg body weight, twice weekly for a period of 2 weeks. Tumor volume for treated animals was assessed and compared to untreated control tumors, thus determining the tumor growth delay.

After reaching a volume of 100 mm3 animals are randomized and individually identified in groups of 5 individuals per cage. Protein or antibody of interest was administered via conventional routes (intraperitoneal, subcutaneous, intravenous, or intramuscular) for a period of 2 weeks. Twice weekly, the animals are evaluated for tumor size using calipers. Daily individual animal weights are recorded throughout the dosing period and twice weekly thereafter. Tumor volume is determined using the formula: Tumor volume (in mm3) = (length x width x height) x 0.536. The volume determinations for the treated groups are compared to the untreated tumor bearing control group. The difference in time for the treated tumors to reach specific volumes is calculated for 500 1000, 1500 and 2000 mm3. Body weights are evaluated for changes when compared to untreated tumor bearing control animals. Data are reported as tumor growth in volume plotted against time. Body weights for each experimental group are also plotted in graph form.

Results show that the treatment is well tolerated by the mice. Specifically, complete regressions were noted in both the IGROV1 ovarian (6.25 mg/kg i.v. q4dX4) and the Caki-1 (3.3 mg/kg i.v. q4dx4) renal cell carcinoma models. No overt toxicity was observed in mice at doses up to 25 mg/kg (cumulative dose of 100 mg/kg). These data indicate that treatment with anti-TIM-1 mAb AE conjugate inhibits tumor growth of established CaKi-1 and OVCAR tumors, thus making these antibodies useful in the treatment of ovarian and renal carcinomas.

Example 10

Treatment of Renal Carcinoma with anti-TIM-1 antibodies

A patient in need of treatment for a renal carcinoma is given an intravenous injection of anti-TIM-1 antibodies coupled to a cytotoxic chemotherapic agent or radiotherapic agent. The progress of the patient is monitored and additional administrations of anti-TIM-1 antibodies are given as needed to inhibit growth of the renal carcinoma. Following such treatment, the level of carcinoma in the patient is decreased.

## Example 11

### FACS analysis of expression of TIM-1 protein on CD4+ T cells

Mononuclear cells were isolated from human blood diluted 1:1 in PBS, by spinning over Ficoll for 20 minutes. The mononuclear cells were washed twice at 1000 rpm with PBS -Mg and Ca and re-suspended in Miltenyi buffer (Miltenyi Biotec Inc., Auburn, CA); PBS, 0.5% BSA, 5 mM EDTA at approximately 108 cells/mL. 20 μL of CD4 Miltenyi beads were added per 107 cells and incubated for 15 minutes on ice. Cells were washed with a 10-fold excess volume of Miltenyi buffer. A positive selection column (type VS+) (Miltenyi Biotec Inc., Auburn, CA) was washed with 3 mL of Miltenyi buffer. The pelleted cells were re-suspended at 108 cells per mL of Miltenyi buffer and applied to the washed VS column. The column was then washed three times with 3 mL of Miltenyi buffer. Following this, the VS column was removed from the magnetic field and CD4+ cells were eluted from the column with 5 mL of Miltenyi buffer. Isolated CD4+ lymphocytes were pelleted and re-suspended in DMEM 5% FCS plus additives (non-essential amino acids, sodium pyruvate, mercaptoethanol, glutamine, penicillin, and streptomycin) at 106 cells/mL. 1x106 freshly isolated resting CD4+ T cells were transferred into flow cytometry tubes and washed with 2 mL/tube FACS staining buffer (FSB) containing PBS, 1% BSA and 0.05% NaN3. Cells were spun down and supernatant removed. Cells were blocked with 20% goat serum in FSB for 30 minutes on ice. Cells were washed as above and incubated with 10 μg/mL of primary human anti-TIM-1 mAb or control PK16.3 mAb in FSB (200 μL) for 45 minutes on ice followed by washing. Secondary goat anti-human PE conjugated antibody was added at 1:50 dilution for 45 minutes on ice in the dark, washed, resuspended in 500 μL of PBS containing 1% formaldehyde and kept at 4°C until flow cytometry analysis was performed.

FACS analysis was performed to determine the expression of TIM-1 protein as detected with five anfi-TIM-1 monoclonal antibodies (2.59.2, 1.29, 2.70.2, 2.56.2, 2.45.1) on human and mouse resting CD4+ T cells, as well as human activated and human polarized CD4+ T cells. These analyses demonstrate that freshly isolated resting human CD4+ T cells do not express TIM-1, while a major fraction of polarized human Th2 and Th1 cells do express TIM-1.

FACS Analysis of the Expression of the TIM-1 protein on human CD4+ Th2 cells using five anti-TIM-1 monoclonal antibodies is shown in Table 13. The experiment is described in the left-hand column and the labeled antibody is specified along the top row. Data is reported as the geometric mean of the fluorescence intensity.

Table 13

| FACS Analysis of the Expression of the TIM-1 protein on human CD4+ Th2 cells | | | | | | |
|---|---|---|---|---|---|---|
| **Experiment** | **Geometric mean of fluorescence intensity** | | | | | |
| | **Control PK16.3** | **Anti-TIM-1 mAb** | | | | |
| | | **1.29** | **2.45.1** | **2.56.2** | **2.59.2** | **2.70.2** |
| **Resting Human CD4+ T cells** | 4.6 | 4.7 | 5.1 | 6 | 4.9 | N/A |
| **Polarized Human CD4+ Th2 Cells** | 8.4 | 22.3 | 42.4 | 564.1 | 22 | 27.8 |

Table 14 demonstrates that over the course of 5 days, continual stimulation of T cells results in an increase in TIM-1 expression, as measured by anti-TIM-1 mAb 2.70.2, as compared to the control PK16.3 antibody. Furthermore, addition of matrix metalloproteinase inhibitor (MMPI) did not measurably increase TIM-1 expression, demonstrating that the receptor is not shed from T cells under these experimental conditions. Thus, expression of the TIM-1 protein and specific antibody binding is specific to activated Th1 and Th2 cells, which in turn, are characteristic of inflammatory response, specifically asthma.

Table 14

| Percent of activated T cells that express TIM-1 | | | | | | |
|---|---|---|---|---|---|---|
| | | ***Day 0*** | ***Day 1*** | ***Day 2*** | ***Day 4*** | ***Day 5*** |
| ***Control PK16.3*** | **- MMPI** | 1 | 3 | 3 | 1 | 1 |
| | **+ MMPI** | 1 | 2 | 6 | 2 | 2 |

(continued)

| Percent of activated T cells that express TIM-1 | | | | | | |
|---|---|---|---|---|---|---|
| | | ***Day 0*** | ***Day 1*** | ***Day 2*** | ***Day 4*** | ***Day 5*** |
| ***TIM-1 2.70.2*** | **- MMPI** | 1 | 8 | 10 | 5 | 13 |
| | **+ MMPI** | 1 | 10 | 14 | 10 | 19 |

Example 12

Cytokine assays

IL-4, IL-5, IL-10, IL-13, and IFNγ production levels by activated Th1 and Th2 cell were measured in culture supernatants treated with anti-TIM-1 antibodies using standard ELISA protocols. Cytokine production by Th1 or Th2 cells treated with anti-TIM-1 antibodies was compared to Th1 or Th2 cells treated with the control PK16.3 antibody. In addition, the following samples were run in parallel as internal controls: i) anti-CD3 treated Th1 or Th2 cells, where no cytokine production is expected because of the absence of co-stimulation, ii) anti-CD3/anti-CD28 stimulated Th1 or Th2 cells, expected to show detectable cytokine production, and iii) untreated Th1 or Th2 cells. CD4+ T cells were isolated as described in the Example above. Isolated CD4+ lymphocytes were then spun down and re-suspended in DMEM 5% FCS plus additives (non-essential amino acids, sodium pyruvate, mercaptoethanol, glutamine, penicillin, and streptomycin) at $10^6$ cells/mL. Falcon 6-well non-tissue culture treated plates were pre-coated overnight with anti-CD3 (2 μg/mL) and anti-CD28 (10 μg/mL) (600 μL total in Dulbecco's PBS) overnight at 4°C. The plates were washed with PBS and CD4+ lymphocytes were suspended at 500,000 cells/mL in Th2 medium: DMEM+ 10% FCS plus supplements and IL-2 5ng/mL, IL-4 5 ng/mL, anti-IFN gamma 5μg/mL and cells were stimulated 4-6 days at 37 °C and 5% CO2 in the presence of 5 μg/mL of mAb recognizing the TIM-1 protein or isotype matched negative control mAb PK16.3.

In another set of experiments, CD4+ lymphocytes were suspended at 500,000 cells/mL in Th1 medium: DMEM+ 10% FCS plus supplements and IL-2 5 ng/mL, IL12 5 ng/mL, anti-IL-4 5μg/mL and stimulated 4-6 days 37°C temp and 5% CO2 in the presence of 5 μg/mL TIM-1 or isotype matched control mAb PK16.3. Cells were washed two times in DMEM and resuspended in DMEM, 10% FCS plus supplements and 2 ng/mL IL-2 (500,000 cells/mL) in the presence of 5 μg/mL TIM-1 mAb or control PK16.3 mAb and cultured (rested) for 4-6 days at 37°C and 5% CO2. The process of activation and resting was repeated at least once more as described above with the addition of anti-CD95L (anti-FAS ligand) to prevent FAS-mediated apoptosis of cells. Falcon 96-well non-tissue culture treated plates pre-coated overnight with anti-CD3 mAb at 500 ng/mL and costimulatory molecule B7H2 (B7 homolog 2) 5μg/mL were washed and 100 μL of TIM-1 mAb treated Th1 or Th2 (200,000 cells) added per well. After 3 days of culture, the supernatants were removed and IL-4, IL-5, IL-10, IL-13, and IFNγ levels were determined by ELISA (Pharmingen, San Diego, CA or R&D Systems, Minneapolis, MN).

As demonstrated below, anti-TIM-1 mAb significantly inhibited release of the tested cytokines by Th1 and Th2 cells (see Figures 8-17). Results where inhibition of cytokine production is significant (p=.02-.008), are marked on the bar graphs with an asterisk. Tables 15 and 16 summarize the bar graphs in Figures 8-17.

Table 15

| Cytokine Inhibition in CD4+ Th1 cells using anti-TIM-1 antibodies in two independent human donors | | | | | | |
|---|---|---|---|---|---|---|
| Experiments that demonstrate significant inhibition of cytokine production are marked with an asterisk: P= 0.01 to 0.05 *; P=0.005 to 0.009 **; P=0.001 to 0.004 *** | | | | | | |
| **Donor 12+17** | | **Percentage of Control Antibody** | | | | |
| **TH1** | **Cytokines Anti-TIM-1 mAbs** | **IL-5** | **IL-4** | **IL-10** | **IL-13** | **INF γ** |
| | 2.56.2 | 100.17 | 28.49 * | 63.76 * | 86.45 | 93.69 |
| | 2.45.1 | 90.23 | 39.78 * | 83.98 | 96.25 | 100.6 |
| | 1.29 | 94.63 | 81.05 | 60.77 ** | 73.95 *** | 93.51 |
| | 2.59.2 | 66.62 * | 31.40 * | 68.99 * | 54.5 *** | 128.12 |

Table 16

| Cytokine Inhibition in CD4+ Th2 cells using anti-TIM-1 antibodies in two independent human donors | | | | | | |
|---|---|---|---|---|---|---|
| Experiments that demonstrate significant inhibition of cytokine production are marked with an asterisk: P= 0.01 to 0.05 *;P=0.005 to 0.009 **; P=0.001 to 0.004 *** | | | | | | |
| **Donor 12+17** | | **Percentage of Control Antibody** | | | | |
| | **Cytokines Anti-TIM-1 mAbs** | **IL-5** | **IL-4** | **IL-10** | **IL-13** | **INF γ** |
| **TH2** | 2.56.2 | 112.07 | 103.46 | 93.97 | 86.45 | 88.30 |
| | 2.45.1 | 148.7 | 25.66 *** | 55.97 * | 86.81 | 25.66 * |
| | 1.29 | 80.26 | 112.54 | 44.45 * | 48.91 ** | 112.54 |
| | 2.59.2 | 23.62 * | 19.17 ** | 43.86 * | 43.71 *** | 19.18 * |

A summary of Th2 cytokine inhibition data obtained from multiple experiments with different donors is provided in Table 17. Each experiment used purified CD4+ cells isolated from whole blood samples from two independent donors. Cytokine production is reported as the percent of cytokine production detected using the control PK16.3 mAb. The anti-TIM-1 mAb used in each experiment is specified along the bottom row. Results that report significant cytokine inhibition are underlined in Table 17 below. The use of "ND" indicates that the experiment was not performed. These results do reflect donor dependent variability but show that mAbs 2.59.2 and 1.29 reproducibly block one or more of the Th2 cytokines.

Table 17

| Summary of Cytokine Inhibition using anti-TIM-1 mAbs 2.59.2 and 1.29 in 5 independent human donor groups | | | | | |
|---|---|---|---|---|---|
| Results of experiments that report inhibition greater than 50% of that seen using the control PK16.3 antibody are underlined | | | | | |
| **Donor ID Cytokine** | **12+17** | **12+14** | **13+14** | **14** | **12** |
| **IL-4** | 19 | 626 | 130 | ND | ND |
| **IL-5** | 24 | 5 | 122 | 67 | 2 |
| **IL-10** | 44 | 83 | 19 | 45 | 109 |
| **IL-13** | 44 | ND | 17 | 100 | 91 |
| | **Anti-TIM-1 mAb 2.59.2** | **Anti-TIM-1 mAb 1.29** | | | |

Example 13

Construction, expression and purification of anti-TIM- scFv.

The VL and VH domains of mAb 2.70 were used to make a scFv construct. The sequence of the anti-TIM-1 scFv was synthesized by methods known in the art.

The nucleotide sequence of anti-TIM-1 scFv is as follows:

ATGAAATACCTGCTGCCGACCGCTGCTGCTGGTCTGCTGCTCCTCGCTGCCCAG
CCGGCCATGGCCGATATTGTGATGACCCAGACTCCACTCTCCCTGCCCGTCACC
CCTGGAGAGCCGGCCTCCATCTCCTGCAGGTCTAGTCGGAGCCTCTTGGATAGT
GATGATGGAAACACCTATTTGGACTGGTACCTGCAGAAGCCAGGGCAGTCTCC
ACAGCTCCTGATCTACACGCTTTCCTATCGGGCCTCTGGAGTCCCAGACAGGTT
CAGTGGCAGTGGGTCAGGCACTGATTTCACACTGAAAATCAGCAGGGTGGAGG
CTGAGGATGTTGGAGTTTATTACTGCATGCAACGTGTAGAGTTTCCTATCACCTT
CGGCCAAGGGACACGACTGGAGATTAAACTTTCCGCGGACGATGCGAAAAAGG
ATGCTGCGAAGAAAGATGACGCTAAGAAAGACGATGCTAAAAAGGACCTCCAG
GTGCAGCTGGTGGAGTCTGGGGGAGGCGTGGTCCAGCCTGGGAGGTCCCTGAG
ACTCTCCTGTGCAGCGTCTGGATTCATCTTCAGTCGCTATGGCATGCACTGGGTC
CGCCAGGCTCCAGGCAAGGGGCTGAAATGGGTGGCAGTTATATGGTATGATGG
AAGTAATAAACTCTATGCAGACTCCGTGAAGGGCCGATTCACCATCTCCAGAGA
CAATTCCAAGAACACGCTGTATCTGCAAATGAACAGCCTGAGAGCCGAGGACA
CGGCTGTGTATTACTGTGCGAGAGATTACTATGATAATAGTAGACATCACTGGG

GGTTTGACTACTGGGGCCAGGGAACCCTGGTCACCGTCTCCTCAGCTAGCGATT
ATAAGGACGATGATGACAAATAG (SEQ ID NO:108)

The amino acid sequence of mature anti-TIM-1 scFv is as follows:

DIVMTQTPLSLPVTPGEPASISCRSSRSLLDSDDGNTYLDWYLQKPGQSPQLLIYTLS
YRASGVPDRFSGSGSGTDFTLKISRVEAEDVGVYYCMQRVEFPITFGQGTRLEIKLS
ADDAKKDAAKKDDAKKDDAKKDLQVQLVESGGGVVQPGRSLRLSCAASGFIFSR
YGMHWVRQAPGKGLKWVAVIWYDGSNKLYADSVKGRFTISRDNSKNTLYLQMN
SLRAEDTAVYYCARDYYDNSRHHWGFDYWGQGTLVTVSSASDYKDDDDK (SEQ
ID NO:109)

The synthesized DNA can be inserted into the pET-20b(+) expression vector, for periplasmic expression in *E. coli.* Cells are grown and the periplasmic proteins prepared using standard protocols. Purification of the anti-TIM-1 scFv is achieved using an anti-FLAG M2 affinity column as per the manufacturer's directions. The predicted molecular weight of the mature protein is 30222.4 daltons. This purified scFv is used in the assays described below to test for biological activity. The scFv construct is comprised of a signal peptide (SP), VL (VL1) derived from mAb 2.70, a linker (L4) based on the 25 amino acid linker 205C, the VH (VH1) derived from mAb 2.70, and a Tag (in this case the FLAG tag). It will be obvious to those skilled in the art that other SP, linker and tag sequences could be utilized to get the same activity as the anti-TIM-1 scFv antibody described herein.

<u>Example 14</u>

<u>Construction, expression and purification of anti-TIM-1 and anti-CD3 bispecific scFv1</u>

The basic formula for the construction of this therapeutic protein is as follows:

SPI - VL1 - L1 - VH1 - L2 - VH2 - L3 - VL2 - Tag

The signal peptide SP1 is the same as IgG kappa signal peptide VKIII A27 from Medical Research Council (MRC) Centre for Protein Engineering, University of Cambridge, UK.

Other signal peptides can also be used and will be obvious to those skilled in the art. This protein is designed to be expressed from mammalian cells. The predicted molecular weight of the mature cleaved protein is 54833.3 dalton. L1 corresponds to the (Gly4Ser)3 linker, while linker 2 (L2) corresponds to the short linker sequence: GGGGS. L3 is an 18 amino acid linker. VH2 corresponds to the anti-CD3 variable heavy chain domain from Genbank (accession number CAE85148) while VL1 corresponds to the anti-CD3 variable light chain domain from Genbank (accession number CAE85148). The tag being used for this construct is a His tag to facilitate purification and detection of this novel protein.

Standard protocols are used to express and purify this His tagged protein, which is tested for activity and tumor cell killing in the protocols described below.

The amino acid and nucleic acid numbering for the components comprising the anti-TIM-1 and anti-CD3 bispecific scFv1 is as follows:

SP: -20 to -1 aa; -60 to -1 nt
VL1: 1-113 aa; 1-339nt
L1: 114-128 aa; 340-384nt
VH1: 129-251 aa; 385-753nt
L2: 252-256 aa; 754-768nt
VH2: 257-375 aa; 769-1125nt
L3: 376-393 aa; 1126-1179nt
VL2: 394-499 aa; 1180-1497nt
Tag: 500-505 aa; 1498-1515nt

The nucleotide sequence of anti-TIM-1 and anti-CD3 bispecific scFv1 is as follows:

ATGGAAACCCCAGCGCAGCTTCTCTTCCTCCTGCTACTCTGGCTCCCAGATACC
ACCGGAGATATTGTGATGACCCAGACTCCACTCTCCCTGCCCGTCACCCCTGGA
GAGCCGGCCTCCATCTCCTGCAGGTCTAGTCGGAGCCTCTTGGATAGTGATGAT
GGAAACACCTATTTGGACTGGTACCTGCAGAAGCCAGGGCAGTCTCCACAGCTC
CTGATCTACACGCTTTCCTATCGGGCCTCTGGAGTCCCAGACAGGTTCAGTGGC
AGTGGGTCAGGCACTGATTTCACACTGAAAATCAGCAGGGTGGAGGCTGAGGA
TGTTGGAGTTTATTACTGCATGCAACGTGTAGAGTTTCCTATCACCTTCGGCCAA
GGGACACGACTGGAGATTAAAGGTGGTGGTGGTTCTGGCGGCGGCGGCTCCGG
TGGTGGTGGTTCCCAGGTGCAGCTGGTGGAGTCTGGGGGAGGCGTGGTCCAGC
CTGGGAGGTCCCTGAGACTCTCCTGTGCAGCGTCTGGATTCATCTTCAGTCGCT
ATGGCATGCACTGGGTCCGCCAGGCTCCAGGCAAGGGGCTGAAATGGGTGGCA
GTTATATGGTATGATGGAAGTAATAAACTCTATGCAGACTCCGTGAAGGGCCGA
TTCACCATCTCCAGAGACAATTCCAAGAACACGCTGTATCTGCAAATGAACAGC
CTGAGAGCCGAGGACACGGCTGTGTATTACTGTGCGAGAGATTACTATGATAAT
AGTAGACATCACTGGGGGTTTGACTACTGGGGCCAGGGAACCCTGGTCACCGTC
TCCTCAGGAGGTGGTGGATCCGATATCAAACTGCAGCAGTCAGGGGCTGAACT
GGCAAGACCTGGGGCCTCAGTGAAGATGTCCTGCAAGACTTCTGGCTACACCTT
TACTAGGTACACGATGCACTGGGTAAAACAGAGGCCTGGACAGGGTCTGGAAT
GGATTGGATACATTAATCCTAGCCGTGGTTATACTAATTACAATCAGAAGTTCA
AGGACAAGGCCACATTGACTACAGACAAATCCTCCAGCACAGCCTACATGCAA
CTGAGCAGCCTGACATCTGAGGACTCTGCAGTCTATTACTGTGCAAGATATTAT
GATGATCATTACTGCCTTGACTACTGGGGCCAAGGCACCACTCTCACAGTCTCC
TCAGTCGAAGGTGGAAGTGGAGGTTCTGGTGGAAGTGGAGGTTCAGGTGGAGT

CGACGACATTCAGCTGACCCAGTCTCCAGCAATCATGTCTGCATCTCCAGGGGA
GAAGGTCACCATGACCTGCAGAGCCAGTTCAAGTGTAAGTTACATGAACTGGT
ACCAGCAGAAGTCAGGCACCTCCCCCAAAAGATGGATTTATGACACATCCAAA
GTGGCTTCTGGAGTCCCTTATCGCTTCAGTGGCAGTGGGTCTGGGACCTCATAC
TCTCTCACAATCAGCAGCATGGAGGCTGAAGATGCTGCCACTTATTACTGCCAA
CAGTGGAGTAGTAACCCGCTCACGTTCGGTGCTGGGACCAAGCTGGAGCTGAA
ATAG (SEQ ID NO:110)

The protein sequence of mature anti-TIM-1 and anti-CD3 bispecific scFv1 is as follows:

DIVMTQTPLSLPVTPGEPASISCRSSRSLLDSDDGNTYLDWYLQKPGQSPQLLIYTLS
YRASGVPDRFSGSGSGTDFTLKISRVEAEDVGVYYCMQRVEFPITFGQGTRLEIKGG
GGSGGGGSGGGGSQVQLVESGGGVVQPGRSLRLSCAASGFIFSRYGMHWVRQAPG
KGLKWVAVIWYDGSNKLYADSVKGRFTISRDNSKNTLYLQMNSLRAEDTAVYYC
ARDYYDNSRHHWGFDYWGQGTLVTVSSGGGGSDIKLQQSGAELARPGASVKMSC
KTSGYTFTRYTMHWVKQRPGQGLEWIGYINPSRGYTNYNQKFKDKATLTTDKSSS
TAYMQLSSLTSEDSAVYYCARYYDDHYCLDYWGQGTTLTVSSVEGGSGGSGGSG
GSGGVDDIQLTQSPAIMSASPGEKVTMTCRASSSVSYMNWYQQKSGTSPKRWIYD
TSKVASGVPYRFSGSGSGTSYSLTISSMEAEDAATYYCQQWSSNPLTFGAGTKLEL
K (SEQ ID NO:111)

Example 15

Construction, expression and purification of anti-TIM-1 and anti-CD3 bispecific scFv2:

The basic formula for the construction of this novel therapeutic protein is as follows:

SP1-VL1-L4-VH1-L2-VH2-L4-VL2-Tag

The signal peptide SP1 is IgG kappa signal peptide VKIII A27 from Medical Research Council (MRC) Centre for Protein Engineering, University of Cambridge, UK. For more information see mrc-cpe.cam.ac.uk/ALIGNMENTS.php?menu=901. Other signal peptides and linkers could also be used to get additional biologically active bispecific single chain antibodies. The protein being described in this example is also designed to be expressed from mammalian cells and is similar to the anti-TIM-1 and anti-CD3 bispecific scFv1, except that it utilizes a different linker as indicated in the basic formula above (L4, as described earlier), and that a Flag tag is used instead of the His tag as in the first example.

The predicted molecular weight of the mature cleaved protein is 58070.0 dalton. The tag being used for this construct is a FLAG tag to facilitate purification and detection of this novel protein. Standard protocols are used to express this secreted protein and purify it, which is tested for activity and tumor cell killing in the protocols described below.

The amino acid and nucleic acid numbering for the components comprising the anti-TIM-1 and anti-CD3 bispecific scFv2 is as follows:

SP: -20 to -1 aa; -60 to -1nt
VL1: 1-113 aa; 1-339nt
L1: 114-138 aa; 340-414nt
VH1: 139-261 aa; 415-783nt
L2: 262-266 aa; 784-798nt
VH2: 267-385 aa; 799-1155nt
L3: 386-410 aa; 1156-1230nt
VL2: 411-516 aa; 1231-1548nt
Tag: 517-524 aa; 1549-1572nt

The nucleotide sequence of anti-TIM-1 and anti-CD3 bispecific scFv2 is as follows:

ATGGAAACCCCAGCGCAGCTTCTCTTCCTCCTGCTACTCTGGCTCCCAGATACC
ACCGGAGATATTGTGATGACCCAGACTCCACTCTCCCTGCCCGTCACCCCTGGA
GAGCCGGCCTCCATCTCCTGCAGGTCTAGTCGGAGCCTCTTGGATAGTGATGAT
GGAAACACCTATTTGGACTGGTACCTGCAGAAGCCAGGGCAGTCTCCACAGCTC
CTGATCTACACGCTTTCCTATCGGGCCTCTGGAGTCCCAGACAGGTTCAGTGGC
AGTGGGTCAGGCACTGATTTCACACTGAAAATCAGCAGGGTGGAGGCTGAGGA
TGTTGGAGTTTATTACTGCATGCAACGTGTAGAGTTTCCTATCACCTTCGGCCAA
GGGACACGACTGGAGATTAAACTTTCCGCGGACGATGCGAAAAAGGATGCTGC
GAAGAAAGATGACGCTAAGAAAGACGATGCTAAAAAGGACCTGCAGGTGCAG
CTGGTGGAGTCTGGGGGAGGCGTGGTCCAGCCTGGGAGGTCCCTGAGACTCTCC
TGTGCAGCGTCTGGATTCATCTTCAGTCGCTATGGCATGCACTGGGTCCGCCAG
GCTCCAGGCAAGGGGCTGAAATGGGTGGCAGTTATATGGTATGATGGAAGTAA
TAAACTCTATGCAGACTCCGTGAAGGGCCGATTCACCATCTCCAGAGACAATTC
CAAGAACACGCTGTATCTGCAAATGAACAGCCTGAGAGCCGAGGACACGGCTG
TGTATTACTGTGCGAGAGATTACTATGATAATAGTAGACATCACTGGGGGTTTG
ACTACTGGGGCCAGGGAACCCTGGTCACCGTCTCCTCAGGAGGTGGTGGATCCG
ATATCAAACTGCAGCAGTCAGGGGCTGAACTGGCAAGACCTGGGGCCTCAGTG
AAGATGTCCTGCAAGACTTCTGGCTACACCTTTACTAGGTACACGATGCACTGG
GTAAAACAGAGGCCTGGACAGGGTCTGGAATGGATTGGATACATTAATCCTAG
CCGTGGTTATACTAATTACAATCAGAAGTTCAAGGACAAGGCCACATTGACTAC
AGACAAATCCTCCAGCACAGCCTACATGCAACTGAGCAGCCTGACATCTGAGG
ACTCTGCAGTCTATTACTGTGCAAGATATTATGATGATCATTACTGCCTTGACTA
CTGGGGCCAAGGCACCACTCTCACAGTCTCCTCACTTTCCGCGGACGATGCGAA
AAAGGATGCTGCGAAGAAAGATGACGCTAAGAAAGACGATGCTAAAAAGGAC
CTGGACATTCAGCTGACCCAGTCTCCAGCAATCATGTCTGCATCTCCAGGGGAG
AAGGTCACCATGACCTGCAGAGCCAGTTCAAGTGTAAGTTACATGAACTGGTAC

CAGCAGAAGTCAGGCACCTCCCCCAAAAGATGGATTTATGACACATCCAAAGT
GGCTTCTGGAGTCCCTTATCGCTTCAGTGGCAGTGGGTCTGGGACCTCATACTCT
CTCACAATCAGCAGCATGGAGGCTGAAGATGCTGCCACTTATTACTGCCAACAG
TGGAGTAGTAACCCGCTCACGTTCGGTGCTGGGACCAAGCTGGAGCTGAAAGA
TTATAAGGACGATGATGACAAATAG (SEQ ID NO:112)

The protein sequence of mature anti-TIM-1 and anti-CD3 bispecific scFv2 is as follows:

DIVMTQTPLSLPVTPGEPASISCRSSRSLLDSDDGNTYLDWYLQKP
GQSPQLLIYTLSYRASGVPDRFSGSGSGTDFTLKISRVEAEDVGVYYCMQRVEFPIT
FGQGTRLEIKLSADDAKKDAAKKDDAKKDDAKKDLQVQLVESGGGVVQPGRSLR
LSCAASGFIFSRYGMHWVRQAPGKGLKWVAVIWYDGSNKLYADSVKGRFTISRDN
SKNTLYLQMNSLRAEDTAVYYCARDYYDNSRHHWGFDYWGQGTLVTVSSGGGG
SDIKLQQSGAELARPGASVKMSCKTSGYTFTRYTMHWVKQRPGQGLEWIGYINPS
RGYTNYNQKFKDKATLTTDKSSSTAYMQLSSLTSEDSAVYYCARYYDDHYCLDY
WGQGTTLTVSSLSADDAKKDAAKKDDAKKDDAKKDLDIQLTQSPAIMSASPGEKV
TMTCRASSSVSYMNWYQQKSGTSPKRWIYDTSKVASGVPYRFSGSGSGTSYSLTIS
SMEAEDAATYYCQQWSSNPLTFGAGTKLELKDYKDDDDK (SEQ ID NO:113)

Example 16

Anti-TIM-1 scFv species biological activity

ELISA Analysis:

To determine if the anti-TIM-1 and anti-CD3 bispecific scFv1 and scFv2 antibodies bind to specific antigen, ELISA analysis is performed. 1ug/ml of specific antigen (TIM-1 antigen (CG57008-02) is bound to ELISA plates overnight in carbonate/bicarbonate buffer (pH approximately 9.2-9.4). Plates are blocked with assay diluent buffer purchased from Pharmingen San Diego, CA), and various concentrations of the anti-TIM-1 scFv bispecific antibodies are added for 1 hour at room temp. Plates are washed in 0.01% Tween 20 in PBS, followed by addition of HRP-conjugated mAb to either the 6-His tag (Invitrogen, Carlsbad, CA) or the FLAG peptide tag or (Sigma, St. Louis, MO) in assay diluent for 60 minutes at room temperature. Color is developed with TMB substrate (Pharmingen), and the reaction stopped with $H_2SO_4$. Plates are read at A450 nm, and the O.D. value taken as a measure of protein binding.

FACS analysis

Binding of the anti-TIM-1 and anti-CD3 bispecific scFv1 and scFv2 antibodies., as well as the anti-TIM-1 scFv antibody to cells expressing the antigens recognized by the anti-TIM-1 human mAbs is examined by FACS analysis. Cells (such as ACHN) are washed in PBS and resuspended in FACS buffer consisting of ice cold PBS with addition of 1% BSA or 1% FBS. The resuspended cells are then incubated on ice with various concentrations of the bispecific antibody for 30 minutes. Cells are washed to remove non-bound antibody. Bound antibody is detected by binding of a secondary labeled mAb (phycoerythrin or FITC labeled) that specifically recognizes the 6-his tag or the FLAG-tag that is engineered on the bispecific antibody sequence. Cells are washed and analyzed for binding of the anti-tag mAb by FACS analysis. Binding of bispecific mAb plus anti-tag mAb is compared to binding of the anti-tag mAb alone.

Cytotoxicity analysis

To determine if the bispecific antibody has functional activity as defined by the ability of the bispecific to target T cells to TIM-1 expressing normal or tumor cells, the bispecific antibody is tested in a Cytotoxicity assay. T cells are obtained from the low density cells derived from centrifugation of blood over density separation medium (specific density 1.077). T cells can be used in a heterogeneous mix from the peripheral blood mononuclear cell fraction (which also contains B cells, NK cells and monocytes) or further purified from the low-density cells using MACS separation and negative or positive selection. Killing in assays with T cells derived from the blood directly will have less cytolytic activity than cells that have been stimulated *in vitro* with PHA, cytokine, activating monoclonal antibodies or other stimulators of polyclonal T cell activation. Therefore, these activators will be used to further boost the activity of T cells in the functional assays. Many variations of cytotoxicity assays are available. Cytotoxicity assays measure the release of natural products of the cells metabolism upon lysis, such as LDH. Other assays are based around labeling cells with various agents such as radioactive chromium (51Cr), DELFIA BATDA, CSFE or similar labeling agents and detecting release or change in live cells bound by the agent.

DELFIA cytotoxicity assays (PerkinElmer Life and Analytical Sciences, Inc. Boston, MA) offer a non-radioactive method to be used in cell mediated cytotoxicity studies. The method is based on loading cells with an acetoxymethyl ester of a fluorescence enhancing ligand. After the ligand has penetrated the cell membrane the ester bonds are hydrolyzed within the cell to form a hydrophilic ligand, which no longer passes through the membrane. After cytolysis the released ligand is introduced to a europium solution to form a fluorescent chelate. The measured signal correlates directly with the amount of lysed cells. Target cells are resuspended to a concentration of $2x10^6$/ml. 10 $\mu$l of DELFIA BATDA was mixed in a tube with 2 ml of target cells according to the manufacturers instructions. Various concentrations of T cells are added to a fixed concentration of labeled target cells (5000 cells per well) in 96 well U-bottom plates, and incubated for at least 2 hours at 37°C. The plates are spun at approximately 200g, followed by the aspiration of 20 $\mu$l of supernatant, which was then added to a europium solution (200 $\mu$l) in a separate plate. The plate is incubated for 15 minutes at room temperature, followed by analysis on a SAFIRE (Tecan, Maennedorf, Switzerland) according to the manufacturer's instructions. Signal in the test wells are compared to signal in 100% lysis well (10% lysis buffer in place of T cells) and cell with medium alone (spontaneous release), and % specific lysis is calculated from the formula

$$\%\text{specific lysis} = (\text{test} - \text{spontaneous release})/100\%\ \text{lysis}\ x100.$$

BIAcore kinetic analysis of scFv constructs

Kinetic measurements to determine the affinity for the scFv constructs (monomer as well as bispecific, containing at least 1 scFv moiety binding to TIM-1) are measured using the methods described earlier for the whole antibodies described herein. scFv-containing antibody protein affinities to TIM-1 are expected to be within a factor of 10, i.e. between 0.271- 27.1 nM, of the affinity given for mAb 2.70.

Example 17

Ability of anti-TIM-1 mAb to inhibit the proliferation of human ovary carcinoma cells

Several fully human monoclonal antibody clones were isolated from the immunizations described above and their ability to inhibit the proliferative potential of OVCAR-5 (human ovary carcinoma) cells was analyzed using the 5-bromo-2-deoxyuridine (BrdU) incorporation assay (described in International Patent Application No. WO 01/25433).

In the BrdU assay, OVCAR-5 cancer cells (Manassas, VA) were cultured in Dulbeccos Modification of Eagles Medium (DMEM) supplemented with 10% fetal bovine serum or 10% calf serum respectively. The ovarian cancer cell line was grown to confluence at 37°C in 10% $CO_2$/air. Cells were then starved in DMEM for 24 hours. Enriched conditioned medium was added (10 μL/100 μL of culture) for 18 hours. BrdU (10 μM) was then added and incubated with the cells for 5 hours. BrdU incorporation was assayed by colorimetric immunoassay according to the manufacturer's specifications (Boehringer Mannheim, Indianapolis, IN).

The capability of various human anti-TIM-1 monoclonal antibodies to neutralize was assessed. The results provided in Figures 18A-18T are presented in a bar graph format to assist in comparing the levels of BrdU incorporation in OVCAR5 cells upon exposure to various human anti-TIM-1monoclonal antibodies described herein. As positive and negative controls, OVCAR5 cells were cultured in the presence of either complete media (complete) or restricted serum-containing media (starved). In addition, the monoclonal antibody PK16.3 was included as a negative treatment control representing a human IgG antibody of irrelevant specificity. Human anti-TIM-1 monoclonal antibodies described herein were used at varying doses (10-1000 ng/mL) as compared to a control run utilizing varying concentrations.

Example 18

Antibody conjugate studies

Additional antibody conjugate studies were performed using the plant toxin saporin conjugated to anti-TIM-1-specific mABs (1.29 and 2.56.2) and various irrelevant antibodies, including, PK16.3 (Figures 19A-19C). Additional negative controls included anti-TIM-1-specific mAB 2.56.2 and irrelevant antibody PK16.3 without toxin (Figure 19D). Four cancer cell lines, three kidney cancer cell lines (ACHN, CAKI, and 7860) and one breast cancer cell line (BT549), were treated for 72 hours with saporin-antibody conjugates or antibodies alone, after which time BrdU was added to monitor proliferation over a 24 hour period. The results are described in Figures 19A-20C for the kidney cancer cell lines and Figure 19D for the breast cancer cell line. All three kidney cancer cell lines were sensitive to treatment with saporin-TIM-1-specific antibody, conjugates as evidenced by a measurable decrease in BrdU incorporation. Treatment of the same cell lines with conjugated irrelevant antibodies had little or no effect demonstrating antigen dependent antiproliferative effects. The same studies performed with the BT549 cell line showed that the TIM-1-specific antibody 2.56.2 showed no antiproliferative effect either alone or when conjugated to saporin. The negative controls for these studies appeared to work well with no cytotoxic effects

Example 19

Sequences

Below are sequences related to monoclonal antibodies against TIM-1. With regard to the amino acid sequences, bold indicates framework regions, underlining indicates CDR regions, and *italics* indicates constant regions.

Anti-TIM-1 mAb 1.29

Nucleotide sequence of heavy chain variable region and a portion of constant region:

5'TGGGTCCTGTCCCAGGTGCAGCTGCAGGAGTCGGGCCCAGGACTGGTGAAGC
CTTCGGAGACCCTGTCCCTCACCTGCACTGTCTCTGGTGGCTCCGTCAGCAGTG
GTGGTTACTACTGGAGCTGGATCCGGCAGCCCCCAGGGAAGGGACTGGAGTGG
ATTGGGTTTATCTATTACACTGGGAGCACCAACTACAACCCCTCCCTCAAGAGT
CGAGTCTCCATATCAGTAGACACGTCCAAGAACCAGTTCTCCCTGAAGCTGAGC
TCTGTGACCGCTGCGGACGCGGCCGTGTATTACTGTGCGAGAGATTATGACTGG
AGCTTCCACTTTGACTACTGGGGCCAGGGAACCCTGGTCACCGTCTCCTCAGCC
TCCACCAAGGGCCCATCGGTCTTCCCCCTGGCGCCCTGCTCCAGGAGCACCTCC
GAGAGCACAGCGGCCCTGGGCTGCCTGGTCAAGGACTACTTCCCCGAACCGGT
GACGGTGTCGTGGAACTCAGGCGCTCT3' (SEQ ID NO:1)

Amino acid sequence of heavy chain variable region and a portion of constant region encoded by SEQ ID NO:1:

*WVLS***QVQLQESGPGLVKPSETLSLTCTVS**GGSVSSGGYYWS**WIRQPPGKGLEWI**
**G**FIYYTGSTNYNPSLKS**RVSISVDTSKNQFSLKLSSVTAADAAVYYCAR**DYDWSF
HFDYWGQGTLVTVSSA*STKGPSVFPLAPCSRSTSESTAALGCLVKDYFPEPVTVSWNSG*
*A* (SEQ ID NO:114)

Nucleotide sequence of light chain variable region and a portion of constant region:

5'CAGCTCCTGGGGCTCCTGCTGCTCTGGTTCCCAGGTGCCAGGTGTGACATCCA
GATGACCCAGTCTCCATCCTCCCTGTCTGCATCTATAGGAGACAGAGTCACCAT
CACTTGCCGGGCAAGTCAGGGCATTAGAAATGATTTAGGCTGGTATCAGCAGA
AACCAGGGAAAGCCCCTAAGCGCCTGATCTATGCTGCATCCAGTTTGCAAAGTG
GGGTCCCATCAAGGTTCAGCGGCAGTGGATCTGGGACAGAATTCACTCTCACAA
TCAGCAGCCTGCAGCCTGAAGATTTTGCAACTTATTACTGTCTACAGCATAATA

GTTACCCTCTCACTTTCGGCGGAGGGACCAAGGTGGAGATCAAACGAACTGTG
GCTGCACCATCTGTCTTCATCTTCCCGCCATCTGATGAGCAGTTGAAATCTGGA
ACTGCCTCTGTTGTGTGCCTGCTGAATAACTTCTATCCCAGAGAGGCCAAAGTA
CAGTGGAAGGTGGATAACGCC3' (SEQ ID NO:3)

Amino acid sequence of light chain variable region and a portion of constant region encoded by SEQ ID NO:3:

*QLLGLLLLWFPGARC***DIQMTQSPSSLSASIGDRVTITC**RASQGIRNDLG**WYQQKPG**
**KAPKRLIY**AASSLQS**GVPSRFSGSGSGTEFTLTISSLQPEDFATYYC**LQHNSYPLT
**FGGGTKVEIKR***TVAAPSVFIFPPSDEQLKSGTASVVCLLNNFYPREAKVQWKVDNA*
(SEQ ID NO:115)

Anti-TIM-1 mAb 1.37

Nucleotide sequence of heavy chain variable region and a portion of constant region:

5'CAGTGTGAGGTGCAGCTGGTGGAGTCTGGGGGAGGCTTGGTCCAGCCTGGGG
GGTCCCTGAGACTCTCCTGTGCAGCCTCTGGATTCACCTTTACTAACTATTGGAT
GAGCTGGGTCCGCCAGGCTCCAGGGAAGGGGCTGGAGTGGGTGGCCAACATAC
AGCAAGATGGAAGTGAGAAATACTATGTGGACTCTGTGAGGGGCCGATTCACC
ATCTCCAGAGACAACGCCAAGAACTCACTGTATCTGCAAATGAACAGCCTGAG
AGCCGAGGACTCGGCTGTGTATTACTGTGCGAGATGGGACTACTGGGGCCAGG
GAACCCTGGTCACCGTCTCCTCAGCCTCCACCAAGGGCCCATCGGTCTTCCCCC
TGGCGCCCTGCTCCAGGAGCACCTCCGAGAGCACAGCGGCCCTGGGCTGCCTG
GTCAAGGACTACTTCCCCGAACCGGTGAGCGGTGTCGTGGAAC3' (SEQ ID NO:5)

Amino acid sequence of heavy chain variable region and a portion of constant region encoded by SEQ ID NO:5:

*QC***EVQLVESGGGLVQPGGSLRLSCAAS**GFTFTNYWMS**WVRQAPGKGLEWVA**N
IQQDGSEKYYVDSVRG**RFTISRDNAKNSLYLQMNSLRAEDSAVYYCAR**WDY**WG
QGTLVTVSSA***STKGPSVFPLAPCSRSTSESTAALGCLVKDYFPEPVSGVVE* (SEQ ID NO:116)

Nucleotide sequence of light chain variable region and a portion of constant region:

5'CTTCTGGGGCTGCTAATGCTCTGGGTCCCTGGATCCAGTGGGGATATTGTGAT
GACCCAGACTCCACTCTCCTCAACTGTCATCCTTGGACAGCCGGCCTCCATCTCC
TGCAGGTCTAGTCAAAGCCTCGTACACAGTGATGGAAACACCTACTTGAATTGG
CTTCAGCAGAGGCCAGGCCAGCCTCCAAGACTCCTAATTTATATGATTTCTAAC
CGGTTCTCTGGGGTCCCAGACAGATTCAGTGGCAGTGGGGCAGGGACAGATTTC
ACACTGAAAATCAGCAGGGTGGAAGCTGAGGATGTCGGGGTTTATTACTGCAT
GCAAGCTACAGAATCTCCTCAGACGTTCGGCCAAGGGACCAAGGTGGAAATCA

AACGAACTGTGGCTGCACCATCTGTCTTCATCTTCCCGCCATCTGATGAGCAGTT
GAAATCTGGAAGGGCCTCTGTTG3' (SEQ ID NO:7)

Amino acid sequence of light chain variable region and a portion of constant region encoded by SEQ ID NO:7:

*LLGLLMLWVPGSSG***DIVMTQTPLSSTVILGQPASISC**RSSQSLVHSDGNTYLN**WLQ
QRPGQPPRLLIY**MISNRFS**GVPDRFSGSGAGTDFTLKISRVEAEDVGVYYC**MQA
TESPQT**FGQGTKVEIKR***TVAAPSVFIFPPSDEQLKSGRASV* (SEQ ID NO:117)

Anti-TIM-1 mAb 2.16

Nucleotide sequence of heavy chain variable region and a portion of constant :

5'GAGCAGTCGGGGGGAGGCGTGGTAAAGCCTGGGGGGTCTCTTAGACTCTCCT
GTGCAGCCTCTGGATTCACTTTCAGTAACGCCTGGATGACCTGGGTCCGCCAGG
CTCCAGGGAAGGGGCTGGAGTGGGTTGGCCGTATTAAAAGGAGAACTGATGGT
GGGACAACAGACTACGCTGCACCCGTGAAAGGCAGATTCACCATCTCAAGAGA
TGATTCAAAAAACACGCTGTATCTGCAAATGAACAACCTGAAAAACGAGGACA
CAGCCGTGTATTACTGTACCTCAGTCGATAATGACGTGGACTACTGGGGCCAGG
GAACCCTGGTCACCGTCTCCTCAGCTTCCACCAAGGGCCCATCCGTCTTCCCCCT
GGCGCCCTGCTCCAGGAGCACCTCCGAGAGCACAGCCGCCCTGGGCTGCCTGGT
CAAGGACTACTTCCCCGAACCGGTGACGGTGTCGTGGAACTCAGGCGCCCTGAC
CAGCGGCGTGCACACCTTCCCGGCTGTCCTACAGTCCTCAGGACTCT3' (SEQ ID
NO:9)

Amino acid sequence of heavy chain variable region and a portion of constant region encoded by SEQ ID NO:9:

**XXXXEQSGGGVVKPGGSLRLSCAAS**GFTFSNAWMT**WVRQAPGKGLEWVG**RIK
RRTDGGTTDYAAPV**KGRFTISRDDSKNTLYLQMNNLKNEDTAVYYCTS**VDNDV
DY**WGQGTLVTVSSA***STKGPSVFPLAPCSRSTSESTAALGCLVKDYFPEPVTVSWNSGA*
*LTSGVHTFPAVLQSSGL* (SEQ ID NO:118)

Nucleotide sequence of light chain variable region and a portion of constant region:

5'CTGACTCAGTCTCCACTCTCCCTGCCCGTCACCCCTGGAGAGCCGGCCTCCAT
CTCCTGCAGGTCTAGTCAGAGCCTCCTGCATAGTAATGGATACAACTATTTGGA
TTGGTACCTGCAGAAGCCAGGGCAGTCTCCACAGCTCCTGATCTATTTGGGTTC
TAATCGGGCCTCCGGGGTCCCTGACAGGTTCAGTGGCAGTGGATCAGGCACAG
ATTTTACACTGAAAATCAGCAGAGTGGAGGCTGAGGATATTGGTCTTTATTACT
GCATGCAAGCTCTACAAACTCCGCTCACTTTCGGCGGAGGGACCAAGGTGGAC
ATCAAACGAACTGTGGCTGCACCATCTGTCTTCATCTTCCCGCCATCTGATGAG
CAGTTGAAATCTGGAACTGCCTCTGTTGTGTGCCTGCTGAATAACTTCTATCCCA
GAGAGGCCAAAGTACAG3' (SEQ ID NO:11)

Amino acid sequence of light chain variable region and a portion of constant region encoded by SEQ ID NO:11:

**XXXLTQSPLSLPVTPGEPASISC**RSSQSLLHSNGYNYLD**WYLQKPGQSPQLLIY**L
GSNRAS**GVPDRFSGSGSGTDFTLKISRVEAEDIGLYYC**MQALQTPLT**FGGGTKV**
**DIKR***TVAAPSVFIFPPSDEQLKSGTASVVCLLNNFYPREAKVQ* (SEQ ID NO:119)

Anti-TIM-1 mAb 2.17

Nucleotide sequence of heavy chain variable region and a portion of constant region:

5'CAGGTGCAGCTGGAGCAGTCGGGGGGAGGCTTGGTACAGCCTGGGGGGTCCC
TGAGACTCTCCTGTGCAGCCTCTGGATTCACCTTCAGTACCTATAGCATGAACT
GGGTCCGCCAGGCTCCAGGGAAGGGGCTGGAGTGGGTTTCATACATTAGAAGT
AGTACTAGTACCATATACTATGCAGAGTCCCTGAAGGGCCGATTCACCATCTCC
AGCGACAATGCCAAGAATTCACTATATCTGCAAATGAACAGCCTGAGAGACGA
GGACACGGCTGTGTATTACTGTGCGCGGGACTTTGACTACTGGGGCCAGGGAAC
CCTGGTCACCGTCTCCTCAGCTTCCACCAAGGGCCCATCCGTCTTCCCCCTGGCG
CCCTGCTCCAGGAGCACCTCCGAGAGCACAGCCGCCCTGGGCTGCCTGGTCAAG
GACTACTTCCCCGAACCGGTGACGGTGTCGTGGAACTCAGGCGCCCTGACCAGC
GGCGTGCACACCTTCCCGGCTGTCCTACAGTCCTCAGGACTCTACTCCCTCAGC
A3' (SEQ ID NO:13)

Amino acid sequence of heavy chain variable region and a portion of constant region encoded by SEQ ID NO:13:

**QVQLEQSGGGLVQPGGSLRLSCAAS**GFTFSTYSMN**WVRQAPGKGLEWVS**YIRS
STSTIYYAESLKG**RFTISSDNAKNSLYLQMNSLRDEDTAVYYCAR**DFDY**WGQGT
LVTVSSA***STKGPSVFPLAPCSRSTSESTAALGCLVKDYFPEPVTVSWNSGALTSGVHTFP
AVLQSSGLYSLS* (SEQ ID NO:120)

Nucleotide sequence of light chain variable region and a portion of constant region:

5'GAAATCCAGCTGACTCAGTCTCCACTCTCCTCACCTGTCACCCTTGGACAGCC
GGCCTCCATCTCCTGCAGGTCTAGTCAAAGCCTCGTACACAGTGATGGAGACAC
CTACTTGAATTGGCTTCAGCAGAGGCCAGGCCAGCCTCCAAGACTCCTAATTTA
TAAGATTTCTACCCGGTTCTCTGGGGTCCCTGACAGATTCAGTGGCAGTGGGGC
AGGGACAGATTTCACACTGAAAATCAGCAGGGTGGAGACTGACGATGTCGGGA
TTTATTACTGCATGCAAACTACACAAATTCCTCAAATCACCTTCGGCCAAGGGA
CACGACTGGAGATTAAACGAACTGTGGCTGCACCATCTGTCTTCATCTTCCCGC
CATCTGATGAGCAGTTGAAATCTGGAACTGCCTCTGTTGTGTGCCTGCTGAATA
ACTTCTATCCCAGAGAGGCCAAAGTACAGTGGAAGGTGGATAACGCCCTCCAA
TCGGGTA3' (SEQ ID NO:15)

Amino acid sequence of light chain variable region and a portion of constant region encoded by SEQ ID NO:15:

**EIQLTQSPLSSPVTLGQPASISC**RSSQSLVHSDGDTYLN**WLQQRPGQPPRLLIY**KI
STRFS**GVPDRFSGSGAGTDFTLKISRVETDDVGIYYC**MQTTQIPQIT**FGQGTRLEI
KR***TVAAPSVFIFPPSDEQLKSGTASVVCLLNNFYPREAKVQWKVDNALQSG* (SEQ ID
NO:121)

Anti-TIM-1 mAb 2.24

Nucleotide sequence of heavy chain variable region and a portion of constant region:

5'CAGGTGCAGCTGGAGCAGTCGGGGGGAGGCGTGGTCCAGCCTGGGAGGTCCCTGAGACTCTCCTGTGCAGCGTCTGGATTCACCTTCAGTCGCTATGGCATGCACTGGGTCCGCCAGGCTCCAGGCAAGGGGCTGAAATGGGTGGCAGTTATATGGTATGATGGAAGTAATAAACTCTATGCAGACTCCGTGAAGGGCCGATTCACCATCTCCAGAGACAATTCCAAGAACACGCTGTATCTGCAAATGAACAGCCTGAGAGCCGAGGACACGGCTGTGTATTACTGTGCGAGAGATTACTATGATAATAGTAGACATCACTGGGGGTTTGACTACTGGGGCCAGGGAACCCTGGTCACCGTCTCCTCAGCTTCCACCAAGGGCCCATCCGTCTTCCCCCTGGCGCCCTGCTCCAGGAGCACCTCCGAGAGCACAGCCGCCCTGGGCTGCCTGGTCAAGGACTACTTCCCCGAACCGGTGACGGTGTCGTGGAACTCAGGCGCCCTGACCAGCGGCGTGCACACCTTCCCGGCTGTCCTACAGTCCTCAGGACTCTACTCCCTCAGCA (SEQ ID NO:17)

Amino acid sequence of heavy chain variable region and a portion of constant region encoded by SEQ ID NO:17:

**QVQLEQSGGGVVQPGRSLRLSCAAS**GFTFSRYGMH**WVRQAPGKGLKWVA**VIWYDGSNKLYADSVK**GRFTISRDNSKNTLYLQMNSLRAEDTAVYYCAR**DYYDNSRHHWGFDY**WGQGTLVTVSSA***STKGPSVFPLAPCSRSTSESTAALGCLVKDYFPEPVTVSWNSGALTSGVHTFPAVLQSSGLYSLS* (SEQ ID NO:122)

Nucleotide sequence of light chain variable region and a portion of constant region:

5'GACATCCAGCTGACCCAGTCTCCATCCTCCCTGTCTGCATCTGTAGGAGACAGAGTCACCATCACTTGCCGGGCAAGTCAGAGTATTTATAGTTATTTAAATTGGTATCAGCAGAAACCAGGGAAAGCCCCTAAGCTCCTGATCTATGCTGCATCCAGTTTGCAAAGTGGGGTCCCATCCAGGTTCAGTGGCAGTGGATCTGGGACAGATTTCACTCTCACCATCAGCAGTCTGCAACCTGAAGATTTTGCAACTTACTACTGTCAACAGAGTTACAGTACCCCTCCGACGTTCGGCCAAGGGACCAAGGTGGAAATCAAACGAACTGTGGCTGCACCATCTGTCTTCATCTTCCCGCCATCTGATGAGCAGTTGAAATCTGGAACTGCCTCTGTTGTGTGCCTGCTGAATAACTTCTATCCCAGAGAGGCCAAAGTACAGTGGAAGGTGGATAACGCCCTCCAATCGGGTA3' (SEQ ID NO:19)

Amino acid sequence of light chain variable region and a portion of constant region encoded by SEQ ID NO:19:

**DIQL/MT/LQSPSSLSASVGDRVTITC**RASQSIYSYLN**WYQQKPGKAPKLLIY**AASSLQ**SGVPSRFSGSGSGTDFTLTISSLQPEDFATYYC**QQSYSTPPT**FGQGTKVEIKR***TVAAPSVFIFPPSDEQLKSGTASVVCLLNNFYPREAKVQWKVDNALQSG* (SEQ ID NO:123)

Anti-TIM-1 mAb 2.45

Nucleotide sequence of heavy chain variable region and a portion of constant region:

5'CAGTCGGGGGGAGGCTTGGTAAAGCCTGGGGGGTCCCTTAGACTCTCCTGTGCAGCCTCTGGATTCACTTTCAGTAACGCCTGGATGACCTGGGTCCGCCAGGCTCCAGGGAAGGGGCTGGAGTGGGTTGGCCGTATTAAAAGGAAAACTGATGGTGGGACAACAGACTACGCTGCACCCGTGAAAGGCAGATTCACCATCTCAAGAGATGATTCAGAAAACACGCTGTATCTGCAAATGAACAGCCTGGAAACCGAGGACACAGCCGTGTATTACTGTACCACAGTCGATAACAGTGGTGACTACTGGGGCCAGGGAACCCTGGTCACCGTCTCCTCAGCTTCCACCAAGGGCCCATCCGTCTTCCCCCTGGCGCCCTGCTCCAGGAGCACCTCCGAGAGCACAGCCGCCCTGGGCTGCCTGGTCAAGGACTACTTCCCCGAACCGGTGACGGTGTCGTGGAACTCAGGCGCCCTGACCAGCGGCGTGCACACCTTCCCGGCTGTCCTACAGTCCTCAGGACTCTCT3' (SEQ ID NO:21)

Amino acid sequence of heavy chain variable region and a portion of constant region encoded by SEQ ID NO:21:

**XXXXXQSGGGLVKPGGSLRLSCAAS**GFTFSNAWMT**WVRQAPGKGLEWVG**RIKRKTDGGTTDYAAPV**KGRFTISRDDSENTLYLQMNSLETEDTAVYYCTT**VDNSGDY**WGQGTLVTVSSA***STKGPSVFPLAPCSRSTSESTAALGCLVKDYFPEPVTVSWNSGALTSGVHTFPAVLQSSGLS* (SEQ ID NO:124)

Nucleotide sequence of light chain variable region and a portion of constant region:

5'ACTCAGTCTCCACTCTCCCTGCCCGTCACCCCTGGAGAGCCGGCCTCCATCTCCTGCAGGTCTAGTCAGAGCCTCCTGCATAGTAATGGATACAACTATTTGGATTGGTACCTGCAGAAGCCAGGGCAGTCTCCACAGCTCCTGATCTATTTGGGTTCTAATCGGGCCTCCGGGGTCCCTGACAGGTTCAGTGGCAGTGGATCAGGCACAGATTTTACACTGAAAATCAGCAGAGTGGAGGCTGAGGATGTTGGGGTTTATTACTGCATGCAAGCTCTACAAACTCCGCTCACTTTCGGCGGAGGGACCAAGGTGGAGATCAAACGAACTGTGGCTGCACCATCTGTCTTCATCTTCCCGCCATCTGATGAGCAGTTGAAATCTGGAACTGCCTCTGTTGTGTGCCTGCTGAATAACTTCTATCCCAGAGAGGCCAAAGTACAGTGGAAGGTGGATAACGCCCTCA3' (SEQ ID NO:23)

Amino acid sequence of light chain variable region and a portion of constant region encoded by SEQ ID NO:23:

**XXXXTQSPLSLPVTPGEPASISC**RSSQSLLHSNGYNYLD**WYLQKPGQSPQLLIY**LGSNRAS**GVPDRFSGSGSGTDFTLKISRVEAEDVGVYYC**MQALQTPLT**FGGGTKVEIKR***TVAAPSVFIFPPSDEQLKSGTASVVCLLNNFYPREAKVQWKVDNAL* (SEQ ID NO:125)

Anti-TIM-1 mAb 2.54

Nucleotide sequence of heavy chain variable region and a portion of constant region:

5'CAGGTGCAGCTGGAGCAGTCGGGGGGAGGCGTGGTCCAGCCTGGGAGGTCCC
TGAGACTCTCCTGTGCAGCGTCTGGATTCACCTTCACTAACTATGGCTTGCACTG
GGTCCGCCAGGCTCCAGGCAAGGGGCTGGATTGGGTGGCAGTTATATGGTATG
ATGGAAGTCATAAATTCTATGCAGACTCCGTGAAGGGCCGATTCACCATCTCCA
GAGACAATTCCAAGAACACGCTCTTTCTGCAAATGAACAGCCTGAGAGCCGAG
GACACGGCTGTGTATTACTGTACGCGAGATCTTGACTACTGGGGCCAGGGAACC
CTGGTCACCGTCTCCTCAGCTTCCACCAAGGGCCCATCCGTCTTCCCCCTGGCGC
CCTGCTCCAGGAGCACCTCCGAGAGCACAGCCGCCCTGGGCTGCCTGGTCAAG
GACTACTTCCCCGAACCGGTGACGGTGTCGTGGAACTCAGGCGCCCTGACCAGC
GGCGTGCACACCTTCCCGGCTGTCCTACAGTCCTCAGGACTCTACTCCCTCAGC3
' (SEQ ID NO:25)

Amino acid sequence of heavy chain variable region and a portion of constant region encoded by SEQ ID NO:25:

**QVQLEQSGGGVVQPGRSLRLSCAAS**GFTFTNYGLH**WVRQAPGKGLDWVA**VIW
YDGSHKFYADSVK**GRFTISRDNSKNTLFLQMNSLRAEDTAVYYCTR**DLDY**WGQ
GTLVTVSSA***STKGPSVFPLAPCSRSTSESTAALGCLVKDYFPEPVTVSWNSGALTSGVHT
FPAVLQSSGLYSLS* (SEQ ID NO:126)

Nucleotide sequence of light chain variable region and a portion of constant region:

5'GAAACGCAGCTGACGCAGTCTCCAGGCACCCTGTCTTTGTCTCCAGGGGAAA
GAGTCACCCTCTCCTGCAGGGCCAGTCAGAGTGTTAGCAACAACTACTTAGCCT
GGTACCAGCAGAAACCTGGCCAGGCTCCCAGGCTCCTCATCTATGGTGCATCCA
GCAGGGCCACTGGCATCCCAGACAGGTTCAGTGGCAGTGGGTCTGGGACAGAC
TTCACTCTCACCATCAGCAGACTGGAGCCTGAAGATTGTGCAGAGTGTTACTGT
CAGCAATATGGTAGCTCACTCCCGCTCACTTTCGGCGGAGGGACCAAGGTGGA
GATCAAACGAACTGTGGCTGCACCATCTGTCTTCATCTTCCCGCCATCTGATGA
GCAGTTGAAATCTGGAACTGCCTCTGTTGTGTGCCTGCTGAATAACTTCTATCCC
AGAGAGGCCAAAGTACAGTGGAAGGTGGATAACGCCCTCCAATCGGGTA3'
(SEQ ID NO:27)

Amino acid sequence of light chain variable region and a portion of constant region encoded by SEQ ID NO:27:

**ETQLTQSPGTLSLSPGERVTLSC**RASQSVSNNYLA**WYQQKPGQAPRLLIY**GASS
RAT**GIPDRFSGSGSGTDFTLTISRLEPEDCAECYC**QQYGSSLPLT**FGGGTKVEIK
R***TVAAPSVFIFPPSDEQLKSGTASVVCLLNNFYPREAKVQWEGGITPSNRV* (SEQ ID
NO:127)

Anti-TIM-1 mAb 2.56

Nucleotide sequence of heavy chain variable region and a portion of constant region:

5'GTCCAGTGTCAGGTGCAGCTGGTGGAGTCTGGGGGAGGCGTGGTCCAGCCTG
GGAGGTCCCTGAGACTCTCCTGTGCAGCGTCTGGATTCACCTTCAGTAGCTATG
GCATGCACTGGGTCCGCCAGGCTCCAGGCAAGGGGCTGGAGTGGGTGGCAGTT
ATATGGTATGATGGAAGTCATAAATACTATGCAGACTCCGTGAAGGGCCGATTC
ACCATCTCCAGAGACAATTCCAAGAACACGCTGTATCTGCAAATGAACAGCCTG
AGAGCCGAGGACACGGCTGTGTATTACTCTGCGAGAGATTACTATGATACGAGT
CGGCATCACTGGGGGTTTGACTGCTGGGGCCAGGGAACCCTGGTCACCGTCTCC
TCTGCTTCCACCAAGGGCCCATCCGTCTTCCCCCTGGCGCCCTGCTCCAGGAGC
ACCTCCGAGAGCACAGCCGCCCTGGGCTGCCTGGTCAAGGACTACTTCCCCGAA
CCGGTGACGGTGTCGTGGAACTCAGGCGCCCTGACCAGCGGCGTGCACACCTTC
CCGGC3' (SEQ ID NO:29)

Amino acid sequence of heavy chain variable region and a portion of constant region encoded by SEQ ID NO: 29:

VQCQVQLVESGGGVVQPGRSLRLSCAASGFTFSSYGMHWVRQAPGKGLEWVA
VIWYDGSHKY/LYA/TDSVKGRFTISRDNSKNTLYLQMNSLRAEDTAVYYSARDY
YDTSRHHWGFDCWGQGTLVTVSSASTKGPSVFPLAPCSRSTSESTAALGCLVKDYFP
EPVTVSWNSGALTSGVHTFP (SEQ ID NO:128)

Nucleotide sequence of light chain variable region and a portion of constant region:

5'CAGCTCCTGGGGCTGCTAATGCTCTGGGTCCCTGGATCCAGTGAGGAAATTGT
GATGACCCAGACTCCACTCTCCCTGCCCGTCACCCCTGGAGAGCCGGCCTCCAT
CTCCTGCAGGTCTAGTCAGAGCCTCTTGGATAGTGAAGATGGAAACACCTATTT
GGACTGGTACCTGCAGAAGCCAGGGCAGTCTCCACAGCTCCTGATCTATACGCT
TTCCCATCGGGCCTCTGGAGTCCCAGACAGGTTCAGTGGCAGTGGGTCAGGCAC
TGATTTCACACTGAAAATCAGCAGGGTGGAGGCTGAGGATGTTGGAGTTTATTG
CTGCATGCAACGTGTAGAGTTTCCTATCACCTTCGGCCAAGGGACACGACTGGA
GATTAAACGAACTGTGGCTGCACCATCTGTCTTCATCTTCCCGCCATCTGATGA
GCAGTTGAAATCTGGAACTGCCTCTGTTGTGTGCCTGCTGAATAACTTCTATCCC
AGAGAGGCCAAAGTACAGTGGAAGGTGGATAACGC3' (SEQ ID NO:31)

Amino acid sequence of light chain variable region and a portion of constant region encoded by SEQ ID NO:31:

QLLGLLMLWVPGSSEEIVMTQTPLSLPVTPGEPASISCRSSQSLLDSEDGNTYLDW
YLQKPGQSPQLLIYTLSHRASGVPDRFSGSGSGTDFTLKISRVEAEDVGVYCCM
QRVEFPITFGQGTRLEIKRTVAAPSVFIFPPSDEQLKSGTASVVCLLNNFYPREAKVQW
KVDN (SEQ ID NO:129)

Anti-TIM-1 mAb 2.59

Nucleotide sequence of heavy chain variable region and a portion of constant region:

5'CAGTCGGGCCCAAGACTGGTGAAGCCTTCACAGACCCTGTCCCTCACCTGCAC
TGTCTCTGGTGGCTCCATCAGTAGTGATGGTTACTACTGGAGCTGGATCCGCCA
GCACCCAGGGAAGGGCCTGGAGTGGATTGGGTACATCTATTACAGTGGGAGCA
CCTTCTACAACCCGTCCCTCAAGAGTCGAGTTGCCATATCAGTGGACACGTCTA
AGAACCAGTTCTCCCTGAAGCTGAGCTCTGTGACTGCCGCGGACACGGCCGTGT
ATTACTGTGCGAGAGAATCCCCTCATAGCAGCAACTGGTACTCGGGCTTTGACT
GCTGGGGCCAGGGAACCCTGGTCACCGTCTCCTCAGCTTCCACCAAGGGCCCAT
CCGTCTTCCCCCTGGCGCCCTGCTCCAGGAGCACCTCCGAGAGCACAGCCGCCC
TGGGCTGCCTGGTCAAGGACTACTTTCCCCGAACCGGTGACGGTGTCGTGGAAC
TCAGGCGCCCTGACCAGCGGCGTGCACACCTTCCCGGCTGTCCTACAGTCCTCA
GGACTCTCT3' (SEQ ID NO:33)

Amino acid sequence of heavy chain variable region and a portion of constant region encoded by SEQ ID NO:33:

**XXXXXQSGPRLVKPSQTLSLTCTVS**GGSISSDGYYWS**WIRQHPGKGLEWIG**YIY
YSGSTFYNPSLKS**RVAISVDTSKNQFSLKLSSVTAADTAVYYCAR**ESPHSSNWYS
GFDC**WGQGTLVTVSSA***STKGPSVFPLAPCSRSTSESTAALGCLVKDYFPRTGDGVVEL
RRPDQRRAHLPGCPTVLRTL* (SEQ ID NO:130)

Nucleotide sequence of light chain variable region and a portion of constant region:

5'ACTCAGTCTCCAGACTTTCAGTCTGTGACTCCAAAGGAGAAAGTCACCATCAC
CTGCCGGGCCAGTCAGAGCATTGGTAGTAGGTTACACTGGTACCAGCAGAAAC
CAGATCAGTCTCCAAAGCTCCTCATCAAGTATGCTTCCCAGTCCTTCTCAGGGG
TCCCCTCGAGGTTCAGTGGCAGTGGATCTGGGACAGATTTCACCCTCACCATCA
ATAGCCTGGAAGCTGAAGATGCTGCAACGTATTACTGTCATCAGAGTAGTAATT
TACCATTCACTTTCGGCCCTGGGACCAAAGTGGATATCAAACGAACTGTGGCTG
CACCATCTGTCTTCATCTTCCCGCCATCTGATGAGCAGTTGAAATCTGGAACTGC
CTCTGTTGTGTGCCTGCTGAATAACTTCTATCCCAGAGAGGCCAAAGTACAGTG
GAAGGTGGATAACGCCCTC3' (SEQ ID NO:35)

Amino acid sequence of light chain variable region and a portion of constant region encoded by SEQ ID NO:35:

**XXXXTQSPDFQSVTPKEKVTITC**RASQSIGSRLH**WYQQKPDQSPKLLIK**YASQSF
S**GVPSRFSGSGSGTDFTLTINSLEAEDAATYYC**HQSSNLPFT**FGPGTKVDIKR***TVA
APSVFIFPPSDEQLKSGTASVVCLLNNFYPREAKVQWKVDNAL* (SEQ ID NO:131)

Anti-TIM-1 mAb 2.61

Nucleotide sequence of heavy chain variable region and a portion of constant region:

5'CAGGTGCAGCTGGTGGAGGCTGGGGGAGGCGTGGTCCAGCCTGGGAGGTCCC
TGAGACTCTCCTGTGCAGCGTCTGGATTCACCTTCAGAAGCTATGGCATGCACT
GGGTCCGCCAGGCTCCAGGCAAGGGGCTGAAATGGGTGGCAGTTATATGGTAT
GATGGAAGTAATAAATACTATACAGACTCCGTGAAGGGCCGATTCACCATCTCC
AGAGACAATTCCAAGAACACGCTGTATCTGCAAATGAACAGCCTGAGAGCCGA
GGACACGGCTGTGTATTACTGTGTGAGAGATTACTATGATAATAGTAGACATCA
CTGGGGGTTTGACTACTGGGGCCAGGGAACCCTGGTCACCGTCTCCTCAGCTTC
CACCAAGGGCCCATCCGTCTTCCCCCTGGCGCCCTGCTCCAGGAGCACCTCCGA
GAGCACAGCCGCCCTGGGCTGCCTGGTCAAGGACTACTTCCCCGAACCGGTGAC
GGTGTCGTGGAACTCAGGCGCCCTGACCAGGCGGCGTGCACACCTTCCCGGC3'
(SEQ ID NO:37)

Amino acid sequence of heavy chain variable region and a portion of constant region encoded by SEQ ID NO:37:

**QVQLVE/QAGGGVVQPGRSLRLSCAAS**GFTFRSYGMH**WVRQAPGKGLKWVA**V
IWYDGSNKY/LYTDSVK**GRFTISRDNSKNTLYLQMNSLRAEDTAVYYCVR**DYYD
NSRHHWGFDY**WGQGTLVTVSSA***STKGPSVFPLAPCSRSTSESTAALGCLVKDYFPEP*
*VTVSWNSGALTRRRAHLPG* (SEQ ID NO:132)

Nucleotide sequence of light chain variable region and a portion of constant region:

5'GACATCCAGATGACCCAGTCTCCATCCTCCCGGTGTGCATCCGTAGGAGACAG
AGTCACCATCACTTGCCGGGCAAGTCAGGGCATCAGAAATGATTTAGCTTGGTA
TCAGCAGAAACCAGGGAAAGCCCCTAAGCGCCTGATCTATGCTGCATCCAGTTT
GCAAAGTGGGGTCCCATCAAGGTTCAGCGGCAGTAGATCTGGGACAGAATTCA
CTCTCACAATCAGCAGCCTGCAGCCTGAAGATTTTGCAGCTTATTACTGTCTCCA
GCATAATAGTTACCCTCCCAGTTTTGGCCAGGGGACCAAGCTGGAGATCAAACG
AACTGTGGCTGCACCATCTGTCTTCATCTTCCCGCCATCTGATGAGCAGTTGAA
ATCTGGAACTGCTAGCGTTGTGTGCCTGCTGAATAACTTCTATCCCAGAGAGGC
CAAAGTACAGTGGAAGGTGGATAACGCCCTCCAATCGGG3' (SEQ ID NO:39)

Amino acid sequence of light chain variable region and a portion of constant region encoded by SEQ ID NO:39:

**DIQMTQSPSSRCASVGDRVTITC**RASQGIRNDLA**WYQQKPGKAPKRLIY**AASSL
QS**GVPSRFSGSRSGTEFTLTISSLQPEDFAAYYC**LQHNSYPPS**FGQGTKLEIKR***TV*
*AAPSVFIFPPSDEQLKSGTASVVCLLNNFYPREAKVQWKVDNALQS* (SEQ ID NO:133)

Anti-TIM-1 mAb 2.70

Nucleotide sequence of heavy chain variable region and a portion of constant region:

5'CATGTGCAGGTGCAGCTGGTGGAGTCTGGGGGAGGCGTGGTCCAGCCTGGGA
GGTCCCTGAGACTCTCCTGTGCAGCGTCTGGATTCATCTTCAGTCGCTATGGCAT
GCACTGGGTCCGCCAGGCTCCAGGCAAGGGGCTGAAATGGGTGGCAGTTATAT
GGTATGATGGAAGTAATAAACTCTATGCAGACTCCGTGAAGGGCCGATTCACC
ATCTCCAGAGACAATTCCAAGAACACGCTGTATCTGCAAATGAACAGCCTGAG
AGCCGAGGACACGGCTGTGTATTACTGTGCGAGAGATTACTATGATAATAGTAG
ACATCACTGGGGGTTTGACTACTGGGGCCAGGGAACCCTGGTCACCGTCTCCTC
AGCTTCCACCAAGGGCCCATCCGTCTTCCCCCTGGCGCCCTGCTCCAGGAGCAC
CTCCGAGAGCACAGCCGCCCTGGGCTGCCTGGTCAAGGACTACTTCCCCGAACC
GGTGACGGTGTCGTGGAACTCAGGCGCCCTGA3' (SEQ ID NO:41)

Amino acid sequence of heavy chain variable region and a portion of constant region encoded by SEQ ID NO:41:

HVQVQLVESGGGVVQPGRSLRLSCAASGFIFSRYGMHWVRQAPGKGLKWVAV
IWYDGSNKLYADSVKGRFTISRDNSKNTLYLQMNSLRAEDTAVYYCARDYYDN
SRHHWGFDYWGQGTLVTVSSASTKGPSVFPLAPCSRSTSESTAALGCLVKDYFPEPVT
VSWNSGAL (SEQ ID NO:134)

Nucleotide sequence of light chain variable region and a portion of constant region:

5'TCAGCTCCTGGGGCTGCTAATGCTCTGGGTCCCTGGATCAGTGAGGATATTGT
GATGACCCAGACTCCACTCTCCCTGCCCGTCACCCCTGGAGAGCCGGCCTCCAT
CTCCTGCAGGTCTAGTCGGAGCCTCTTGGATAGTGATGATGGAAACACCTATTT
GGACTGGTACCTGCAGAAGCCAGGGCAGTCTCCACAGCTCCTGATCTACACGCT
TTCCTATCGGGCCTCTGGAGTCCCAGACAGGTTCAGTGGCAGTGGGTCAGGCAC
TGATTTCACACTGAAAATCAGCAGGGTGGAGGCTGAGGATGTTGGAGTTTATTA
CTGCATGCAACGTGTAGAGTTTCCTATCACCTTCGGCCAAGGGACACGACTGGA
GATTAAACGAACTGTGGCTGCACCATCTGTCTTCATCTTCCCGCCATCTGATGA
GCAGTTGAAATCTGGAACTGCCTCTGTTGTGTGCCTGCTGAATAACTTCTATCCC
AGAGAGGCCAAAGTACAGTGGAAGGTGGATAACGCCT3' (SEQ ID NO:43)

Amino acid sequence of light chain variable region and a portion of constant region encoded by SEQ ID NO:43:

SAPGAANALGPWISEDIVMTQTPLSLPVTPGEPASISCRSSRSLLDSDDGNTYLDWY
LQKPGQSPQLLIYTLSYRASGVPDRFSGSGSGTDFTLKISRVEAEDVGVYYCMQ
RVEFPITFGQGTRLEIKRTVAAPSVFIFPPSDEQLKSGTASVVCLLNNFYPREAKVQWK
VDNA (SEQ ID NO:135)

Anti-TIM-1 mAb 2.70.2

Nucleotide sequence of heavy chain variable region and a portion of constant region:

5'CGGCCGCCTATTTACCCAGAGACAGGGAGAGGCTCTTCTGTGTGTAGTGGTTG
TGCAGAGCCTCATGCATCACGGAGCATGAGAAGACATTCCCCTCCTGCCACCTG
CTCTTGTCCACGGTTAGCCTGCTGTAGAGGAAGAAGGAGCCGTCGGAGTCCAGC
ACGGGAGGCGTGGTCTTGTAGTTGTTCTCCGGCTGCCCATTGCTCTCCCACTCCA
CGGCGATGTCGCTGGGGTAGAAGCCTTTGACCAGGCAGGTCAGGCTGACCTGG
TTCTTGGTCATCTCCTCCTGGGATGGGGGCAGGGTGTACACCTGTGGCTCTCGG
GGCTGCCCTTTGGCTTTGGAGATGGTTTTCTCGATGGAGGACGGGAGGCCTTTG
TTGGAGACCTTGCACTTGTACTCCTTGCCGTTCAGCCAGTCCTGGTGCAGGACG
GTGAGGACGCTGACCACACGGTACGTGCTGTTGAACTGCTCCTCCCGCGGCTTT
GTCTTGGCATTATGCACCTCCACGCCATCCACGTACCAGTTGAACTGGACCTCG
GGGTCTTCCTGGCTCACGTCCACCACCACGCACGTGACCTCAGGGGTCCGGGAG
ATCATGAGAGTGTCCTTGGGTTTTGGGGGGAACAGGAAGACTGATGGTCCCCCC
AGGAACTCAGGTGCTGGGCATGATGGGCATGGGGGACCATATTTGGACTCAAC
TCTCTTGTCCACCTTGGTGTTGCTGGGCTTGTGATCTACGTTGCAGGTGTAGGTC
TTCGTGCCCAAGCTGCTGGAGGGCACGGTCACCACGCTGCTGAGGGAGTAGAG
TCCTGAGGACTGTAGGACAGCCGGGAAGGTGTGCACGCCGCTGGTCAGGGCGC
CTGAGTTCCACGACACCGTCACCGGTTCGGGGAAGTAGTCCTTGACCAGGCAGC
CCAGGGCGGCTGTGCTCTCGGAGGTGCTCCTGGAGCAGGGCGCCAGGGGGAAG
ACGGATGGGCCCTTGGTGGAAGCTGAGGAGACGGTGACCAGGGTTCCCTGGCC
CCAGTAGTCAAACCCCCAGTGATGTCTACTATTATCATAGTAATCTCTCGCACA
GTAATACACAGCCGTGTCCTCGGCTCTCAGGCTGTTCATTTGCAGATACAGCGT
GTTCTTGGAATTGTCTCTGGAGATGGTGAATCGGCCCTTCACGGAGTCTGCATA
GAGTTTATTACTTCCATCATACCATATAACTGCCACCCATTTCAGCCCCTTGCCT
GGAGCCTGGCGGACCCAGTGCATGCCATAGCGACTGAAGATGAATCCAGACGC
TGCACAGGAGAGTCTCAGGGACCTCCCAGGCTGGACCACGCCTCCCCCAGACTC
CACCAGCTGCACCTGACACTGGACACCTTTTAAAATAGCCACAAGAAAAAGCC
AGCTCAGCCCAAACTCCATGGTGGTCGACT3' (SEQ ID NO:136)

Amino acid sequence of heavy chain variable region and a portion of constant region encoded by SEQ ID NO: 136:

*MEFGLSWLFLVAILKGVQC***QVQLVESGGGVVQPGRSLRLSCAAS**GFIFSRYGMH**W**
**VRQAPGKGLKWVA**VIWYDGSNKLYADSVK**GRFTISRDNSKNTLYLQMNSLRA**
**EDTAVYYCAR**DYYDNSRHHWGFDY**WGQGTLVTVSSA***STKGPSVFPLAPCSRSTSE*
*STAALGCLVKDYFPEPVTVSWNSGALTSGVHTFPAVLQSSGLYSLSSVVTVPSSSLGTKTYT*

*CNVDHKPSNTKVDKRVESKYGPPCPSCPAPEFLGGPSVFLFPPKPKDTLMISRTPEVTC*
*VVVDVSQEDPEVQFNWYVDGVEVHNAKTKPREEQFNSTYRVVSVLTVLHQDWLNGKEY*
*KCKVSNKGLPSSIEKTISKAKGQPREPQVYTLPPSQEEMTKNQVSLTCLVKGFYPSDIAVE*
*WESNGQPENNYKTTPPVLDSDGSFFLYSRLTVDKSRWQEGNVFSCSVMHEALHNHYTQ*
*KSLSLSLGK* (SEQ ID NO:137)

Nucleotide sequence of light chain variable region and a portion of constant region:

5'AGTCGACCACCATGGAAACCCCAGCGCAGCTTCTCTTCCTCCTGCTACTCTGG CTCCCAGATACCACCGGAGATATTGTGATGACCCAGACTCCACTCTCCCTGCCC GTCACCCCTGGAGAGCCGGCCTCCATCTCCTGCAGGTCTAGTCGGAGCCTCTTG GATAGTGATGATGGAAACACCTATTTGGACTGGTACCTGCAGAAGCCAGGGCA GTCTCCACAGCTCCTGATCTACACGCTTTCCTATCGGGCCTCTGGAGTCCCAGAC AGGTTCAGTGGCAGTGGGTCAGGCACTGATTTCACACTGAAAATCAGCAGGGT GGAGGCTGAGGATGTTGGAGTTTATTACTGCATGCAACGTGTAGAGTTTCCTAT CACCTTCGGCCAAGGGACACGACTGGAGATTAAACGAACTGTGGCTGCACCAT CTGTCTTCATCTTCCCGCCATCTGATGAGCAGTTGAAATCTGGAACTGCCTCTGT TGTGTGCCTGCTGAATAACTTCTATCCCAGAGAGGCCAAAGTACAGTGGAAGGT GGATAACGCCCTCCAATCGGGTAACTCCCAGGAGAGTGTCACAGAGCAGGACA GCAAGGACAGCACCTACAGCCTCAGCAGCACCCTGACGCTGAGCAAAGCAGAC TACGAGAAACACAAAGTCTACGCCTGCGAAGTCACCCATCAGGGCCTGAGCTC GCCCGTCACAAAGAGCTTCAACAGGGGAGAGTGTTAGGCGGCCG3' (SEQ ID NO:138)

Amino acid sequence of light chain variable region and portion constant region by SEQ ID NO:138:

*METPAQLLFLLLLWLPDTTG***DIVMTQTPLSLPVTPGEPASISC**<u>RSSRSLLDSDDGNTYLD</u>**WYLQKPGQSPQLLIY**<u>TLSYRAS</u>**GVPDRFSGSGSGTDFTLKISRVEAEDVGVYYC**<u>MQRVEFPIT</u>**FGQGTRLEIKR***TVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC* (SEQ ID NO:139)

<u>Anti-TIM-1 mAb 2.76</u>

Nucleotide sequence of heavy chain variable region and a portion of constant region:

5'GAGCAGTCGGGGGGCGGCGTGGTCCAGCCTGGGAGGTCCCTGAGACTCTCCT GTGCAGCGTCTGGATTCACCTTCAGTAGCTATGGCATGTACTGGGTCCGCCAGG CTCCAGGCAAGGGGCTGGAGTGGGTGGCAGTTATATGGTATGATGGAAGCAAT AAATACTATGCAGACTCCGTGAAGGGCCGATTCACCATCTCCAGAGACAATTCC AAGAACACGCTGTATCTGCAAATGAACAGCCTGAGAGCCGAGGACACGGCTGT GTATTACTGTGCGAGGGATTTCTATGATAGTAGTCGTTACCACTACGGTATGGA CGTCTGGGGCCAAGGGACCACGGTCACCGTCTCCTCAGCTTCCACCAAGGGCCC ATCCGTCTTCCCCCTGGCGCCCTGCTCCAGGAGCACCTCCGAGAGCACAGCCGC

CCTGGGCTGCCTGGTCAAGGACTACTTCCCCGAACCGGTGACGGTGTCGTGGAA CTCAGGCGCCCTGACCAGCGGCGTGCACACCTTCCCGGCTGTCCTACAGTCCTC AGGACTCTCT3' (SEQ ID NO:45)

Amino acid sequence of heavy chain variable region and a portion of constant region encoded by SEQ ID NO:45:

**XXXXEQSGGGVVQPGRSLRLSCAAS**<u>GFTFSSYGMY</u>**WVRQAPGKGLEWVA**<u>VIWYDGSNKYYADSVK</u>**GRFTISRDNSKNTLYLQMNSLRAEDTAVYYCAR**<u>DFYDSSRYHYGMDV</u>**WGQGTTVTVSSA***STKGPSVFPLAPCSRSTSESTAALGCLVKDYFPEPVTVSWNSGALTSGVHTFPAVLQSSGLS* (SEQ ID NO:140)

Nucleotide sequence of light chain variable region and a portion of constant region:

**5'ACTCAGTGTCCACTCTCCCTGCCCGTCACCCCTGGAGAGCCGGCCTCCATCTC
CTGCAGGTCTAGTCAGAGCCTCTTGGATAGTGATGATGGAAACACCTATTTGGA
CTGGTACCTGCAGAAGCCAGGGCAGTCTCCACAGCTCCTGATCTATACGGTTTC
CTATCGGGCCTCTGGAGTCCCAGACAGGTTCAGTGGCAGTGGGTCAGGCACTGA
TTTCACACTGAAAATCAGCAGGGTGGAGGCTGAGGATGTTGGAGTTTATTACTG
CATGCAACGTATAGAGTTTCCGATCACCTTCGGCCAAGGGACCCGACTGGAGAT
TAAACGAACTGTGGCTGCACCATCTGTCTTCATCTTCCCGCCATCTGATGAGCA
GTTGAAATCTGGAACTGCCTCTGTTGTGTGCCTGCTGAATAA3' (SEQ ID NO:47)**

Amino acid sequence of light chain variable region and a portion of constant region encoded by SEQ ID NO:47:

**XXXXTQCPLSLPVTPGEPASISC<u>RSSQSLLDSDDGNTYLD</u>WYLQKPGQSPQLLIY
<u>TVSYRAS</u>GVPDRFSGSGSGTDFTLKISRVEAEDVGVYYC<u>MQRIEFPIT</u>FGQGTRL
EIKR*TVAAPSVFIFPPSDEQLKSGTASVVCLLN*** (SEQ ID NO:141)

Example 20

In Vivo Studies Demonstrating Usefulness of Anti-Tim-1 Antibodies For the Treatment of Ovarian Cancer

An *in vivo* study was performed to assess the potency and therapeutic efficacy of the antibody-drug conjugate, CR014-vcMMAE, against an established human IGROV-1 ovarian xenograft in athymic mice.

**Materials and Methods:**

Test Animals: Five- to 6-week old athymic mice (CD-1 *nu/nu* females), used for human tumor xenografts, were obtained from Charles Rivers Laboratories (Wilmington, DE). Animals were housed in specific pathogen-free conditions, according to the guidelines of the Association for Assessment and Accreditation of Laboratory Animal Care International (AAALAC International). Test animals were provided pelleted food and water *ad libitum* and kept in a room with conditioned ventilation (HVAC), temperature (22° ± 2°C), relative humidity (53% ± 15%), and photoperiod (12 hr). All studies were carried out with approved institutional animal care and use protocols. Contract Research Organizations. Experiments *in vivo* were conducted at Southern Research Institute (Birmingham, AL).

Human Ovarian Carcinoma Xenograft Model. The tumor inhibitory activity of the CR014-MMAE immunoconjugate was measured in an anti-tumor xenograft model using athymic mice, according to published methods (Geran RI, Greenberg NH, Macdonald MM, Schumacher AM and Abbott BJ (1972) Protocols for screening chemical agents and natural products against animal tumors and other biological systems. *Cancer Chemother Rep* **3**:1-104).

Briefly, test animals were implanted subcutaneously by trocar with small fragments of the IGROV1 carcinoma (30-60 mg) excised from athymic mouse tumor donors. When tumors became established (day 20, 95 mg), the animals were pair-matched into groups (n= 6 mice/group), and treatment was administered by intravenous injection (tail vein).

The IGROV1 ovarian carcinoma was derived from a 47 yr. old woman in 1985, and was obtained from the American Type Culture Collection. The effects of treatment were monitored by repetitive tumor measurements across 2 diameters with Vernier calipers; tumor size (in mg) was calculated using a standard formula, $(W^2 \times L)/2$, assuming a specific gravity of 1.0. Tumor size and body weights were assessed twice weekly. Mice were examined daily, however, and moribund animals were humanely euthanized if clinical indications of excessive pain or distress were noted (i.e., prostration, hunched posture, paralysis/paresis, distended abdomen, ulcerations, abscesses, seizures, and/or hemorrhages). Animals with tumors exceeding 2,000 mg were removed from the study and euthanized humanely.

Xenograft studies in the athymic mouse have been shown to effectively demonstrate anti-tumor effects for a variety of agents which have been found subsequently to have activity against clinical cancer Johnson JI, Decker S, Zaharevitz D, Rubinstein LV, Venditti JM, Schepartz S, Kalyandrug S, Christian M, Arbuck S, Hollingshead M and Sausville EA (2001) Relationships between drug activity in NCI preclinical in vitro and in vivo models and early clinical trials. Br J Cancer 84:1424-1431.

**Results:**

Anti-Tumor Effects *In Vivo* vs. IGROV1. Based on the potency and cytotoxicity of CR014-vcMMAE against TIM-1-expressing cells *in vitro,* the anti-tumor effects were examined *in vivo.*

The effects of vehicle control groups, reference agents and the CR014-vcMMAE immunoconjugate on the growth of subcutaneous human IGROV1 ovarian carcinoma are shown in Figure 20.

Tumors in animals treated with saline or PBS grew progressively until the tumor mass reached 2,000 mg at which time the animals were removed from the study and euthanized humanely. IGROV1 tumors have a high "take" rate in immunocompromised hosts (93 %) and a very low rate of spontaneous regression (0 %) (Dykes DJ, Abbott BJ, Mayo JG, Harrison Jr. SD, Laster Jr WR, Simpson-Herren L and Griswold Jr. DP (1992) Development of human tumor xenograft models for in vivo evaluation of new antitumor drugs, in Immunodeficient mice in Oncology, vol. 42 (Fiebig HH and Berger DPe eds) pp 1-22, Contrib. Oncol. Basel, Karger).

Two known anti-tumor reference agents, vinblastine sulfate (i.v., 1.7 mg/kg, q4d X4) and paclitaxel (i.v., 24 mg/kg, q2d X4) were used in this study; these agents were administered at the maximum tolerated dose (MTD) determined in prior studies. Vinblastine produced a very slight, but not significant, anti-tumor effect ($P \leq 0.20$); Paclitaxel, however, showed significant tumor growth inhibition and produced complete regression of the ovarian tumors (n= 6/6); re-growth of tumors was not observed during the observation period (i.e., 101 days after the commencement of treatment). Paclitaxel, but not vinblastine, has known efficacy in clinical ovarian carcinoma (Markman, M., Taxol: an important new drug in the management of epithelial ovarian cancer. Yale J Biol Med, 1991. 64(6): p. 583-90).

The anti-tumor effects of CR014-vcMMAE administered i.v. to IGROV1-bearing mice were remarkable. The CR014 immunoconjugate, when dosed at very high levels, however, produced lethal toxicity at 50 mg/kg/treatment (1/6= 17 %) and 100 mg/kg/treatment (6/6= 100 %). Nevertheless, 5/6 animals dosed at 50 mg/kg/treatment showed complete regression of the human ovarian carcinoma. Lower doses, such as 25, 12.5 and 6.25 mg/kg/treatment were therapeutically effective producing tumor growth inhibition which led to complete regressions for the majority of test animals. Tumors that regressed did not re-grow during the observation period.

The animals in this study (CR014-ONC-1, CGC-17) showed no abnormal treatment effects on gross examination at doses below 100 mg/kg; at 50 mg/kg inhibition of body weight and fatal toxicity occurred in only one of six mice. Below 50 mg/kg/treatment, twice weekly body weight determinations showed no observable or statistically significant effects of treatment with CR014-vcMMAE on body weight or weight gain.

Conclusions: CR014-vcMMAE produces substantial, dose-dependent anti-tumor effects that began as tumor growth inhibition but soon led to complete regression of established human ovarian xenografts; the regressions were long-lived and re-growth of tumors after successful therapy was not been noted during the observation period (101 days after first day of treatment).

**Claims**

1. An antibody or binding fragment thereof, that specifically binds to T cell, immunoglobulin domain and mucin domain (TIM-1), for use in treating ovarian cancer or renal cancer, wherein said antibody or binding fragment thereof is conjugated to a monomethyl auristatin E (MMAE) toxin.

2. The antibody or binding fragment for use of Claim 1, wherein said antibody or binding fragment is a monoclonal antibody.

3. The antibody or binding fragment for use of Claim 1, wherein said antibody or binding fragment binds to TIM-1 with a Kd between $10^{-7}$ and $10^{-14}$ M.

4. The antibody or binding fragment for use of Claim 1, wherein the antibody or binding fragment is for use in treating ovarian cancer.

5. The antibody or binding fragment for use of Claim 1, wherein the antibody or binding fragment is for use in treating renal cancer.

6. Use of an antibody or binding fragment thereof as defined in any one of the preceding claims in the manufacture of a medicament for the treatment of ovarian or renal cancer.

## Patentansprüche

1. Antikörper oder bindendes Fragment davon, das spezifisch an T-Zell-Immunglobulindomäne und -Mucindomäne 1 (TIM-1) bindet, zur Verwendung bei der Behandlung von Eierstockkrebs oder Nierenkrebs, wobei der Antikörper oder das bindende Fragment davon an ein Monomethylauristatin E (MMAE)-Toxin gebunden ist.

2. Antikörper oder bindendes Fragment zur Verwendung nach Anspruch 1, wobei der Antikörper oder das bindende Fragment ein monoklonaler Antikörper ist.

3. Antikörper oder bindendes Fragment zur Verwendung nach Anspruch 1, wobei der Antikörper oder das bindende Fragment an TIM-1 mit einer Kd zwischen $10^{-7}$ und $10^{-14}$ M bindet.

4. Antikörper oder bindendes Fragment zur Verwendung nach Anspruch 1, wobei der Antikörper oder das bindende Fragment zur Behandlung von Eierstockkrebs verwendet wird.

5. Antikörper oder bindendes Fragment zur Verwendung nach Anspruch 1, wobei der Antikörper oder das bindende Fragment zur Behandlung von Nierenkrebs verwendet wird.

6. Verwendung eines Antikörpers oder eines bindenden Fragments davon gemäß der Definition in einem der vorhergehenden Ansprüche bei der Herstellung eines Medikaments zur Behandlung von Eierstock- oder Nierenkrebs.

## Revendications

1. Anticorps ou fragment de liaison de celui-ci, qui se lie spécifiquement à l’antigène de domaine d’immunoglobuline et de domaine 1 de mucine (TIM-1) de cellules T, pour utilisation dans le traitement d’un cancer ovarien ou d’un cancer rénal, lequel anticorps ou fragment de celui-ci est conjugué à une toxine monométhylauristatine E (MMAE).

2. Anticorps ou fragment de liaison pour utilisation selon la revendication 1, lequel anticorps ou fragment de liaison est un anticorps monoclonal.

3. Anticorps ou fragment de liaison pour utilisation selon la revendication 1, lequel anticorps ou fragment de liaison se lie à TIM-1 avec une constante Kd comprise entre $10^{-7}$ et $10^{-14}$ M.

4. Anticorps ou fragment de liaison pour utilisation selon la revendication 1, lequel anticorps ou fragment de liaison est destiné à être utilisé dans le traitement d’un cancer ovarien.

5. Anticorps ou fragment de liaison pour utilisation selon la revendication 1, lequel anticorps ou fragment de liaison est destiné à être utilisé dans le traitement d’un cancer rénal.

6. Utilisation d’un anticorps ou d’un fragment de liaison de celui-ci tel que défini dans l’une quelconque des revendications précédentes, dans la fabrication d’un médicament destiné au traitement d’un cancer ovarien ou rénal.

Figure 1

ELISA assay of anti-TIM-1 mAbs 1.29, 2.56.2, 2.59.2, and 2.45.1 against the TIM-1 antigen

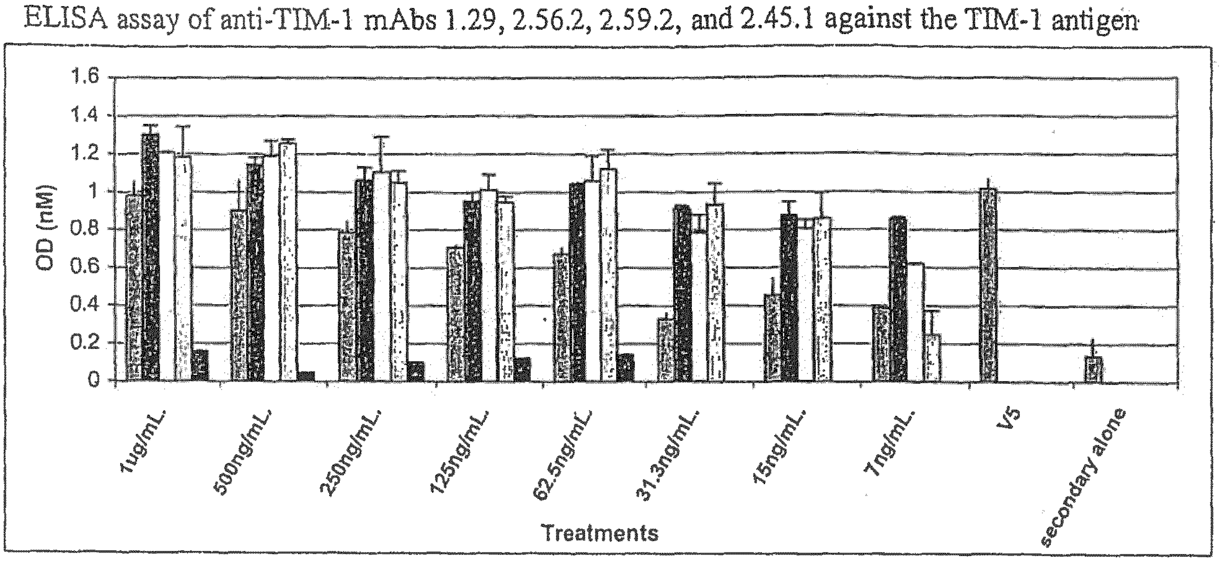

Figure 2

ELISA assay of anti-TIM-1 mAbs 1.29, 2.56.2, 2.59.2, and 2.45.1 against irrelevant protein

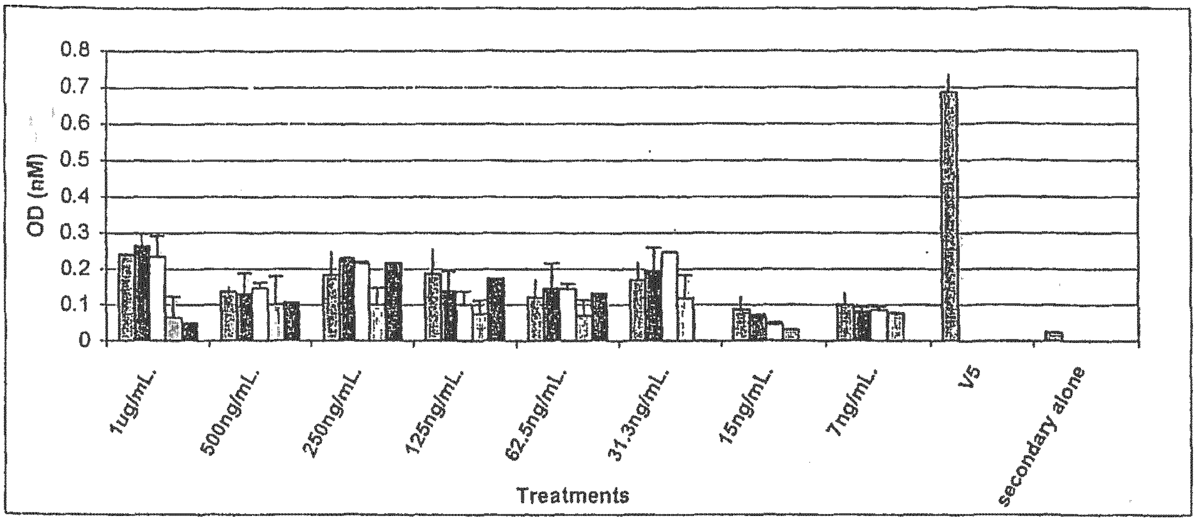

Figure 3A

Renal Cell Cancer

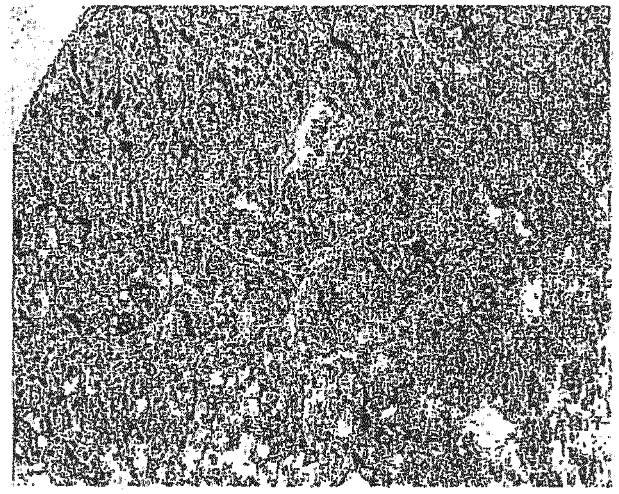

Figure 3B

Pancreatic Cancer

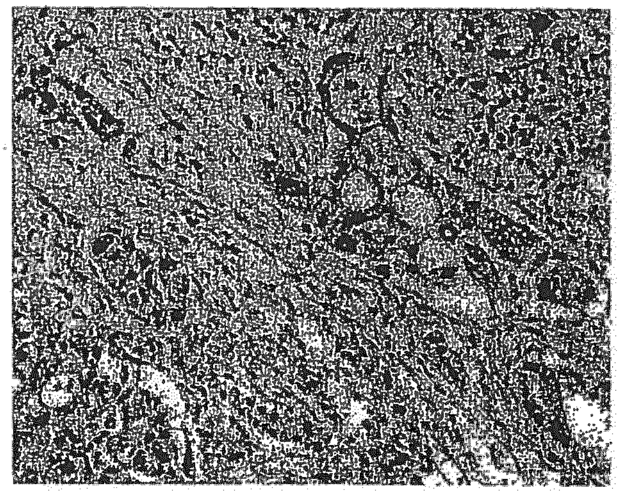

Figure 4

Clonogenic assay results of anti-TIM-1 monoclonal antibody mediated toxin killing in the ACHN kidney cancer cell line

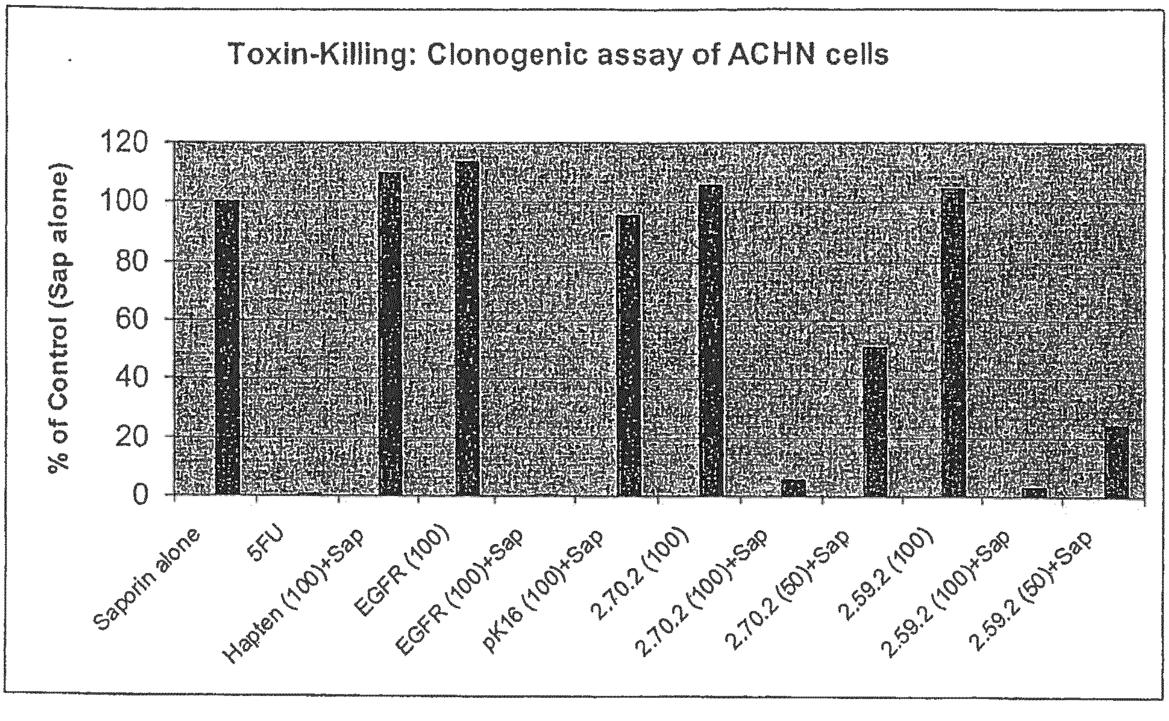

Figure 5

Clonogenic assay results of anti-TIM-1 monoclonal antibody mediated toxin killing in the BT549 breast cancer cell line

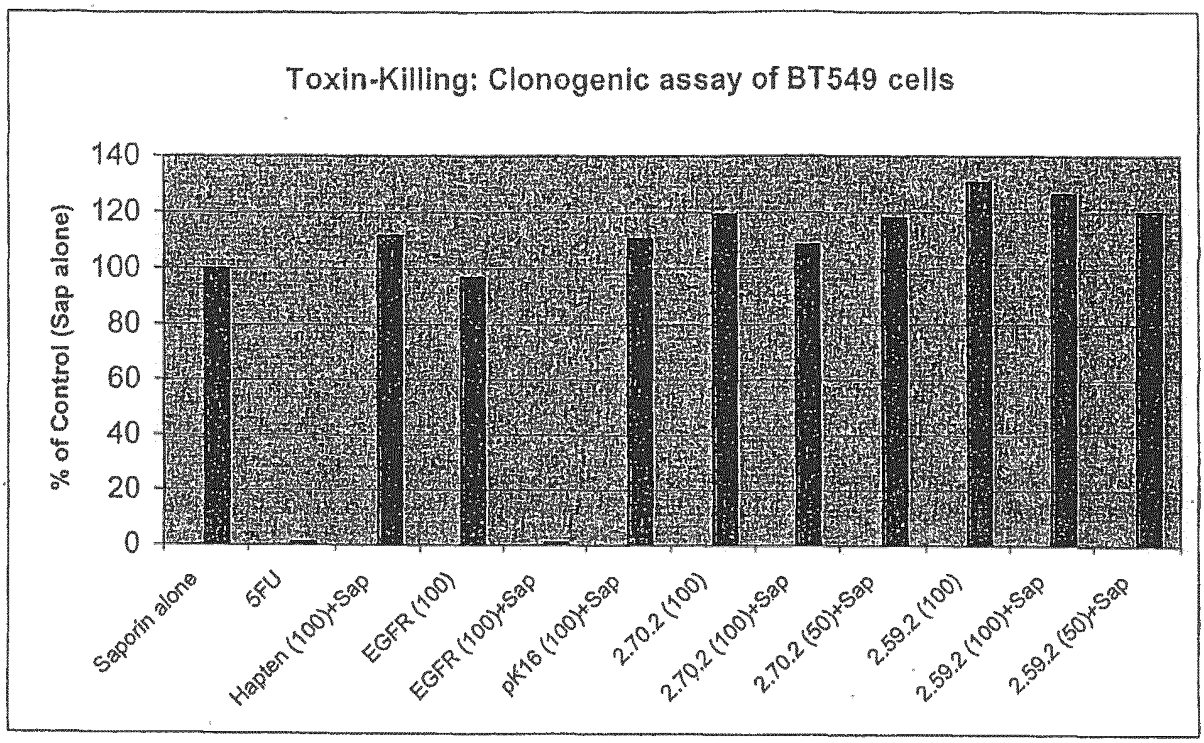

Figure 6

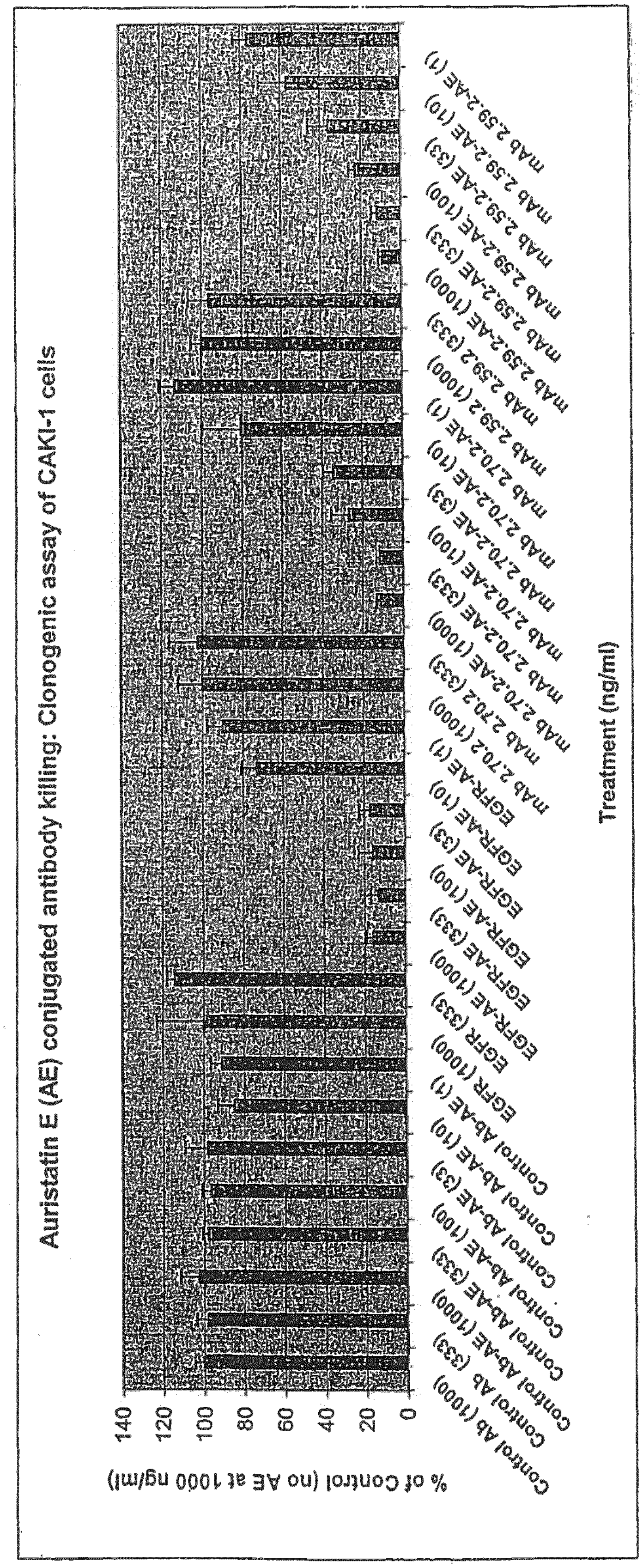
Auristatin E (AE) conjugated antibody killing: Clonogenic assay of CAKI-1 cells
% of Control (no AE at 1000 ng/ml)
140
120
100
80
60
40
20
0
Control Ab (1000)
Control Ab (333)
Control Ab-AE (1000)
Control Ab-AE (333)
Control Ab-AE (100)
Control Ab-AE (33)
Control Ab-AE (10)
Control Ab-AE (1)
EGFR (1000)
EGFR (333)
EGFR-AE (1000)
EGFR-AE (333)
EGFR-AE (100)
EGFR-AE (33)
EGFR-AE (10)
EGFR-AE (1)
mAb 2.70.2 (1000)
mAb 2.70.2 (333)
mAb 2.70.2-AE (1000)
mAb 2.70.2-AE (333)
mAb 2.70.2-AE (100)
mAb 2.70.2-AE (33)
mAb 2.70.2-AE (10)
mAb 2.70.2-AE (1)
mAb 2.59.2 (1000)
mAb 2.59.2 (333)
mAb 2.59.2-AE (1000)
mAb 2.59.2-AE (333)
mAb 2.59.2-AE (100)
mAb 2.59.2-AE (33)
mAb 2.59.2-AE (10)
mAb 2.59.2-AE (1)
Treatment (ng/ml)

Figure 7

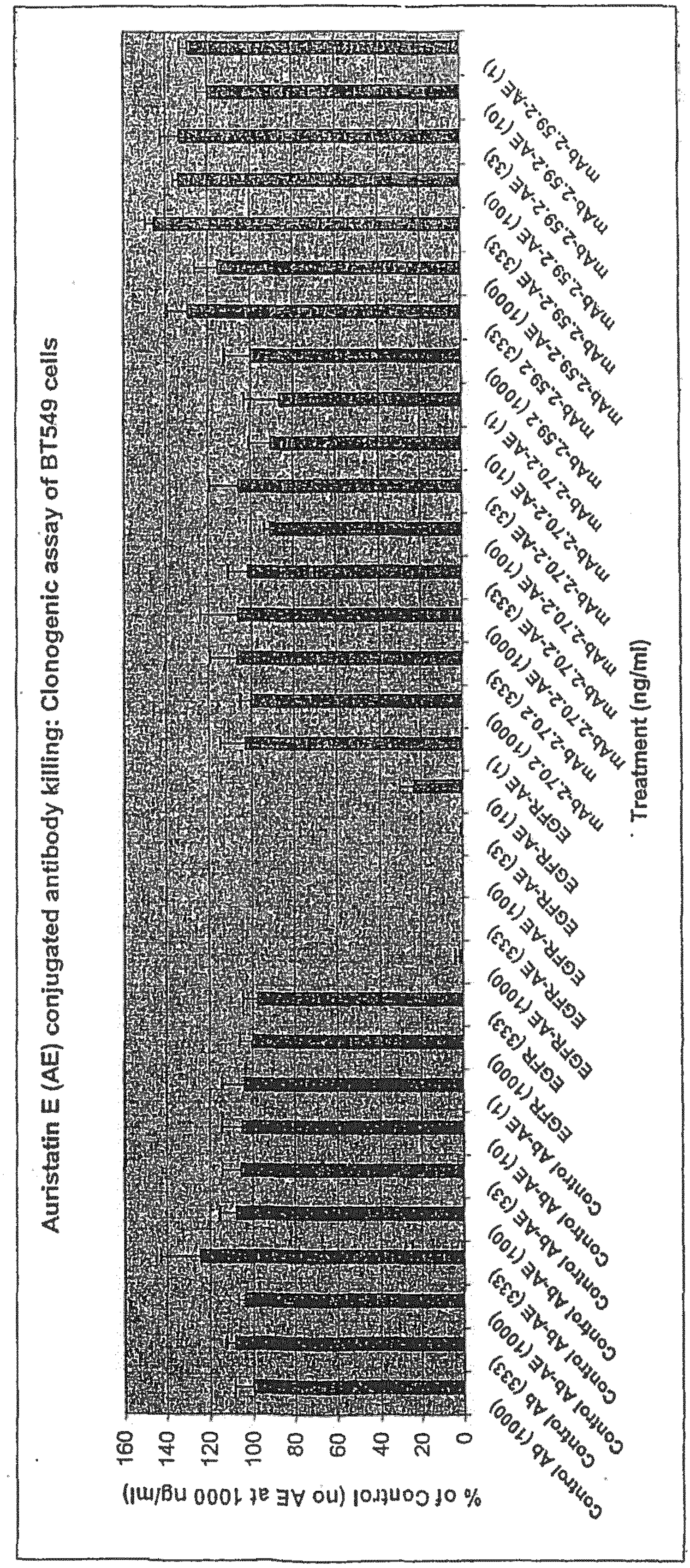
Auristatin E (AE) conjugated antibody killing: Clonogenic assay of BT549 cells
% of Control (no AE at 1000 ng/ml)
160
140
120
100
80
60
40
20
0
Control Ab (1000)
Control Ab (333)
Control Ab-AE (1000)
Control Ab-AE (333)
Control Ab-AE (100)
Control Ab-AE (33)
Control Ab-AE (10)
Control Ab-AE (1)
EGFR (1000)
EGFR (333)
EGFR-AE (1000)
EGFR-AE (333)
EGFR-AE (100)
EGFR-AE (33)
EGFR-AE (10)
EGFR-AE (1)
mAb-2.70.2 (1000)
mAb-2.70.2 (333)
mAb-2.70.2-AE (1000)
mAb-2.70.2-AE (333)
mAb-2.70.2-AE (100)
mAb-2.70.2-AE (33)
mAb-2.70.2-AE (10)
mAb-2.70.2-AE (1)
mAb-2.59.2 (1000)
mAb-2.59.2 (333)
mAb-2.59.2-AE (1000)
mAb-2.59.2-AE (333)
mAb-2.59.2-AE (100)
mAb-2.59.2-AE (33)
mAb-2.59.2-AE (10)
mAb-2.59.2-AE (1)
Treatment (ng/ml)

Figure 8

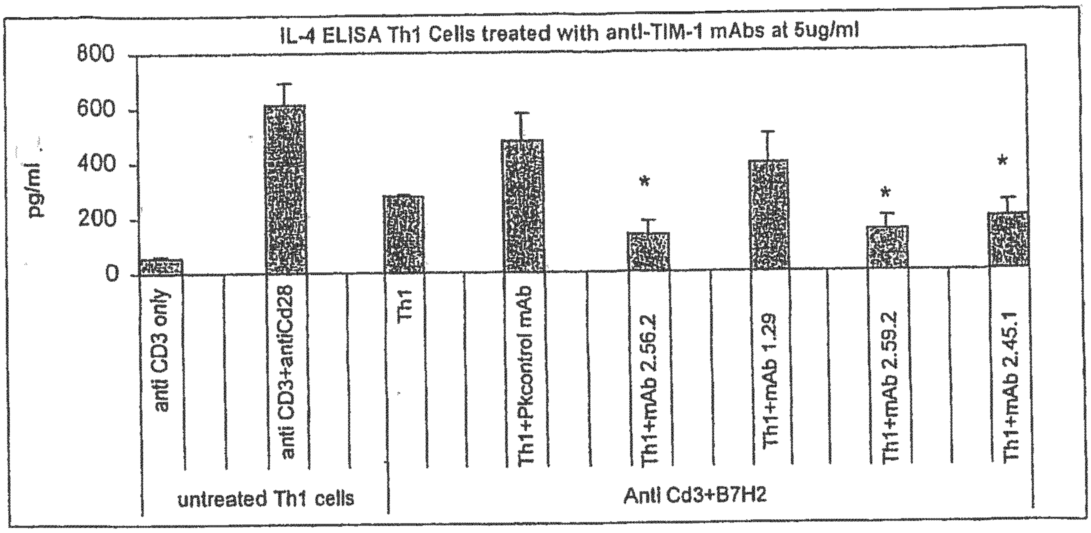
IL-4 ELISA Th1 Cells treated with anti-TIM-1 mAbs at 5ug/ml
800
600
400
200
0
pg/ml
anti CD3 only
anti CD3+antiCd28
Th1
Th1+Pkcontrol mAb
Th1+mAb 2.56.2
Th1+mAb 1.29
Th1+mAb 2.59.2
Th1+mAb 2.45.1
untreated Th1 cells
Anti Cd3+B7H2
*
*
*

Figure 9

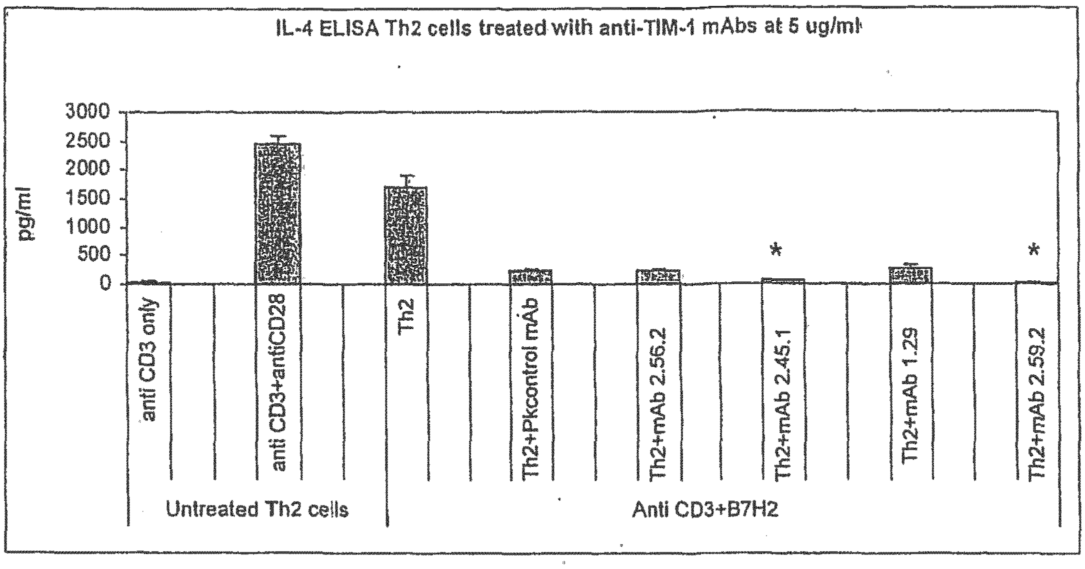
IL-4 ELISA Th2 cells treated with anti-TIM-1 mAbs at 5 ug/ml
pg/ml
3000
2500
2000
1500
1000
500
0
*
*
anti CD3 only
anti CD3+antiCD28
Th2
Th2+Pkcontrol mAb
Th2+mAb 2.56.2
Th2+mAb 2.45.1
Th2+mAb 1.29
Th2+mAb 2.59.2
Untreated Th2 cells
Anti CD3+B7H2

Figure 10

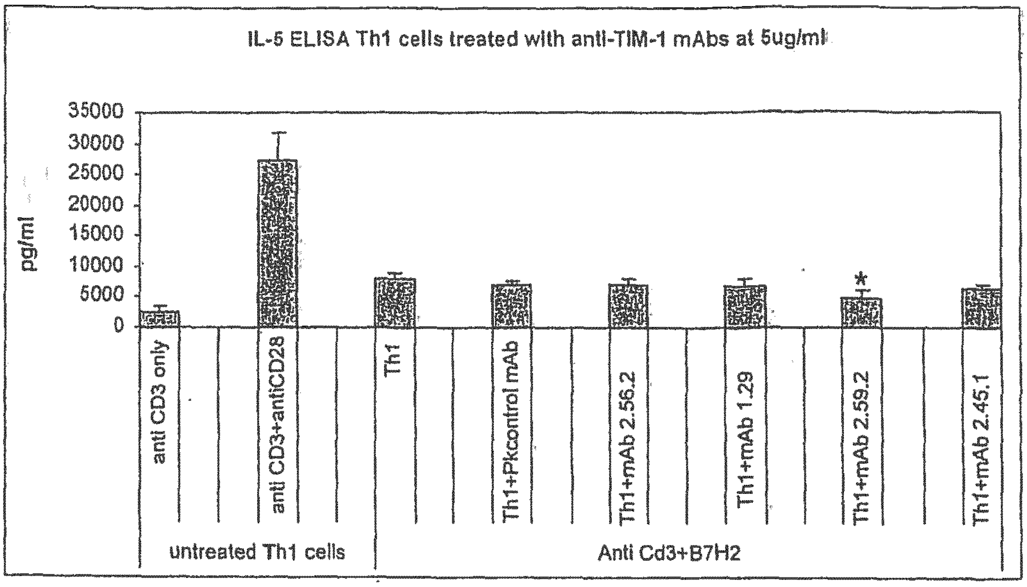
IL-5 ELISA Th1 cells treated with anti-TIM-1 mAbs at 5ug/ml
pg/ml
35000
30000
25000
20000
15000
10000
5000
0
anti CD3 only
anti CD3+antiCD28
Th1
Th1+Pkcontrol mAb
Th1+mAb 2.56.2
Th1+mAb 1.29
Th1+mAb 2.59.2
Th1+mAb 2.45.1
*
untreated Th1 cells
Anti Cd3+B7H2

Figure 11

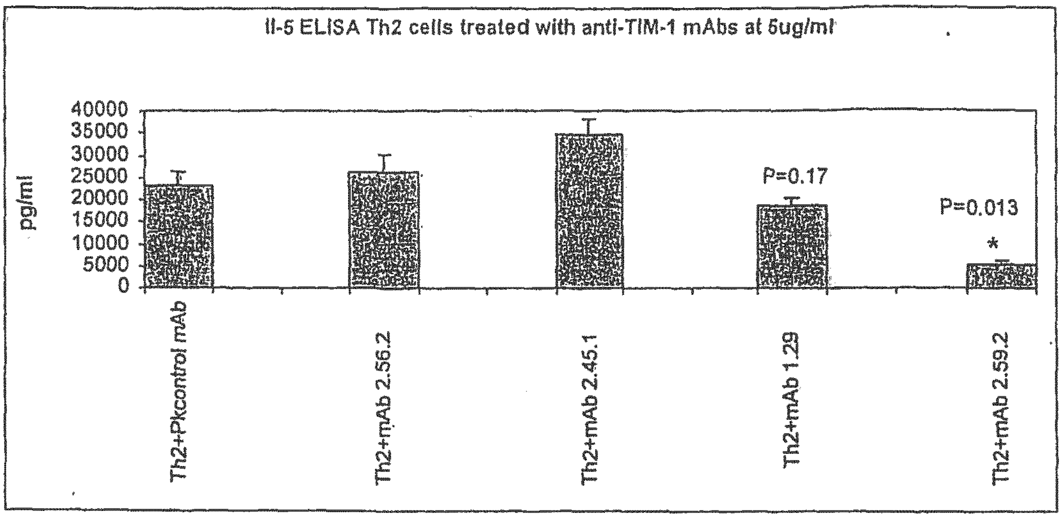
Il-5 ELISA Th2 cells treated with anti-TIM-1 mAbs at 5ug/ml
pg/ml
40000
35000
30000
25000
20000
15000
10000
5000
0
P=0.17
P=0.013
*
Th2+Pkcontrol mAb
Th2+mAb 2.56.2
Th2+mAb 2.45.1
Th2+mAb 1.29
Th2+mAb 2.59.2

Figure 12

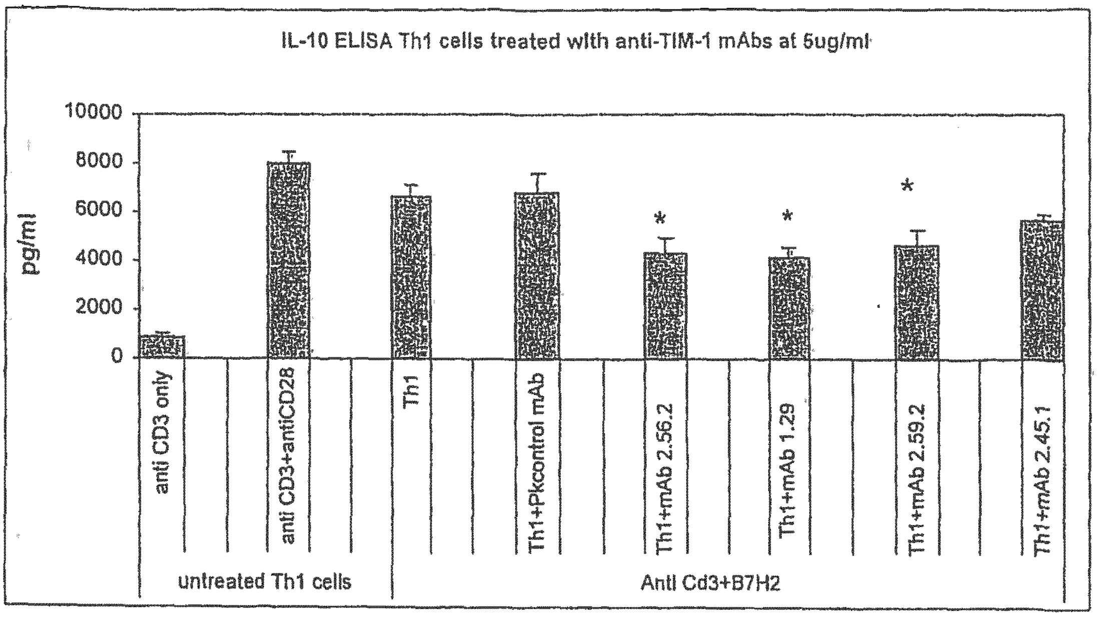
IL-10 ELISA Th1 cells treated with anti-TIM-1 mAbs at 5ug/ml
pg/ml
10000
8000
6000
4000
2000
0
*
*
*
anti CD3 only
anti CD3+antiCD28
Th1
Th1+Pkcontrol mAb
Th1+mAb 2.56.2
Th1+mAb 1.29
Th1+mAb 2.59.2
Th1+mAb 2.45.1
untreated Th1 cells
Anti Cd3+B7H2

Figure 13

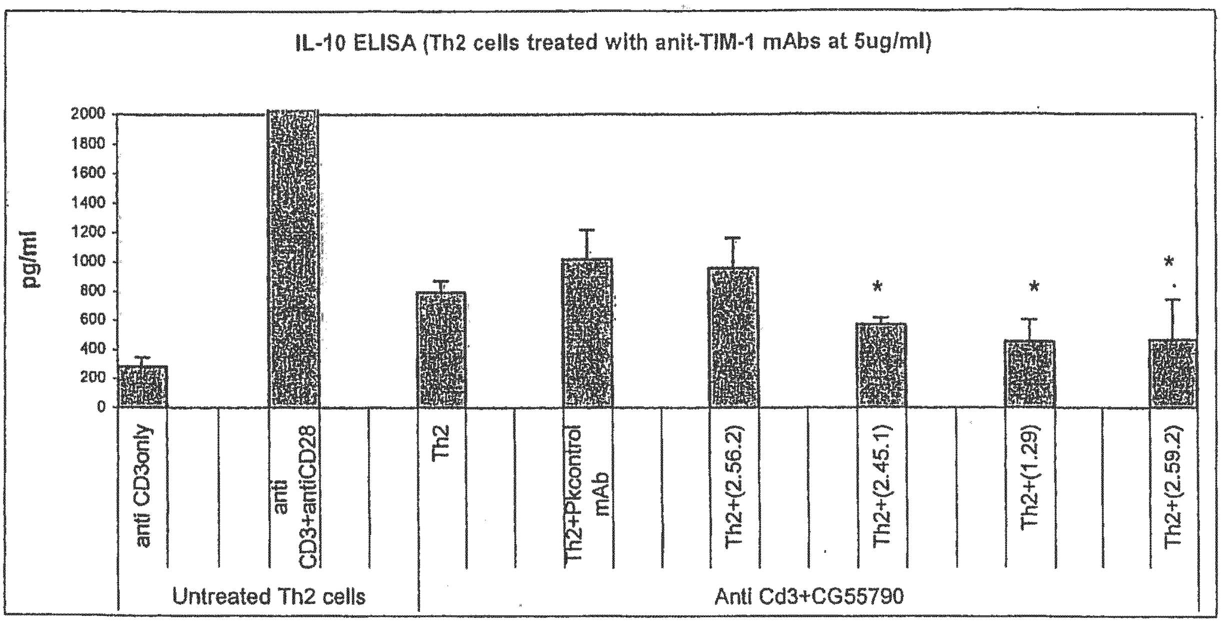
IL-10 ELISA (Th2 cells treated with anit-TIM-1 mAbs at 5ug/ml)
pg/ml
2000
1800
1600
1400
1200
1000
800
600
400
200
0
*
*
*
anti CD3only
anti CD3+antiCD28
Th2
Th2+Pkcontrol mAb
Th2+(2.56.2)
Th2+(2.45.1)
Th2+(1.29)
Th2+(2.59.2)
Untreated Th2 cells
Anti Cd3+CG55790

Figure 14

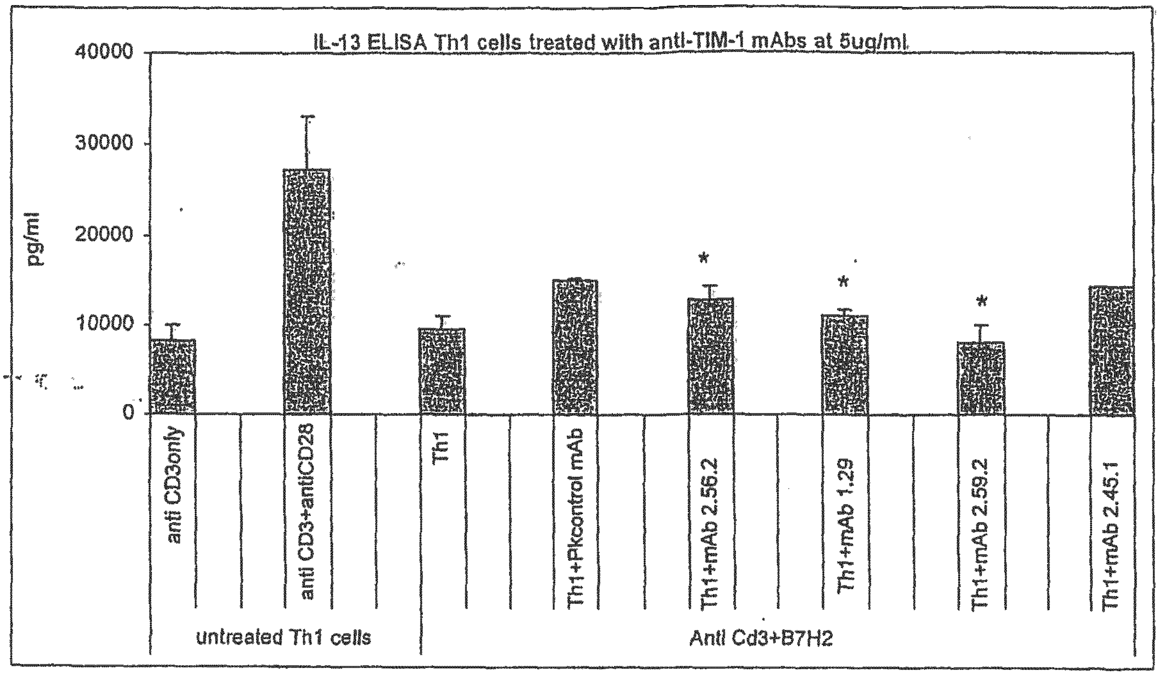
IL-13 ELISA Th1 cells treated with anti-TIM-1 mAbs at 5ug/ml
40000
30000
20000
10000
0
pg/ml
anti CD3only
anti CD3+antiCD28
Th1
Th1+Pkcontrol mAb
Th1+mAb 2.56.2
Th1+mAb 1.29
Th1+mAb 2.59.2
Th1+mAb 2.45.1
untreated Th1 cells
Anti Cd3+B7H2

Figure 15

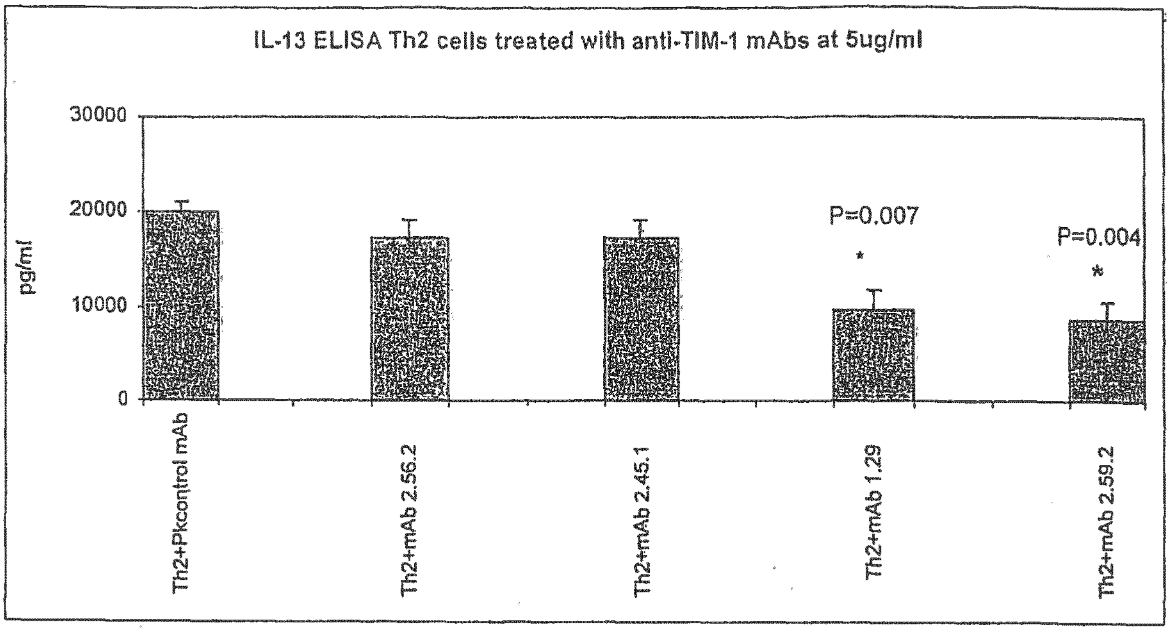
IL-13 ELISA Th2 cells treated with anti-TIM-1 mAbs at 5ug/ml
pg/ml
30000
20000
10000
0
P=0.007
*
P=0.004
*
Th2+Pkcontrol mAb
Th2+mAb 2.56.2
Th2+mAb 2.45.1
Th2+mAb 1.29
Th2+mAb 2.59.2

Figure 16

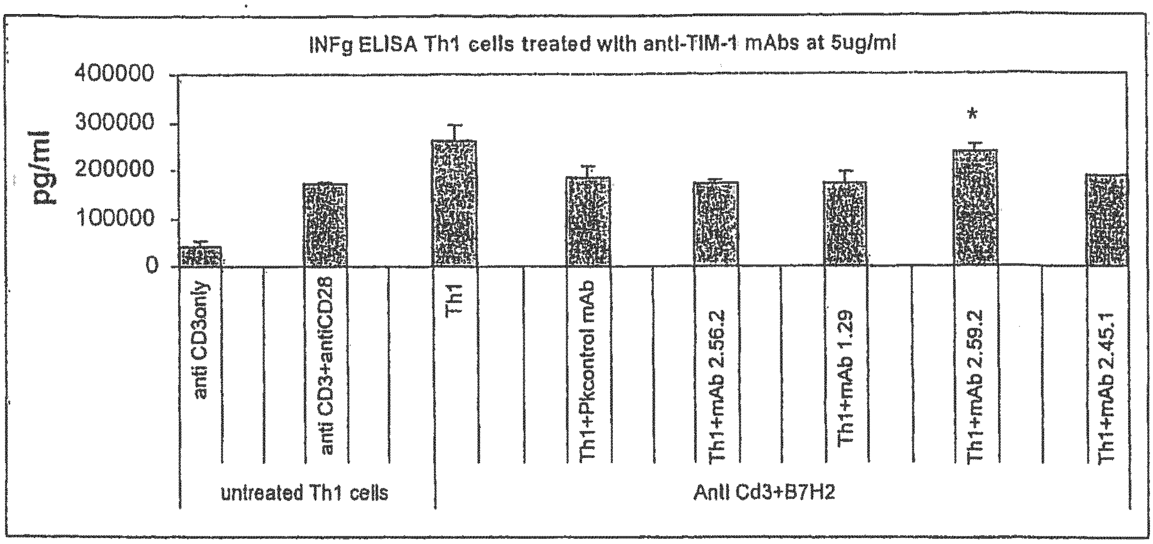
INFg ELISA Th1 cells treated with anti-TIM-1 mAbs at 5ug/ml
400000
300000
200000
100000
0
pg/ml
*
anti CD3only
anti CD3+antiCD28
Th1
Th1+Pkcontrol mAb
Th1+mAb 2.56.2
Th1+mAb 1.29
Th1+mAb 2.59.2
Th1+mAb 2.45.1
untreated Th1 cells
Anti Cd3+B7H2

Figure 17

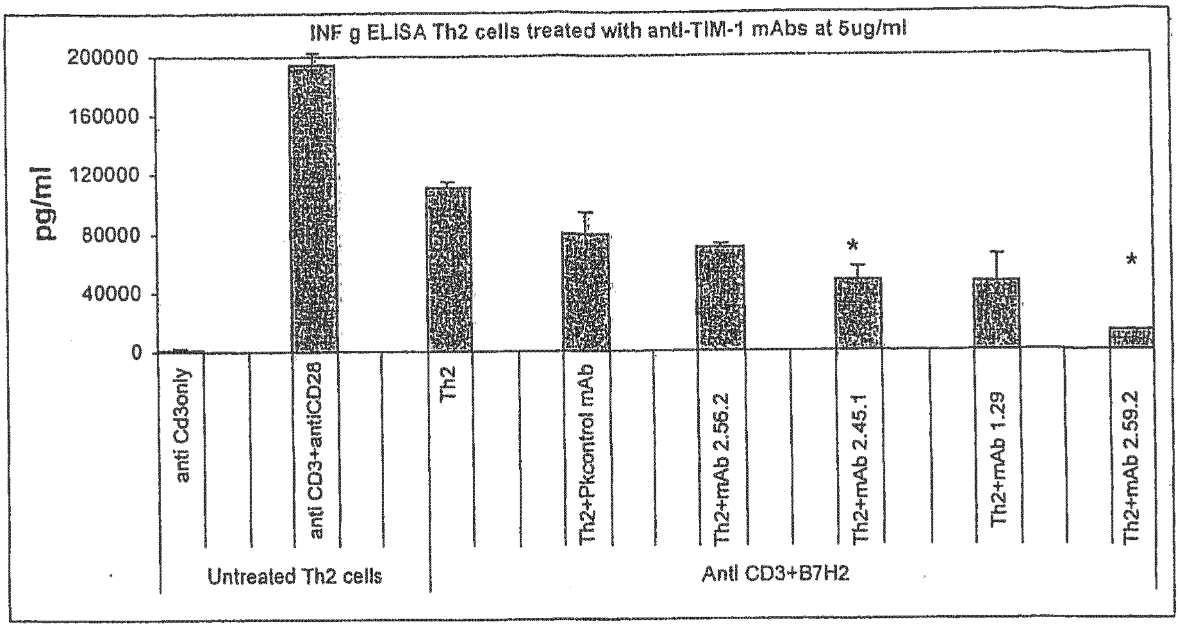
INF g ELISA Th2 cells treated with anti-TIM-1 mAbs at 5ug/ml
pg/ml
200000
160000
120000
80000
40000
0
anti Cd3only
anti CD3+antiCD28
Th2
Th2+Pkcontrol mAb
Th2+mAb 2.56.2
Th2+mAb 2.45.1
Th2+mAb 1.29
Th2+mAb 2.59.2
*
*
Untreated Th2 cells
Anti CD3+B7H2

Figure 18A

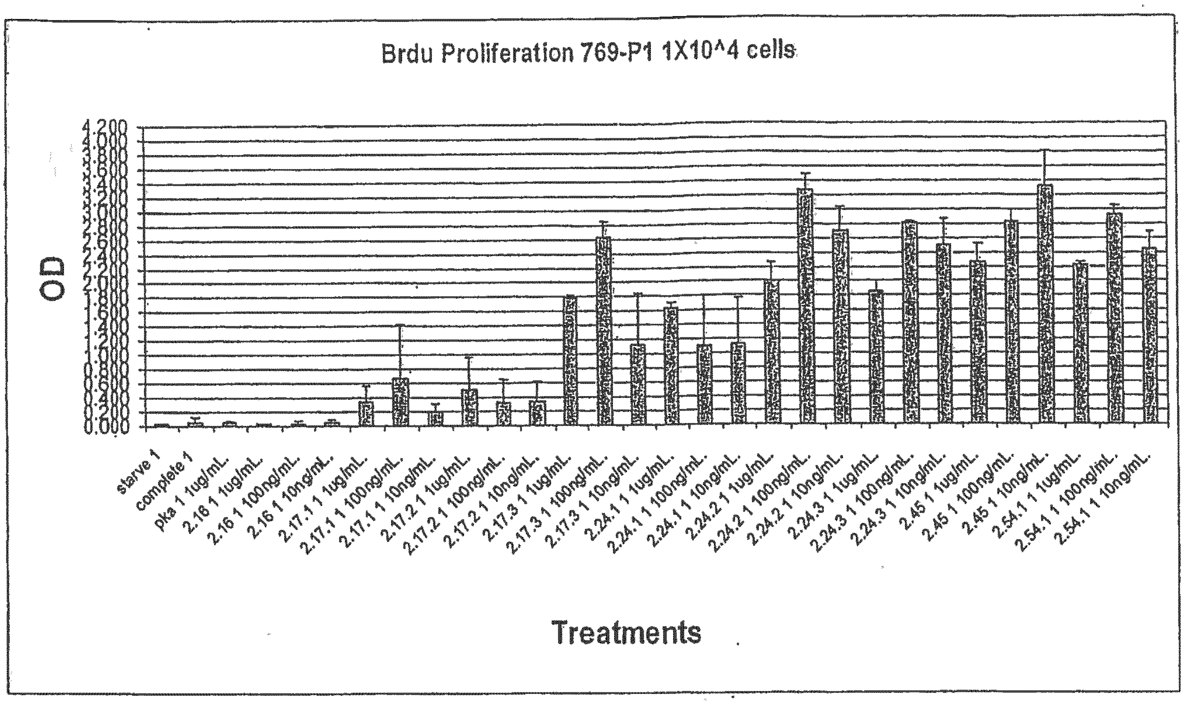


Figure 18B

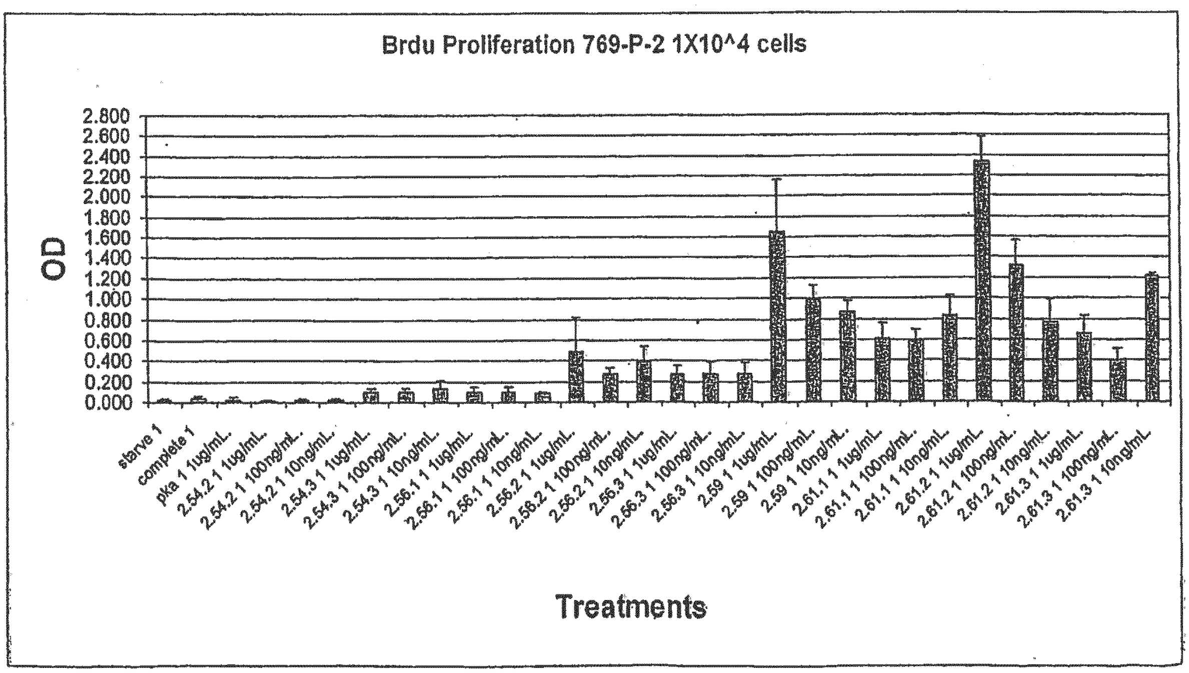

Figure 18C

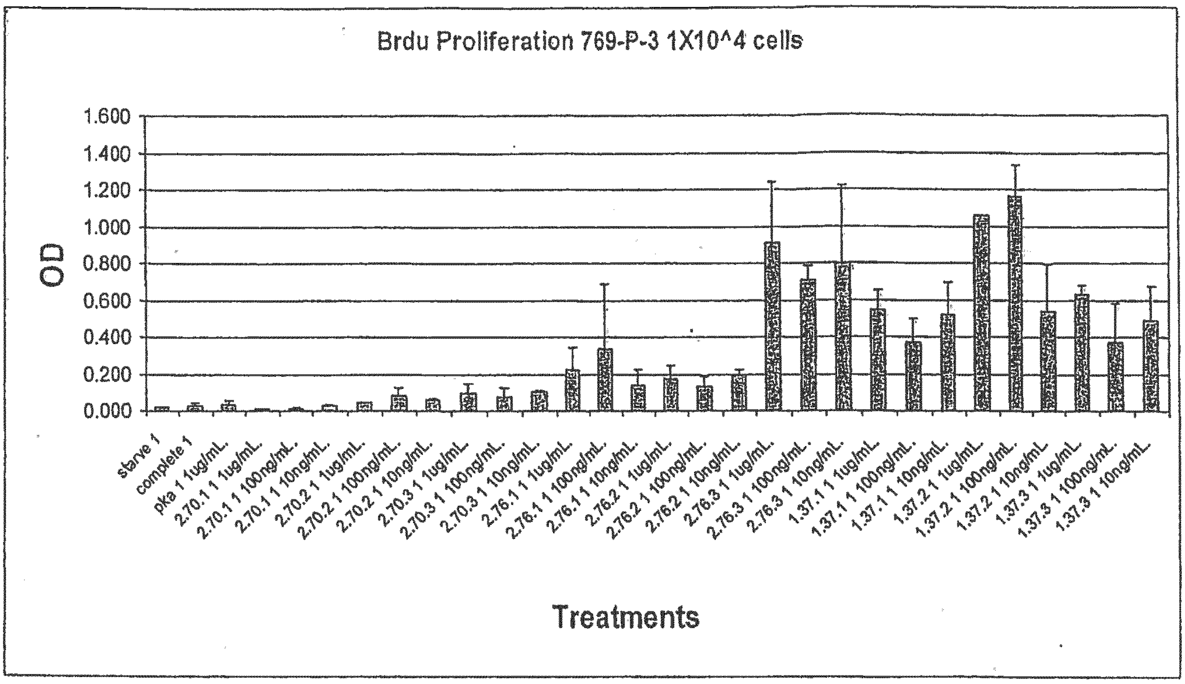


Figure 18D

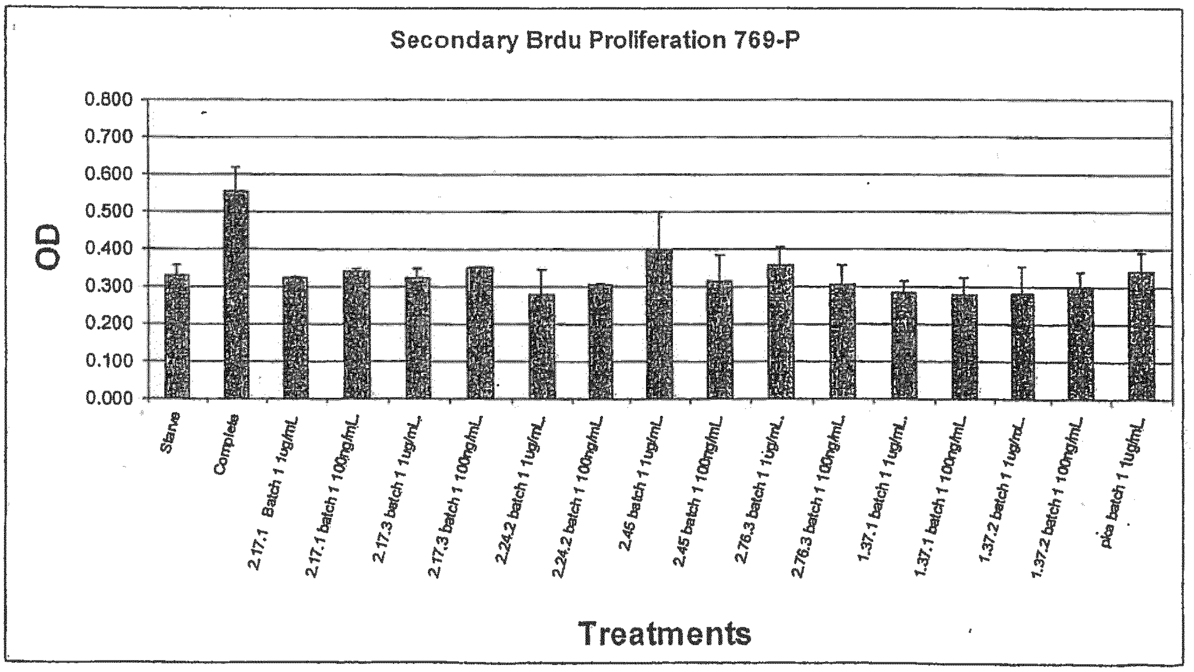

Figure 18E

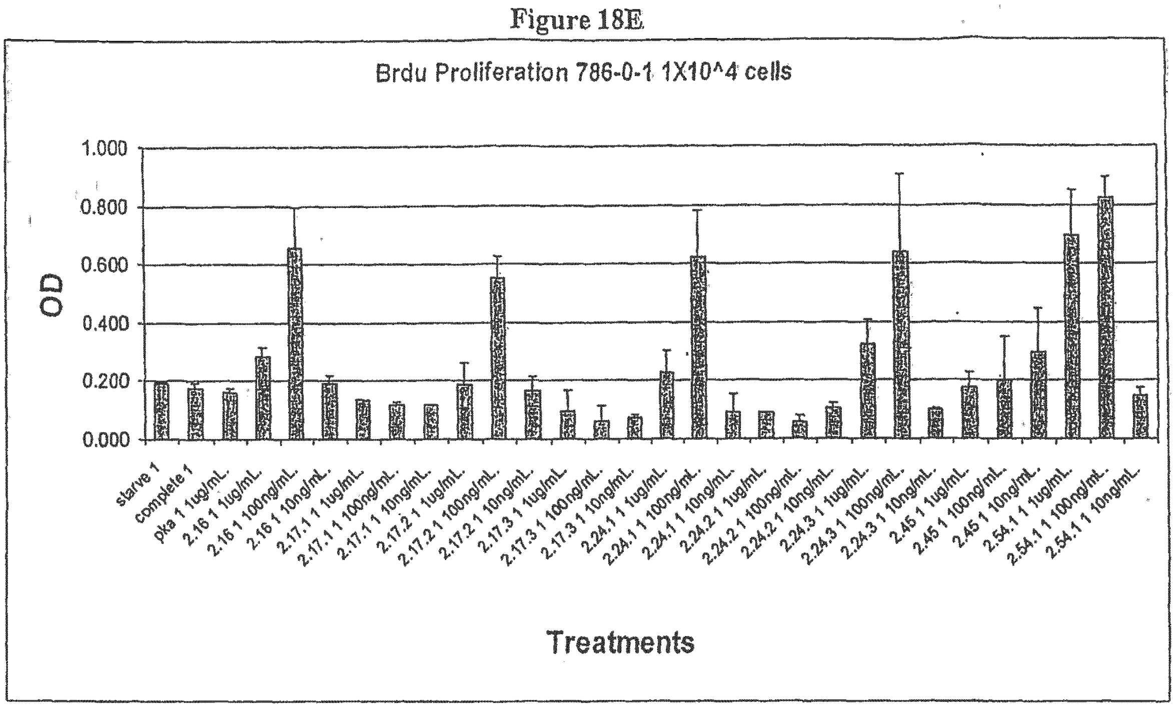


Figure 18F

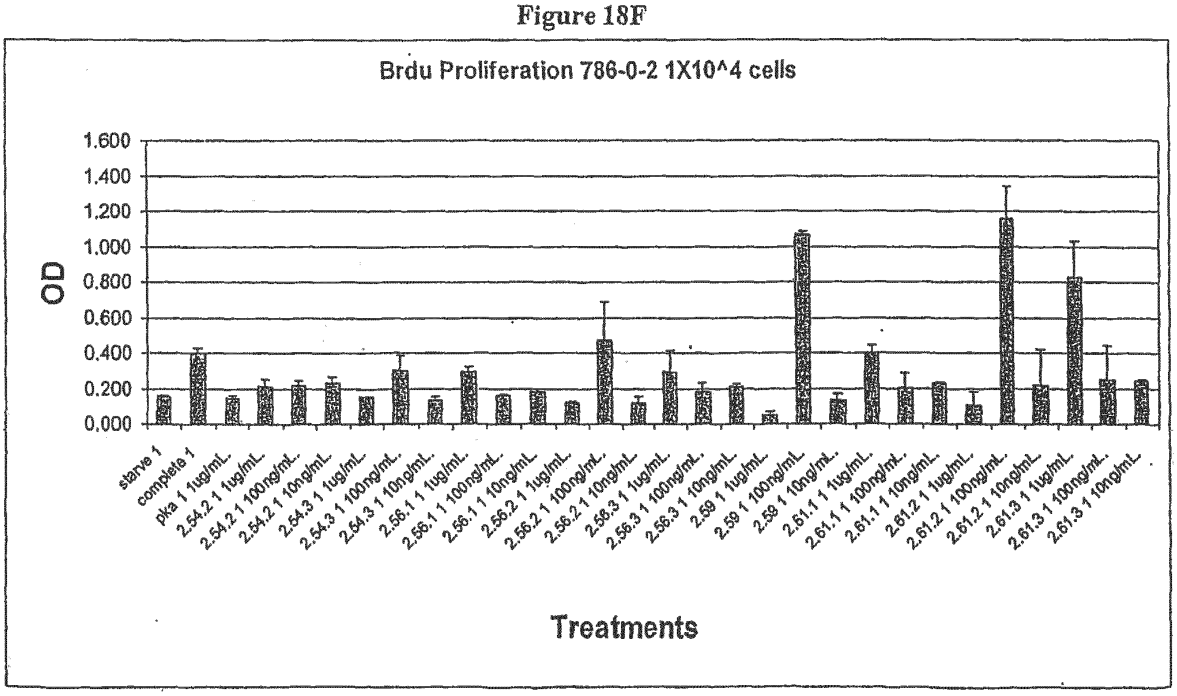

Figure 18G

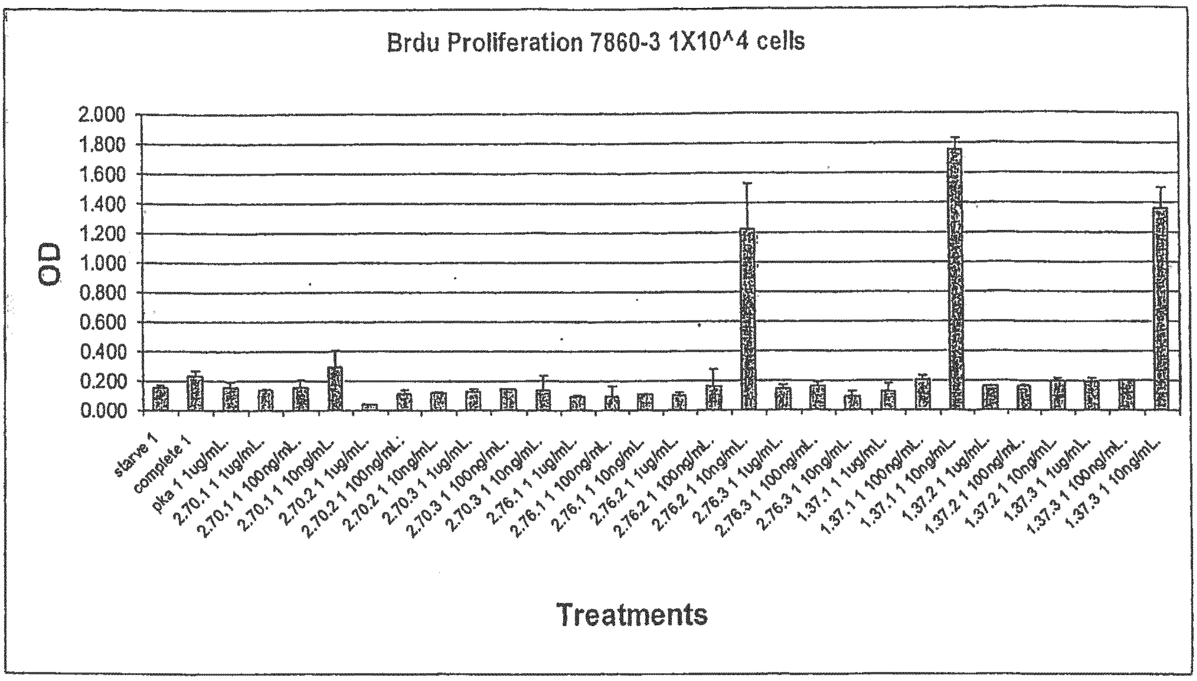


Figure 18H

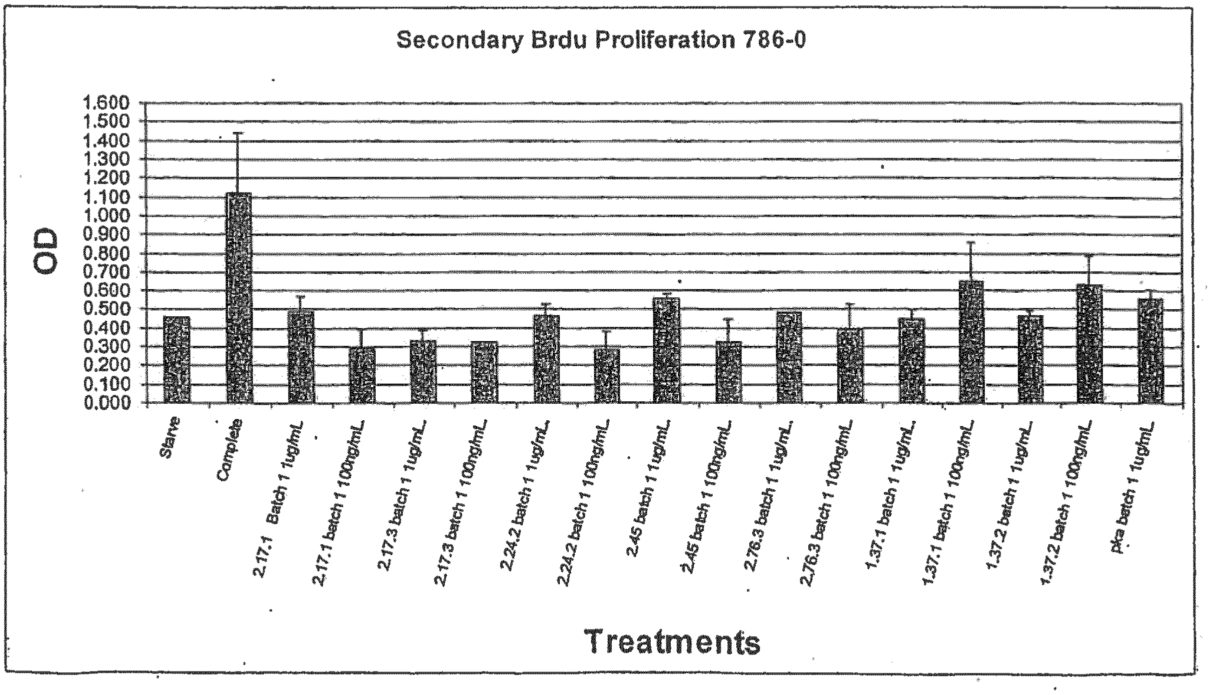

Figure 18I

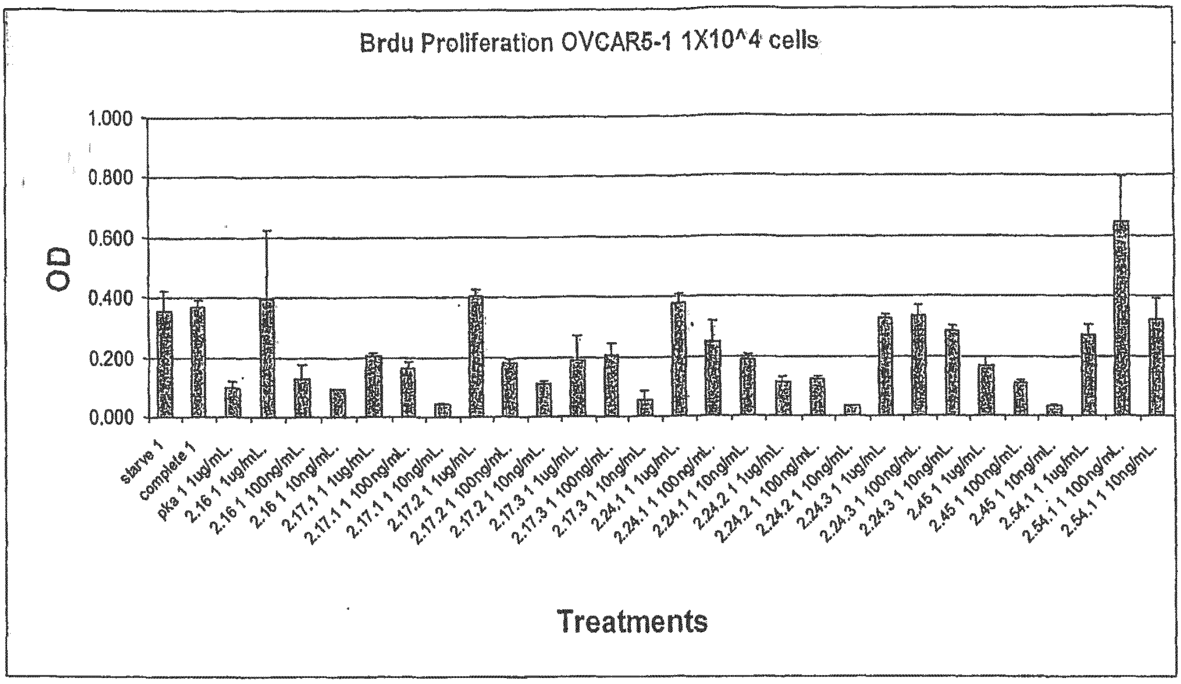


Figure 18J

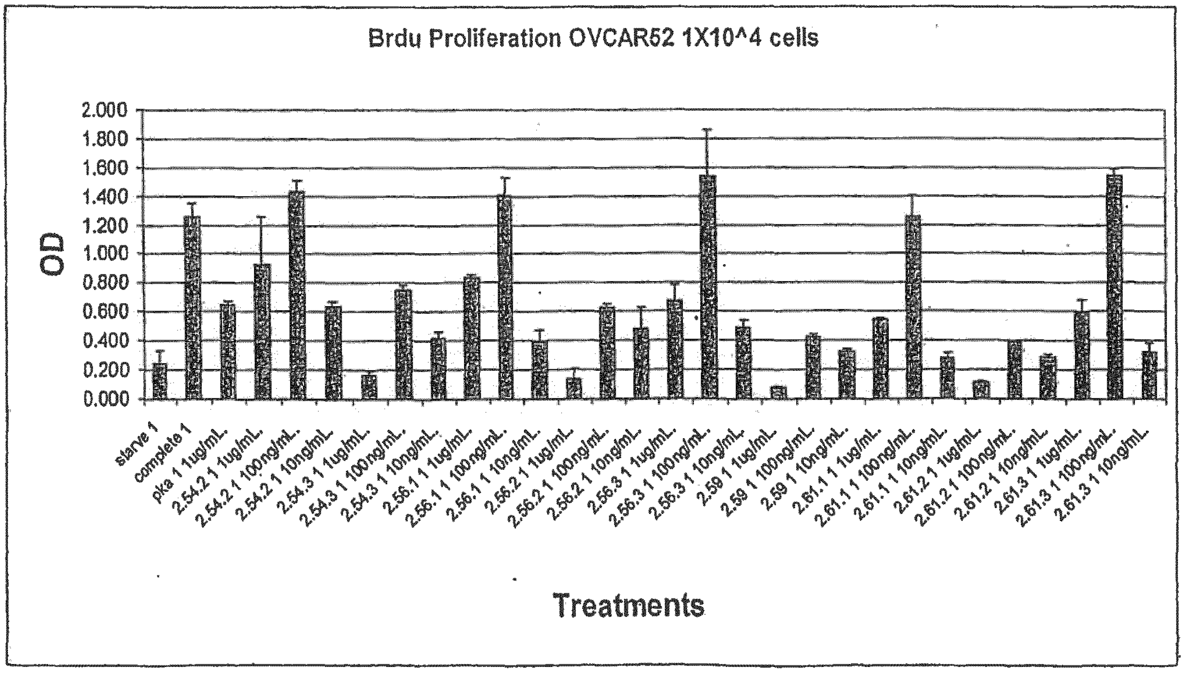

Figure 18K

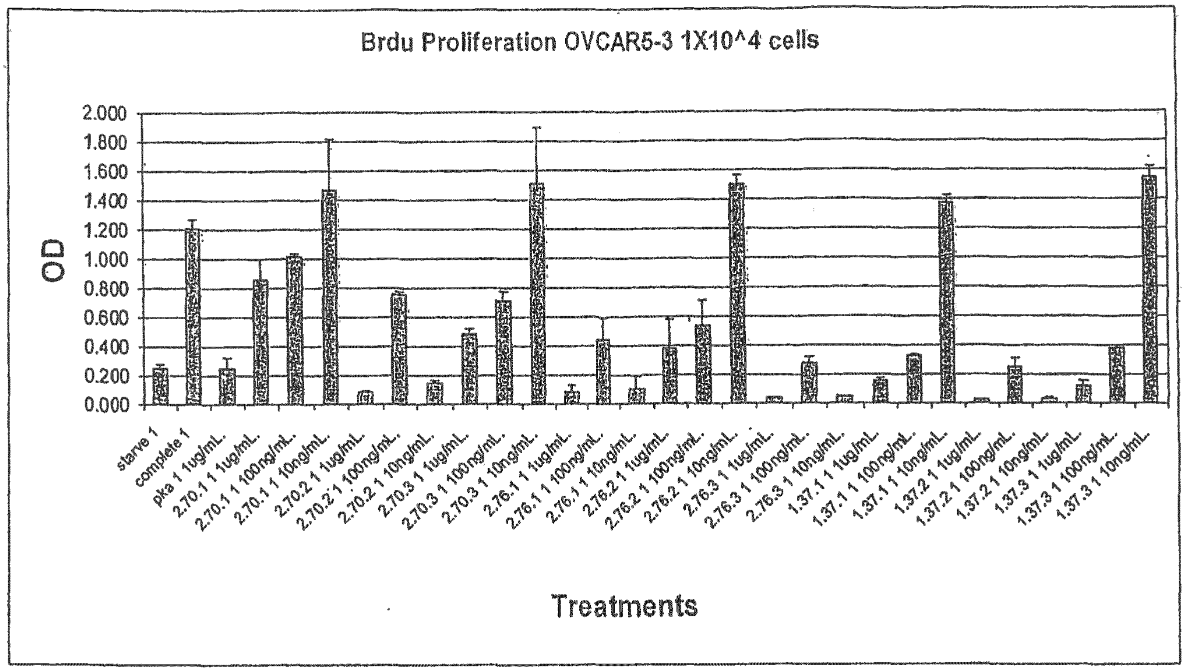


Figure 18L

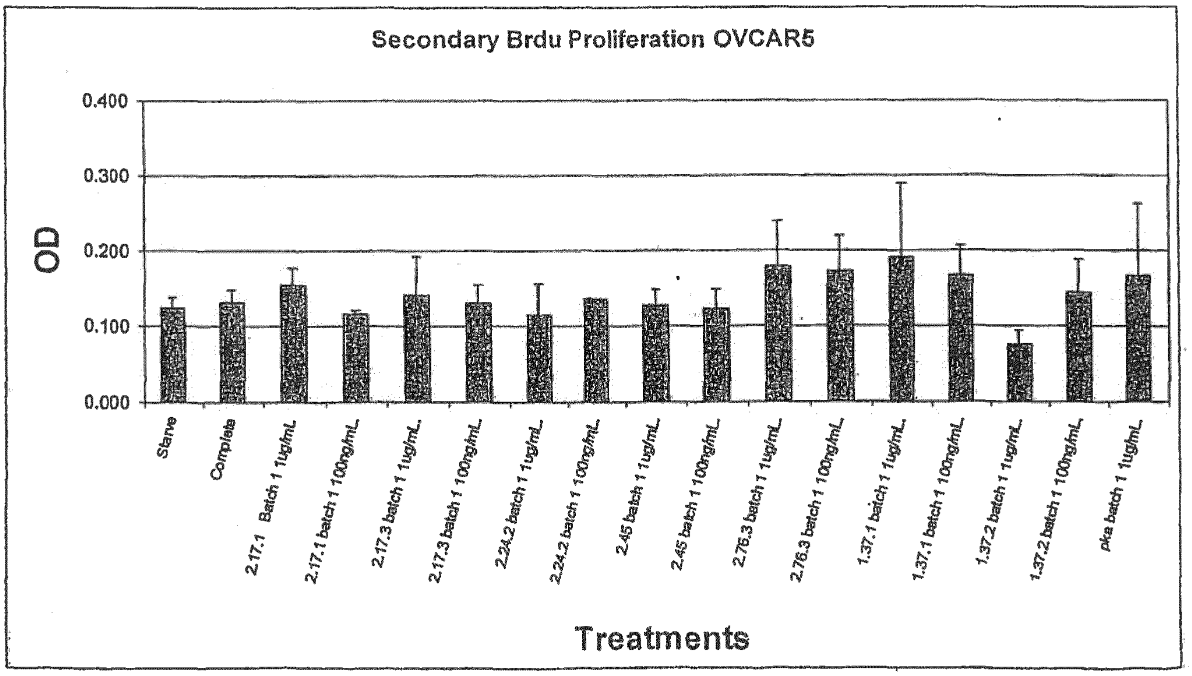

18M

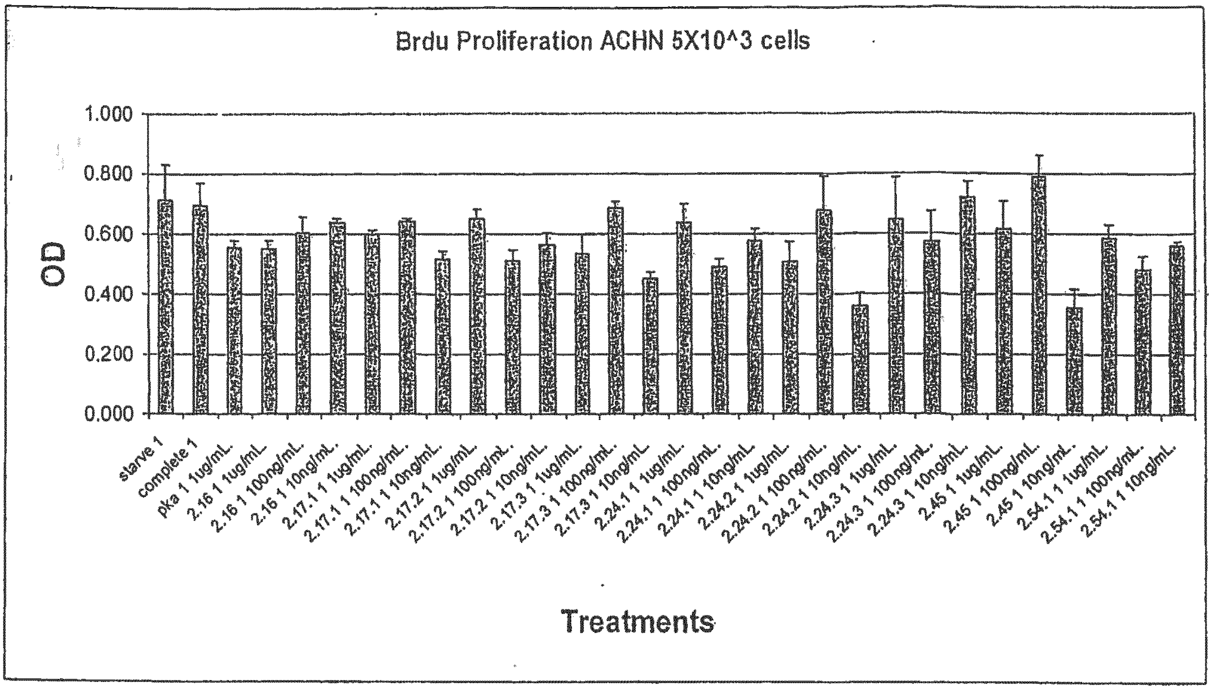
Brdu Proliferation ACHN 5X10^3 cells
OD
1.000
0.800
0.600
0.400
0.200
0.000
starve 1
complete 1
pka 1 1ug/mL.
2.16 1 1ug/mL.
2.16 1 100ng/mL.
2.16 1 10ng/mL.
2.17.1 1 1ug/mL.
2.17.1 1 100ng/mL.
2.17.1 1 10ng/mL.
2.17.2 1 1ug/mL.
2.17.2 1 100ng/mL.
2.17.2 1 10ng/mL.
2.17.3 1 1ug/mL.
2.17.3 1 100ng/mL.
2.17.3 1 10ng/mL.
2.24.1 1 1ug/mL.
2.24.1 1 100ng/mL.
2.24.1 1 10ng/mL.
2.24.2 1 1ug/mL.
2.24.2 1 100ng/mL.
2.24.2 1 10ng/mL.
2.24.3 1 1ug/mL.
2.24.3 1 100ng/mL.
2.24.3 1 10ng/mL.
2.45 1 1ug/mL.
2.45 1 100ng/mL.
2.45 1 10ng/mL.
2.54.1 1 1ug/mL.
2.54.1 1 100ng/mL.
2.54.1 1 10ng/mL.
Treatments

18N

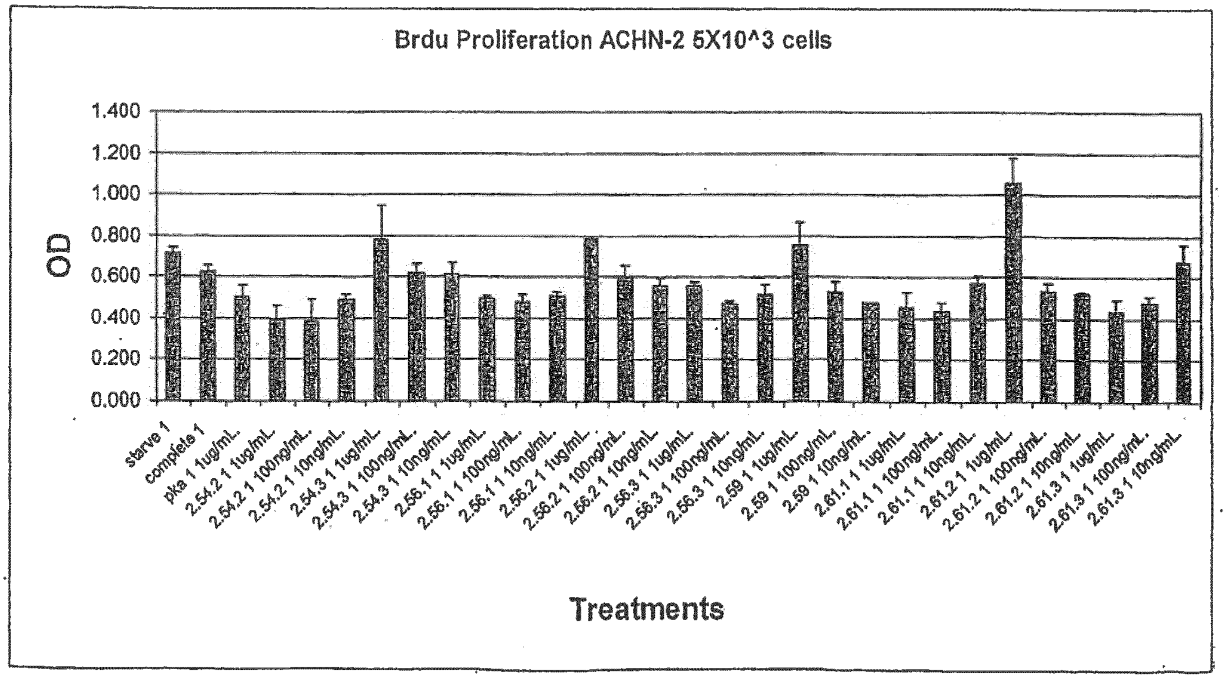
Brdu Proliferation ACHN-2 5X10^3 cells
OD
1.400
1.200
1.000
0.800
0.600
0.400
0.200
0.000
starve 1
complete 1
pka 1 1ug/mL.
2.54.2 1 1ug/mL.
2.54.2 1 100ng/mL.
2.54.2 1 10ng/mL.
2.54.3 1 1ug/mL.
2.54.3 1 100ng/mL.
2.54.3 1 10ng/mL.
2.56.1 1 1ug/mL.
2.56.1 1 100ng/mL.
2.56.1 1 10ng/mL.
2.56.2 1 1ug/mL.
2.56.2 1 100ng/mL.
2.56.2 1 10ng/mL.
2.56.3 1 1ug/mL.
2.56.3 1 100ng/mL.
2.56.3 1 10ng/mL.
2.59 1 1ug/mL.
2.59 1 100ng/mL.
2.59 1 10ng/mL.
2.61.1 1 1ug/mL.
2.61.1 1 100ng/mL.
2.61.1 1 10ng/mL.
2.61.2 1 1ug/mL.
2.61.2 1 100ng/mL.
2.61.2 1 10ng/mL.
2.61.3 1 1ug/mL.
2.61.3 1 100ng/mL.
2.61.3 1 10ng/mL.
Treatments

18O

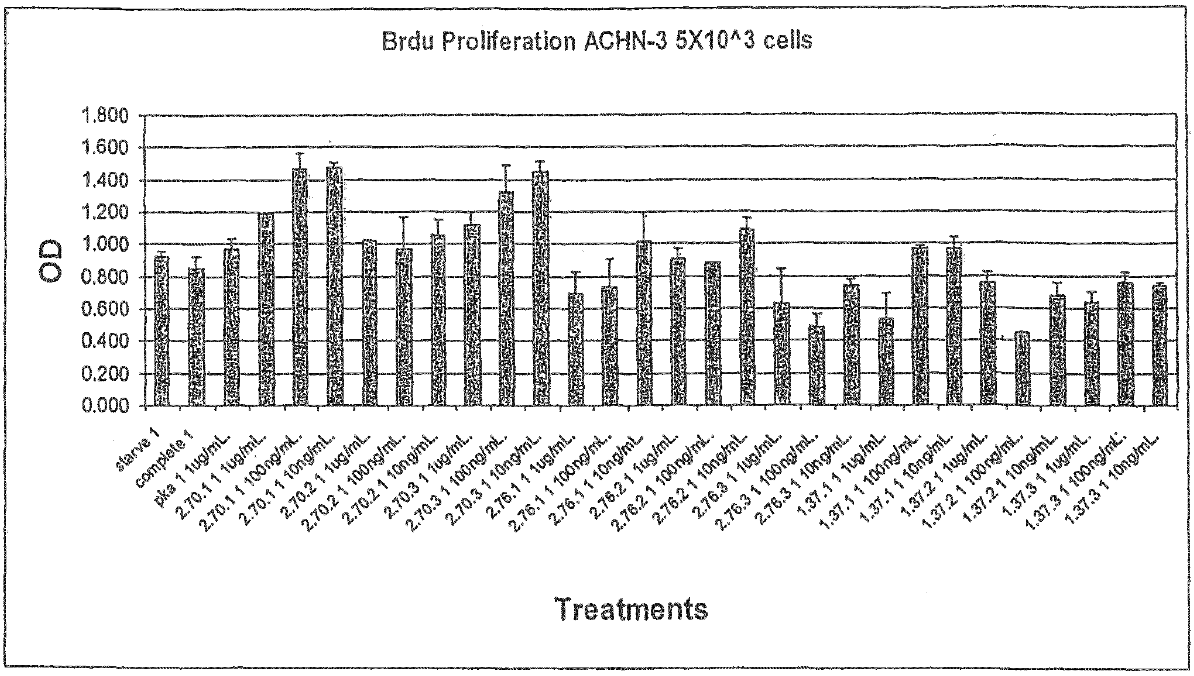
Brdu Proliferation ACHN-3 5X10^3 cells
OD
1.800
1.600
1.400
1.200
1.000
0.800
0.600
0.400
0.200
0.000
starve 1
complete 1
pka 1 1ug/mL.
2.70.1 1 1ug/mL.
2.70.1 1 100ng/mL.
2.70.1 1 10ng/mL.
2.70.2 1 1ug/mL.
2.70.2 1 100ng/mL.
2.70.2 1 10ng/mL.
2.70.3 1 1ug/mL.
2.70.3 1 100ng/mL.
2.70.3 1 10ng/mL.
2.76.1 1 1ug/mL.
2.76.1 1 100ng/mL.
2.76.1 1 10ng/mL.
2.76.2 1 1ug/mL.
2.76.2 1 100ng/mL.
2.76.2 1 10ng/mL.
2.76.3 1 1ug/mL.
2.76.3 1 100ng/mL.
2.76.3 1 10ng/mL.
1.37.1 1 1ug/mL.
1.37.1 1 100ng/mL.
1.37.1 1 10ng/mL.
1.37.2 1 1ug/mL.
1.37.2 1 100ng/mL.
1.37.2 1 10ng/mL.
1.37.3 1 1ug/mL.
1.37.3 1 100ng/mL.
1.37.3 1 10ng/mL.
Treatments

18P

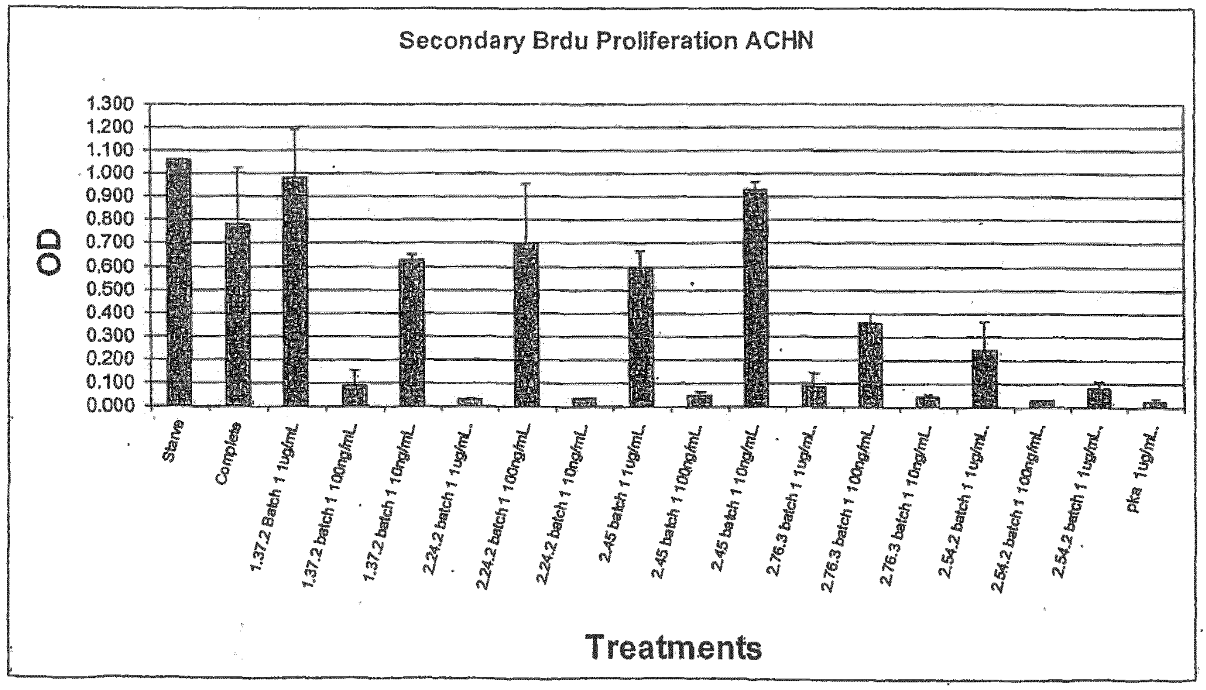
Secondary Brdu Proliferation ACHN
OD
1.300
1.200
1.100
1.000
0.900
0.800
0.700
0.600
0.500
0.400
0.300
0.200
0.100
0.000
Starve
Complete
1.37.2 Batch 1 1ug/mL
1.37.2 batch 1 100ng/mL
1.37.2 batch 1 10ng/mL
2.24.2 batch 1 1ug/mL.
2.24.2 batch 1 100ng/mL
2.24.2 batch 1 10ng/mL
2.45 batch 1 1ug/mL
2.45 batch 1 100ng/mL
2.45 batch 1 10ng/mL
2.76.3 batch 1 1ug/mL
2.76.3 batch 1 100ng/mL
2.76.3 batch 1 10ng/mL
2.54.2 batch 1 1ug/mL.
2.54.2 batch 1 100ng/mL
2.54.2 batch 1 1ug/mL.
pka 1ug/mL.
Treatments

18Q

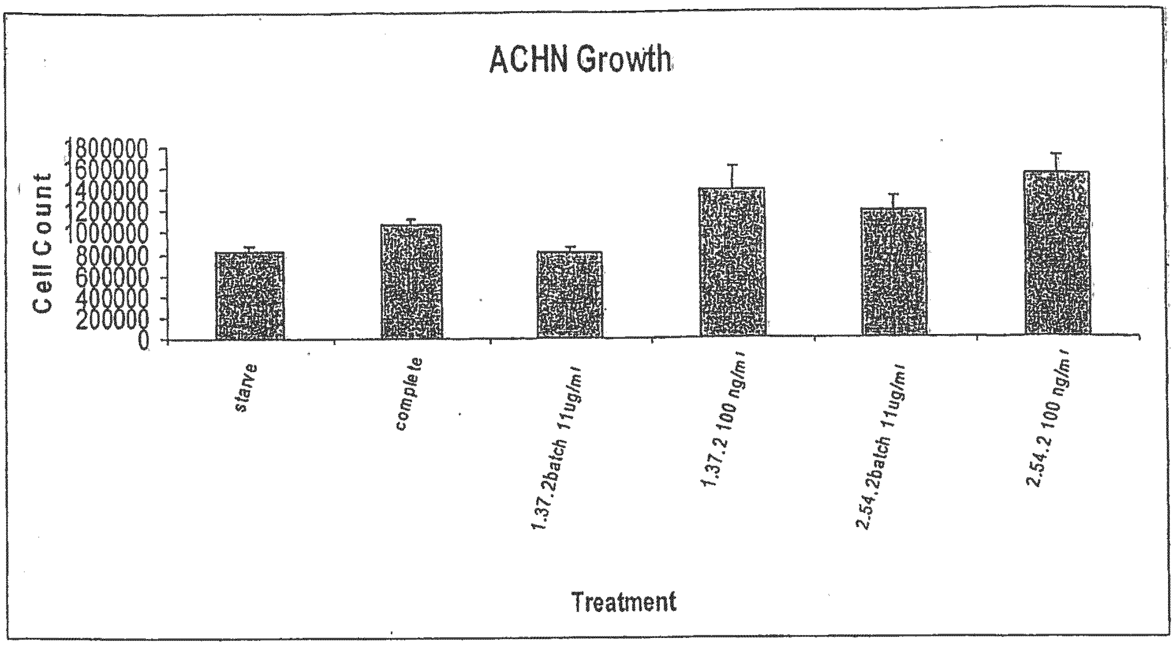
ACHN Growth
Cell Count
1800000
1600000
1400000
1200000
1000000
800000
600000
400000
200000
0
starve
complete
1.37.2batch 11ug/ml
1.37.2 100 ng/ml
2.54.2batch 11ug/ml
2.54.2 100 ng/ml
Treatment

18R

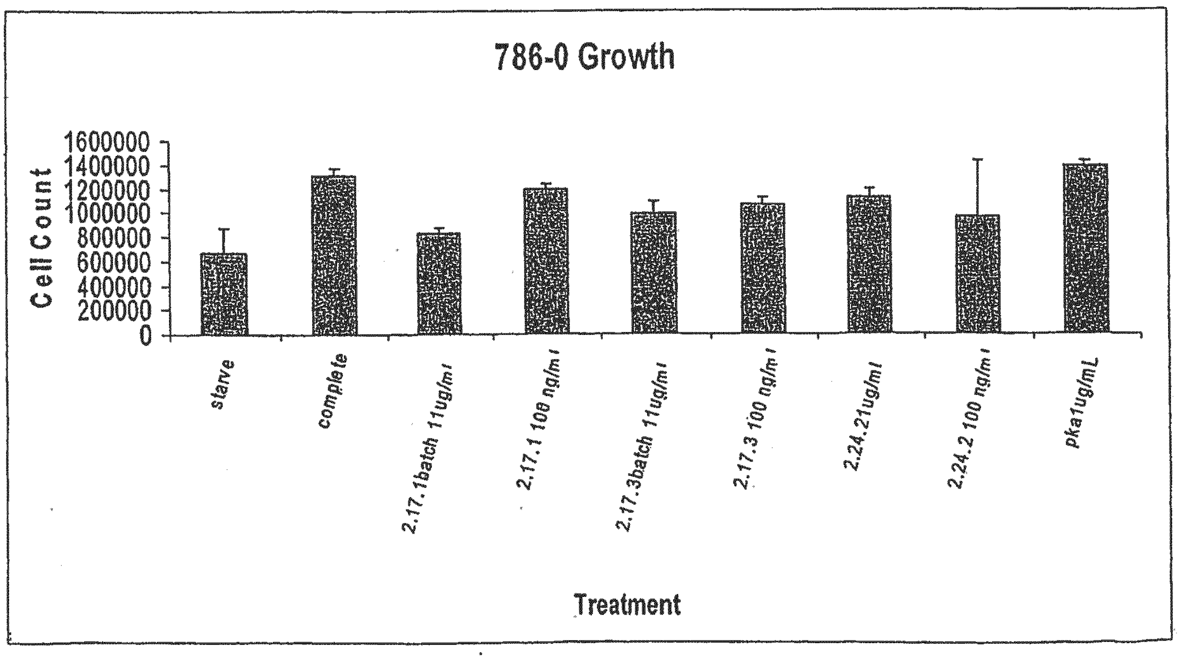
786-0 Growth
Cell Count
1600000
1400000
1200000
1000000
800000
600000
400000
200000
0
starve
complete
2.17.1batch 11ug/ml
2.17.1 100 ng/ml
2.17.3batch 11ug/ml
2.17.3 100 ng/ml
2.24.21ug/ml
2.24.2 100 ng/ml
pka1ug/mL
Treatment

18S

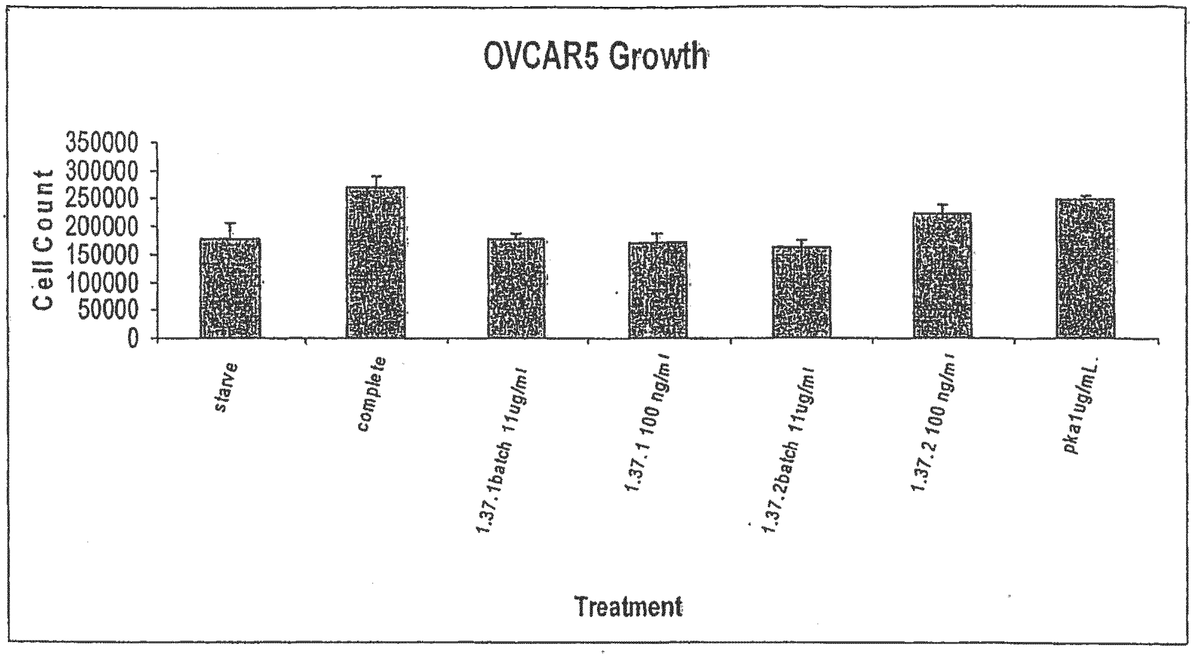
OVCAR5 Growth
Cell Count
350000
300000
250000
200000
150000
100000
50000
0
starve
complete
1.37.1batch 11ug/ml
1.37.1 100 ng/ml
1.37.2batch 11ug/ml
1.37.2 100 ng/ml
pka1ug/mL
Treatment

18T

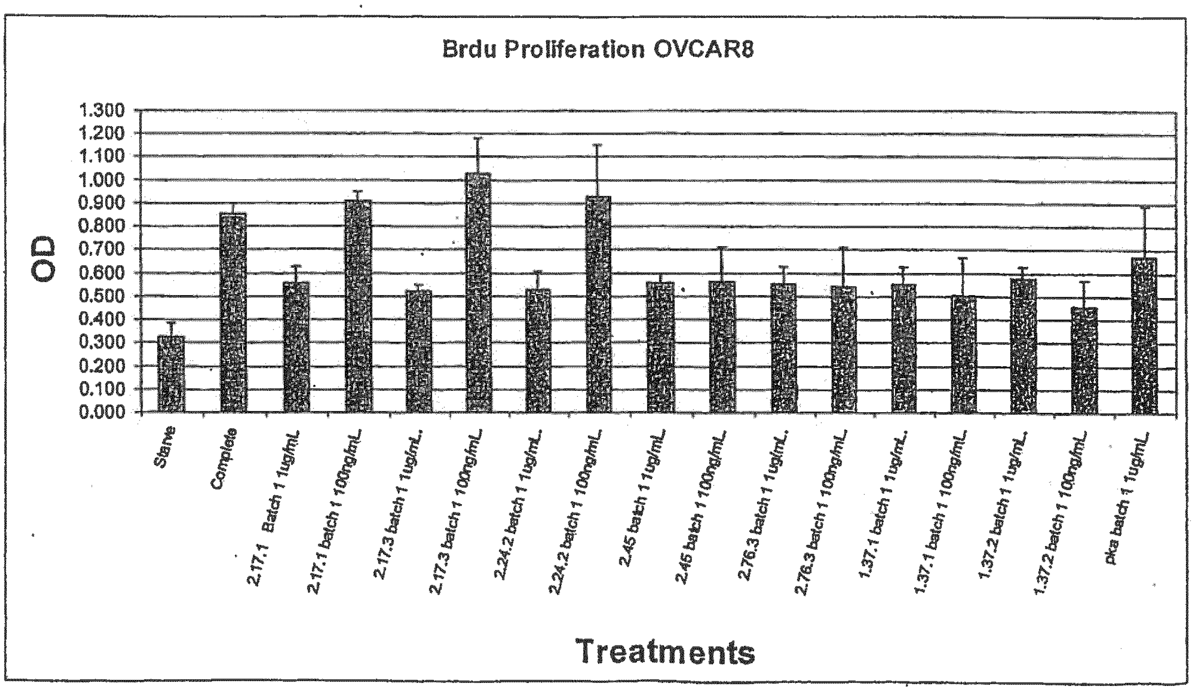
Brdu Proliferation OVCAR8
OD
1.300
1.200
1.100
1.000
0.900
0.800
0.700
0.600
0.500
0.400
0.300
0.200
0.100
0.000
Starve
Complete
2.17.1 Batch 1 1ug/mL
2.17.1 batch 1 100ng/mL
2.17.3 batch 1 1ug/mL
2.17.3 batch 1 100ng/mL
2.24.2 batch 1 1ug/mL
2.24.2 batch 1 100ng/mL
2.45 batch 1 1ug/mL
2.45 batch 1 100ng/mL
2.76.3 batch 1 1ug/mL
2.76.3 batch 1 100ng/mL
1.37.1 batch 1 1ug/mL
1.37.1 batch 1 100ng/mL
1.37.2 batch 1 1ug/mL
1.37.2 batch 1 100ng/mL
pka batch 1 1ug/mL
Treatments

Figure 19A

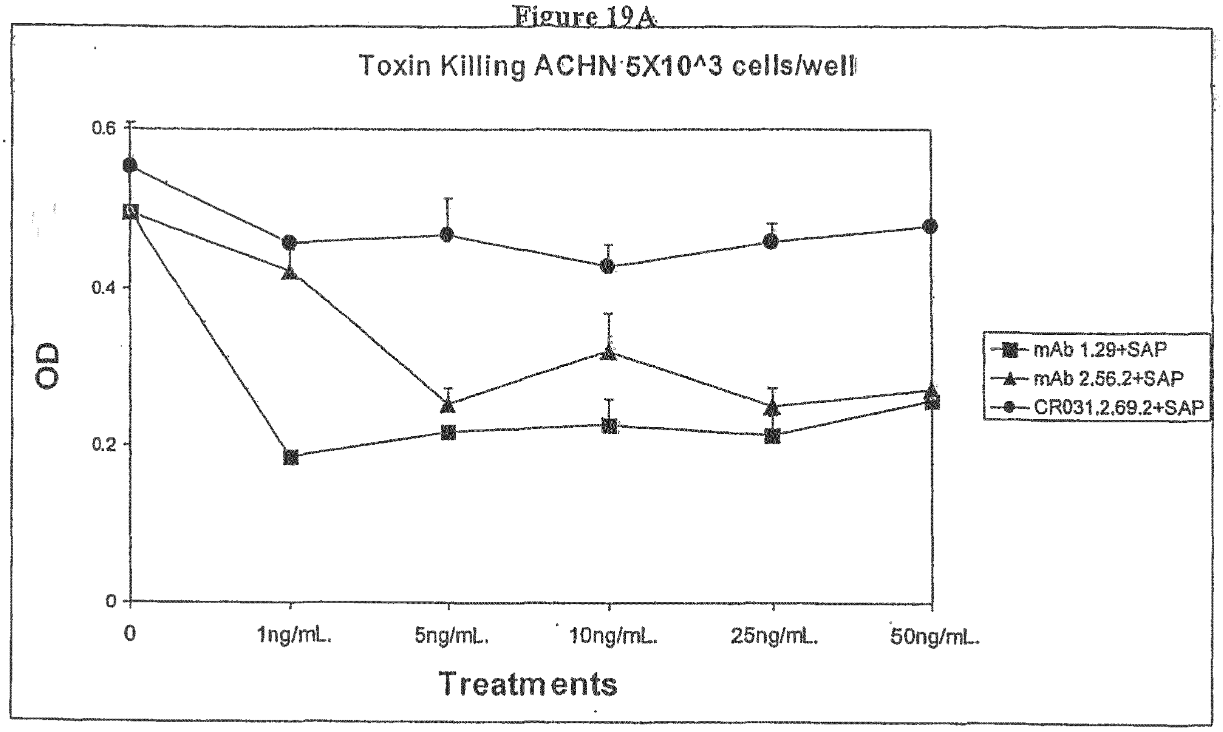


Figure 19B

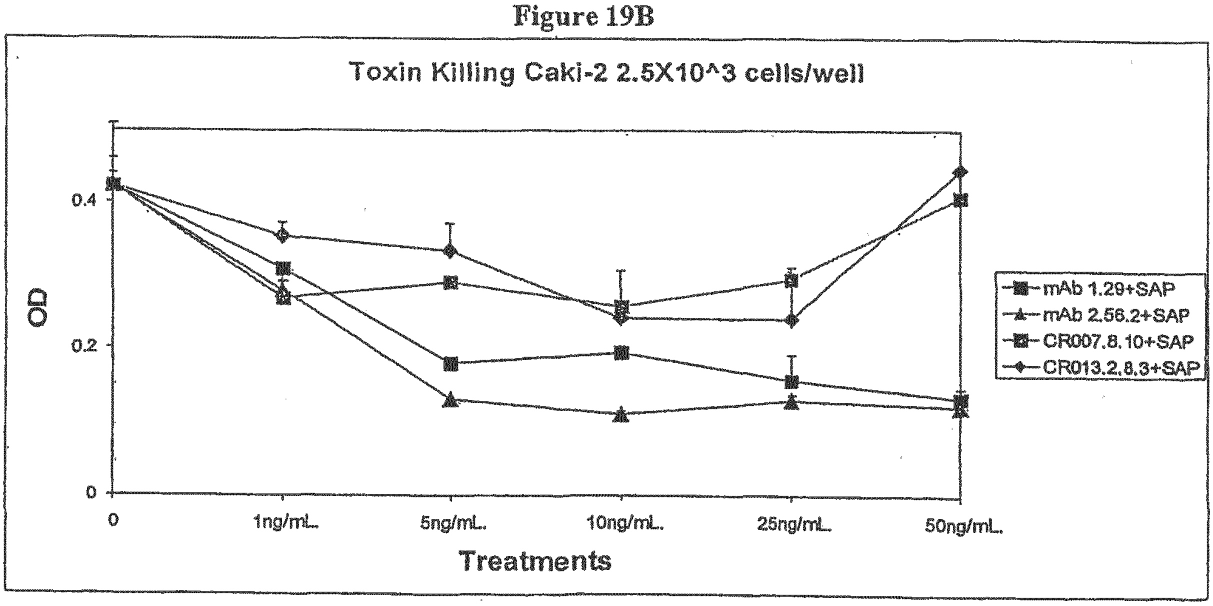

Figure 19C

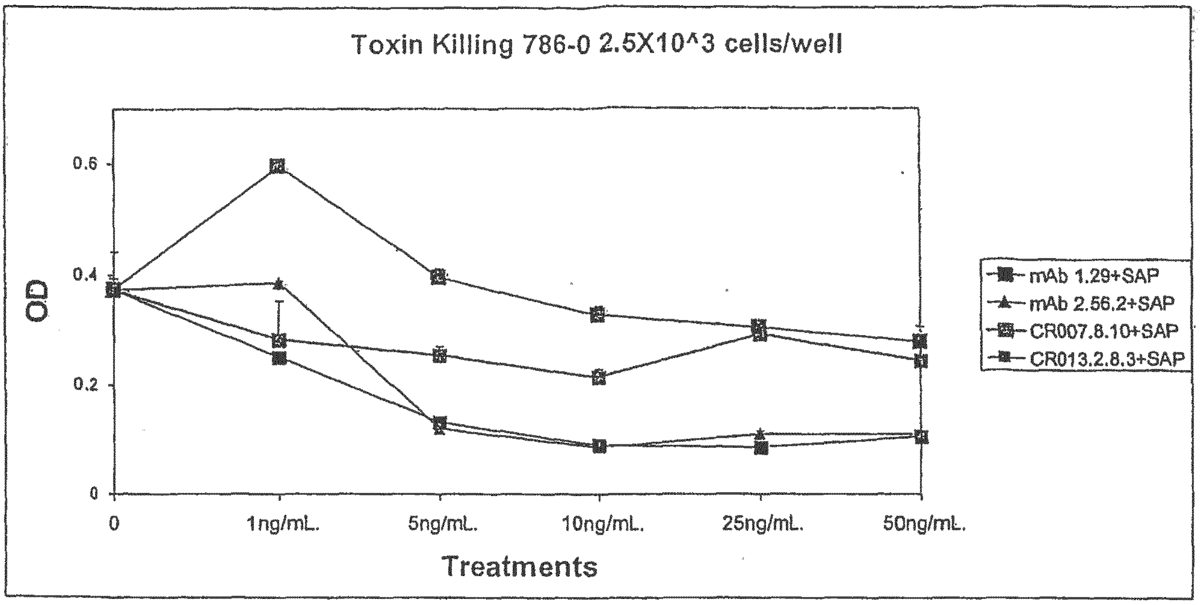


Figure 19D

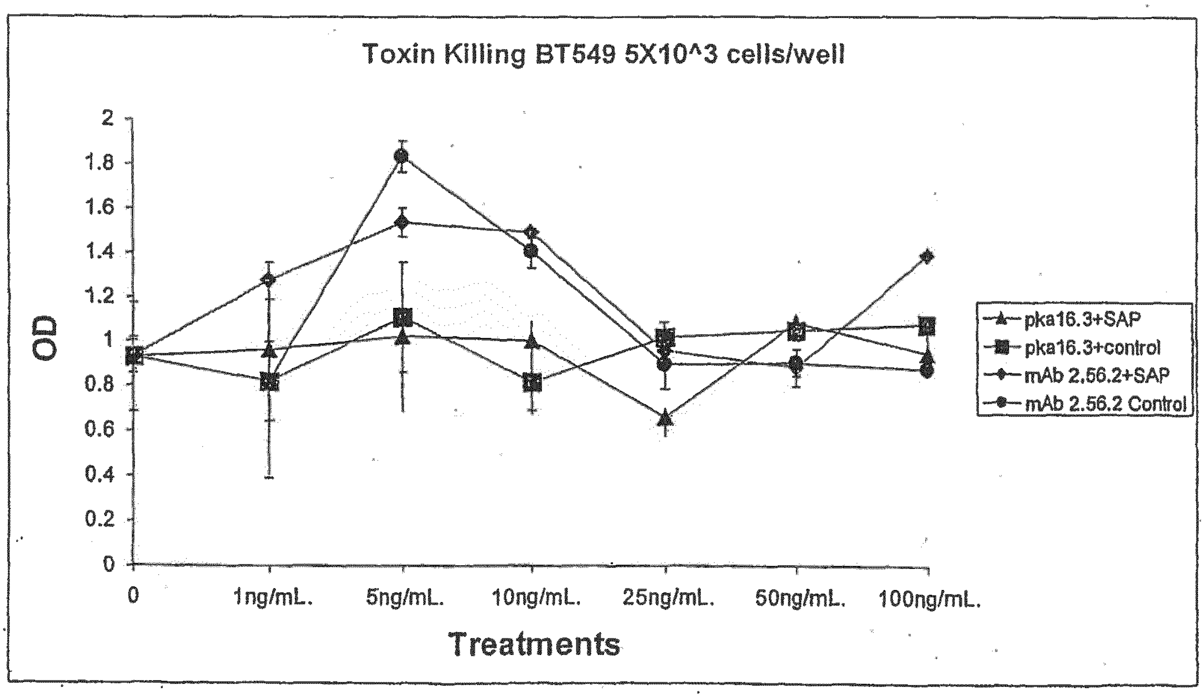

Figure 20

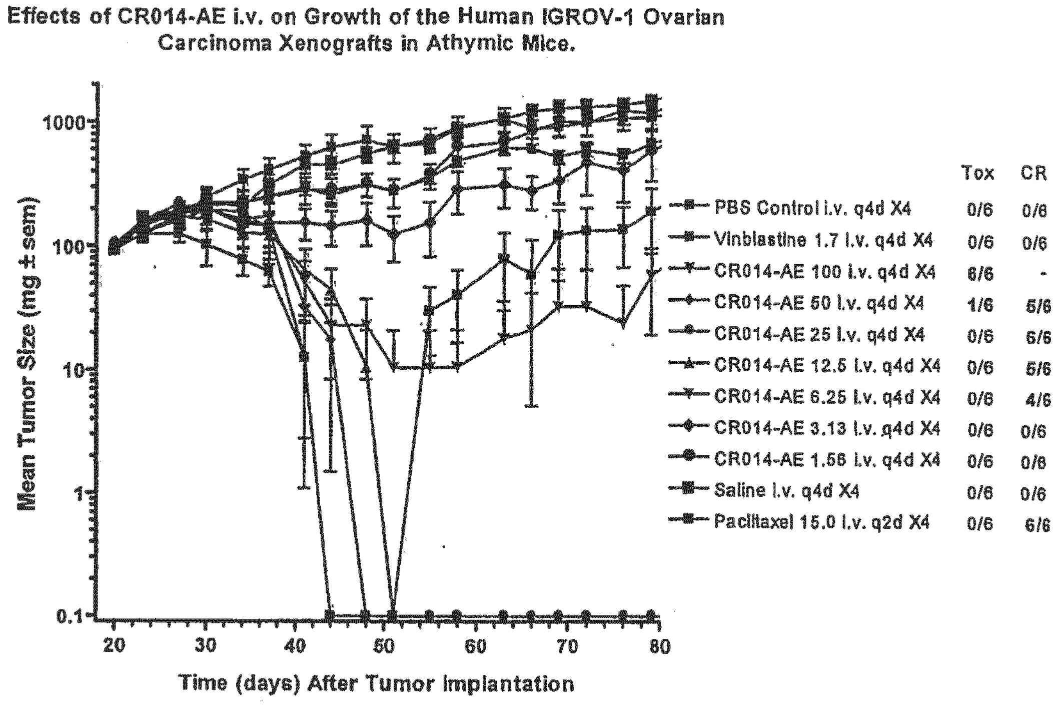
Effects of CR014-AE i.v. on Growth of the Human IGROV-1 Ovarian Carcinoma Xenografts in Athymic Mice.
Mean Tumor Size (mg ± sem)
1000
100
10
1
0.1
20
30
40
50
60
70
80
Time (days) After Tumor Implantation
Tox CR
PBS Control i.v. q4d X4 0/6 0/6
Vinblastine 1.7 i.v. q4d X4 0/6 0/6
CR014-AE 100 i.v. q4d X4 6/6 -
CR014-AE 50 i.v. q4d X4 1/6 5/6
CR014-AE 25 i.v. q4d X4 0/6 6/6
CR014-AE 12.5 i.v. q4d X4 0/6 5/6
CR014-AE 6.25 i.v. q4d X4 0/6 4/6
CR014-AE 3.13 i.v. q4d X4 0/6 0/6
CR014-AE 1.56 i.v. q4d X4 0/6 0/6
Saline i.v. q4d X4 0/6 0/6
Paclitaxel 15.0 i.v. q2d X4 0/6 6/6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5622861 A
- WO 9744460 A
- WO 9853071 A
- US 6664385 B
- WO 02098920 A
- WO 2004084823 A
- US 5151510 A, Stec
- US 46600890 A
- US 07610515 A
- US 07919297 A
- US 07922649 A
- US 08031801 B
- US 08112848 B
- US 08234145 B
- US 08376279 B
- US 08430938 B
- US 08464584 B
- US 08464582 B
- US 08463191 B
- US 08462837 B
- US 08486853 B
- US 08486857 B
- US 08486859 B
- US 08462513 B
- US 08724752 B
- US 08759620 B
- US 6162963 A
- US 6150584 A
- US 6114598 A
- US 6075181 A
- US 5939598 A
- JP 3068180 B
- JP 3068506 B
- JP 3068507 B
- EP 0463151 B1
- WO 9402602 A
- WO 9634096 A
- WO 9824893 A
- WO 0076310 A
- US 5545807 A, Surani
- US 5545806 A
- US 5625825 A
- US 5625126 A
- US 5633425 A
- US 5661016 A
- US 5770429 A
- US 5789650 A
- US 5814318 A
- US 5877397 A
- US 5874299 A
- US 6255458 B, Lonberg and Kay
- US 5591669 A
- US 6023010 A, Krimpenfort and Berns
- US 5612205 A
- US 5721367 A
- US 5789215 A, Berns
- US 5643763 A, Choi and Dunn
- US 57474890 A
- US 07575962 A
- US 07810279 A
- US 07853408 A
- US 07904068 A
- US 07990860 A
- US 08053131 A
- US 08096762 A
- US 08155301 A
- US 08161739 A
- US 08165699 A
- US 08209741 A
- EP 0546073 B1
- WO 9203918 A
- WO 9222645 A
- WO 9222647 A
- WO 9222670 A
- WO 9312227 A
- WO 9400569 A
- WO 9425585 A
- WO 9614436 A
- WO 9713852 A
- WO 9824884 A
- US 5981175 A
- WO 9202190 A
- US 5530101 A
- US 5585089 A
- US 5693761 A
- US 5693792 A
- US 5714350 A
- US 5777085 A
- US 4683195 A
- US 4683202 A
- US 5733743 A
- US 5703057 A
- US 4816397 A
- US 5916771 A
- US 6207418 A
- US 5194594 A
- US 4681581 A
- US 4735210 A

- US 5101827 A
- US 5102990 A
- US RE35500 E
- US 5648471 A
- US 5697902 A
- WO 9429444 A
- WO 9738137 A
- US 5460785 A
- US 4831175 A
- US 5099069 A
- US 5246692 A
- US 5286850 A
- US 5124471 A
- US 6441163 B
- US 4399216 A
- US 4912040 A
- US 4740461 A
- US 4959455 A
- US 3773919 A
- EP 58481 A
- EP 133988 A
- US DE3218121 A
- EP 52322 A
- EP 36676 A
- EP 88046 A
- EP 143949 A
- EP 142641 A
- EP 83118008 A
- US 4485045 A
- US 4544545 A
- EP 102324 A
- US 20030157730 A
- WO 0125433 A
- US 60735574 B

**Non-patent literature cited in the description**

- **KUCHROO et al.** *Nat Rev Immunol,* 2003, vol. 3, 454-462
- **MCINTIRE et al.** *Nat Immunol,* 2001, vol. 2, 1109-1116
- **SHEVACH.** *Nat Rev Immunol,* 2002, vol. 2, 389-400
- **WILLS-KARP et al.** *Nat Immunol,* 2003, vol. 4, 1050-1052
- **KAPLAN et al.** *EMBO J,* 1996, vol. 15 (16), 4282-96
- **ICHIMURA et al.** *J Biol Chem,* 1998, vol. 273, 4135-4142
- **HAN et al.** *Kidney Int,* 2002, vol. 62, 237-244
- **FEIGELSTOCK et al.** *J Virology,* 1998, vol. 72 (8), 6621-6628
- **FEIGELSTOCK et al.** *J Virol,* 1998, vol. 72 (8), 6621-8
- **SILBERSTEIN et al.** *J Virol,* 2001, vol. 75 (2), 717-25
- **ICHIMURA et al.** *J Biol Chem,* 1998, vol. 273 (7), 4135-42
- **PREST et al.** *FASEB J,* 2002, vol. 16 (6), 592-4
- **CHEN et al.** *Biochem Biophys Res Commun,* 2000, vol. 274 (3), 576-82
- **WILLS-KARP.** *Nature Immunology,* 2001, vol. 2, 1095-1096
- **MCINTIRE et al.** *Nature Immunology,* 2001, vol. 2, 1109-1116
- **KABAT et al.** Sequences of Proteins of Immunological Interest. NIH Publication 91-3242, 1991, 1-3
- **SAMBROOK et al.** Molecular Cloning: A Laboratory Manual. Cold Spring Harbor Laboratory Press, 1989
- **LA PLANCHE et al.** *Nucl. Acids Res.,* 1986, vol. 14, 9081
- **STEC et al.** *J. Am. Chem. Soc.,* 1984, vol. 106, 6077
- **STEIN et al.** *Nucl. Acids Res.,* 1988, vol. 16, 3209
- **ZON et al.** *Anti-Cancer Drug Design,* 1991, vol. 6, 539
- **ZON et al.** Oligonucleotides and Analogues: A Practical Approach. Oxford University Press, 1991, 87-108
- **UHLMANN ; PEYMAN.** *Chemical Reviews,* 1990, vol. 90, 543
- **DAYHOFF, M.O.** Atlas of Protein Sequence and Structure. National Biomedical Research Foundation, 1972, vol. 5, 101-110
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. (U.SA.),* 1988, vol. 85, 2444
- Immunology - A Synthesis. Sinauer Associates, 1991
- **BOWIE et al.** *Science,* 1991, vol. 253, 164
- Proteins, Structures and Molecular Principles. W. H. Freeman and Company, 1984
- Introduction to Protein Structure. Garland Publishing, 1991
- **THORNTON et al.** *Nature,* 1991, vol. 354, 105
- **FAUCHERE.** *J. Adv. Drug Res.,* 1986, vol. 15, 29
- **VEBER ; FREIDINGER.** *TINS,* 1985, 392
- **EVANS et al.** *J. Med. Chem.,* 1987, vol. 30, 1229
- **RIZO ; GIERASCH.** *Ann. Rev. Biochem.,* 1992, vol. 61, 387
- The McGraw-Hill Dictionary of Chemical Terms. McGraw-Hill, 1985

- Fundamental Immunology. Raven Press, 1989
- **KABAT.** Sequences of Proteins of Immunological Interest. National Institutes of Health, 1987
- **CHOTHIA ; LESK.** *J. Mol. Biol.,* 1987, vol. 196, 901-917
- **CHOTHIA et al.** *Nature,* 1989, vol. 342, 878-883
- **SONGSIVILAI ; LACHMANN.** *Clin. Exp. Immunol.,* 1990, vol. 79, 315-321
- **KOSTELNY et al.** *J. Immunol.,* 1992, vol. 148, 1547-1553
- **GREEN et al.** *Nature Genetics,* 1994, vol. 7, 13-21
- **MENDEZ et al.** *Nature Genetics,* 1997, vol. 15, 146-156
- **GREEN ; JAKOBOVITS.** *J. Exp. Med.,* 1998, vol. 188, 483-495
- **WINTER ; HARRIS.** *Immunol Today,* 1993, vol. 14, 43-46
- **WRIGHT et al.** *Crit, Reviews in Immunol.,* 1992, vol. 12, 125-168
- **LIU et al.** *P.N.A.S,* 1987, vol. 84, 3439
- *J. Immunol.,* 1987, vol. 139, 3521
- **KABAT et al.** Sequences of Proteins of Immunological Interest. N.I.H. publication no. 91-3242, 1991
- **OKAYAMA et al.** *Mol. Cell. Bio.,* 1983, vol. 3, 280
- **GORMAN et al.** *P.N.A.S.,* 1982, vol. 79, 6777
- **GROSSCHEDL et al.** *Cell,* 1985, vol. 41, 885
- **HANES ; PLUCTHAU.** *PNAS USA,* 1997, vol. 94, 4937-4942
- **SMITH.** *Gene,* 1988, vol. 73, 305-318
- **SCOTT.** *TIBS,* 1992, vol. 17, 241-245
- **CWIRLA et al.** *PNAS USA,* 1990, vol. 87, 6378-6382
- **RUSSEL et al.** *Nucl. Acids Res.,* 1993, vol. 21, 1081-1085
- **HOGANBOOM et al.** *Immunol. Reviews,* 1992, vol. 130, 43-68
- **CHISWELL ; MCCAFFERTY.** *TIBTECH,* 1992, vol. 10, 80-84
- **FANGER et al.** *Immunol Methods,* 1994, vol. 4, 72-81
- **TRAUNECKER et al.** *Int. J. Cancer,* 1992, vol. 7, 51-52
- **DEO et al.** *Blood,* 1997, vol. 18, 127
- **VALERIUS et al.** *Blood,* 1997, vol. 90, 4485-4492
- **VITETTA.** *Immunol Today,* 1993, vol. 14, 252
- **JUNGHANS et al.** Cancer Chemotherapy and Biotherapy. Lippincott Raven, 1996, 655-686
- **HOUGHTEN et al.** *Biotechniques,* 1992, vol. 13, 412-421
- **HOUGHTEN.** *PNAS USA,* 1985, vol. 82, 5131-5135
- **PINALLA et al.** *Biotechniques,* 1992, vol. 13, 901-905
- **BLAKE ; LITZI-DAVIS.** *BioConjugate Chem.,* 1992, vol. 3, 510-513
- **CHEN et al.** *Human Gene Therapy,* 1994, vol. 5, 595-601
- **MARASCO.** *Gene Therapy,* 1997, vol. 4, 11-15
- **CAPSEY et al.** Genetically Engineered Human Therapeutic Drugs. Stockton Press, 1988
- **NARANG.** *Tetrahedron,* 1983, vol. 39, 3
- **ITAKURA et al.** *Annu. Rev. Biochem.,* 1984, vol. 53, 323
- **ITAKURA et al.** *Science,* 1984, vol. 198, 1056
- **IKE et al.** *Nucl. Acid Res.,* 1993, vol. 11, 477
- **ALLEN.** *Nat. Rev. Cancer,* 2002, vol. 2 (10), 750-63
- *Immunol. Cell Biol.,* vol. 65, 111-125
- **MOHAMMAD et al.** *Int. J. Oncol.,* 1999, vol. 15 (2), 367-72
- **DORONINA et al.** *Nature Biotechnology,* 2003, vol. 21 (7), 778-784
- **OGAWA et al.** *Toxicol Lett.,* 2001, vol. 121 (2), 97-106
- *TOXICOL LETT.,* vol. 21 (3), 778-784
- **MOHAMMAD et al.** *Int. J. Oncol.,* August 1999, vol. 15 (2), 367-72
- **BABCOOK et al.** *Proc. Natl. Acad. Sci. USA,* 1996, vol. 93, 7843-7848
- **CHEN et al.** *Anal. Biochem.,* 1995, vol. 227, 168-175
- Remington: The Science and Practice of Pharmacy. Lippincott Williams & Wilkens Publishers, 2003
- **LANGER et al.** *J. Biomed Mater. Res.,* 1981, vol. 15, 167-277
- **LANGER.** *Chem. Tech.,* 1982, vol. 12, 98-105
- **SIDMAN et al.** *Biopolymers,* 1983, vol. 22, 547-556
- **EPSTEIN et al.** *Proc. Natl. Acad. Sci. USA,* 1985, vol. 82, 3688-3692
- **HWANG et al.** *Proc. Natl. Acad. Sci. USA,* 1980, vol. 77, 4030-4034
- **BALDRICK P.** Pharmaceutical excipient development: the need for preclinical guidance. *Regul. Toxicol. Pharmacol.,* 2000, vol. 32 (2), 210-8
- **WANG W.** Lyophilization and development of solid protein pharmaceuticals. *Int. J. Pharm.,* vol. 203 (1-2), 1-60
- **CHARMAN WN.** Lipids, lipophilic drugs, and oral drug delivery-some emerging concepts. *J Pharm Sci .,* 2000, vol. 89 (8), 967-78
- **POWELL et al.** Compendium of excipients for parenteral formulations. *PDA J Pharm Sci Technol.,* 1998, vol. 52, 238-311

- **ICHIMURA et al.** *J. Biol. Chem.,* 1998, vol. 273 (7), 4135-42
- **YANG et al.** *Cancer Res.,* 1999
- **JOHNSON JI ; DECKER S ; ZAHAREVITZ D ; RUBINSTEIN LV ; VENDITTI JM ; SCHEPARTZ S ; KALYANDRUG S ; CHRISTIAN M ; ARBUCK S ; HOLLINGSHEAD M.** Relationships between drug activity in NCI preclinical in vitro and in vivo models and early clinical trials. *Br J Cancer,* 2001, vol. 84, 1424-1431
- Development of human tumor xenograft models for in vivo evaluation of new antitumor drugs. **DYKES DJ ; ABBOTT BJ ; MAYO JG ; HARRISON JR. SD ; LASTER JR WR ; SIMPSON-HERREN L ; GRISWOLD JR. DP.** Immunodeficient mice in Oncology. 1992, vol. 42, 1-22
- **MARKMAN, M.** Taxol: an important new drug in the management of epithelial ovarian cancer. *Yale J Biol Med,* 1991, vol. 64 (6), 583-90

SEQUENCE LISTING

<110> CuraGen Corporation, et al.

<120> METHOD OF TREATING OVARIAN AND RENAL CANCER USING ANTIBODIES AGAINST T CELL IMMUNOGLOBULIN DOMAIN AND MUCIN DOMAIN 1 (TIM-1) ANTIGEN

<130> Cura 965A WO

<150> 60/735,574
<151> 2005-11-10

<160> 141

<170> FastSEQ for Windows Version 4.0

<210> 1
<211> 509
<212> DNA
<213> Homo Sapiens

<400> 1

```
tgggtcctgt cccaggtgca gctgcaggag tcgggcccag gactggtgaa gccttcggag 60
accctgtccc tcacctgcac tgtctctggt ggctccgtca gcagtggtgg ttactactgg 120
agctggatcc ggcagccccc agggaaggga ctggagtgga ttgggtttat ctattacact 180
gggagcacca actacaaccc ctccctcaag agtcgagtct ccatatcagt agacacgtcc 240
aagaaccagt tctccctgaa gctgagctct gtgaccgctg cggacgcggc cgtgtattac 300
tgtgcgagag attatgactg gagcttccac tttgactact ggggccaggg aaccctggtc 360
accgtctcct cagcctccac caagggccca tcggtcttcc ccctggcgcc ctgctccagg 420
agcacctccg agagcacagc ggccctgggc tgcctggtca aggactactt ccccgaaccg 480
gtgacggtgt cgtggaactc aggcgctct                                   509
```

<210> 2
<211> 121
<212> PRT
<213> Homo Sapiens

<400> 2

```
Gln Val Gln Leu Gln Glu Ser Gly Pro Gly Leu Val Lys Pro Ser Glu
1               5                   10                  15
Thr Leu Ser Leu Thr Cys Thr Val Ser Gly Gly Ser Val Ser Ser Gly
            20                  25                  30
Gly Tyr Tyr Trp Ser Trp Ile Arg Gln Pro Pro Gly Lys Gly Leu Glu
        35                  40                  45
Trp Ile Gly Phe Ile Tyr Tyr Thr Gly Ser Thr Asn Tyr Asn Pro Ser
    50                  55                  60
Leu Lys Ser Arg Val Ser Ile Ser Val Asp Thr Ser Lys Asn Gln Phe
65                  70                  75                  80
Ser Leu Lys Leu Ser Ser Val Thr Ala Ala Asp Ala Ala Val Tyr Tyr
                85                  90                  95
Cys Ala Arg Asp Tyr Asp Trp Ser Phe His Phe Asp Tyr Trp Gly Gln
            100                 105                 110
Gly Thr Leu Val Thr Val Ser Ser Ala
        115                 120
```

<210> 3
<211> 504
<212> DNA
<213> Homo Sapiens

<400> 3

```
cagctcctgg ggctcctgct gctctggttc ccaggtgcca ggtgtgacat ccagatgacc 60
cagtctccat cctccctgtc tgcatctata ggagacagag tcaccatcac ttgccgggca 120
agtcagggca ttagaaatga tttaggctgg tatcagcaga aaccagggaa agcccctaag 180
cgcctgatct atgctgcatc cagtttgcaa agtggggtcc catcaaggtt cagcggcagt 240
ggatctggga cagaattcac tctcacaatc agcagcctgc agcctgaaga ttttgcaact 300
tattactgtc tacagcataa tagttaccct ctcactttcg gcggagggac caaggtggag 360
atcaaacgaa ctgtggctgc accatctgtc ttcatcttcc cgccatctga tgagcagttg 420
aaatctggaa ctgcctctgt tgtgtgcctg ctgaataact tctatcccag agaggccaaa 480
gtacagtgga aggtggataa cgcc                                        504
```

<210> 4
<211> 108
<212> PRT
<213> Homo Sapiens

<400> 4

```
Asp Ile Gln Met Thr Gln Ser Pro Ser Ser Leu Ser Ala Ser Ile Gly
 1               5                   10                  15
Asp Arg Val Thr Ile Thr Cys Arg Ala Ser Gln Gly Ile Arg Asn Asp
            20                  25                  30
Leu Gly Trp Tyr Gln Gln Lys Pro Gly Lys Ala Pro Lys Arg Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Leu Gln His Asn Ser Tyr Pro Leu
                85                  90                  95
Thr Phe Gly Gly Gly Thr Lys Val Glu Ile Lys Arg
            100                 105
```

<210> 5
<211> 469
<212> DNA
<213> Homo Sapiens

<400> 5

```
cagtgtgagg tgcagctggt ggagtctggg ggaggcttgg tccagcctgg ggggtccctg 60
agactctcct gtgcagcctc tggattcacc tttactaact attggatgag ctgggtccgc 120
caggctccag ggaaggggct ggagtgggtg gccaacatac agcaagatgg aagtgagaaa 180
tactatgtgg actctgtgag gggccgattc accatctcca gagacaacgc caagaactca 240
ctgtatctgc aaatgaacag cctgagagcc gaggactcgg ctgtgtatta ctgtgcgaga 300
tgggactact ggggccaggg aaccctggtc accgtctcct cagcctccac caagggccca 360
tcggtcttcc ccctggcgcc ctgctccagg agcacctccg agagcacagc ggccctgggc 420
tgcctggtca aggactactt ccccgaaccg gtgagcggtg tcgtggaac             469
```

<210> 6
<211> 113
<212> PRT
<213> Homo Sapiens

<400> 6

```
Glu Val Gln Leu Val Glu Ser Gly Gly Gly Leu Val Gln Pro Gly Gly
 1               5                   10                  15
Ser Leu Arg Leu Ser Cys Ala Ala Ser Gly Phe Thr Phe Thr Asn Tyr
            20                  25                  30
Trp Met Ser Trp Val Arg Gln Ala Pro Gly Lys Gly Leu Glu Trp Val


        35                  40                  45
Ala Asn Ile Gln Gln Asp Gly Ser Glu Lys Tyr Tyr Val Asp Ser Val
    50                  55                  60
Arg Gly Arg Phe Thr Ile Ser Arg Asp Asn Ala Lys Asn Ser Leu Tyr
65                  70                  75                  80
Leu Gln Met Asn Ser Leu Arg Ala Glu Asp Ser Ala Val Tyr Tyr Cys
                85                  90                  95
Ala Arg Trp Asp Tyr Trp Gly Gln Gly Thr Leu Val Thr Val Ser Ser
            100                 105                 110
Ala
```

<210> 7
<211> 454
<212> DNA
<213> Homo Sapiens

<400> 7

```
cttctggggc tgctaatgct ctgggtccct ggatccagtg gggatattgt gatgacccag 60
actccactct cctcaactgt catccttgga cagccggcct ccatctcctg caggtctagt 120
caaagcctcg tacacagtga tggaaacacc tacttgaatt ggcttcagca gaggccaggc 180
cagcctccaa gactcctaat ttatatgatt tctaaccggt tctctggggt cccagacaga 240
ttcagtggca gtggggcagg gacagatttc acactgaaaa tcagcagggt ggaagctgag 300
gatgtcgggg tttattactg catgcaagct acagaatctc ctcagacgtt cggccaaggg 360
accaaggtgg aaatcaaacg aactgtggct gcaccatctg tcttcatctt cccgccatct 420
gatgagcagt tgaaatctgg aagggcctct gttg                             454
```

<210> 8
<211> 113
<212> PRT
<213> Homo Sapiens

<400> 8

```
Asp Ile Val Met Thr Gln Thr Pro Leu Ser Ser Thr Val Ile Leu Gly
1               5                   10                  15
Gln Pro Ala Ser Ile Ser Cys Arg Ser Ser Gln Ser Leu Val His Ser
            20                  25                  30
Asp Gly Asn Thr Tyr Leu Asn Trp Leu Gln Gln Arg Pro Gly Gln Pro
        35                  40                  45
Pro Arg Leu Leu Ile Tyr Met Ile Ser Asn Arg Phe Ser Gly Val Pro
    50                  55                  60
Asp Arg Phe Ser Gly Ser Gly Ala Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Thr Glu Ser Pro Gln Thr Phe Gly Gln Gly Thr Lys Val Glu Ile Lys
            100                 105                 110
Arg
```

<210> 9
<211> 529
<212> DNA
<213> Homo Sapiens

<400> 9

```
gagcagtcgg ggggaggcgt ggtaaagcct gggggtctc ttagactctc ctgtgcagcc 60
tctggattca ctttcagtaa cgcctggatg acctgggtcc gccaggctcc agggaagggg 120
ctggagtggg ttggccgtat taaaaggaga actgatggtg ggacaacaga ctacgctgca 180

cccgtgaaag gcagattcac catctcaaga gatgattcaa aaaacacgct gtatctgcaa 240
atgaacaacc tgaaaaacga ggacacagcc gtgtattact gtacctcagt cgataatgac 300
gtggactact ggggccaggg aaccctggtc accgtctcct cagcttccac caagggccca 360
tccgtcttcc ccctggcgcc ctgctccagg agcacctccg agagcacagc cgccctgggc 420
tgcctggtca aggactactt ccccgaaccg gtgacggtgt cgtggaactc aggcgccctg 480
accagcggcg tgcacacctt cccggctgtc ctacagtcct caggactct             529
```

<210> 10
<211> 119
<212> PRT
<213> Homo Sapiens

<400> 10

```
Asn Asn Asn Asn Glu Gln Ser Gly Gly Gly Val Val Lys Pro Gly Gly
 1               5                  10                  15
Ser Leu Arg Leu Ser Cys Ala Ala Ser Gly Phe Thr Phe Ser Asn Ala
            20                  25                  30
Trp Met Thr Trp Val Arg Gln Ala Pro Gly Lys Gly Leu Glu Trp Val
        35                  40                  45
Gly Arg Ile Lys Arg Arg Thr Asp Gly Gly Thr Thr Asp Tyr Ala Ala
    50                  55                  60
Pro Val Lys Gly Arg Phe Thr Ile Ser Arg Asp Asp Ser Lys Asn Thr
65                  70                  75                  80
Leu Tyr Leu Gln Met Asn Asn Leu Lys Asn Glu Asp Thr Ala Val Tyr
                85                  90                  95
Tyr Cys Thr Ser Val Asp Asn Asp Val Asp Tyr Trp Gly Gln Gly Thr
            100                 105                 110
Leu Val Thr Val Ser Ser Ala
        115
```

<210> 11
<211> 447
<212> DNA
<213> Homo Sapiens

<400> 11

```
ctgactcagt ctccactctc cctgcccgtc acccctggag agccggcctc catctcctgc 60
aggtctagtc agagcctcct gcatagtaat ggatacaact atttggattg gtacctgcag 120
aagccagggc agtctccaca gctcctgatc tatttgggtt ctaatcgggc ctccggggtc 180
cctgacaggt tcagtggcag tggatcaggc acagatttta cactgaaaat cagcagagtg 240
gaggctgagg atattggtct ttattactgc atgcaagctc tacaaactcc gctcactttc 300
ggcggaggga ccaaggtgga catcaaacga actgtggctg caccatctgt cttcatcttc 360
ccgccatctg atgagcagtt gaaatctgga actgcctctg ttgtgtgcct gctgaataac 420
ttctatccca gagaggccaa agtacag                                     447
```

<210> 12
<211> 113
<212> PRT
<213> Homo Sapiens

<400> 12

```
Asn Asn Asn Leu Thr Gln Ser Pro Leu Ser Leu Pro Val Thr Pro Gly
 1               5                  10                  15
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile


65                  70                  75                  80
Ser Arg Val Glu Ala Glu Asp Ile Gly Leu Tyr Tyr Cys Met Gln Ala
                85                  90                  95
Leu Gln Thr Pro Leu Thr Phe Gly Gly Gly Thr Lys Val Asp Ile Lys
            100                 105                 110
Arg
```

<210> 13
<211> 538

<212> DNA
<213> Homo Sapiens

<400> 13

```
caggtgcagc tggagcagtc gggggaggc ttggtacagc ctgggggtc cctgagactc 60
tcctgtgcag cctctggatt caccttcagt acctatagca tgaactgggt ccgccaggct 120
ccagggaagg ggctggagtg ggtttcatac attagaagta gtactagtac catatactat 180
gcagagtccc tgaagggccg attcaccatc tccagcgaca atgccaagaa ttcactatat 240
ctgcaaatga acagcctgag agacgaggac acggctgtgt attactgtgc gcgggacttt 300
gactactggg gccagggaac cctggtcacc gtctcctcag cttccaccaa gggcccatcc 360
gtcttccccc tggcgccctg ctccaggagc acctccgaga gcacagccgc cctgggctgc 420
ctggtcaagg actacttccc cgaaccggtg acggtgtcgt ggaactcagg cgccctgacc 480
agcggcgtgc acaccttccc ggctgtccta cagtcctcag gactctactc cctcagca   538
```

<210> 14
<211> 114
<212> PRT
<213> Homo Sapiens

<400> 14

```
Gln Val Gln Leu Glu Gln Ser Gly Gly Gly Leu Val Gln Pro Gly Gly
 1               5                  10                  15
Ser Leu Arg Leu Ser Cys Ala Ala Ser Gly Phe Thr Phe Ser Thr Tyr
            20                  25                  30
Ser Met Asn Trp Val Arg Gln Ala Pro Gly Lys Gly Leu Glu Trp Val
        35                  40                  45
Ser Tyr Ile Arg Ser Ser Thr Ser Thr Ile Tyr Tyr Ala Glu Ser Leu
    50                  55                  60
Lys Gly Arg Phe Thr Ile Ser Ser Asp Asn Ala Lys Asn Ser Leu Tyr
65                  70                  75                  80
Leu Gln Met Asn Ser Leu Arg Asp Glu Asp Thr Ala Val Tyr Tyr Cys
                85                  90                  95
Ala Arg Asp Phe Asp Tyr Trp Gly Gln Gly Thr Leu Val Thr Val Ser
            100                 105                 110
Ser Ala
```

<210> 15
<211> 490
<212> DNA
<213> Homo Sapiens

<400> 15

```
gaaatccagc tgactcagtc tccactctcc tcacctgtca cccttggaca gccggcctcc 60
atctcctgca ggtctagtca aagcctcgta cacagtgatg gagacaccta cttgaattgg 120
cttcagcaga ggccaggcca gcctccaaga ctcctaattt ataagatttc tacccggttc 180
tctggggtcc ctgacagatt cagtggcagt ggggcaggga cagatttcac actgaaaatc 240
agcagggtgg agactgacga tgtcgggatt tattactgca tgcaaactac acaaattcct 300
caaatcacct tcggccaagg gacacgactg gagattaaac gaactgtggc tgcaccatct 360

gtcttcatct tcccgccatc tgatgagcag ttgaaatctg gaactgcctc tgttgtgtgc 420
ctgctgaata acttctatcc cagagaggcc aaagtacagt ggaaggtgga taacgccctc 480
caatcgggta                                                        490
```

<210> 16
<211> 114
<212> PRT

<213> Homo Sapiens

<400> 16

```
Glu Ile Gln Leu Thr Gln Ser Pro Leu Ser Ser Pro Val Thr Leu Gly
 1               5                  10                  15
Gln Pro Ala Ser Ile Ser Cys Arg Ser Ser Gln Ser Leu Val His Ser
            20                  25                  30
Asp Gly Asp Thr Tyr Leu Asn Trp Leu Gln Gln Arg Pro Gly Gln Pro
        35                  40                  45
Pro Arg Leu Leu Ile Tyr Lys Ile Ser Thr Arg Phe Ser Gly Val Pro
    50                  55                  60
Asp Arg Phe Ser Gly Ser Gly Ala Gly Thr Asp Phe Thr Leu Lys Ile
65                  70                  75                  80
Ser Arg Val Glu Thr Asp Asp Val Gly Ile Tyr Tyr Cys Met Gln Thr
                85                  90                  95
Thr Gln Ile Pro Gln Ile Thr Phe Gly Gln Gly Thr Arg Leu Glu Ile
            100                 105                 110
Lys Arg
```

<210> 17
<211> 568
<212> DNA
<213> Homo Sapiens

<400> 17

```
caggtgcagc tggagcagtc gggggaggc gtggtccagc ctgggaggtc cctgagactc 60
tcctgtgcag cgtctggatt caccttcagt cgctatggca tgcactgggt ccgccaggct 120
ccaggcaagg ggctgaaatg ggtggcagtt atatggtatg atggaagtaa taaactctat 180
gcagactccg tgaagggccg attcaccatc tccagagaca attccaagaa cacgctgtat 240
ctgcaaatga acagcctgag agccgaggac acggctgtgt attactgtgc gagagattac 300
tatgataata gtagacatca ctgggggttt gactactggg gccagggaac cctggtcacc 360
gtctcctcag cttccaccaa gggcccatcc gtcttccccc tggcgccctg ctccaggagc 420
acctccgaga gcacagccgc cctgggctgc ctggtcaagg actacttccc cgaaccggtg 480
acggtgtcgt ggaactcagg cgccctgacc agcggcgtgc acaccttccc ggctgtccta 540
cagtcctcag gactctactc cctcagca                                   568
```

<210> 18
<211> 124
<212> PRT
<213> Homo Sapiens

<400> 18

```
Gln Val Gln Leu Glu Gln Ser Gly Gly Gly Val Val Gln Pro Gly Arg
 1               5                  10                  15
Ser Leu Arg Leu Ser Cys Ala Ala Ser Gly Phe Thr Phe Ser Arg Tyr
            20                  25                  30
Gly Met His Trp Val Arg Gln Ala Pro Gly Lys Gly Leu Lys Trp Val
        35                  40                  45
Ala Val Ile Trp Tyr Asp Gly Ser Asn Lys Leu Tyr Ala Asp Ser Val
    50                  55                  60
Lys Gly Arg Phe Thr Ile Ser Arg Asp Asn Ser Lys Asn Thr Leu Tyr
65                  70                  75                  80
```

```
Leu Gln Met Asn Ser Leu Arg Ala Glu Asp Thr Ala Val Tyr Tyr Cys
                85                  90                  95
Ala Arg Asp Tyr Tyr Asp Asn Ser Arg His His Trp Gly Phe Asp Tyr
            100                 105                 110
Trp Gly Gln Gly Thr Leu Val Thr Val Ser Ser Ala
        115                 120
```

<210> 19
<211> 472
<212> DNA
<213> Homo Sapiens

<400> 19

```
gacatccagc tgacccagtc tccatcctcc ctgtctgcat ctgtaggaga cagagtcacc 60
atcacttgcc gggcaagtca gagtatttat agttatttaa attggtatca gcagaaacca 120
gggaaagccc ctaagctcct gatctatgct gcatccagtt tgcaaagtgg ggtcccatcc 180
aggttcagtg gcagtggatc tgggacagat ttcactctca ccatcagcag tctgcaacct 240
gaagattttg caacttacta ctgtcaacag agttacagta cccctccgac gttcggccaa 300
gggaccaagg tggaaatcaa acgaactgtg gctgcaccat ctgtcttcat cttcccgcca 360
tctgatgagc agttgaaatc tggaactgcc tctgttgtgt gcctgctgaa taacttctat 420
cccagagagg ccaaagtaca gtggaaggtg gataacgccc tccaatcggg ta         472
```

<210> 20
<211> 108
<212> PRT
<213> Homo Sapiens

<400> 20

```
Asp Ile Gln Leu Thr Gln Ser Pro Ser Ser Leu Ser Ala Ser Val Gly
 1              5                   10                  15
Asp Arg Val Thr Ile Thr Cys Arg Ala Ser Gln Ser Ile Tyr Ser Tyr
            20                  25                  30
Leu Asn Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Ser Tyr Ser Thr Pro Pro
                85                  90                  95
Thr Phe Gly Gln Gly Thr Lys Val Glu Ile Lys Arg
            100                 105
```

<210> 21
<211> 528
<212> DNA
<213> Homo Sapiens

<400> 21

```
cagtcggggg gaggcttggt aaagcctggg gggtccctta gactctcctg tgcagcctct 60
ggattcactt tcagtaacgc ctggatgacc tgggtccgcc aggctccagg gaaggggctg 120
gagtgggttg gccgtattaa aaggaaaact gatggtggga caacagacta cgctgcaccc 180
gtgaaaggca gattcaccat ctcaagagat gattcagaaa acacgctgta tctgcaaatg 240
aacagcctgg aaaccgagga cacagccgtg tattactgta ccacagtcga taacagtggt 300
gactactggg gccagggaac cctggtcacc gtctcctcag cttccaccaa gggcccatcc 360
gtcttccccc tggcgccctg ctccaggagc acctccgaga gcacagccgc cctgggctgc 420
ctggtcaagg actacttccc cgaaccggtg acggtgtcgt ggaactcagg cgccctgacc 480
agcggcgtgc acaccttccc ggctgtccta cagtcctcag gactctct              528
```

<210> 22
<211> 119
<212> PRT
<213> Homo Sapiens

<400> 22

```
Asn Asn Asn Asn Asn Gln Ser Gly Gly Gly Leu Val Lys Pro Gly Gly
 1               5                   10                  15
Ser Leu Arg Leu Ser Cys Ala Ala Ser Gly Phe Thr Phe Ser Asn Ala
            20                  25                  30
Trp Met Thr Trp Val Arg Gln Ala Pro Gly Lys Gly Leu Glu Trp Val
        35                  40                  45
Gly Arg Ile Lys Arg Lys Thr Asp Gly Gly Thr Thr Asp Tyr Ala Ala
    50                  55                  60
Pro Val Lys Gly Arg Phe Thr Ile Ser Arg Asp Asp Ser Glu Asn Thr
65                  70                  75                  80
Leu Tyr Leu Gln Met Asn Ser Leu Glu Thr Glu Asp Thr Ala Val Tyr
                85                  90                  95
Tyr Cys Thr Thr Val Asp Asn Ser Gly Asp Tyr Trp Gly Gln Gly Thr
            100                 105                 110
Leu Val Thr Val Ser Ser Ala
        115
```

<210> 23
<211> 466
<212> DNA
<213> Homo Sapiens

<400> 23

```
actcagtctc cactctccct gcccgtcacc cctggagagc cggcctccat ctcctgcagg 60
tctagtcaga gcctcctgca tagtaatgga tacaactatt tggattggta cctgcagaag 120
ccagggcagt ctccacagct cctgatctat ttgggttcta atcgggcctc cggggtccct 180
gacaggttca gtggcagtgg atcaggcaca gattttacac tgaaaatcag cagagtggag 240
gctgaggatg ttggggttta ttactgcatg caagctctac aaactccgct cactttcggc 300
ggagggacca aggtggagat caaacgaact gtggctgcac catctgtctt catcttcccg 360
ccatctgatg agcagttgaa atctggaact gcctctgttg tgtgcctgct gaataacttc 420
tatcccagag aggccaaagt acagtggaag gtggataacg ccctca                466
```

<210> 24
<211> 113
<212> PRT
<213> Homo Sapiens

<400> 24

```
Asn Asn Asn Asn Thr Gln Ser Pro Leu Ser Leu Pro Val Thr Pro Gly
 1               5                  10                  15
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Lys
            100                 105                 110
Arg
```

<210> 25
<211> 537
<212> DNA
<213> Homo Sapiens

<400> 25

```
caggtgcagc tggagcagtc gggggaggc gtggtccagc ctgggaggtc cctgagactc 60
tcctgtgcag cgtctggatt caccttcact aactatggct tgcactgggt ccgccaggct 120
ccaggcaagg ggctggattg ggtggcagtt atatggtatg atggaagtca taaattctat 180
gcagactccg tgaagggccg attcaccatc tccagagaca attccaagaa cacgctcttt 240
ctgcaaatga acagcctgag agccgaggac acggctgtgt attactgtac gcgagatctt 300
gactactggg gccagggaac cctggtcacc gtctcctcag cttccaccaa gggcccatcc 360
gtcttccccc tggcgccctg ctccaggagc acctccgaga gcacagccgc cctgggctgc 420
ctggtcaagg actacttccc cgaaccggtg acggtgtcgt ggaactcagg cgccctgacc 480
agcggcgtgc acaccttccc ggctgtccta cagtcctcag gactctactc cctcagc    537
```

<210> 26
<211> 114
<212> PRT
<213> Homo Sapiens

<400> 26

```
Gln Val Gln Leu Glu Gln Ser Gly Gly Gly Val Val Gln Pro Gly Arg
 1               5                  10                  15
Ser Leu Arg Leu Ser Cys Ala Ala Ser Gly Phe Thr Phe Thr Asn Tyr
            20                  25                  30
Gly Leu His Trp Val Arg Gln Ala Pro Gly Lys Gly Leu Asp Trp Val
        35                  40                  45
Ala Val Ile Trp Tyr Asp Gly Ser His Lys Phe Tyr Ala Asp Ser Val
    50                  55                  60
Lys Gly Arg Phe Thr Ile Ser Arg Asp Asn Ser Lys Asn Thr Leu Phe
65                  70                  75                  80
Leu Gln Met Asn Ser Leu Arg Ala Glu Asp Thr Ala Val Tyr Tyr Cys
                85                  90                  95
Thr Arg Asp Leu Asp Tyr Trp Gly Gln Gly Thr Leu Val Thr Val Ser
            100                 105                 110
Ser Ala
```

<210> 27
<211> 480
<212 DNA
<213> Homo Sapiens

<400> 27

```
gaaacgcagc tgacgcagtc tccaggcacc ctgtctttgt ctccagggga aagagtcacc 60
ctctcctgca gggccagtca gagtgttagc aacaactact tagcctggta ccagcagaaa 120
cctggccagg ctcccaggct cctcatctat ggtgcatcca gcagggccac tggcatccca 180
gacaggttca gtggcagtgg gtctgggaca gacttcactc tcaccatcag cagactggag 240
cctgaagatt gtgcagagtg ttactgtcag caatatggta gctcactccc gctcactttc 300
ggcggaggga ccaaggtgga gatcaaacga actgtggctg caccatctgt cttcatcttc 360
ccgccatctg atgagcagtt gaaatctgga actgcctctg ttgtgtgcct gctgaataac 420
ttctatccca gagaggccaa agtacagtgg gaaggtggga taacgccctc caatcgggta 480
```

<210> 28
<211> 110
<212> PRT
<213> Homo Sapiens

<400> 28

```
Glu Thr Gln Leu Thr Gln Ser Pro Gly Thr Leu Ser Leu Ser Pro Gly
 1               5                  10                  15
Glu Arg Val Thr Leu Ser Cys Arg Ala Ser Gln Ser Val Ser Asn Asn
            20                  25                  30
Tyr Leu Ala Trp Tyr Gln Gln Lys Pro Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Cys Ala Glu Cys Tyr Cys Gln Gln Tyr Gly Ser Ser Leu
                85                  90                  95
Pro Leu Thr Phe Gly Gly Gly Thr Lys Val Glu Ile Lys Arg
            100                 105                 110
```

<210> 29
<211> 542
<212> DNA
<213> Homo Sapiens

<400> 29

```
gtccagtgtc aggtgcagct ggtggagtct gggggaggcg tggtccagcc tgggaggtcc 60
ctgagactct cctgtgcagc gtctggattc accttcagta gctatggcat gcactgggtc 120
cgccaggctc caggcaaggg gctggagtgg gtggcagtta tatggtatga tggaagtcat 180
aaatactatg cagactccgt gaagggccga ttcaccatct ccagagacaa ttccaagaac 240
acgctgtatc tgcaaatgaa cagcctgaga gccgaggaca cggctgtgta ttactctgcg 300
agagattact atgatacgag tcggcatcac tggggggtttg actgctgggg ccagggaacc 360
ctggtcaccg tctcctctgc ttccaccaag ggcccatccg tcttcccct ggcgccctgc 420
tccaggagca cctccgagag cacagccgcc ctgggctgcc tggtcaagga ctacttcccc 480
gaaccggtga cggtgtcgtg gaactcaggc gccctgacca gcggcgtgca caccttcccg 540
gc                                                                542
```

<210> 30
<211> 124
<212> PRT
<213> Homo Sapiens

<400> 30

```
Gln Val Gln Leu Val Glu Ser Gly Gly Gly Val Val Gln Pro Gly Arg
 1               5                  10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Val Ile Trp Tyr Asp Gly Ser His Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Ser
                85                  90                  95
Ala Arg Asp Tyr Tyr Asp Thr Ser Arg His His Trp Gly Phe Asp Cys
            100                 105                 110
Trp Gly Gln Gly Thr Leu Val Thr Val Ser Ser Ala
        115                 120
```

<210> 31
<211> 521
<212> DNA
<213> Homo Sapiens

<400> 31

```
cagctcctgg ggctgctaat gctctgggtc cctggatcca gtgaggaaat tgtgatgacc 60
cagactccac tctccctgcc cgtcacccct ggagagccgg cctccatctc ctgcaggtct 120
agtcagagcc tcttggatag tgaagatgga aacacctatt tggactggta cctgcagaag 180
ccagggcagt ctccacagct cctgatctat acgctttccc atcgggcctc tggagtccca 240
gacaggttca gtggcagtgg gtcaggcact gatttcacac tgaaaatcag cagggtggag 300
gctgaggatg ttggagttta ttgctgcatg caacgtgtag agtttcctat caccttcggc 360
caagggacac gactggagat taaacgaact gtggctgcac catctgtctt catcttcccg 420
ccatctgatg agcagttgaa atctggaact gcctctgttg tgtgcctgct gaataacttc 480
tatcccagag aggccaaagt acagtggaag gtggataacg c                     521
```

<210> 32
<211> 114
<212> PRT
<213> Homo Sapiens

<400> 32

```
Glu Ile Val Met Thr Gln Thr Pro Leu Ser Leu Pro Val Thr Pro Gly
 1               5                  10                  15
Glu Pro Ala Ser Ile Ser Cys Arg Ser Ser Gln Ser Leu Leu Asp Ser
            20                  25                  30
Glu Asp Gly Asn Thr Tyr Leu Asp Trp Tyr Leu Gln Lys Pro Gly Gln
        35                  40                  45
Ser Pro Gln Leu Leu Ile Tyr Thr Leu Ser His Arg Ala Ser Gly Val
    50                  55                  60
Pro Asp Arg Phe Ser Gly Ser Gly Ser Gly Thr Asp Phe Thr Leu Lys
65                  70                  75                  80
Ile Ser Arg Val Glu Ala Glu Asp Val Gly Val Tyr Cys Cys Met Gln
                85                  90                  95
Arg Val Glu Phe Pro Ile Thr Phe Gly Gln Gly Thr Arg Leu Glu Ile
            100                 105                 110
Lys Arg
```

<210> 33
<211> 547
<212> DNA

<213> Homo Sapiens

<400> 33

```
cagtcgggcc caagactggt gaagccttca cagaccctgt ccctcacctg cactgtctct 60
ggtggctcca tcagtagtga tggttactac tggagctgga tccgccagca cccagggaag 120
ggcctggagt ggattgggta catctattac agtgggagca ccttctacaa cccgtccctc 180
aagagtcgag ttgccatatc agtggacacg tctaagaacc agttctccct gaagctgagc 240
tctgtgactg ccgcggacac ggccgtgtat tactgtgcga gagaatcccc tcatagcagc 300
aactggtact cgggctttga ctgctggggc cagggaaccc tggtcaccgt ctcctcagct 360
tccaccaagg gcccatccgt cttccccctg gcgccctgct ccaggagcac ctccgagagc 420
acagccgccc tgggctgcct ggtcaaggac tactttcccc gaaccggtga cggtgtcgtg 480
gaactcaggc gccctgacca gcggcgtgca caccttcccg gctgtcctac agtcctcagg 540
actctct                                                           547
```

<210> 34
<211> 125
<212> PRT
<213> Homo Sapiens

<400> 34

```
Asn Asn Asn Asn Asn Gln Ser Gly Pro Arg Leu Val Lys Pro Ser Gln
 1               5                  10                  15
Thr Leu Ser Leu Thr Cys Thr Val Ser Gly Gly Ser Ile Ser Ser Asp
            20                  25                  30
Gly Tyr Tyr Trp Ser Trp Ile Arg Gln His Pro Gly Lys Gly Leu Glu
        35                  40                  45
Trp Ile Gly Tyr Ile Tyr Tyr Ser Gly Ser Thr Phe Tyr Asn Pro Ser
    50                  55                  60
Leu Lys Ser Arg Val Ala Ile Ser Val Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Glu Ser Pro His Ser Ser Asn Trp Tyr Ser Gly Phe Asp
            100                 105                 110
Cys Trp Gly Gln Gly Thr Leu Val Thr Val Ser Ser Ala
        115                 120                 125
```

<210> 35
<211> 450
<212> DNA
<213> Homo Sapiens

<400> 35

```
actcagtctc cagactttca gtctgtgact ccaaaggaga aagtcaccat cacctgccgg 60
gccagtcaga gcattggtag taggttacac tggtaccagc agaaaccaga tcagtctcca 120
aagctcctca tcaagtatgc ttcccagtcc ttctcagggg tcccctcgag gttcagtggc 180
agtggatctg ggacagattt caccctcacc atcaatagcc tggaagctga agatgctgca 240
acgtattact gtcatcagag tagtaattta ccattcactt tcggccctgg gaccaaagtg 300
gatatcaaac gaactgtggc tgcaccatct gtcttcatct tcccgccatc tgatgagcag 360
ttgaaatctg gaactgcctc tgttgtgtgc ctgctgaata acttctatcc cagagaggcc 420
aaagtacagt ggaaggtgga taacgccctc                                  450
```

<210> 36
<211> 108
<212> PRT
<213> Homo Sapiens

<400> 36

```
Asn Asn Asn Asn Thr Gln Ser Pro Asp Phe Gln Ser Val Thr Pro Lys
 1               5                  10                  15
Glu Lys Val Thr Ile Thr Cys Arg Ala Ser Gln Ser Ile Gly Ser Arg
            20                  25                  30
Leu His Trp Tyr Gln Gln Lys Pro Asp Gln Ser Pro Lys Leu Leu Ile
        35                  40                  45
Lys Tyr Ala Ser Gln Ser Phe Ser Gly Val Pro Ser Arg Phe Ser Gly
    50                  55                  60
Ser Gly Ser Gly Thr Asp Phe Thr Leu Thr Ile Asn Ser Leu Glu Ala
65                  70                  75                  80
Glu Asp Ala Ala Thr Tyr Tyr Cys His Gln Ser Ser Asn Leu Pro Phe
                85                  90                  95
Thr Phe Gly Pro Gly Thr Lys Val Asp Ile Lys Arg
            100                 105
```

<210> 37
<211> 534
<212> DNA
<213> Homo Sapiens

<400> 37

```
caggtgcagc tggtggaggc tgggggaggc gtggtccagc ctgggaggtc cctgagactc 60
tcctgtgcag cgtctggatt caccttcaga agctatggca tgcactgggt ccgccaggct 120
ccaggcaagg ggctgaaatg ggtggcagtt atatggtatg atggaagtaa taaatactat 180
acagactccg tgaagggccg attcaccatc tccagagaca attccaagaa cacgctgtat 240
ctgcaaatga acagcctgag agccgaggac acggctgtgt attactgtgt gagagattac 300
tatgataata gtagacatca ctggggggttt gactactggg gccagggaac cctggtcacc 360
gtctcctcag cttccaccaa gggcccatcc gtcttccccc tggcgccctg ctccaggagc 420
acctccgaga gcacagccgc cctgggctgc ctggtcaagg actacttccc cgaaccggtg 480
acggtgtcgt ggaactcagg cgccctgacc aggcggcgtg cacaccttcc cggc       534
```

<210> 38
<211> 124
<212> PRT
<213> Homo Sapiens

<400> 38

```
Gln Val Gln Leu Val Glu Ala Gly Gly Gly Val Val Gln Pro Gly Arg
 1               5                  10                  15
Ser Leu Arg Leu Ser Cys Ala Ala Ser Gly Phe Thr Phe Arg Ser Tyr
            20                  25                  30
Gly Met His Trp Val Arg Gln Ala Pro Gly Lys Gly Leu Lys Trp Val
        35                  40                  45
Ala Val Ile Trp Tyr Asp Gly Ser Asn Lys Tyr Tyr Thr Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Val Arg Asp Tyr Tyr Asp Asn Ser Arg His His Trp Gly Phe Asp Tyr
            100                 105                 110
Trp Gly Gln Gly Thr Leu Val Thr Val Ser Ser Ala
        115                 120
```

<210> 39
<211> 470

<212> DNA
<213> Homo Sapiens

<400> 39

```
gacatccaga tgacccagtc tccatcctcc cggtgtgcat ccgtaggaga cagagtcacc 60
atcacttgcc gggcaagtca gggcatcaga aatgatttag cttggtatca gcagaaacca 120
gggaaagccc ctaagcgcct gatctatgct gcatccagtt tgcaaagtgg ggtcccatca 180
aggttcagcg gcagtagatc tgggacagaa ttcactctca caatcagcag cctgcagcct 240
gaagattttg cagcttatta ctgtctccag cataatagtt accctcccag ttttggccag 300
gggaccaagc tggagatcaa acgaactgtg gctgcaccat ctgtcttcat cttcccgcca 360
tctgatgagc agttgaaatc tggaactgct agcgttgtgt gcctgctgaa taacttctat 420
cccagagagg ccaaagtaca gtggaaggtg gataacgccc tccaatcggg            470
```

<210> 40
<211> 108
<212> PRT
<213> Homo Sapiens

<400> 40

```
Asp Ile Gln Met Thr Gln Ser Pro Ser Ser Arg Cys Ala Ser Val Gly
 1               5                  10                  15
Asp Arg Val Thr Ile Thr Cys Arg Ala Ser Gln Gly Ile Arg Asn Asp
            20                  25                  30
Leu Ala Trp Tyr Gln Gln Lys Pro Gly Lys Ala Pro Lys Arg Leu Ile


        35                  40                  45
Tyr Ala Ala Ser Ser Leu Gln Ser Gly Val Pro Ser Arg Phe Ser Gly
    50                  55                  60
Ser Arg Ser Gly Thr Glu Phe Thr Leu Thr Ile Ser Ser Leu Gln Pro
65                  70                  75                  80
Glu Asp Phe Ala Ala Tyr Tyr Cys Leu Gln His Asn Ser Tyr Pro Pro
                85                  90                  95
Ser Phe Gly Gln Gly Thr Lys Leu Glu Ile Lys Arg
            100                 105
```

<210> 41
<211> 514
<212> DNA
<213> Homo Sapiens

<400> 41

```
catgtgcagg tgcagctggt ggagtctggg ggaggcgtgg tccagcctgg gaggtccctg 60
agactctcct gtgcagcgtc tggattcatc ttcagtcgct atggcatgca ctgggtccgc 120
caggctccag gcaaggggct gaaatgggtg gcagttatat ggtatgatgg aagtaataaa 180
ctctatgcag actccgtgaa gggccgattc accatctcca gagacaattc caagaacacg 240
ctgtatctgc aaatgaacag cctgagagcc gaggacacgg ctgtgtatta ctgtgcgaga 300
gattactatg ataatagtag acatcactgg gggtttgact actggggcca gggaaccctg 360
gtcaccgtct cctcagcttc caccaagggc ccatccgtct tccccctggc gccctgctcc 420
aggagcacct ccgagagcac agccgccctg ggctgcctgg tcaaggacta cttccccgaa 480
ccggtgacgg tgtcgtggaa ctcaggcgcc ctga                             514
```

<210> 42
<211> 124
<212> PRT
<213> Homo Sapiens

<400> 42

```
Gln Val Gln Leu Val Glu Ser Gly Gly Gly Val Val Gln Pro Gly Arg
 1               5                  10                  15
Ser Leu Arg Leu Ser Cys Ala Ala Ser Gly Phe Ile Phe Ser Arg Tyr
            20                  25                  30
Gly Met His Trp Val Arg Gln Ala Pro Gly Lys Gly Leu Lys Trp Val
        35                  40                  45
Ala Val Ile Trp Tyr Asp Gly Ser Asn Lys Leu Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Tyr Tyr Asp Asn Ser Arg His His Trp Gly Phe Asp Tyr
            100                 105                 110
Trp Gly Gln Gly Thr Leu Val Thr Val Ser Ser Ala
        115                 120
```

<210> 43
<211> 523
<212> DNA
<213> Homo Sapiens

<400> 43

```
tcagctcctg gggctgctaa tgctctgggt ccctggatca gtgaggatat tgtgatgacc 60
cagactccac tctccctgcc cgtcacccct ggagagccgg cctccatctc ctgcaggtct 120
agtcggagcc tcttggatag tgatgatgga aacacctatt tggactggta cctgcagaag 180
ccagggcagt ctccacagct cctgatctac acgctttcct atcgggcctc tggagtccca 240

gacaggttca gtggcagtgg gtcaggcact gatttcacac tgaaaatcag cagggtggag 300
gctgaggatg ttggagttta ttactgcatg caacgtgtag agtttcctat caccttcggc 360
caagggacac gactggagat taaacgaact gtggctgcac catctgtctt catcttcccg 420
ccatctgatg agcagttgaa atctggaact gcctctgttg tgtgcctgct gaataacttc 480
tatcccagag aggccaaagt acagtggaag gtggataacg cct                   523
```

<210> 44
<211> 114
<212> PRT
<213> Homo Sapiens

<400> 44

```
Asp Ile Val Met Thr Gln Thr Pro Leu Ser Leu Pro Val Thr Pro Gly
 1               5                  10                  15
Glu Pro Ala Ser Ile Ser Cys Arg Ser Ser Arg Ser Leu Leu Asp Ser
            20                  25                  30
Asp Asp Gly Asn Thr Tyr Leu Asp Trp Tyr Leu Gln Lys Pro Gly Gln
        35                  40                  45
Ser Pro Gln Leu Leu Ile Tyr Thr Leu Ser Tyr Arg Ala Ser Gly Val
    50                  55                  60
Pro Asp Arg Phe Ser Gly Ser Gly Ser Gly Thr Asp Phe Thr Leu Lys
65                  70                  75                  80
Ile Ser Arg Val Glu Ala Glu Asp Val Gly Val Tyr Tyr Cys Met Gln
                85                  90                  95
Arg Val Glu Phe Pro Ile Thr Phe Gly Gln Gly Thr Arg Leu Glu Ile
            100                 105                 110
Lys Arg
```

<210> 45
<211> 546
<212> DNA
<213> Homo Sapiens

<400> 45

```
gagcagtcgg ggggcggcgt ggtccagcct gggaggtccc tgagactctc ctgtgcagcg 60
tctggattca ccttcagtag ctatggcatg tactgggtcc gccaggctcc aggcaagggg 120
ctggagtggg tggcagttat atggtatgat ggaagcaata aatactatgc agactccgtg 180
aagggccgat tcaccatctc cagagacaat tccaagaaca cgctgtatct gcaaatgaac 240
agcctgagag ccgaggacac ggctgtgtat tactgtgcga gggatttcta tgatagtagt 300
cgttaccact acggtatgga cgtctggggc caagggacca cggtcaccgt ctcctcagct 360
tccaccaagg gcccatccgt cttccccctg gcgccctgct ccaggagcac ctccgagagc 420
acagccgccc tgggctgcct ggtcaaggac tacttccccg aaccggtgac ggtgtcgtgg 480
aactcaggcg ccctgaccag cggcgtgcac accttcccgg ctgtcctaca gtcctcagga 540
ctctct                                                            546
```

<210> 46
<211> 124
<212> PRT
<213> Homo Sapiens

<400> 46

```
Asn Asn Asn Asn Glu Gln Ser Gly Gly Gly Val Val Gln Pro Gly Arg
 1               5                  10                  15
Ser Leu Arg Leu Ser Cys Ala Ala Ser Gly Phe Thr Phe Ser Ser Tyr
            20                  25                  30
Gly Met Tyr Trp Val Arg Gln Ala Pro Gly Lys Gly Leu Glu Trp Val
        35                  40                  45
Ala Val Ile Trp Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Phe Tyr Asp Ser Ser Arg Tyr His Tyr Gly Met Asp Val
            100                 105                 110
Trp Gly Gln Gly Thr Thr Val Thr Val Ser Ser Ala
        115                 120
```

<210> 47
<211> 419
<212> DNA
<213> Homo Sapiens

<400> 47

```
actcagtgtc cactctccct gcccgtcacc cctggagagc cggcctccat ctcctgcagg 60
tctagtcaga gcctcttgga tagtgatgat ggaaacacct atttggactg gtacctgcag 120
aagccagggc agtctccaca gctcctgatc tatacggttt cctatcgggc ctctggagtc 180
ccagacaggt tcagtggcag tgggtcaggc actgatttca cactgaaaat cagcagggtg 240
gaggctgagg atgttggagt ttattactgc atgcaacgta tagagtttcc gatcaccttc 300
ggccaaggga cccgactgga gattaaacga actgtggctg caccatctgt cttcatcttc 360
ccgccatctg atgagcagtt gaaatctgga actgcctctg ttgtgtgcct gctgaataa  419
```

<210> 48
<211> 114
<212> PRT
<213> Homo Sapiens

<400> 48

```
Asn Asn Asn Asn Thr Gln Cys Pro Leu Ser Leu Pro Val Thr Pro Gly
1               5                   10                  15
Glu Pro Ala Ser Ile Ser Cys Arg Ser Ser Gln Ser Leu Leu Asp Ser
            20                  25                  30
Asp Asp Gly Asn Thr Tyr Leu Asp Trp Tyr Leu Gln Lys Pro Gly Gln
        35                  40                  45
Ser Pro Gln Leu Leu Ile Tyr Thr Val Ser Tyr Arg Ala Ser Gly Val
    50                  55                  60
Pro Asp Arg Phe Ser Gly Ser Gly Ser Gly Thr Asp Phe Thr Leu Lys
65                  70                  75                  80
Ile Ser Arg Val Glu Ala Glu Asp Val Gly Val Tyr Tyr Cys Met Gln
                85                  90                  95
Arg Ile Glu Phe Pro Ile Thr Phe Gly Gln Gly Thr Arg Leu Glu Ile
            100                 105                 110
Lys Arg
```

<210> 49
<211> 789
<212> DNA
<213> Homo Sapiens

<400> 49

```
tctgtaaagg ttggtggaga ggcaggtcca tctgtcacac taccctgcca ctacagtgga 60
gctgtcacat caatgtgctg gaatagaggc tcatgttctc tattcacatg ccaaaatggc 120
attgtctgga ccaatggaac ccacgtcacc tatcggaagg acacacgcta taagctattg 180
ggggaccttt caagaaggga tgtctctttg accatagaaa atacagctgt gtctgacagt 240
ggcgtatatt gttgccgtgt tgagcaccgt gggtggttca atgacatgaa aatcaccgta 300
tcattggaga ttgtgccacc caaggtcacg actactccaa ttgtcacaac tgttccaacc 360
gtcacgactg ttcgaacgag caccactgtt ccaacgacaa cgactgttcc aacgacaact 420

gttccaacaa caatgagcat tccaacgaca acgactgttc cgacgacaat gactgtttca 480
acgacaacga gcgttccaac gacaacgagc attccaacaa caacaagtgt tccagtgaca 540
acaacggtct ctacctttgt tcctccaatg cctttgccca ggcagaacca tgaaccagta 600
gccacttcac catcttcacc tcagccagca gaaacccacc ctacgacact gcagggagca 660
ataaggagag aacccaccag ctcaccattg tactcttaca caacagatgg gaatgacacc 720
gtgacagagt cttcagatgg cctttggaat aacaatcaaa ctcaactgtt cctagaacat 780
agtctactg                                                         789
```

<210> 50
<211> 263
<212> PRT
<213> Homo Sapiens

<400> 50

```
Ser Val Lys Val Gly Gly Glu Ala Gly Pro Ser Val Thr Leu Pro Cys
 1               5                  10                  15
His Tyr Ser Gly Ala Val Thr Ser Met Cys Trp Asn Arg Gly Ser Cys
            20                  25                  30
Ser Leu Phe Thr Cys Gln Asn Gly Ile Val Trp Thr Asn Gly Thr His
        35                  40                  45
Val Thr Tyr Arg Lys Asp Thr Arg Tyr Lys Leu Leu Gly Asp Leu Ser
    50                  55                  60
Arg Arg Asp Val Ser Leu Thr Ile Glu Asn Thr Ala Val Ser Asp Ser
65                  70                  75                  80
Gly Val Tyr Cys Cys Arg Val Glu His Arg Gly Trp Phe Asn Asp Met
                85                  90                  95
Lys Ile Thr Val Ser Leu Glu Ile Val Pro Pro Lys Val Thr Thr Thr
            100                 105                 110
Pro Ile Val Thr Thr Val Pro Thr Val Thr Thr Val Arg Thr Ser Thr
        115                 120                 125
Thr Val Pro Thr Thr Thr Thr Val Pro Thr Thr Thr Val Pro Thr Thr
    130                 135                 140
Met Ser Ile Pro Thr Thr Thr Thr Val Pro Thr Thr Met Thr Val Ser
145                 150                 155                 160
Thr Thr Thr Ser Val Pro Thr Thr Thr Ser Ile Pro Thr Thr Thr Ser
                165                 170                 175
Val Pro Val Thr Thr Thr Val Ser Thr Phe Val Pro Pro Met Pro Leu
            180                 185                 190
Pro Arg Gln Asn His Glu Pro Val Ala Thr Ser Pro Ser Ser Pro Gln
        195                 200                 205
Pro Ala Glu Thr His Pro Thr Thr Leu Gln Gly Ala Ile Arg Arg Glu
    210                 215                 220
Pro Thr Ser Ser Pro Leu Tyr Ser Tyr Thr Thr Asp Gly Asn Asp Thr
225                 230                 235                 240
Val Thr Glu Ser Ser Asp Gly Leu Trp Asn Asn Asn Gln Thr Gln Leu
                245                 250                 255
Phe Leu Glu His Ser Leu Leu
            260
```

<210> 51
<211> 114
<212> PRT
<213> Homo Sapiens

<400> 51

```
Gln Val Gln Leu Val Glu Ser Gly Gly Gly Val Val Gln Pro Gly Arg
 1               5                  10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val


        35                  40                  45
Ala Val Ile Trp Tyr Asp Gly Ser Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asn Asn Asp Tyr Trp Gly Gln Gly Thr Leu Val Thr Val Ser
            100                 105                 110
Ser Ala
```

<210> 52
<211> 124

<212> PRT
<213> Homo Sapiens

<400> 52

```
Gln Val Gln Leu Val Glu Ser Gly Gly Gly Val Val Gln Pro Gly Arg
 1               5                  10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Val Ile Trp Tyr Asp Gly Ser Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Asn Asn Asn Tyr Asp Ser Ser Asn Asn Asn Tyr Gly Met Asp Val
            100                 105                 110
Trp Gly Gln Gly Thr Thr Val Thr Val Ser Ser Ala
        115                 120
```

<210> 53
<211> 125
<212> PRT
<213> Homo Sapiens

<400> 53

```
Gln Val Gln Leu Gln Glu Ser Gly Pro Gly Leu Val Lys Pro Ser Gln
 1               5                  10                  15
Thr Leu Ser Leu Thr Cys Thr Val Ser Gly Gly Ser Ile Ser Ser Gly
            20                  25                  30
Gly Tyr Tyr Trp Ser Trp Ile Arg Gln His Pro Gly Lys Gly Leu Glu
        35                  40                  45
Trp Ile Gly Tyr Ile Tyr Tyr Ser Gly Ser Thr Tyr Tyr Asn Pro Ser
    50                  55                  60
Leu Lys Ser Arg Val Thr Ile Ser Val Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Asn Asn Asn Asn Ser Ser Ser Trp Tyr Asn Asn Phe Asp
            100                 105                 110
Tyr Trp Gly Gln Gly Thr Leu Val Thr Val Ser Ser Ala
        115                 120                 125
```

<210> 54
<211> 124
<212> PRT
<213> Homo Sapiens

<400> 54

```
Gln Val Gln Leu Val Glu Ser Gly Gly Gly Val Val Gln Pro Gly Arg
 1               5                  10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Val Ile Trp Tyr Asp Gly Ser Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Tyr Tyr Asp Ser Ser Asn Asn Asn Asn Asn Phe Asp Tyr
            100                 105                 110
Trp Gly Gln Gly Thr Leu Val Thr Val Ser Ser Ala
        115                 120
```

<210> 55
<211> 119
<212> PRT
<213> Homo Sapiens

<400> 55

```
Glu Val Gln Leu Val Glu Ser Gly Gly Gly Leu Val Lys Pro Gly Gly
 1               5                  10                  15
Ser Leu Arg Leu Ser Cys Ala Ala Ser Gly Phe Thr Phe Ser Asn Ala
            20                  25                  30
Trp Met Ser Trp Val Arg Gln Ala Pro Gly Lys Gly Leu Glu Trp Val
        35                  40                  45
Gly Arg Ile Lys Ser Lys Thr Asp Gly Gly Thr Thr Asp Tyr Ala Ala
    50                  55                  60
Pro Val Lys Gly Arg Phe Thr Ile Ser Arg Asp Asp Ser Lys Asn Thr
65                  70                  75                  80
Leu Tyr Leu Gln Met Asn Ser Leu Lys Thr Glu Asp Thr Ala Val Tyr
                85                  90                  95
Tyr Cys Thr Asn Asn Asp Asn Asn Asn Asp Tyr Trp Gly Gln Gly Thr
            100                 105                 110
Leu Val Thr Val Ser Ser Ala
        115
```

<210> 56
<211> 121
<212> PRT
<213> Homo Sapiens

<400> 56

```
Gln Val Gln Leu Gln Glu Ser Gly Pro Gly Leu Val Lys Pro Ser Glu
 1               5                  10                  15
Thr Leu Ser Leu Thr Cys Thr Val Ser Gly Gly Ser Val Ser Ser Gly
            20                  25                  30
Gly Tyr Tyr Trp Ser Trp Ile Arg Gln Pro Pro Gly Lys Gly Leu Glu
        35                  40                  45
Trp Ile Gly Tyr Ile Tyr Tyr Ser Gly Ser Thr Asn Tyr Asn Pro Ser
```

```
        50                  55                  60
Leu Lys Ser Arg Val Thr Ile Ser Val Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Asn Asn Asn Trp Asn Asn Asn Phe Asp Tyr Trp Gly Gln
            100                 105                 110
Gly Thr Leu Val Thr Val Ser Ser Ala
        115                 120
```

<210> 57
<211> 119
<212> PRT
<213> Homo Sapiens

<400> 57

```
Glu Val Gln Leu Val Glu Ser Gly Gly Gly Leu Val Lys Pro Gly Gly
 1               5                  10                  15
Ser Leu Arg Leu Ser Cys Ala Ala Ser Gly Phe Thr Phe Ser Asn Ala
            20                  25                  30
Trp Met Ser Trp Val Arg Gln Ala Pro Gly Lys Gly Leu Glu Trp Val
        35                  40                  45
Gly Arg Ile Lys Ser Lys Thr Asp Gly Gly Thr Thr Asp Tyr Ala Ala
    50                  55                  60
Pro Val Lys Gly Arg Phe Thr Ile Ser Arg Asp Asp Ser Lys Asn Thr
65                  70                  75                  80
Leu Tyr Leu Gln Met Asn Ser Leu Lys Thr Glu Asp Thr Ala Val Tyr
                85                  90                  95
Tyr Cys Thr Thr Asn Asn Asn Ser Gly Asp Tyr Trp Gly Gln Gly Thr
            100                 105                 110
Leu Val Thr Val Ser Ser Ala
        115
```

<210> 58
<211> 113
<212> PRT
<213> Homo Sapiens

<400> 58

```
Glu Val Gln Leu Val Glu Ser Gly Gly Gly Leu Val Gln Pro Gly Gly
 1               5                  10                  15
Ser Leu Arg Leu Ser Cys Ala Ala Ser Gly Phe Thr Phe Ser Ser Tyr
            20                  25                  30
Trp Met Ser Trp Val Arg Gln Ala Pro Gly Lys Gly Leu Glu Trp Val
        35                  40                  45
Ala Asn Ile Lys Gln Asp Gly Ser Glu Lys Tyr Tyr Val Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asn Asp Tyr Trp Gly Gln Gly Thr Leu Val Thr Val Ser Ser
            100                 105                 110
Ala
```

<210> 59
<211> 114
<212> PRT

<213> Homo Sapiens

<400> 59

```
Glu Val Gln Leu Val Glu Ser Gly Gly Gly Leu Val Gln Pro Gly Gly
 1               5                  10                  15
Ser Leu Arg Leu Ser Cys Ala Ala Ser Gly Phe Thr Phe Ser Ser Tyr
            20                  25                  30
Ser Met Asn Trp Val Arg Gln Ala Pro Gly Lys Gly Leu Glu Trp Val
        35                  40                  45
Ser Tyr Ile Ser Ser Ser Ser Ser Thr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Asp Glu Asp Thr Ala Val Tyr Tyr Cys
                85                  90                  95
Ala Asn Asn Phe Asp Tyr Trp Gly Gln Gly Thr Leu Val Thr Val Ser
            100                 105                 110
Ser Ala
```

<210> 60
<211> 110
<212> PRT
<213> Homo Sapiens

<400> 60

```
Glu Ile Val Leu Thr Gln Ser Pro Gly Thr Leu Ser Leu Ser Pro Gly
 1               5                  10                  15
Glu Arg Ala Thr Leu Ser Cys Arg Ala Ser Gln Ser Val Ser Ser Ser
            20                  25                  30
Tyr Leu Ala Trp Tyr Gln Gln Lys Pro Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Asn
                85                  90                  95
Asn Leu Thr Phe Gly Gly Gly Thr Lys Val Glu Ile Lys Arg
            100                 105                 110
```

<210> 61
<211> 113
<212> PRT
<213> Homo Sapiens

<400> 61

```
Asp Ile Val Met Thr Gln Ser Pro Leu Ser Leu Pro Val Thr Pro Gly
 1               5                  10                  15
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                                      21

                85                  90                  95
Leu Gln Thr Asn Asn Thr Phe Gly Gly Gly Thr Lys Val Glu Ile Lys
            100                 105                 110
Arg
```

<210> 62
<211> 108
<212> PRT
<213> Homo Sapiens

<400> 62

```
Asp Ile Gln Met Thr Gln Ser Pro Ser Ser Leu Ser Ala Ser Val Gly
 1               5                  10                  15
Asp Arg Val Thr Ile Thr Cys Arg Ala Ser Gln Gly Ile Arg Asn Asp
            20                  25                  30
Leu Gly Trp Tyr Gln Gln Lys Pro Gly Lys Ala Pro Lys Arg Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Leu Gln His Asn Ser Tyr Pro Leu
                85                  90                  95
Thr Phe Gly Gly Gly Thr Lys Val Glu Ile Lys Arg
            100                 105
```

<210> 63
<211> 114
<212> PRT
<213> Homo Sapiens

<400> 63

Asp Ile Val Met Thr Gln Thr Pro Leu Ser Ser Pro Val Thr Leu Gly
1 5 10 15
Gln Pro Ala Ser Ile Ser Cys Arg Ser Ser Gln Ser Leu Val His Ser
20 25 30
Asp Gly Asn Thr Tyr Leu Ser Trp Leu Gln Gln Arg Pro Gly Gln Pro
35 40 45
Pro Arg Leu Leu Ile Tyr Lys Ile Ser Asn Arg Phe Ser Gly Val Pro
50 55 60
Asp Arg Phe Ser Gly Ser Gly Ala Gly Thr Asp Phe Thr Leu Lys Ile
65 70 75 80
Ser Arg Val Glu Ala Glu Asp Val Gly Val Tyr Tyr Cys Met Gln Ala
85 90 95
Thr Gln Phe Pro Asn Ile Thr Phe Gly Gln Gly Thr Arg Leu Glu Ile
100 105 110
Lys Arg

<210> 64
<211> 108
<212> PRT
<213> Homo Sapiens

<400> 64

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Ser Tyr
20 25 30
Leu Asn Trp Tyr Gln Gln Lys Pro Gly Lys Ala Pro Lys Leu Leu Ile
35 40 45
Tyr Ala Ala Ser Ser Leu Gln Ser Gly Val Pro Ser Arg Phe Ser Gly
50 55 60
Ser Gly Ser Gly Thr Asp Phe Thr Leu Thr Ile Ser Ser Leu Gln Pro
65 70 75 80
Glu Asp Phe Ala Thr Tyr Tyr Cys Gln Gln Ser Tyr Ser Thr Pro Pro
85 90 95
Thr Phe Gly Gln Gly Thr Lys Val Glu Ile Lys Arg
100 105

<210> 65
<211> 113
<212> PRT
<213> Homo Sapiens

<400> 65

```
Asp Ile Val Met Thr Gln Thr Pro Leu Ser Ser Pro Val Thr Leu Gly
 1               5                  10                  15
Gln Pro Ala Ser Ile Ser Cys Arg Ser Ser Gln Ser Leu Val His Ser
            20                  25                  30
Asp Gly Asn Thr Tyr Leu Ser Trp Leu Gln Gln Arg Pro Gly Gln Pro
        35                  40                  45
Pro Arg Leu Leu Ile Tyr Lys Ile Ser Asn Arg Phe Ser Gly Val Pro
    50                  55                  60
Asp Arg Phe Ser Gly Ser Gly Ala Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Thr Gln Phe Pro Gln Thr Phe Gly Gln Gly Thr Lys Val Glu Ile Lys
            100                 105                 110
Arg
```

<210> 66
<211> 114
<212> PRT
<213> Homo Sapiens

<400> 66

```
Asp Ile Val Met Thr Gln Thr Pro Leu Ser Leu Pro Val Thr Pro Gly
 1               5                  10                  15
Glu Pro Ala Ser Ile Ser Cys Arg Ser Ser Gln Ser Leu Leu Asp Ser
            20                  25                  30
Asp Asp Gly Asn Thr Tyr Leu Asp Trp Tyr Leu Gln Lys Pro Gly Gln
        35                  40                  45
Ser Pro Gln Leu Leu Ile Tyr Thr Leu Ser Tyr Arg Ala Ser Gly Val
    50                  55                  60
Pro Asp Arg Phe Ser Gly Ser Gly Ser Gly Thr Asp Phe Thr Leu Lys
65                  70                  75                  80
Ile Ser Arg Val Glu Ala Glu Asp Val Gly Val Tyr Tyr Cys Met Gln
                85                  90                  95
Arg Ile Glu Phe Pro Ile Thr Phe Gly Gln Gly Thr Arg Leu Glu Ile
            100                 105                 110
Lys Arg
```

<210> 67
<211> 108
<212> PRT
<213> Homo Sapiens

<400> 67

```
Glu Ile Val Leu Thr Gln Ser Pro Asp Phe Gln Ser Val Thr Pro Lys
 1               5                  10                  15
Glu Lys Val Thr Ile Thr Cys Arg Ala Ser Gln Ser Ile Gly Ser Ser
            20                  25                  30
Leu His Trp Tyr Gln Gln Lys Pro Asp Gln Ser Pro Lys Leu Leu Ile
        35                  40                  45
Lys Tyr Ala Ser Gln Ser Phe Ser Gly Val Pro Ser Arg Phe Ser Gly
    50                  55                  60
Ser Gly Ser Gly Thr Asp Phe Thr Leu Thr Ile Asn Ser Leu Glu Ala
65                  70                  75                  80
Glu Asp Ala Ala Thr Tyr Tyr Cys His Gln Ser Ser Ser Leu Pro Phe
                85                  90                  95
Thr Phe Gly Pro Gly Thr Lys Val Asp Ile Lys Arg
            100                 105
```

<210> 68 -
<211> 108
<212> PRT
<213> Homo Sapiens

<400> 68

```
Asp Ile Gln Met Thr Gln Ser Pro Ser Ser Leu Ser Ala Ser Val Gly
 1               5                  10                  15
Asp Arg Val Thr Ile Thr Cys Arg Ala Ser Gln Gly Ile Arg Asn Asp
            20                  25                  30
Leu Gly Trp Tyr Gln Gln Lys Pro Gly Lys Ala Pro Lys Arg Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Leu Gln His Asn Ser Tyr Pro Asn
                85                  90                  95
Asn Phe Gly Gln Gly Thr Lys Leu Glu Ile Lys Arg
            100                 105
```

<210> 69
<211> 113
<212> PRT
<213> Homo Sapiens

<400> 69

```
Asp Ile Val Met Thr Gln Thr Pro Leu Ser Ser Pro Val Thr Leu Gly
 1               5                  10                  15
Gln Pro Ala Ser Ile Ser Cys Arg Ser Ser Gln Ser Leu Val His Ser
            20                  25                  30
Asp Gly Asn Thr Tyr Leu Ser Trp Leu Gln Gln Arg Pro Gly Gln Pro
        35                  40                  45
Pro Arg Leu Leu Ile Tyr Lys Ile Ser Asn Arg Phe Ser Gly Val Pro
    50                  55                  60
Asp Arg Phe Ser Gly Ser Gly Ala Gly Thr Asp Phe Thr Leu Lys Ile
65                  70                  75                  80

Ser Arg Val Glu Ala Glu Asp Val Gly Val Tyr Tyr Cys Met Gln Ala
                85                  90                  95
Thr Gln Phe Pro Gln Thr Phe Gly Gln Gly Thr Lys Val Glu Ile Lys
            100                 105                 110
Arg
```

<210> 70
<211> 114
<212> PRT
<213> Homo Sapiens

<400> 70

Asp Ile Val Met Thr Gln Thr Pro Leu Ser Leu Pro Val Thr Pro Gly
1 5 10 15
Glu Pro Ala Ser Ile Ser Cys Arg Ser Ser Gln Ser Leu Leu Asp Ser
20 25 30
Asp Asp Gly Asn Thr Tyr Leu Asp Trp Tyr Leu Gln Lys Pro Gly Gln
35 40 45
Ser Pro Gln Leu Leu Ile Tyr Thr Leu Ser Tyr Arg Ala Ser Gly Val
50 55 60
Pro Asp Arg Phe Ser Gly Ser Gly Ser Gly Thr Asp Phe Thr Leu Lys
65 70 75 80
Ile Ser Arg Val Glu Ala Glu Asp Val Gly Val Tyr Tyr Cys Met Gln
85 90 95
Arg Ile Glu Phe Pro Ile Thr Phe Gly Gln Gly Thr Arg Leu Glu Ile
100 105 110
Lys Arg

<210> 71
<211> 108
<212> PRT
<213> Homo Sapiens

<400> 71

Glu Ile Val Leu Thr Gln Ser Pro Asp Phe Gln Ser Val Thr Pro Lys
1 5 10 15
Glu Lys Val Thr Ile Thr Cys Arg Ala Ser Gln Ser Ile Gly Ser Ser
20 25 30
Leu His Trp Tyr Gln Gln Lys Pro Asp Gln Ser Pro Lys Leu Leu Ile
35 40 45
Lys Tyr Ala Ser Gln Ser Phe Ser Gly Val Pro Ser Arg Phe Ser Gly
50 55 60
Ser Gly Ser Gly Thr Asp Phe Thr Leu Thr Ile Asn Ser Leu Glu Ala
65 70 75 80
Glu Asp Ala Ala Thr Tyr Tyr Cys His Gln Ser Ser Ser Leu Pro Phe
85 90 95
Thr Phe Gly Pro Gly Thr Lys Val Asp Ile Lys Arg
100 105

<210> 72
<211> 108
<212> PRT
<213> Homo Sapiens

<220>
<221> misc_feature
<222> (96) .. (96)
<223> Wherein Xaa may be any amino acid

<220>
<221> misc_feature
<222> (97) .. (97)
<223> Wherein Xaa may be any amino acid

<400> 72

```
Asp Ile Gln Met Thr Gln Ser Pro Ser Ser Leu Ser Ala Ser Val Gly
 1               5                  10                  15
Asp Arg Val Thr Ile Thr Cys Arg Ala Ser Gln Gly Ile Arg Asn Asp
            20                  25                  30
Leu Gly Trp Tyr Gln Gln Lys Pro Gly Lys Ala Pro Lys Arg Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Leu Gln His Asn Ser Tyr Pro Xaa
                85                  90                  95
Xaa Phe Gly Gln Gly Thr Lys Leu Glu Ile Lys Arg
            100                 105
```

<210> 73
<211> 16
<212> DNA
<213> Homo Sapiens

<400> 73
ttactatgat aatagt 16

<210> 74
<211> 15
<212> DNA
<213> Homo Sapiens

<400> 74
agacatcact ggggg 15

<210> 75
<211> 17
<212> DNA
<213> Homo Sapiens

<400> 75
atagcagcaa ctggtac 17

<210> 76
<211> 16
<212> DNA
<213> Homo Sapiens

<400> 76
ttactatgat aatagt 16

<210> 77
<211> 15
<212> DNA
<213> Homo Sapiens

<400> 77
agacatcact ggggg 15

<210> 78
<211> 16
<212> DNA
<213> Homo Sapiens

<400> 78
ttactatgat aatagt 16

<210> 79
<211> 15
<212> DNA
<213> Homo Sapiens

<400> 79
agacatcact ggggg 15

<210> 80
<211> 13
<212> DNA
<213> Homo Sapiens

<400> 80
ctatgatagt agt 13

<210> 81
<211> 11
<212> DNA
<213> Homo Sapiens

<400> 81
ttactatgat a 11

<210> 82
<211> 20
<212 DNA
<213> Homo Sapiens

<400> 82
cgagtcggca tcactggggg 20

<210> 83
<211> 22
<212> DNA
<213> Homo Sapiens

<400> 83
caggtgcagc tggagcagtc gg 22

<210> 84
<211> 24
<212> DNA
<213> Homo Sapiens

<400> 84
gctgagggag tagagtcctg agga 24

<210> 85
<211> 19
<212> DNA
<213> Homo Sapiens

<400> 85
cacaccgcgg tcacatggc 19

<210> 86
<211> 20
<212> DNA
<213> Homo Sapiens

<400> 86
ctactctagg gcacctgtcc 20

<210> 87
<211> 14
<212> PRT
<213> Homo Sapiens

<400> 87

```
Pro Met Pro Leu Pro Arg Gln Asn His Glu Pro Val Ala Thr
1               5                   10
```

<210> 88
<211> 12
<212> PRT
<213> Homo Sapiens

<400> 88

```
Pro Met Pro Leu Pro Arg Gln Asn His Glu Pro Val
1               5                   10
```

<210> 89
<211> 10
<212> PRT
<213> Homo Sapiens

<400> 89

```
Pro Met Pro Leu Pro Arg Gln Asn His Glu
1               5                   10
```

<210> 90
<211> 8
<212 PRT
<213> Homo Sapiens

<400> 90

```
Pro Met Pro Leu Pro Arg Gln Asn
1               5
```

<210> 91
<211> 6
<212> PRT
<213> Homo Sapiens

<400> 91

```
Pro Met Pro Leu Pro Arg
1               5
```

<210> 92
<211> 12
<212> PRT
<213> Homo Sapiens

<400> 92

Pro Leu Pro Arg Gln Asn His Glu Pro Val Ala Thr
1 5 10

<210> 93
<211> 10
<212> PRT
<213> Homo Sapiens

<400> 93

Pro Arg Gln Asn His Glu Pro Val Ala Thr
1 5 10

<210> 94
<211> 8
<212> PRT
<213> Homo Sapiens

<400> 94

Gln Asn His Glu Pro Val Ala Thr
1 5

<210> 95
<211> 6
<212> PRT
<213> Homo Sapiens

<400> 95

His Glu Pro Val Ala Thr
1 5

<210> 96
<211> 7
<212> PRT
<213> Homo Sapiens

<400> 96

Pro Leu Pro Arg Asn His Glu
1 5

<210> 97
<211> 6
<212> PRT
<213> Homo Sapiens

<400> 97

Leu Pro Arg Gln Asn His
1 5

<210> 98
<211> 10
<212> PRT
<213> Homo Sapiens

<400> 98

Pro Met Pro Ala Pro Arg Gln Asn His Glu
1 5 10

<210> 99
<211> 10
<212> PRT
<213> Homo Sapiens

<400> 99

Pro Met Pro Leu Ala Arg Gln Asn His Glu
1 5 10

<210> 100
<211> 10
<212> PRT
<213> Homo Sapiens

<400> 100

Pro Met Pro Leu Pro Ala Gln Asn His Glu
1 5 10

<210> 101
<211> 10
<212> PRT
<213> Homo Sapiens

<400> 101

Pro Met Pro Leu Pro Arg Ala Asn His Glu
1 5 10

<210> 102
<211> 10
<212> PRT
<213> Homo Sapiens

<400> 102

Pro Met Pro Leu Pro Arg Gln Ala His Glu
1 5 10

<210> 103
<211> 10
<212> PRT

<213> Homo Sapiens

<400> 103

Pro Met Pro Leu Pro Arg Gln Asn Ala Glu
1 5 10

<210> 104
<211> 8
<212> PRT
<213> Homo Sapiens

<400> 104

Pro Leu Pro Arg Gln Asn His Glu
1 5

<210> 105
<211> 7
<212> PRT
<213> Homo Sapiens

<400> 105

Leu Pro Arg Gln Asn His Glu
1 5

<210> 106
<211> 8
<212> PRT
<213> Homo Sapiens

<400> 106

Pro Leu Pro Arg Gln Asn His Glu
1 5

<210> 107
<211> 7
<212> PRT
<213> Homo Sapiens

<400> 107

Leu Pro Arg Gln Asn His Glu
1 5

<210> 108
<211> 882
<212> DNA
<213> Homo Sapiens

<400> 108

```
atgaaatacc tgctgccgac cgctgctgct ggtctgctgc tcctcgctgc ccagccggcc 60
atggccgata ttgtgatgac ccagactcca ctctccctgc ccgtcacccc tggagagccg 120
gcctccatct cctgcaggtc tagtcggagc ctcttggata gtgatgatgg aaacacctat 180
ttggactggt acctgcagaa gccagggcag tctccacagc tcctgatcta cacgctttcc 240
tatcggccct ctggagtccc agacaggttc agtggcagtg ggtcaggcac tgatttcaca 300
ctgaaaatca gcagggtgga ggctgaggat gttggagttt attactgcat gcaacgtgta 360
gagtttccta tcaccttcgg ccaagggaca cgactggaga ttaaactttc cgcggacgat 420
gcgaaaaagg atgctgcgaa gaaagatgac gctaagaaag acgatgctaa aaaggacctc 480
caggtgcagc tggtggagtc tgggggaggc gtggtccagc ctgggaggtc cctgagactc 540
tcctgtgcag cgtctggatt catcttcagt cgctatggca tgcactgggt ccgccaggct 600
ccaggcaagg ggctgaaatg ggtggcagtt atatggtatg atggaagtaa taaactctat 660
gcagactccg tgaagggccg attcaccatc tccagagaca attccaagaa cacgctgtat 720
ctgcaaatga acagcctgag agccgaggac acggctgtgt attactgtgc gagagattac 780

tatgataata gtagacatca ctgggggttt gactactggg gccagggaac cctggtcacc 840
gtctcctcag ctagcgatta taaggacgat gatgacaaat ag                    882
```

<210> 109
<211> 271
<212> PRT
<213> Homo Sapiens

<400> 109

```
Asp Ile Val Met Thr Gln Thr Pro Leu Ser Leu Pro Val Thr Pro Gly
1               5                   10                  15
Glu Pro Ala Ser Ile Ser Cys Arg Ser Ser Arg Ser Leu Leu Asp Ser
            20                  25                  30
Asp Asp Gly Asn Thr Tyr Leu Asp Trp Tyr Leu Gln Lys Pro Gly Gln
        35                  40                  45
Ser Pro Gln Leu Leu Ile Tyr Thr Leu Ser Tyr Arg Ala Ser Gly Val
    50                  55                  60
Pro Asp Arg Phe Ser Gly Ser Gly Ser Gly Thr Asp Phe Thr Leu Lys
65                  70                  75                  80
Ile Ser Arg Val Glu Ala Glu Asp Val Gly Val Tyr Tyr Cys Met Gln
                85                  90                  95
Arg Val Glu Phe Pro Ile Thr Phe Gly Gln Gly Thr Arg Leu Glu Ile
            100                 105                 110
Lys Leu Ser Ala Asp Asp Ala Lys Lys Asp Ala Ala Lys Lys Asp Asp
        115                 120                 125
Ala Lys Lys Asp Asp Ala Lys Lys Asp Leu Gln Val Gln Leu Val Glu
    130                 135                 140
Ser Gly Gly Gly Val Val Gln Pro Gly Arg Ser Leu Arg Leu Ser Cys
145                 150                 155                 160
Ala Ala Ser Gly Phe Ile Phe Ser Arg Tyr Gly Met His Trp Val Arg
                165                 170                 175
Gln Ala Pro Gly Lys Gly Leu Lys Trp Val Ala Val Ile Trp Tyr Asp
            180                 185                 190
Gly Ser Asn Lys Leu Tyr Ala Asp Ser Val Lys Gly Arg Phe Thr Ile
        195                 200                 205
Ser Arg Asp Asn Ser Lys Asn Thr Leu Tyr Leu Gln Met Asn Ser Leu
    210                 215                 220
Arg Ala Glu Asp Thr Ala Val Tyr Tyr Cys Ala Arg Asp Tyr Tyr Asp
225                 230                 235                 240
Asn Ser Arg His His Trp Gly Phe Asp Tyr Trp Gly Gln Gly Thr Leu
                245                 250                 255
Val Thr Val Ser Ser Ala Ser Asp Tyr Lys Asp Asp Asp Asp Lys
            260                 265                 270
```

<210> 110

<211> 1560
<212> DNA
<213> Homo Sapiens

<400> 110

```
atggaaaccc cagcgcagct tctcttcctc ctgctactct ggctcccaga taccaccgga 60
gatattgtga tgacccagac tccactctcc ctgcccgtca cccctggaga gccggcctcc 120
atctcctgca ggtctagtcg gagcctcttg gatagtgatg atggaaacac ctatttggac 180
tggtacctgc agaagccagg gcagtctcca cagctcctga tctacacgct ttcctatcgg 240
gcctctggag tcccagacag gttcagtggc agtgggtcag gcactgattt cacactgaaa 300
atcagcaggg tggaggctga ggatgttgga gtttattact gcatgcaacg tgtagagttt 360
cctatcacct tcggccaagg gacacgactg gagattaaag gtggtggtgg ttctggcggc 420
ggcggctccg gtggtggtgg ttcccaggtg cagctggtgg agtctggggg aggcgtggtc 480
cagcctggga ggtccctgag actctcctgt gcagcgtctg gattcatctt cagtcgctat 540
ggcatgcact gggtccgcca ggctccaggc aaggggctga aatgggtggc agttatatgg 600

tatgatggaa gtaataaact ctatgcagac tccgtgaagg gccgattcac catctccaga 660
gacaattcca agaacacgct gtatctgcaa atgaacagcc tgagagccga ggacacggct 720
gtgtattact gtgcgagaga ttactatgat aatagtagac atcactgggg gtttgactac 780
tggggccagg gaaccctggt caccgtctcc tcaggaggtg gtggatccga tatcaaactg 840
cagcagtcag gggctgaact ggcaagacct ggggcctcag tgaagatgtc ctgcaagact 900
tctggctaca cctttactag gtacacgatg cactgggtaa aacagaggcc tggacagggt 960
ctggaatgga ttggatacat taatcctagc cgtggttata ctaattacaa tcagaagttc 1020
aaggacaagg ccacattgac tacagacaaa tcctccagca cagcctacat gcaactgagc 1080
agcctgacat ctgaggactc tgcagtctat tactgtgcaa gatattatga tgatcattac 1140
tgccttgact actggggcca aggcaccact ctcacagtct cctcagtcga aggtggaagt 1200
ggaggttctg gtggaagtgg aggttcaggt ggagtcgacg acattcagct gacccagtct 1260
ccagcaatca tgtctgcatc tccaggggag aaggtcacca tgacctgcag agccagttca 1320
agtgtaagtt acatgaactg gtaccagcag aagtcaggca cctcccccaa aagatggatt 1380
tatgacacat ccaaagtggc ttctggagtc ccttatcgct tcagtggcag tgggtctggg 1440
acctcatact ctctcacaat cagcagcatg gaggctgaag atgctgccac ttattactgc 1500
caacagtgga gtagtaaccc gctcacgttc ggtgctggga ccaagctgga gctgaaatag 1560
```

<210> 111
<211> 499
<212> PRT
<213> Homo Sapiens

<400> 111

```
Asp Ile Val Met Thr Gln Thr Pro Leu Ser Leu Pro Val Thr Pro Gly
 1               5                  10                  15
Glu Pro Ala Ser Ile Ser Cys Arg Ser Ser Arg Ser Leu Leu Asp Ser
            20                  25                  30
Asp Asp Gly Asn Thr Tyr Leu Asp Trp Tyr Leu Gln Lys Pro Gly Gln
        35                  40                  45
Ser Pro Gln Leu Leu Ile Tyr Thr Leu Ser Tyr Arg Ala Ser Gly Val
    50                  55                  60
Pro Asp Arg Phe Ser Gly Ser Gly Ser Gly Thr Asp Phe Thr Leu Lys
65                  70                  75                  80
Ile Ser Arg Val Glu Ala Glu Asp Val Gly Val Tyr Tyr Cys Met Gln
                85                  90                  95
Arg Val Glu Phe Pro Ile Thr Phe Gly Gln Gly Thr Arg Leu Glu Ile
            100                 105                 110
Lys Gly Gly Gly Gly Ser Gly Gly Gly Gly Ser Gly Gly Gly Gly Ser
        115                 120                 125
Gln Val Gln Leu Val Glu Ser Gly Gly Gly Val Val Gln Pro Gly Arg
    130                 135                 140
Ser Leu Arg Leu Ser Cys Ala Ala Ser Gly Phe Ile Phe Ser Arg Tyr
145                 150                 155                 160
Gly Met His Trp Val Arg Gln Ala Pro Gly Lys Gly Leu Lys Trp Val
                165                 170                 175
Ala Val Ile Trp Tyr Asp Gly Ser Asn Lys Leu Tyr Ala Asp Ser Val
            180                 185                 190
Lys Gly Arg Phe Thr Ile Ser Arg Asp Asn Ser Lys Asn Thr Leu Tyr
        195                 200                 205
Leu Gln Met Asn Ser Leu Arg Ala Glu Asp Thr Ala Val Tyr Tyr Cys
    210                 215                 220
Ala Arg Asp Tyr Tyr Asp Asn Ser Arg His His Trp Gly Phe Asp Tyr
225                 230                 235                 240
Trp Gly Gln Gly Thr Leu Val Thr Val Ser Ser Gly Gly Gly Gly Ser
                245                 250                 255
Asp Ile Lys Leu Gln Gln Ser Gly Ala Glu Leu Ala Arg Pro Gly Ala
            260                 265                 270
Ser Val Lys Met Ser Cys Lys Thr Ser Gly Tyr Thr Phe Thr Arg Tyr
        275                 280                 285
Thr Met His Trp Val Lys Gln Arg Pro Gly Gln Gly Leu Glu Trp Ile
```

```
        290                 295                 300
    Gly Tyr Ile Asn Pro Ser Arg Gly Tyr Thr Asn Tyr Asn Gln Lys Phe
    305                 310                 315                 320
    Lys Asp Lys Ala Thr Leu Thr Thr Asp Lys Ser Ser Ser Thr Ala Tyr
                    325                 330                 335
    Met Gln Leu Ser Ser Leu Thr Ser Glu Asp Ser Ala Val Tyr Tyr Cys
                340                 345                 350
    Ala Arg Tyr Tyr Asp Asp His Tyr Cys Leu Asp Tyr Trp Gly Gln Gly
            355                 360                 365
    Thr Thr Leu Thr Val Ser Ser Val Glu Gly Gly Ser Gly Gly Ser Gly
        370                 375                 380
    Gly Ser Gly Gly Ser Gly Gly Val Asp Asp Ile Gln Leu Thr Gln Ser
    385                 390                 395                 400
    Pro Ala Ile Met Ser Ala Ser Pro Gly Glu Lys Val Thr Met Thr Cys
                    405                 410                 415
    Arg Ala Ser Ser Ser Val Ser Tyr Met Asn Trp Tyr Gln Gln Lys Ser
                420                 425                 430
    Gly Thr Ser Pro Lys Arg Trp Ile Tyr Asp Thr Ser Lys Val Ala Ser
            435                 440                 445
    Gly Val Pro Tyr Arg Phe Ser Gly Ser Gly Ser Gly Thr Ser Tyr Ser
        450                 455                 460
    Leu Thr Ile Ser Ser Met Glu Ala Glu Asp Ala Ala Thr Tyr Tyr Cys
    465                 470                 475                 480
    Gln Gln Trp Ser Ser Asn Pro Leu Thr Phe Gly Ala Gly Thr Lys Leu
                    485                 490                 495
    Glu Leu Lys
```

<210> 112
<211> 1635
<212> DNA
<213> Homo Sapiens

<400> 112

```
atggaaaccc cagcgcagct tctcttcctc ctgctactct ggctcccaga taccaccgga 60
gatattgtga tgacccagac tccactctcc ctgcccgtca cccctggaga gccggcctcc 120
atctcctgca ggtctagtcg gagcctcttg gatagtgatg atggaaacac ctatttggac 180
tggtacctgc agaagccagg gcagtctcca cagctcctga tctacacgct ttcctatcgg 240
gcctctggag tcccagacag gttcagtggc agtgggtcag gcactgattt cacactgaaa 300
atcagcaggg tggaggctga ggatgttgga gtttattact gcatgcaacg tgtagagttt 360
cctatcacct tcggccaagg gacacgactg gagattaaac tttccgcgga cgatgcgaaa 420
aaggatgctg cgaagaaaga tgacgctaag aaagacgatg ctaaaaagga cctgcaggtg 480
cagctggtgg agtctggggg aggcgtggtc cagcctggga ggtccctgag actctcctgt 540
gcagcgtctg gattcatctt cagtcgctat ggcatgcact gggtccgcca ggctccaggc 600
aaggggctga aatgggtggc agttatatgg tatgatggaa gtaataaact ctatgcagac 660
tccgtgaagg gccgattcac catctccaga gacaattcca agaacacgct gtatctgcaa 720
atgaacagcc tgagagccga ggacacggct gtgtattact gtgcgagaga ttactatgat 780
aatagtagac atcactgggg gtttgactac tggggccagg gaaccctggt caccgtctcc 840
tcaggaggtg gtggatccga tatcaaactg cagcagtcag gggctgaact ggcaagacct 900
ggggcctcag tgaagatgtc ctgcaagact tctggctaca cctttactag gtacacgatg 960
cactgggtaa aacagaggcc tggacagggt ctggaatgga ttggatacat taatcctagc 1020
cgtggttata ctaattacaa tcagaagttc aaggacaagg ccacattgac tacagacaaa 1080
tcctccagca cagcctacat gcaactgagc agcctgacat ctgaggactc tgcagtctat 1140
tactgtgcaa gatattatga tgatcattac tgccttgact actggggcca aggcaccact 1200
ctcacagtct cctcactttc cgcggacgat gcgaaaaagg atgctgcgaa gaaagatgac 1260
gctaagaaag acgatgctaa aaaggacctg gacattcagc tgacccagtc tccagcaatc 1320
atgtctgcat ctccagggga gaaggtcacc atgacctgca gagccagttc aagtgtaagt 1380
tacatgaact ggtaccagca gaagtcaggc acctccccca aaagatggat ttatgacaca 1440
tccaaagtgg cttctggagt cccttatcgc ttcagtggca gtgggtctgg gacctcatac 1500
tctctcacaa tcagcagcat ggaggctgaa gatgctgcca cttattactg ccaacagtgg 1560
```

```
agtagtaacc cgctcacgtt cggtgctggg accaagctgg agctgaaaga ttataaggac 1620
gatgatgaca aatag                                                  1635
```

<210> 113
<211> 524
<212> PRT
<213> Homo Sapiens

<400> 113

```
Asp Ile Val Met Thr Gln Thr Pro Leu Ser Leu Pro Val Thr Pro Gly
 1               5                  10                  15
Glu Pro Ala Ser Ile Ser Cys Arg Ser Ser Arg Ser Leu Leu Asp Ser
            20                  25                  30
Asp Asp Gly Asn Thr Tyr Leu Asp Trp Tyr Leu Gln Lys Pro Gly Gln
        35                  40                  45
Ser Pro Gln Leu Leu Ile Tyr Thr Leu Ser Tyr Arg Ala Ser Gly Val
    50                  55                  60
Pro Asp Arg Phe Ser Gly Ser Gly Ser Gly Thr Asp Phe Thr Leu Lys
65                  70                  75                  80
Ile Ser Arg Val Glu Ala Glu Asp Val Gly Val Tyr Tyr Cys Met Gln
                85                  90                  95
Arg Val Glu Phe Pro Ile Thr Phe Gly Gln Gly Thr Arg Leu Glu Ile
            100                 105                 110
Lys Leu Ser Ala Asp Asp Ala Lys Lys Asp Ala Ala Lys Lys Asp Asp
        115                 120                 125
Ala Lys Lys Asp Asp Ala Lys Lys Asp Leu Gln Val Gln Leu Val Glu
    130                 135                 140
Ser Gly Gly Gly Val Val Gln Pro Gly Arg Ser Leu Arg Leu Ser Cys
145                 150                 155                 160
Ala Ala Ser Gly Phe Ile Phe Ser Arg Tyr Gly Met His Trp Val Arg
                165                 170                 175
Gln Ala Pro Gly Lys Gly Leu Lys Trp Val Ala Val Ile Trp Tyr Asp
            180                 185                 190
Gly Ser Asn Lys Leu Tyr Ala Asp Ser Val Lys Gly Arg Phe Thr Ile
        195                 200                 205
Ser Arg Asp Asn Ser Lys Asn Thr Leu Tyr Leu Gln Met Asn Ser Leu
    210                 215                 220
Arg Ala Glu Asp Thr Ala Val Tyr Tyr Cys Ala Arg Asp Tyr Tyr Asp
225                 230                 235                 240
Asn Ser Arg His His Trp Gly Phe Asp Tyr Trp Gly Gln Gly Thr Leu
                245                 250                 255
Val Thr Val Ser Ser Gly Gly Gly Gly Ser Asp Ile Lys Leu Gln Gln
            260                 265                 270
Ser Gly Ala Glu Leu Ala Arg Pro Gly Ala Ser Val Lys Met Ser Cys
        275                 280                 285
Lys Thr Ser Gly Tyr Thr Phe Thr Arg Tyr Thr Met His Trp Val Lys
    290                 295                 300
Gln Arg Pro Gly Gln Gly Leu Glu Trp Ile Gly Tyr Ile Asn Pro Ser
305                 310                 315                 320
Arg Gly Tyr Thr Asn Tyr Asn Gln Lys Phe Lys Asp Lys Ala Thr Leu
                325                 330                 335
Thr Thr Asp Lys Ser Ser Ser Thr Ala Tyr Met Gln Leu Ser Ser Leu
            340                 345                 350
Thr Ser Glu Asp Ser Ala Val Tyr Tyr Cys Ala Arg Tyr Tyr Asp Asp
        355                 360                 365
His Tyr Cys Leu Asp Tyr Trp Gly Gln Gly Thr Thr Leu Thr Val Ser
    370                 375                 380
Ser Leu Ser Ala Asp Asp Ala Lys Lys Asp Ala Ala Lys Lys Asp Asp
385                 390                 395                 400
Ala Lys Lys Asp Asp Ala Lys Lys Asp Leu Asp Ile Gln Leu Thr Gln
                405                 410                 415
```

Ser Pro Ala Ile Met Ser Ala Ser Pro Gly Glu Lys Val Thr Met Thr
420 425 430
Cys Arg Ala Ser Ser Ser Val Ser Tyr Met Asn Trp Tyr Gln Gln Lys
435 440 445
Ser Gly Thr Ser Pro Lys Arg Trp Ile Tyr Asp Thr Ser Lys Val Ala
450 455 460
Ser Gly Val Pro Tyr Arg Phe Ser Gly Ser Gly Ser Gly Thr Ser Tyr
465 470 475 480
Ser Leu Thr Ile Ser Ser Met Glu Ala Glu Asp Ala Ala Thr Tyr Tyr
485 490 495
Cys Gln Gln Trp Ser Ser Asn Pro Leu Thr Phe Gly Ala Gly Thr Lys
500 505 510
Leu Glu Leu Lys Asp Tyr Lys Asp Asp Asp Asp Lys
515 520

<210> 114
<211> 169
<212> PRT
<213> Homo Sapiens

<400> 114

Trp Val Leu Ser Gln Val Gln Leu Gln Glu Ser Gly Pro Gly Leu Val
1 5 10 15
Lys Pro Ser Glu Thr Leu Ser Leu Thr Cys Thr Val Ser Gly Gly Ser
20 25 30
Val Ser Ser Gly Gly Tyr Tyr Trp Ser Trp Ile Arg Gln Pro Pro Gly
35 40 45
Lys Gly Leu Glu Trp Ile Gly Phe Ile Tyr Tyr Thr Gly Ser Thr Asn
50 55 60
Tyr Asn Pro Ser Leu Lys Ser Arg Val Ser Ile Ser Val Asp Thr Ser
65 70 75 80
Lys Asn Gln Phe Ser Leu Lys Leu Ser Ser Val Thr Ala Ala Asp Ala
85 90 95
Ala Val Tyr Tyr Cys Ala Arg Asp Tyr Asp Trp Ser Phe His Phe Asp
100 105 110
Tyr Trp Gly Gln Gly Thr Leu Val Thr Val Ser Ser Ala Ser Thr Lys
115 120 125
Gly Pro Ser Val Phe Pro Leu Ala Pro Cys Ser Arg Ser Thr Ser Glu
130 135 140
Ser Thr Ala Ala Leu Gly Cys Leu Val Lys Asp Tyr Phe Pro Glu Pro
145 150 155 160
Val Thr Val Ser Trp Asn Ser Gly Ala
165

<210> 115
<211> 168
<212> PRT
<213> Homo Sapiens

<400> 115

Gln Leu Leu Gly Leu Leu Leu Leu Trp Phe Pro Gly Ala Arg Cys Asp
1 5 10 15
Ile Gln Met Thr Gln Ser Pro Ser Ser Leu Ser Ala Ser Ile Gly Asp
20 25 30
Arg Val Thr Ile Thr Cys Arg Ala Ser Gln Gly Ile Arg Asn Asp Leu
35 40 45
Gly Trp Tyr Gln Gln Lys Pro Gly Lys Ala Pro Lys Arg Leu Ile Tyr
50 55 60
Ala Ala Ser Ser Leu Gln Ser Gly Val Pro Ser Arg Phe Ser Gly Ser

```
65                  70                  75                  80
Gly Ser Gly Thr Glu Phe Thr Leu Thr Ile Ser Ser Leu Gln Pro Glu
                85                  90                  95
Asp Phe Ala Thr Tyr Tyr Cys Leu Gln His Asn Ser Tyr Pro Leu Thr
            100                 105                 110
Phe Gly Gly Gly Thr Lys Val Glu Ile Lys Arg Thr Val Ala Ala Pro
        115                 120                 125
Ser Val Phe Ile Phe Pro Pro Ser Asp Glu Gln Leu Lys Ser Gly Thr
    130                 135                 140
Ala Ser Val Val Cys Leu Leu Asn Asn Phe Tyr Pro Arg Glu Ala Lys
145                 150                 155                 160
Val Gln Trp Lys Val Asp Asn Ala
                165
```

<210> 116
<211> 156
<212> PRT
<213> Homo Sapiens

<400> 116

```
Gln Cys Glu Val Gln Leu Val Glu Ser Gly Gly Gly Leu Val Gln Pro
 1               5                  10                  15
Gly Gly Ser Leu Arg Leu Ser Cys Ala Ala Ser Gly Phe Thr Phe Thr
            20                  25                  30
Asn Tyr Trp Met Ser Trp Val Arg Gln Ala Pro Gly Lys Gly Leu Glu
        35                  40                  45
Trp Val Ala Asn Ile Gln Gln Asp Gly Ser Glu Lys Tyr Tyr Val Asp
    50                  55                  60
Ser Val Arg Gly Arg Phe Thr Ile Ser Arg Asp Asn Ala Lys Asn Ser
65                  70                  75                  80
Leu Tyr Leu Gln Met Asn Ser Leu Arg Ala Glu Asp Ser Ala Val Tyr
                85                  90                  95
Tyr Cys Ala Arg Trp Asp Tyr Trp Gly Gln Gly Thr Leu Val Thr Val
            100                 105                 110
Ser Ser Ala Ser Thr Lys Gly Pro Ser Val Phe Pro Leu Ala Pro Cys
        115                 120                 125
Ser Arg Ser Thr Ser Glu Ser Thr Ala Ala Leu Gly Cys Leu Val Lys
    130                 135                 140
Asp Tyr Phe Pro Glu Pro Val Ser Gly Val Val Glu
145                 150                 155
```

<210> 117
<211> 151
<212> PRT
<213> Homo Sapiens

<400> 117

```
Leu Leu Gly Leu Leu Met Leu Trp Val Pro Gly Ser Ser Gly Asp Ile
 1               5                  10                  15
Val Met Thr Gln Thr Pro Leu Ser Ser Thr Val Ile Leu Gly Gln Pro
            20                  25                  30
Ala Ser Ile Ser Cys Arg Ser Ser Gln Ser Leu Val His Ser Asp Gly
        35                  40                  45
Asn Thr Tyr Leu Asn Trp Leu Gln Gln Arg Pro Gly Gln Pro Pro Arg
    50                  55                  60
Leu Leu Ile Tyr Met Ile Ser Asn Arg Phe Ser Gly Val Pro Asp Arg
65                  70                  75                  80
Phe Ser Gly Ser Gly Ala Gly Thr Asp Phe Thr Leu Lys Ile Ser Arg
                85                  90                  95

Val Glu Ala Glu Asp Val Gly Val Tyr Tyr Cys Met Gln Ala Thr Glu
            100                 105                 110
Ser Pro Gln Thr Phe Gly Gln Gly Thr Lys Val Glu Ile Lys Arg Thr
        115                 120                 125
Val Ala Ala Pro Ser Val Phe Ile Phe Pro Pro Ser Asp Glu Gln Leu
    130                 135                 140
Lys Ser Gly Arg Ala Ser Val
145                 150
```

<210> 118
<211> 180
<212> PRT
<213> Homo Sapiens

<220>
<221> misc_feature
<222> (1) .. (1)
<223> Wherein Xaa may be any amino acid

<220>
<221> misc_feature
<222> (2) .. (2)
<223> Wherein Xaa may be any amino acid

<220>
<221> misc_feature
<222> (3) .. (3)
<223> Wherein Xaa may be any amino acid

<220>
<221> misc_feature
<222> (4) .. (4)
<223> Wherein Xaa may be any amino acid

<400> 118

```
Xaa Xaa Xaa Xaa Glu Gln Ser Gly Gly Gly Val Val Lys Pro Gly Gly
 1               5                  10                  15
Ser Leu Arg Leu Ser Cys Ala Ala Ser Gly Phe Thr Phe Ser Asn Ala
            20                  25                  30
Trp Met Thr Trp Val Arg Gln Ala Pro Gly Lys Gly Leu Glu Trp Val
        35                  40                  45
Gly Arg Ile Lys Arg Arg Thr Asp Gly Gly Thr Thr Asp Tyr Ala Ala
    50                  55                  60
Pro Val Lys Gly Arg Phe Thr Ile Ser Arg Asp Asp Ser Lys Asn Thr
65                  70                  75                  80
Leu Tyr Leu Gln Met Asn Asn Leu Lys Asn Glu Asp Thr Ala Val Tyr
                85                  90                  95
Tyr Cys Thr Ser Val Asp Asn Asp Val Asp Tyr Trp Gly Gln Gly Thr
            100                 105                 110
Leu Val Thr Val Ser Ser Ala Ser Thr Lys Gly Pro Ser Val Phe Pro
        115                 120                 125
Leu Ala Pro Cys Ser Arg Ser Thr Ser Glu Ser Thr Ala Ala Leu Gly
    130                 135                 140
Cys Leu Val Lys Asp Tyr Phe Pro Glu Pro Val Thr Val Ser Trp Asn
145                 150                 155                 160
Ser Gly Ala Leu Thr Ser Gly Val His Thr Phe Pro Ala Val Leu Gln
                165                 170                 175
Ser Ser Gly Leu
            180
```

<210> 119
<211> 152
<212> PRT
<213> Homo Sapiens

<220>
<221> misc_feature
<222> (1) .. (1)
<223> Wherein Xaa may be any amino acid

<220>
<221> misc_feature
<222> (2) .. (2)
<223> Wherein Xaa may be any amino acid

<220>
<221> misc_feature
<222> (3) .. (3)
<223> Wherein Xaa may be any amino acid

<400> 119

```
Xaa Xaa Xaa Leu Thr Gln Ser Pro Leu Ser Leu Pro Val Thr Pro Gly
 1               5                  10                  15
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Ile Gly Leu Tyr Tyr Cys Met Gln Ala
                85                  90                  95
Leu Gln Thr Pro Leu Thr Phe Gly Gly Gly Thr Lys Val Asp Ile Lys
            100                 105                 110
Arg Thr Val Ala Ala Pro Ser Val Phe Ile Phe Pro Pro Ser Asp Glu
        115                 120                 125
Gln Leu Lys Ser Gly Thr Ala Ser Val Val Cys Leu Leu Asn Asn Phe
    130                 135                 140
Tyr Pro Arg Glu Ala Lys Val Gln
145                 150
```

<210> 120
<211> 179
<212> PRT
<213> Homo Sapiens

<400> 120

```
Gln Val Gln Leu Glu Gln Ser Gly Gly Gly Leu Val Gln Pro Gly Gly
 1               5                  10                  15
Ser Leu Arg Leu Ser Cys Ala Ala Ser Gly Phe Thr Phe Ser Thr Tyr
            20                  25                  30
Ser Met Asn Trp Val Arg Gln Ala Pro Gly Lys Gly Leu Glu Trp Val
        35                  40                  45
Ser Tyr Ile Arg Ser Ser Thr Ser Thr Ile Tyr Tyr Ala Glu Ser Leu
    50                  55                  60
Lys Gly Arg Phe Thr Ile Ser Ser Asp Asn Ala Lys Asn Ser Leu Tyr
65                  70                  75                  80
Leu Gln Met Asn Ser Leu Arg Asp Glu Asp Thr Ala Val Tyr Tyr Cys


                85                  90                  95
Ala Arg Asp Phe Asp Tyr Trp Gly Gln Gly Thr Leu Val Thr Val Ser
            100                 105                 110
Ser Ala Ser Thr Lys Gly Pro Ser Val Phe Pro Leu Ala Pro Cys Ser
        115                 120                 125
Arg Ser Thr Ser Glu Ser Thr Ala Ala Leu Gly Cys Leu Val Lys Asp
    130                 135                 140
Tyr Phe Pro Glu Pro Val Thr Val Ser Trp Asn Ser Gly Ala Leu Thr
145                 150                 155                 160
Ser Gly Val His Thr Phe Pro Ala Val Leu Gln Ser Ser Gly Leu Tyr
                165                 170                 175
Ser Leu Ser
```

<210> 121
<211> 163
<212> PRT
<213> Homo Sapiens

<400> 121

```
Glu Ile Gln Leu Thr Gln Ser Pro Leu Ser Ser Pro Val Thr Leu Gly
 1               5                  10                  15
Gln Pro Ala Ser Ile Ser Cys Arg Ser Ser Gln Ser Leu Val His Ser
            20                  25                  30
Asp Gly Asp Thr Tyr Leu Asn Trp Leu Gln Gln Arg Pro Gly Gln Pro
        35                  40                  45
Pro Arg Leu Leu Ile Tyr Lys Ile Ser Thr Arg Phe Ser Gly Val Pro
    50                  55                  60
Asp Arg Phe Ser Gly Ser Gly Ala Gly Thr Asp Phe Thr Leu Lys Ile
65                  70                  75                  80
Ser Arg Val Glu Thr Asp Asp Val Gly Ile Tyr Tyr Cys Met Gln Thr
                85                  90                  95
Thr Gln Ile Pro Gln Ile Thr Phe Gly Gln Gly Thr Arg Leu Glu Ile
            100                 105                 110
Lys Arg Thr Val Ala Ala Pro Ser Val Phe Ile Phe Pro Pro Ser Asp
        115                 120                 125
Glu Gln Leu Lys Ser Gly Thr Ala Ser Val Val Cys Leu Leu Asn Asn
    130                 135                 140
Phe Tyr Pro Arg Glu Ala Lys Val Gln Trp Lys Val Asp Asn Ala Leu
145                 150                 155                 160
Gln Ser Gly
```

<210> 122
<211> 189
<212> PRT
<213> Homo Sapiens

<400> 122

```
Gln Val Gln Leu Glu Gln Ser Gly Gly Gly Val Val Gln Pro Gly Arg
 1               5                  10                  15
Ser Leu Arg Leu Ser Cys Ala Ala Ser Gly Phe Thr Phe Ser Arg Tyr
            20                  25                  30
Gly Met His Trp Val Arg Gln Ala Pro Gly Lys Gly Leu Lys Trp Val
        35                  40                  45
Ala Val Ile Trp Tyr Asp Gly Ser Asn Lys Leu Tyr Ala Asp Ser Val
    50                  55                  60
Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95
Ala Arg Asp Tyr Tyr Asp Asn Ser Arg His His Trp Gly Phe Asp Tyr
            100                 105                 110
Trp Gly Gln Gly Thr Leu Val Thr Val Ser Ser Ala Ser Thr Lys Gly
        115                 120                 125
Pro Ser Val Phe Pro Leu Ala Pro Cys Ser Arg Ser Thr Ser Glu Ser
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser
            180                 185
```

<210> 123
<211> 159
<212> PRT
<213> Homo Sapiens

<400> 123

```
Asp Ile Gln Leu Met Thr Leu Gln Ser Pro Ser Ser Leu Ser Ala Ser
 1               5                  10                  15
Val Gly Asp Arg Val Thr Ile Thr Cys Arg Ala Ser Gln Ser Ile Tyr
            20                  25                  30
Ser Tyr Leu Asn Trp Tyr Gln Gln Lys Pro Gly Lys Ala Pro Lys Leu
        35                  40                  45
Leu Ile Tyr Ala Ala Ser Ser Leu Gln Ser Gly Val Pro Ser Arg Phe
    50                  55                  60
Ser Gly Ser Gly Ser Gly Thr Asp Phe Thr Leu Thr Ile Ser Ser Leu
65                  70                  75                  80
Gln Pro Glu Asp Phe Ala Thr Tyr Tyr Cys Gln Gln Ser Tyr Ser Thr
                85                  90                  95
Pro Pro Thr Phe Gly Gln Gly Thr Lys Val Glu Ile Lys Arg Thr Val
            100                 105                 110
Ala Ala Pro Ser Val Phe Ile Phe Pro Pro Ser Asp Glu Gln Leu Lys
        115                 120                 125
Ser Gly Thr Ala Ser Val Val Cys Leu Leu Asn Asn Phe Tyr Pro Arg
    130                 135                 140
Glu Ala Lys Val Gln Trp Lys Val Asp Asn Ala Leu Gln Ser Gly
145                 150                 155
```

<210> 124
<211> 181
<212> PRT
<213> Homo Sapiens

<220>
<221> misc_feature
<222> (1) .. (1)
<223> Wherein Xaa may be any amino acid

<220>
<221> misc_feature
<222> (2) .. (2)
<223> Wherein Xaa may be any amino acid

<220>
<221> misc_feature
<222> (3) .. (3)
<223> Wherein Xaa may be any amino acid

<220>
<221> misc_feature
<222> (4) .. (4)
<223> Wherein Xaa may be any amino acid

<220>
<221> misc_feature
<222> (5) .. (5)
<223> Wherein Xaa may be any amino acid

<400> 124

```
Xaa Xaa Xaa Xaa Xaa Gln Ser Gly Gly Gly Leu Val Lys Pro Gly Gly
 1               5                   10                  15
Ser Leu Arg Leu Ser Cys Ala Ala Ser Gly Phe Thr Phe Ser Asn Ala
            20                  25                  30
Trp Met Thr Trp Val Arg Gln Ala Pro Gly Lys Gly Leu Glu Trp Val
        35                  40                  45
Gly Arg Ile Lys Arg Lys Thr Asp Gly Gly Thr Thr Asp Tyr Ala Ala
    50                  55                  60
Pro Val Lys Gly Arg Phe Thr Ile Ser Arg Asp Asp Ser Glu Asn Thr
65                  70                  75                  80
Leu Tyr Leu Gln Met Asn Ser Leu Glu Thr Glu Asp Thr Ala Val Tyr
                85                  90                  95
Tyr Cys Thr Thr Val Asp Asn Ser Gly Asp Tyr Trp Gly Gln Gly Thr
            100                 105                 110
Leu Val Thr Val Ser Ser Ala Ser Thr Lys Gly Pro Ser Val Phe Pro
        115                 120                 125
Leu Ala Pro Cys Ser Arg Ser Thr Ser Glu Ser Thr Ala Ala Leu Gly
    130                 135                 140
Cys Leu Val Lys Asp Tyr Phe Pro Glu Pro Val Thr Val Ser Trp Asn
145                 150                 155                 160
Ser Gly Ala Leu Thr Ser Gly Val His Thr Phe Pro Ala Val Leu Gln
                165                 170                 175
Ser Ser Gly Leu Ser
            180
```

<210> 125
<211> 159
<212> PRT
<213> Homo Sapiens

<220>
<221> misc_feature
<222> (1) .. (1)
<223> Wherein Xaa may be any amino acid

<220>
<221> misc_feature
<222> (2) .. (2)
<223> Wherein Xaa may be any amino acid

<220>
<221> misc_feature
<222> (3) .. (3)
<223> Wherein Xaa may be any amino acid

<220>
<221> misc_feature
<222> (4) .. (4)
<223> Wherein Xaa may be any amino acid

<400> 125

Xaa Xaa Xaa Xaa Thr Gln Ser Pro Leu Ser Leu Pro Val Thr Pro Gly
1 5 10 15
Glu Pro Ala Ser Ile Ser Cys Arg Ser Ser Gln Ser Leu Leu His Ser
20 25 30
Asn Gly Tyr Asn Tyr Leu Asp Trp Tyr Leu Gln Lys Pro Gly Gln Ser
35 40 45
Pro Gln Leu Leu Ile Tyr Leu Gly Ser Asn Arg Ala Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Ala
85 90 95
Leu Gln Thr Pro Leu Thr Phe Gly Gly Gly Thr Lys Val Glu Ile Lys
100 105 110
Arg Thr Val Ala Ala Pro Ser Val Phe Ile Phe Pro Pro Ser Asp Glu
115 120 125
Gln Leu Lys Ser Gly Thr Ala Ser Val Val Cys Leu Leu Asn Asn Phe
130 135 140
Tyr Pro Arg Glu Ala Lys Val Gln Trp Lys Val Asp Asn Ala Leu
145 150 155

<210> 126
<211> 179
<212> PRT
<213> Homo Sapiens

<400> 126

Gln Val Gln Leu Glu Gln Ser Gly Gly Gly Val Val Gln Pro Gly Arg
1 5 10 15
Ser Leu Arg Leu Ser Cys Ala Ala Ser Gly Phe Thr Phe Thr Asn Tyr
20 25 30
Gly Leu His Trp Val Arg Gln Ala Pro Gly Lys Gly Leu Asp Trp Val
35 40 45
Ala Val Ile Trp Tyr Asp Gly Ser His Lys Phe Tyr Ala Asp Ser Val
50 55 60
Lys Gly Arg Phe Thr Ile Ser Arg Asp Asn Ser Lys Asn Thr Leu Phe
65 70 75 80
Leu Gln Met Asn Ser Leu Arg Ala Glu Asp Thr Ala Val Tyr Tyr Cys
85 90 95
Thr Arg Asp Leu Asp Tyr Trp Gly Gln Gly Thr Leu Val Thr Val Ser
100 105 110
Ser Ala Ser Thr Lys Gly Pro Ser Val Phe Pro Leu Ala Pro Cys Ser
115 120 125
Arg Ser Thr Ser Glu Ser Thr Ala Ala Leu Gly Cys Leu Val Lys Asp
130 135 140
Tyr Phe Pro Glu Pro Val Thr Val Ser Trp Asn Ser Gly Ala Leu Thr
145 150 155 160
Ser Gly Val His Thr Phe Pro Ala Val Leu Gln Ser Ser Gly Leu Tyr
165 170 175
Ser Leu Ser

<210> 127
<211> 160
<212> PRT
<213> Homo Sapiens

<400> 127

Glu Thr Gln Leu Thr Gln Ser Pro Gly Thr Leu Ser Leu Ser Pro Gly
1 5 10 15
Glu Arg Val Thr Leu Ser Cys Arg Ala Ser Gln Ser Val Ser Asn Asn
20 25 30
Tyr Leu Ala Trp Tyr Gln Gln Lys Pro Gly Gln Ala Pro Arg Leu Leu
35 40 45
Ile Tyr Gly Ala Ser Ser Arg Ala Thr Gly Ile Pro Asp Arg Phe Ser
50 55 60
Gly Ser Gly Ser Gly Thr Asp Phe Thr Leu Thr Ile Ser Arg Leu Glu
65 70 75 80
Pro Glu Asp Cys Ala Glu Cys Tyr Cys Gln Gln Tyr Gly Ser Ser Leu
85 90 95
Pro Leu Thr Phe Gly Gly Gly Thr Lys Val Glu Ile Lys Arg Thr Val
100 105 110
Ala Ala Pro Ser Val Phe Ile Phe Pro Pro Ser Asp Glu Gln Leu Lys
115 120 125
Ser Gly Thr Ala Ser Val Val Cys Leu Leu Asn Asn Phe Tyr Pro Arg
130 135 140
Glu Ala Lys Val Gln Trp Glu Gly Gly Ile Thr Pro Ser Asn Arg Val
145 150 155 160

<210> 128
<211> 182
<212> PRT
<213> Homo Sapiens

<400> 128

Val Gln Cys Gln Val Gln Leu Val Glu Ser Gly Gly Gly Val Val Gln
1 5 10 15
Pro Gly Arg Ser Leu Arg Leu Ser Cys Ala Ala Ser Gly Phe Thr Phe
20 25 30
Ser Ser Tyr Gly Met His Trp Val Arg Gln Ala Pro Gly Lys Gly Leu
35 40 45
Glu Trp Val Ala Val Ile Trp Tyr Asp Gly Ser His Lys Tyr Leu Tyr
50 55 60
Ala Thr Asp Ser Val Lys Gly Arg Phe Thr Ile Ser Arg Asp Asn Ser
65 70 75 80
Lys Asn Thr Leu Tyr Leu Gln Met Asn Ser Leu Arg Ala Glu Asp Thr
85 90 95
Ala Val Tyr Tyr Ser Ala Arg Asp Tyr Tyr Asp Thr Ser Arg His His
100 105 110
Trp Gly Phe Asp Cys Trp Gly Gln Gly Thr Leu Val Thr Val Ser Ser
115 120 125
Ala Ser Thr Lys Gly Pro Ser Val Phe Pro Leu Ala Pro Cys Ser Arg
130 135 140
Ser Thr Ser Glu Ser Thr Ala Ala Leu Gly Cys Leu Val Lys Asp Tyr
145 150 155 160
Phe Pro Glu Pro Val Thr Val Ser Trp Asn Ser Gly Ala Leu Thr Ser
165 170 175
Gly Val His Thr Phe Pro
180

<210> 129
<211> 173
<212> PRT
<213> Homo Sapiens

<400> 129

```
Gln Leu Leu Gly Leu Leu Met Leu Trp Val Pro Gly Ser Ser Glu Glu
 1               5                  10                  15
Ile Val Met Thr Gln Thr Pro Leu Ser Leu Pro Val Thr Pro Gly Glu
            20                  25                  30
Pro Ala Ser Ile Ser Cys Arg Ser Ser Gln Ser Leu Leu Asp Ser Glu
        35                  40                  45
Asp Gly Asn Thr Tyr Leu Asp Trp Tyr Leu Gln Lys Pro Gly Gln Ser
    50                  55                  60
Pro Gln Leu Leu Ile Tyr Thr Leu Ser His Arg Ala Ser Gly Val Pro
65                  70                  75                  80
Asp Arg Phe Ser Gly Ser Gly Ser Gly Thr Asp Phe Thr Leu Lys Ile
                85                  90                  95
Ser Arg Val Glu Ala Glu Asp Val Gly Val Tyr Cys Cys Met Gln Arg
            100                 105                 110
Val Glu Phe Pro Ile Thr Phe Gly Gln Gly Thr Arg Leu Glu Ile Lys
        115                 120                 125
Arg Thr Val Ala Ala Pro Ser Val Phe Ile Phe Pro Pro Ser Asp Glu
    130                 135                 140
Gln Leu Lys Ser Gly Thr Ala Ser Val Val Cys Leu Leu Asn Asn Phe
145                 150                 155                 160
Tyr Pro Arg Glu Ala Lys Val Gln Trp Lys Val Asp Asn
                165                 170
```

<210> 130
<211> 187
<212> PRT
<213> Homo Sapiens

<220>
<221> misc_feature
<222> (1) .. (1)
<223> Wherein Xaa may be any amino acid

<220>
<221> misc_feature
<222> (2) .. (2)
<223> Wherein Xaa may be any amino acid

<220>
<221> misc_feature
<222> (3) .. (3)
<223> Wherein Xaa may be any amino acid

<220>
<221> misc_feature
<222> (4) .. (4)
<223> Wherein Xaa may be any amino acid

<220>
<221> misc_feature
<222> (5) .. (5)
<223> Wherein Xaa may be any amino acid

<400> 130

```
Xaa Xaa Xaa Xaa Xaa Gln Ser Gly Pro Arg Leu Val Lys Pro Ser Gln
 1               5                  10                  15
Thr Leu Ser Leu Thr Cys Thr Val Ser Gly Gly Ser Ile Ser Ser Asp
```

```
                20                  25                  30
Gly Tyr Tyr Trp Ser Trp Ile Arg Gln His Pro Gly Lys Gly Leu Glu
            35                  40                  45
Trp Ile Gly Tyr Ile Tyr Tyr Ser Gly Ser Thr Phe Tyr Asn Pro Ser
    50                  55                  60
Leu Lys Ser Arg Val Ala Ile Ser Val Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg Glu Ser Pro His Ser Ser Asn Trp Tyr Ser Gly Phe Asp
            100                 105                 110
Cys Trp Gly Gln Gly Thr Leu Val Thr Val Ser Ser Ala Ser Thr Lys
        115                 120                 125
Gly Pro Ser Val Phe Pro Leu Ala Pro Cys Ser Arg Ser Thr Ser Glu
    130                 135                 140
Ser Thr Ala Ala Leu Gly Cys Leu Val Lys Asp Tyr Phe Pro Arg Thr
145                 150                 155                 160
Gly Asp Gly Val Val Glu Leu Arg Arg Pro Asp Gln Arg Arg Ala His
                165                 170                 175
Leu Pro Gly Cys Pro Thr Val Leu Arg Thr Leu
            180                 185
```

<210> 131
<211> 154
<212> PRT
<213> Homo Sapiens

<220>
<221> misc_feature
<222> (1) .. (1)
<223> Wherein Xaa may be any amino acid

<220>
<221> misc_feature
<222> (2) .. (2)
<223> Wherein Xaa may be any amino acid

<220>
<221> misc_feature
<222> (3) .. (3)
<223> Wherein Xaa may be any amino acid

<220>
<221> misc_feature
<222> (4) .. (4)
<223> Wherein Xaa may be any amino acid

<400> 131

```
Xaa Xaa Xaa Xaa Thr Gln Ser Pro Asp Phe Gln Ser Val Thr Pro Lys
 1               5                  10                  15
Glu Lys Val Thr Ile Thr Cys Arg Ala Ser Gln Ser Ile Gly Ser Arg
            20                  25                  30
Leu His Trp Tyr Gln Gln Lys Pro Asp Gln Ser Pro Lys Leu Leu Ile
        35                  40                  45
Lys Tyr Ala Ser Gln Ser Phe Ser Gly Val Pro Ser Arg Phe Ser Gly
    50                  55                  60
Ser Gly Ser Gly Thr Asp Phe Thr Leu Thr Ile Asn Ser Leu Glu Ala
65                  70                  75                  80
Glu Asp Ala Ala Thr Tyr Tyr Cys His Gln Ser Ser Asn Leu Pro Phe
                85                  90                  95


Thr Phe Gly Pro Gly Thr Lys Val Asp Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu
145                 150
```

<210> 132
<211> 180
<212> PRT
<213> Homo Sapiens

<400> 132

```
Gln Val Gln Leu Val Glu Gln Ala Gly Gly Gly Val Val Gln Pro Gly
 1               5                  10                  15
Arg Ser Leu Arg Leu Ser Cys Ala Ala Ser Gly Phe Thr Phe Arg Ser
            20                  25                  30
Tyr Gly Met His Trp Val Arg Gln Ala Pro Gly Lys Gly Leu Lys Trp
        35                  40                  45
Val Ala Val Ile Trp Tyr Asp Gly Ser Asn Lys Tyr Leu Tyr Thr Asp
    50                  55                  60
Ser Val Lys Gly Arg Phe Thr Ile Ser Arg Asp Asn Ser Lys Asn Thr
65                  70                  75                  80
Leu Tyr Leu Gln Met Asn Ser Leu Arg Ala Glu Asp Thr Ala Val Tyr
                85                  90                  95
Tyr Cys Val Arg Asp Tyr Tyr Asp Asn Ser Arg His His Trp Gly Phe
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Cys Ser Arg Ser Thr Ser
    130                 135                 140
Glu Ser Thr Ala Ala Leu Gly Cys Leu Val Lys Asp Tyr Phe Pro Glu
145                 150                 155                 160
Pro Val Thr Val Ser Trp Asn Ser Gly Ala Leu Thr Arg Arg Arg Ala
                165                 170                 175
His Leu Pro Gly
            180
```

<210> 133
<211> 156
<212> PRT
<213> Homo Sapiens

<400> 133

```
Asp Ile Gln Met Thr Gln Ser Pro Ser Ser Arg Cys Ala Ser Val Gly
 1               5                  10                  15
Asp Arg Val Thr Ile Thr Cys Arg Ala Ser Gln Gly Ile Arg Asn Asp
            20                  25                  30
Leu Ala Trp Tyr Gln Gln Lys Pro Gly Lys Ala Pro Lys Arg Leu Ile
        35                  40                  45
Tyr Ala Ala Ser Ser Leu Gln Ser Gly Val Pro Ser Arg Phe Ser Gly
    50                  55                  60
Ser Arg Ser Gly Thr Glu Phe Thr Leu Thr Ile Ser Ser Leu Gln Pro
65                  70                  75                  80
Glu Asp Phe Ala Ala Tyr Tyr Cys Leu Gln His Asn Ser Tyr Pro Pro
                85                  90                  95
Ser Phe Gly Gln Gly Thr Lys Leu Glu Ile Lys Arg Thr Val Ala Ala


            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser
145                 150                 155
```

<210> 134
<211> 171
<212> PRT
<213> Homo Sapiens

<400> 134

```
His Val Gln Val Gln Leu Val Glu Ser Gly Gly Gly Val Val Gln Pro
 1               5                  10                  15
Gly Arg Ser Leu Arg Leu Ser Cys Ala Ala Ser Gly Phe Ile Phe Ser
            20                  25                  30
Arg Tyr Gly Met His Trp Val Arg Gln Ala Pro Gly Lys Gly Leu Lys
        35                  40                  45
Trp Val Ala Val Ile Trp Tyr Asp Gly Ser Asn Lys Leu Tyr Ala Asp
    50                  55                  60
Ser Val Lys Gly Arg Phe Thr Ile Ser Arg Asp Asn Ser Lys Asn Thr
65                  70                  75                  80
Leu Tyr Leu Gln Met Asn Ser Leu Arg Ala Glu Asp Thr Ala Val Tyr
                85                  90                  95
Tyr Cys Ala Arg Asp Tyr Tyr Asp Asn Ser Arg His His Trp Gly Phe
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Cys Ser Arg Ser Thr Ser
    130                 135                 140
Glu Ser Thr Ala Ala Leu Gly Cys Leu Val Lys Asp Tyr Phe Pro Glu
145                 150                 155                 160
Pro Val Thr Val Ser Trp Asn Ser Gly Ala Leu
                165                 170
```

<210> 135
<211> 174
<212> PRT
<213> Homo Sapiens

<400> 135

```
Ser Ala Pro Gly Ala Ala Asn Ala Leu Gly Pro Trp Ile Ser Glu Asp
 1               5                  10                  15
Ile Val Met Thr Gln Thr Pro Leu Ser Leu Pro Val Thr Pro Gly Glu
            20                  25                  30
Pro Ala Ser Ile Ser Cys Arg Ser Ser Arg Ser Leu Leu Asp Ser Asp
        35                  40                  45
Asp Gly Asn Thr Tyr Leu Asp Trp Tyr Leu Gln Lys Pro Gly Gln Ser
    50                  55                  60
Pro Gln Leu Leu Ile Tyr Thr Leu Ser Tyr Arg Ala Ser Gly Val Pro
65                  70                  75                  80
Asp Arg Phe Ser Gly Ser Gly Ser Gly Thr Asp Phe Thr Leu Lys Ile
                85                  90                  95
Ser Arg Val Glu Ala Glu Asp Val Gly Val Tyr Tyr Cys Met Gln Arg
            100                 105                 110
Val Glu Phe Pro Ile Thr Phe Gly Gln Gly Thr Arg Leu Glu Ile Lys
        115                 120                 125

Arg Thr Val Ala Ala Pro Ser Val Phe Ile Phe Pro Pro Ser Asp Glu
    130                 135                 140
Gln Leu Lys Ser Gly Thr Ala Ser Val Val Cys Leu Leu Asn Asn Phe
145                 150                 155                 160
Tyr Pro Arg Glu Ala Lys Val Gln Trp Lys Val Asp Asn Ala
                165                 170
```

<210> 136
<211> 1428
<212> DNA
<213> Homo Sapiens

<400> 136

```
cggccgccta tttacccaga gacagggaga ggctcttctg tgtgtagtgg ttgtgcagag 60
cctcatgcat cacggagcat gagaagacat tcccctcctg ccacctgctc ttgtccacgg 120
ttagcctgct gtagaggaag aaggagccgt cggagtccag cacgggaggc gtggtcttgt 180
agttgttctc cggctgccca ttgctctccc actccacggc gatgtcgctg ggtagaagcc 240
ctttgaccag gcaggtcagg ctgacctggt tcttggtcat ctcctcctgg gatgggggca 300
gggtgtacac ctgtggctct cggggctgcc ctttggcttt ggagatggtt ttctcgatgg 360
aggacgggag gcctttgttg gagaccttgc acttgtactc cttgccgttc agccagtcct 420
ggtgcaggac ggtgaggacg ctgaccacac ggtacgtgct gttgaactgc tcctcccgcg 480
gctttgtctt ggcattatgc acctccacgc catccacgta ccagttgaac tggacctcgg 540
ggtcttcctg gctcacgtcc accaccacgc acgtgacctc aggggtccgg gagatcatga 600
gagtgtcctt gggttttggg gggaacagga agactgatgg tccccccagg aactcaggtg 660
ctgggcatga tgggcatggg ggaccatatt tggactcaac tctcttgtcc accttggtgt 720
tgctgggctt gtgatctacg ttgcaggtgt aggtcttcgt gcccaagctg ctggagggca 780
cggtcaccac gctgctgagg gagtagagtc ctgaggactg taggacagcc gggaaggtgt 840
gcacgccgct ggtcagggcg cctgagttcc acgacaccgt caccggttcg gggaagtagt 900
ccttgaccag gcagcccagg gcggctgtgc tctcggaggt gctcctggag caggcgcgca 960
gggggaagac ggatgggccc ttggtggaag ctgaggagac ggtgaccagg gttccctggc 1020
cccagtagtc aaacccccag tgatgtctac tattatcata gtaatctctc gcacagtaat 1080
acacagccgt gtcctcggct ctcaggctgt tcatttgcag atacagcgtg ttcttggaat 1140
tgtctctgga gatggtgaat cggcccttca cggagtctgc atagagttta ttacttccat 1200
cataccatat aactgccacc catttcagcc ccttgcctgg agcctggcgg acccagtgca 1260
tgccatagcg actgaagatg aatccagacg ctgcacagga gagtctcagg gacctcccag 1320
gctggaccac gcctccccca gactccacca gctgcacctg acactggaca ccttttaaaa 1380
tagccacaag aaaaagccag ctcagcccaa actccatggt ggtcgact             1428
```

<210> 137
<211> 469
<212> PRT
<213> Homo Sapiens

<400> 137

```
Met Glu Phe Gly Leu Ser Trp Leu Phe Leu Val Ala Ile Leu Lys Gly
 1               5                  10                  15
Val Gln Cys Gln Val Gln Leu Val Glu Ser Gly Gly Gly Val Val Gln
            20                  25                  30
Pro Gly Arg Ser Leu Arg Leu Ser Cys Ala Ala Ser Gly Phe Ile Phe
        35                  40                  45
Ser Arg Tyr Gly Met His Trp Val Arg Gln Ala Pro Gly Lys Gly Leu
    50                  55                  60
Lys Trp Val Ala Val Ile Trp Tyr Asp Gly Ser Asn Lys Leu Tyr Ala
65                  70                  75                  80
Asp Ser Val Lys Gly Arg Phe Thr Ile Ser Arg Asp Asn Ser Lys Asn
                85                  90                  95
Thr Leu Tyr Leu Gln Met Asn Ser Leu Arg Ala Glu Asp Thr Ala Val
            100                 105                 110
Tyr Tyr Cys Ala Arg Asp Tyr Tyr Asp Asn Ser Arg His His Trp Gly
        115                 120                 125

Phe Asp Tyr Trp Gly Gln Gly Thr Leu Val Thr Val Ser Ser Ala Ser
    130                 135                 140
Thr Lys Gly Pro Ser Val Phe Pro Leu Ala Pro Cys Ser Arg Ser Thr
145                 150                 155                 160
Ser Glu Ser Thr Ala Ala Leu Gly Cys Leu Val Lys Asp Tyr Phe Pro
                165                 170                 175
Glu Pro Val Thr Val Ser Trp Asn Ser Gly Ala Leu Thr Ser Gly Val
            180                 185                 190
His Thr Phe Pro Ala Val Leu Gln Ser Ser Gly Leu Tyr Ser Leu Ser
        195                 200                 205
Ser Val Val Thr Val Pro Ser Ser Ser Leu Gly Thr Lys Thr Tyr Thr
    210                 215                 220
Cys Asn Val Asp His Lys Pro Ser Asn Thr Lys Val Asp Lys Arg Val
225                 230                 235                 240
Glu Ser Lys Tyr Gly Pro Pro Cys Pro Ser Cys Pro Ala Pro Glu Phe
                245                 250                 255
Leu Gly Gly Pro Ser Val Phe Leu Phe Pro Pro Lys Pro Lys Asp Thr
            260                 265                 270
Leu Met Ile Ser Arg Thr Pro Glu Val Thr Cys Val Val Val Asp Val
        275                 280                 285
Ser Gln Glu Asp Pro Glu Val Gln Phe Asn Trp Tyr Val Asp Gly Val
    290                 295                 300
Glu Val His Asn Ala Lys Thr Lys Pro Arg Glu Glu Gln Phe Asn Ser
305                 310                 315                 320
Thr Tyr Arg Val Val Ser Val Leu Thr Val Leu His Gln Asp Trp Leu
                325                 330                 335
Asn Gly Lys Glu Tyr Lys Cys Lys Val Ser Asn Lys Gly Leu Pro Ser
            340                 345                 350
Ser Ile Glu Lys Thr Ile Ser Lys Ala Lys Gly Gln Pro Arg Glu Pro
        355                 360                 365
Gln Val Tyr Thr Leu Pro Pro Ser Gln Glu Glu Met Thr Lys Asn Gln
    370                 375                 380
Val Ser Leu Thr Cys Leu Val Lys Gly Phe Tyr Pro Ser Asp Ile Ala
385                 390                 395                 400
Val Glu Trp Glu Ser Asn Gly Gln Pro Glu Asn Asn Tyr Lys Thr Thr
                405                 410                 415
Pro Pro Val Leu Asp Ser Asp Gly Ser Phe Phe Leu Tyr Ser Arg Leu
            420                 425                 430
Thr Val Asp Lys Ser Arg Trp Gln Glu Gly Asn Val Phe Ser Cys Ser
        435                 440                 445
Val Met His Glu Ala Leu His Asn His Tyr Thr Gln Lys Ser Leu Ser
    450                 455                 460
Leu Ser Leu Gly Lys
465
```

<210> 138
<211> 741
<212 DNA
<213> Homo Sapiens

<400> 138

```
agtcgaccac catggaaacc ccagcgcagc ttctcttcct cctgctactc tggctcccag 60
ataccaccgg agatattgtg atgacccaga ctccactctc cctgcccgtc acccctggag 120
agccggcctc catctcctgc aggtctagtc ggagcctctt ggatagtgat gatggaaaca 180
cctatttgga ctggtacctg cagaagccag ggcagtctcc acagctcctg atctacacgc 240
tttcctatcg ggcctctgga gtcccagaca ggttcagtgg cagtgggtca ggcactgatt 300
tcacactgaa aatcagcagg gtggaggctg aggatgttgg agtttattac tgcatgcaac 360
gtgtagagtt tcctatcacc ttcggccaag ggacacgact ggagattaaa cgaactgtgg 420
ctgcaccatc tgtcttcatc ttcccgccat ctgatgagca gttgaaatct ggaactgcct 480
ctgttgtgtg cctgctgaat aacttctatc ccagagaggc caaagtacag tggaaggtgg 540

ataacgccct ccaatcgggt aactcccagg agagtgtcac agagcaggac agcaaggaca 600
gcacctacag cctcagcagc accctgacgc tgagcaaagc agactacgag aaacacaaag 660
tctacgcctg cgaagtcacc catcagggcc tgagctcgcc cgtcacaaag agcttcaaca 720
ggggagagtg ttaggcggcc g                                          741
```

<210> 139
<211> 240
<212> PRT
<213> Homo Sapiens

<400> 139

```
Met Glu Thr Pro Ala Gln Leu Leu Phe Leu Leu Leu Leu Trp Leu Pro
1               5                   10                  15
Asp Thr Thr Gly Asp Ile Val Met Thr Gln Thr Pro Leu Ser Leu Pro
            20                  25                  30
Val Thr Pro Gly Glu Pro Ala Ser Ile Ser Cys Arg Ser Ser Arg Ser
        35                  40                  45
Leu Leu Asp Ser Asp Asp Gly Asn Thr Tyr Leu Asp Trp Tyr Leu Gln
    50                  55                  60
Lys Pro Gly Gln Ser Pro Gln Leu Leu Ile Tyr Thr Leu Ser Tyr Arg
65                  70                  75                  80
Ala Ser Gly Val Pro Asp Arg Phe Ser Gly Ser Gly Ser Gly Thr Asp
                85                  90                  95
Phe Thr Leu Lys Ile Ser Arg Val Glu Ala Glu Asp Val Gly Val Tyr
            100                 105                 110
Tyr Cys Met Gln Arg Val Glu Phe Pro Ile Thr Phe Gly Gln Gly Thr
        115                 120                 125
Arg Leu Glu Ile Lys Arg Thr Val Ala Ala Pro Ser Val Phe Ile Phe
    130                 135                 140
Pro Pro Ser Asp Glu Gln Leu Lys Ser Gly Thr Ala Ser Val Val Cys
145                 150                 155                 160
Leu Leu Asn Asn Phe Tyr Pro Arg Glu Ala Lys Val Gln Trp Lys Val
                165                 170                 175
Asp Asn Ala Leu Gln Ser Gly Asn Ser Gln Glu Ser Val Thr Glu Gln
            180                 185                 190
Asp Ser Lys Asp Ser Thr Tyr Ser Leu Ser Ser Thr Leu Thr Leu Ser
        195                 200                 205
Lys Ala Asp Tyr Glu Lys His Lys Val Tyr Ala Cys Glu Val Thr His
    210                 215                 220
Gln Gly Leu Ser Ser Pro Val Thr Lys Ser Phe Asn Arg Gly Glu Cys
225                 230                 235                 240
```

<210> 140
<211> 186
<212> PRT
<213> Homo Sapiens

<220>
<221> misc_feature
<222> (1) .. (1)
<223> Wherein Xaa may be any amino acid

<220>
<221> misc_feature
<222> (2) .. (2)
<223> Wherein Xaa may be any amino acid

<220>
<221> misc_feature
<222> (3) .. (3)
<223> Wherein Xaa may be any amino acid

<220>
<221> misc_feature
<222> (4) .. (4)
<223> Wherein Xaa may be any amino acid

<400> 140

```
Xaa Xaa Xaa Xaa Glu Gln Ser Gly Gly Gly Val Val Gln Pro Gly Arg
 1               5                  10                  15
Ser Leu Arg Leu Ser Cys Ala Ala Ser Gly Phe Thr Phe Ser Ser Tyr
            20                  25                  30
Gly Met Tyr Trp Val Arg Gln Ala Pro Gly Lys Gly Leu Glu Trp Val
        35                  40                  45
Ala Val Ile Trp Tyr Asp Gly Ser Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Phe Tyr Asp Ser Ser Arg Tyr His Tyr Gly Met Asp Val
            100                 105                 110
Trp Gly Gln Gly Thr Thr Val Thr Val Ser Ser Ala Ser Thr Lys Gly
        115                 120                 125
Pro Ser Val Phe Pro Leu Ala Pro Cys Ser Arg Ser Thr Ser Glu Ser
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Ser
            180                 185
```

<210> 141
<211> 143
<212> PRT
<213> Homo Sapiens

<220>
<221> misc_feature
<222> (1) .. (1)

<223> Wherein Xaa may be any amino acid

<220>
<221> misc_feature
<222> (2) .. (2)
<223> Wherein Xaa may be any amino acid

<220>
<221> misc_feature
<222> (3) .. (3)
<223> Wherein Xaa may be any amino acid

<220>
<221> misc_feature
<222> (4) .. (4)
<223> Wherein Xaa may be any amino acid

<400> 141

```
Xaa Xaa Xaa Xaa Thr Gln Cys Pro Leu Ser Leu Pro Val Thr Pro Gly


 1               5                  10                  15
Glu Pro Ala Ser Ile Ser Cys Arg Ser Ser Gln Ser Leu Leu Asp Ser
            20                  25                  30
Asp Asp Gly Asn Thr Tyr Leu Asp Trp Tyr Leu Gln Lys Pro Gly Gln
        35                  40                  45
Ser Pro Gln Leu Leu Ile Tyr Thr Val Ser Tyr Arg Ala Ser Gly Val
    50                  55                  60
Pro Asp Arg Phe Ser Gly Ser Gly Ser Gly Thr Asp Phe Thr Leu Lys
65                  70                  75                  80
Ile Ser Arg Val Glu Ala Glu Asp Val Gly Val Tyr Tyr Cys Met Gln
                85                  90                  95
Arg Ile Glu Phe Pro Ile Thr Phe Gly Gln Gly Thr Arg Leu Glu Ile
            100                 105                 110
Lys Arg Thr Val Ala Ala Pro Ser Val Phe Ile Phe Pro Pro Ser Asp
        115                 120                 125
Glu Gln Leu Lys Ser Gly Thr Ala Ser Val Val Cys Leu Leu Asn
    130                 135                 140
```